# EUROPEAN PATENT SPECIFICATION

(11) **EP 2 115 165 B1**
(45) Date of publication and mention of the grant of the patent: **14.03.2012**
(21) Application number: 08701160.7
(22) Date of filing: 18.01.2008
(51) Int. Cl.: C12Q 1/68

(54) **METHODS AND NUCLEIC ACIDS FOR ANALYSES OF CELL PROLIFERATIVE DISORDERS**
VERFAHREN UND NUKLEINSÄUREN ZUR ANALYSE PROLIFERATIVER ZELLERKRANKUNGEN
PROCEDES ET ACIDES NUCLEIQUES POUR ANALYSES DES TROUBLES PROLIFERATIFS CELLULAIRES

(30) Priority: 19.01.2007 EP 07100829; 11.06.2007 EP 07110019; 30.07.2007 EP 07113449
(43) Date of publication of application: 11.11.2009
(60) Divisional application: 10156846.7
(73) Proprietor: Epigenomics AG, 10178 Berlin (DE)
(72) Inventor: LIEBENBERG, Volker, 14050 Berlin (DE); DISTLER, Jürgen, 12163 Berlin (DE); LEWIN, Jörn, 10115 Berlin (DE); MODEL, Fabian, 12683 Berlin (DE); TETZNER, Reimo, 10439 Berlin (DE); CORTESE, Rene, M5R2N9 Toronto, ON (CA)
(74) Representative: Krauss, Jan
(86) International application number: PCT/EP2008/000384
(87) International publication number: WO 2008/087040

(56) References cited:
- WO-A-2005/054517
- WO-A-2005/111232
- WO-A-2005/123945
- WO-A-2006/008128
- WO-A-2007/009755
- DOHADWALA MARIAM ET AL: "Autocrine/paracrine prostaglandin E2 production by non-small cell lung cancer cells regulates matrix metalloproteinase-2 and CD44 in cyclooxygenase-2-dependent invasion." JOURNAL OF BIOLOGICAL CHEMISTRY, vol. 277, no. 52, 27 December 2002 (2002-12-27), pages 50828-50833, XP002482805 ISSN: 0021-9258
- HAN SHOUWEI ET AL: "Activation of peroxisome proliferator-activated receptor beta/delta (PPAR beta/delta) increases the expression of prostaglandin E-2 receptor subtype EP4 - The roles of phosphatidylinositol 3-kinase and CCAAT/enhancer-binding protein beta" JOURNAL OF BIOLOGICAL CHEMISTRY, vol. 280, no. 39, September 2005 (2005-09), pages 33240-33249, XP002482806 ISSN: 0021-9258
- PIAZUELO ET AL: "Effects of selective PGE2 receptor antagonists in esophageal adenocarcinoma cells derived from Barrett's esophagus" PROSTAGLANDINS AND OTHER LIPID MEDIATORS, ELSEVIER, vol. 81, no. 3-4, 3 November 2006 (2006-11-03), pages 150-161, XP005732409 ISSN: 1098-8823

## Description

### FIELD OF THE INVENTION

The present invention relates to genomic DNA sequences that exhibit altered expression patterns in disease states relative to normal. Particular embodiments provide methods, nucleic acids, nucleic acid arrays and kits useful for detecting, or for diagnosing cell proliferative disorders.

### BACKGROUND

*CpG island methylation.* Apart from mutations aberrant methylation of CpG islands has been shown to lead to the transcriptional silencing of certain genes that have been previously linked to the pathogenesis of various cell proliferative disorders, including cancer. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, i.e., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, *i.e.*, individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cell proliferative disorders, often involve a plurality of genes and proteins. Additionally, certain proteins may be elevated for reasons unrelated to a disease state. Specificity is important when the cost or risk associated with further diagnostic procedures or further medical intervention are very high.

WO 2006/008128 discloses a method and nucleic acids for the detection and differential of breast cancers by analysis of the methylation patterns of genes and discloses PTGER4 However, there is no disclosure in WO 2006/008128 that hypermethylation of PTGER4 as a marker can be used for detection of lung cancer.

### SUMMARY OF THE INVENTION

The present invention provides a method for detecting lung carcinoma in a subject comprising detecting the presence of absence of CpG methylation within the PTGER44 gene and/or promoter or regulatory elements wherein hyper-methylation is indicative of the presence of lung carcinoma. The present invention provides an efficient and unique genetic marker. Preferred is a lung carcinoma selected from the group consisting of Lung adenocarcinoma; Large cell lung cancer; squamous cell lung carcinoma; Small cell lung carcinoma.

Said expression is determined by detecting the presence or absence of CpG methylation within said gene, wherein under-expression indicates the presence of lung carcinoma.

Said method comprises the following steps: i) contacting genomic DNA isolated from a biological sample (preferably selected from the group consisting of cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion) obtained from the subject with at least one reagent, or series of reagents that distinguishes between methylated and non-methylated CpG dinucleotides within at least one target region of the genomic DNA, wherein the nucleotide sequence of said target region comprises, or hybridizes under stringent conditions to a sequence of at least 16 contiguous nucleotides of SEQ ID NO: 4, wherein said contiguous nucleotides comprise at least one CpG dinucleotide sequence and ii) detecting lung carcinoma.

Preferably, the sensitivity of said detection is from about 75% to about 96%, or from about 80% to about 90%, or from about 80% to about 85%. Preferably, the specificity is from about 75% to about 96%, or from about 80% to about 90%, or from about 80% to about 85%.

The detection of lung carcinoma is enabled by means of analysis of the *methylation status* of gene or genomic sequence of PTGER4, and/or promoter or regulatory elements thereof.

The present invention provides a method for ascertaining epigenetic parameters of genomic DNA associated with the development of lung carcinoma. The method has utility for the improved detection and diagnosis of said disease.

Preferably, the source of the test sample is selected from the group consisting of cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion, and combinations thereof. More preferably the samnpel type is selected form the group consisting of blood plasma, sputum and biological matter derived from bronchoscopy (including but not limited to bronchial lavage, bronchial alveolar lavage, bronchial brushing, bronchial abrasion) and all possible combinations thereof.

Specifically, the present invention provides a method for detecting lung carcinoma suitable for use in a diagnostic tool, comprising: obtaining a biological sample comprising genomic nucleic acid(s); contacting the nucleic acid(s), or a fragment thereof, with a reagent or a plurality of reagents sufficient for distinguishing between methylated and non methylated CpG dinucleotide sequences within a target sequence of the subject nucleic acid, wherein the target sequence comprises, or hybridises under stringent conditions to, a sequence comprising at least 16 contiguous nucleotides of SEQ ID NO: 4 said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences.

Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO: 19, 20, 43 and 44, and contiguous regions thereof corresponding to the target sequence.

Additional embodiments provide a method for the detection of lung carcinoma comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting of SEQ ID NO: 19, 20, 43 and 44, and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one , but more preferably a plurality of CpG dinucleotides of SEQ ID NO: 4.

Preferably, determining comprises use of at least one method selected from the group consisting of: i) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 19, 20, 43 and 44, and complements thereof; ii) hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 19, 20,

43 and 44, and complements thereof; iii) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 19, 20, 43 and 44, and complements thereof, and extending at least one such hybridized nucleic acid molecule by at least one nucleotide base; and iv) sequencing of the amplificate.

Further embodiments provide a method for the analysis (i.e. detection or diagnosis) of lung carcinoma, comprising: obtaining a biological sample having subject genomic DNA; extracting the genomic DNA; contacting the genomic DNA, or a fragment thereof, comprising one or more sequences selected from the group consisting of SEQ ID NO: 4 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of SEQ ID NO: 4 or an average, or a value reflecting an average methylation state of a plurality of CpG dinucleotide sequences thereof. Preferably, the digested or undigested genomic DNA is amplified prior to said determining.

Additional embodiments describe novel genomic and chemically modified nucleic acid sequences, as well as oligonucleotides and/or PNA-oligomers for analysis of cytosine methylation patterns within SEQ ID NO: 4.

### DETAILED DESCRIPTION OF THE INVENTION

### Definitions:

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 KB, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analysed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., cell proliferative disorders, preferably those according to Cancer Research 57:594-599, 1997.

The term "MethyLight™" refers to the art-recognized fluorescence-based real-time PCR technique described by Eads et al., cell proliferative disorders, preferably those according to Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., cell proliferative disorders, preferably those according to Cancer Res. 59:2307-12, 1999, and in WO 00/26401 A1.

The term "hybridisation" is to be understood as a bond of an oligonucleotide to a complementary sequence along the lines of the Watson-Crick base pairings in the sample DNA, forming a duplex structure.

"Stringent hybridisation conditions," as defined herein, involve hybridising at 68°C in 5x SSC/5x Denhardt's solution/1.0% SDS, and washing in 0.2x SSC/0.1% SDS at room temperature, or involve the art-recognized equivalent thereof (e.g., conditions in which a hybridisation is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "Methylation-specific restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5. "Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."

In reference to composite array sequences, the phrase "contiguous nucleotides" refers to a contiguous sequence region of any individual contiguous sequence of the composite array, but does not include a region of the composite array sequence that includes a "node," as defined herein above.

The term "at least one gene or genomic sequence selected from the group consisting of PTGER4" shall be taken to include all transcript variants thereof and all promoter and regulatory elements thereof. Furthermore as a plurality of SNPs are known within said gene the term shall be taken to include all sequence variants thereof.

### Overview:

The present invention provides a method for detecting lung carcinoma-in a subject comprising detecting the presence of absence of CpG methylation within the PTGER4 gene and/or promoter or regulatory elements wherein hyper-methylation is indicative of the presence of lung carcinoma.

In addition to the embodiments above wherein the methylation analysis of the gene or genomic sequence of PTGER4 is analysed, the invention describes panels of genes comprising at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1 with novel utility for the detection of lung carcinoma.

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g.,* PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analysed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyse individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g.*, Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridisation has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 97/46705 and WO 95/15373).

The present invention provides for the use of the bisulfite technique, in combination with one or more methylation assays, for determination of the methylation status of CpG dinucleotide sequences within SEQ ID NO: 4. Genomic CpG dinucleotides can be methylated or unmethylated (alternatively known as up- and down- methylated respectively). However the methods of the present invention are suitable for the analysis of biological samples of a heterogeneous nature e.g. a low concentration of tumor cells within a background of blood. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position. Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) PTGER4.

According to the present invention, determination of the methylation status of CpG dinucleotide sequences within SEQ ID NO: 4 have utility in the diagnosis and detection of lung carcinoma.

*Methylation Assay Procedures.* Various methylation assay procedures are known in the art, and can be used in conjunction with the present invention. These assays allow for determination of the methylation state of one or a plurality of CpG dinucleotides (*e.g.*, CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad Sci. USA 89:1827-1831, 1992). Additionally, restriction enzyme digestion of PCR products amplified from bisulfite-converted DNA is used, *e.g.*, the method described by Sadri & Homsby (Nucl. Acids Res. 24:5058-5059,1996), or COBRA (Combined Bisulfite Restriction Analysis) (Xiong & Laird, Nucleic Acids Res. 25:2532-2534, 1997).

*COBRA.* COBRA^{™} analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (*e.g.*, as might be found in a typical COBRA^{™}-based kit) for COBRA^{™} analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer, sulfonation buffer; DNA recovery reagents or kits (*e.g.*, precipitation, ultrafiltration, affinity column); desulfonation buffer; and DNA recovery components.

Preferably, assays such as "MethyLight^{™}" (a fluorescence-based real-time PCR technique) (Eads et al., cell proliferative disorders, preferably those according to Cancer Res. 59:2302-2306, 1999), Ms-SNuPE^{™} (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., cell proliferative disorders, preferably those according to Cancer Res. 59:2307-12, 1999) are used alone or in combination with other of these methods.

The "HeavyMethyl™" assay, technique is a quantitative method for assessing methylation differences based on methylation specific amplification of bisulfite treated DNA. Methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers. The HeavyMethyl™ assay may also be used in combination with methylation specific amplification primers.

Typical reagents (*e.g.*, as might be found in a typical MethyLight□-based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (*e.g.,* as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MethyLight*^{™}*.* The MethyLight^{™} assay is a high-throughput quantitative methylation assay that utilizes fluorescence-based real-time PCR (TaqMan□) technology that requires no further manipulations after the PCR step (Eads et al., Cancer Res. 59:2302-2306, 1999). Briefly, the MethyLight^{™} process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process.

The MethyLight^{™} assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLight^{™} process can by used with any suitable probes e.g. "TaqMan®", Lightcycler® etc.... For example, double-stranded genomic DNA is treated with sodium bisulfite and subjected to one of two sets of PCR reactions using TaqMan® probes; *e.g.*, with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g.*, as might be found in a typical MethyLight□-based kit) for MethyLight^{™} analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The QM^{™} (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The QM^{™} process can by used with any suitable probes e.g. "TaqMan®", Lightcycler® etc... in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.
Typical reagents (*e.g.*, as might be found in a typical QM^{™} -based kit) for QM^{™} analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

*Ms-SNuPE.* The Ms-SNuPE^{™} technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g.*, microdissected pathology sections), and it avoids utilization of restriction enzymes for determining the methylation status at CpG sites.

Typical reagents (*e.g*., as might be found in a typical Ms-SNuPE^{™}-based kit) for Ms-SNuPE^{™} analysis may include, but are not limited to: PCR primers for specific gene (or bisulfite treated DNA sequence or CpG island); optimized PCR buffers and deoxynucleotides; gel extraction kit; positive control primers; Ms-SNuPE^{™} primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (*e.g.,* precipitation, ultrafiltration, affinity column); desulfonation buffer; and DNA recovery components.

The Genomic Sequence(s) According to SEQ ID NO: 4 and Non-naturally Occurring Treated Variants Thereof According to_SEQ ID NO: 19, 20, 43 and 44 were Determined to have Novel Utility for the Detection of lung carcinoma.

Preferred is a lung carcinoma selected from the group consisting of Lung adenocarcinoma; Large cell lung cancer, squamous cell lung carcinoma; Small cell lung carcinoma.

In the invention the detection of lung carcinoma is enabled by means of analysis of the *methylation status* of the gene or genomic sequence of PTGER4, and/or promoter or regulatory elements thereof. According to the present invention, hyper-methylation is associated with the presence of cell lung carcinoma.

To detect the presence of mRNA encoding a gene or genomic sequence, a sample is obtained from a patient. The sample may be any suitable sample comprising cellular matter of the tumor. Suitable sample types include cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion and all possible combinations thereof. More preferably the samnpel type is selected form the group consisting of blood plasma, sputum and biological matter derived from bronchoscopy (including but not limited to bronchial lavage, bronchial alveolar lavage, bronchial brushing, bronchial abrasion) and all possible combinations thereof.

Particularly preferred is the use of microarrays. The microarray analysis process can be divided into two main parts. First is the immobilization of known gene sequences onto glass slides or other solid support followed by hybridisation of the fluorescently labelled cDNA (comprising the sequences to be interrogated) to the known genes immobilized on the glass slide (or other solid phase). After hybridisation, arrays are scanned using a fluorescent microarray scanner. Analysing the relative fluorescent intensity of different genes provides a measure of the differences in gene expression.

DNA arrays can be generated by immobilizing presynthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1 and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Preferably said synthesized nucleic acids are locked nucleic acids.

Described is a kit for use in diagnosis of lung carcinoma and further preferred is a lung cancer selected from the group consisting of lung adenocarcinoma; large cell lung cancer; squamous cell lung carcinoma; small cell lung carcinoma.) in a subject according to the methods of the present invention, said kit comprising: a means for measuring the level of transcription of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1. In a preferred embodiment, the means for measuring the level of transcription comprise oligonucleotides or polynucleotides able to hybridise under stringent or moderately stringent conditions to the transcription products of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1. The level of transcription can be determined by techniques selected from the group of Northern Blot analysis, reverse transcriptase PCR, real-time PCR, RNAse protection, and microarray. In another embodiment the kit further comprises means for obtaining a biological sample of the patient. Preferred is a kit, which further comprises a container which is most preferably suitable for containing the means for measuring the level of transcription and the biological sample of the patient, and most preferably further comprises instructions for use and interpretation of the kit results.

The kit can comprise (a) a plurality of oligonucleotides or polynucleotides able to hybridise under stringent or moderately stringent conditions to the transcription products of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1; (b) a container, preferably suitable for containing the oligonucleotides or polynucleotides and a biological sample of the patient comprising the transcription products wherein the oligonucleotides or polynucleotides can hybridise under stringent or moderately stringent conditions to the transcription products, (c) means to detect the hybridisation of (b); and optionally, (d) instructions for use and interpretation of the kit results

The kit may also contain other components such as hybridisation buffer (where the oligonucleotides are to be used as a probe) packaged in a separate container. Alternatively, where the oligonucleotides are to be used to amplify a target region, the kit may contain, packaged in separate containers, a polymerase and a reaction buffer optimised for primer extension mediated by the polymerase, such as PCR. Preferably said polymerase is a reverse transcriptase. It is further preferred that said kit further contains an Rnase reagent.

The kit may also contain other components such as buffers or solutions suitable for blocking, washing or coating , packaged in a separate container.

Particular embodiments of the present invention provide a novel application of the analysis of methylation levels and/or patterns within the gene or genomic sequence of PTGER4 that enables a precise detection, characterisation and/or treatment of lung carcinoma. Particularly preferred is a lung carcinoma selected from the group consisting of lung adenocarcinoma; large cell lung cancer; squamous cell lung carcinoma; small cell lung carcinoma. Early detection of lung carcinoma is directly linked with disease prognosis, and the disclosed method thereby enables the physician and patient to make better and more informed treatment decisions.

In the most preferred embodiment of the method, the presence or absence lung carcinoma, in particular a lung cancer selected from the group consisting of lung adenocarcinoma; large cell lung cancer; squamous cell lung carcinoma; small cell lung carcinoma is determined by analysis of the methylation status of one or more CpG dinucleotides of the gene or genomic sequence.

In one embodiment the invention of said method comprises the following steps: i) contacting genomic DNA (preferably isolated from body fluids) obtained from the subject with at least one reagent, or series of reagents that distinguishes between methylated and non-methylated CpG dinucleotides within at least one gene or genomic sequence selected from the group consisting of PTGER44 (including promoter and regulatory regions thereof) and ii) detecting lung carcinoma. Particularly preferred is a lung cancer selected from the group consisting of lung adenocarcinoma; large cell lung cancer; squamous cell lung carcinoma; small cell lung carcinoma.

It is preferred that said one or more CpG dinucleotides of at least one gene or genomic sequence selected from the group consisting of PTGER4 are comprised within a respective genomic target sequence thereof as provided in SEQ ID NO: 4 and complements thereof. The present invention further provides a method for ascertaining genetic and/or epigenetic parameters of the gene or genomic sequence and/or the genomic sequence according to SEQ ID NO: 4 within a subject by analysing cytosine methylation. Said method comprising contacting a nucleic acid comprising SEQ ID NO: 4 in a biological sample obtained from said subject with at least one reagent or a series of reagents, wherein said reagent or series of reagents, distinguishes between methylated and non-methylated CpG dinucleotides within the target nucleic acid.

In a preferred embodiment, said method comprises the following steps: In the *first step,* a sample of the tissue to be analysed is obtained. The source may be any suitable source, such as cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion and all possible combinations thereof. More preferably the samnpel type is selected form the group consisting of blood plasma, sputum and biological matter derived from bronchoscopy (including but not limited to bronchial lavage, bronchial alveolar lavage, bronchial brushing, bronchial abrasion) and all possible combinations thereof.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g.ultrafiltration , silica surfaces or membranes, magnetic particles, polystyrol particles, polystyrol surfaces, positively charged surfaces, and positively charged membranse, charged membranes, charged surfaces, charged switch membranes, charged switched surfaces.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pre-treatment' or 'treatment' herein.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. PCT/EP2004/011715). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2, 5,7,8, - tetramethylchromane 2-carboxylic acid or trihydroxybenzoe acid and derivates thereof, e.g. Gallic acid (see: PCT/EP2004/011715). The bisulfite conversion is preferably carried out at a reaction temperature between 30°C and 70°C, whereby the temperature is increased to over 85°C for short periods of times during the reaction (see: PCT/EP2004/011715). The bisulfite treated DNA is preferably purified priori to the quantification. This may be conducted by any means known in the art, such as but not limited to ultrafiltration, preferably carried out by means of Microcon^{™} columns (manufactured by Millipore). The purification is carried out according to a modified manufacturer's protocol (see: PCT/EP2004/011715).

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridise under stringent or highly stringent conditions to an at least 16-base-pair long segment of the base sequences of one of SEQ ID NO: 19, 20, 43 and 44 and sequences complementary thereto.

In an alternate embodiment of the method, the methylation status of pre-selected CpG positions within the gene or genomic sequence and preferably within the nucleic acid sequences according to SEQ ID NO: 4 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridises to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridises to a treated nucleic acid sequence according to one of SEQ ID NO: 19, 20, 43 and 44 and sequences complementary thereto, wherein the base sequence of said oligomers comprises at least one CpG dinucleotide .A further preferred embodiment of the method comprises the use of *blocker* oligonucleotides (the HeavyMethyl™ assay). The use of such blocker oligonucleotides has been described by Yu et al., BioTechniques 23:714-720, 1997. Blocking probe oligonucleotides are hybridised to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (*e.g.*, with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridises to a treated nucleic acid sequence according to one of SEQ ID NO: 19, 20, 43 and 44 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labelled amplificates have a single positive or negative net charge, allowing for better delectability in the mass spectrometer. The detection may be carried out and visualized by means of, *e.g.,* matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analysed by means of based-based methods such as, but not limited to, array technology and probe based technologies as well as by means of techniques such as sequencing and template directed extension.

In one embodiment of the method, the amplificates synthesised in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide. The hybridizing portion of the hybridizing nucleic acids is typically at least 9, 15, 20, 25, 30 or 35 nucleotides in length. However, longer molecules have inventive utility, and are thus within the scope of the present invention.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence selected from the group consisting SEQ ID NO: 4, and the equivalent positions within SEQ ID NO: 19, 20, 43 and 44. Said oligonucleotides may also be present in the form of peptide nucleic acids. The non-hybridised amplificates are then removed. The hybridised amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labelled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridise to a CpG-rich sequence located between the forward and reverse amplification primers. The TaqMan™ probe further comprises a fluorescent "reporter moiety" and a "quencher moiety" covalently bound to linker moieties (*e.g.*, phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLightTM™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the *fourth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467, 1977).

### Best mode

In the most preferred embodiment of the method the genomic nucleic acids are isolated and treated according to the first three steps of the method outlined above, namely:
a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides,* and further wherein
e) detecting of the amplificates is carried out by means of a real-time detection probe, as described above.
Preferably, where the subsequent amplification of d) is carried out by means of methylation specific primers, as described above, said methylation specific primers comprise a sequence having a length of at least 9 nucleotides which hybridises to a treated nucleic acid sequence according to one of SEQ ID NO: 19, 20, 43 and 44-and sequences complementary thereto, wherein the base sequence of said oligomers comprise at least one CpG dinucleotide.

Step e) of the method, namely the detection of the specific amplificates indicative of the methylation status of one or more CpG positions according to SEQ ID NO: 4 is carried out by means of *real-time* detection methods as described above.

Additional embodiments of the invention provide a method for the analysis of the methylation status of the gene or genomic sequence of PTGER4 (preferably SEQ SEQ ID NO: 4, and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH.

In the *first step* of such additional embodiments, the genomic DNA sample is isolated from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, *e.g.*, by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic or potentially neoplastic matter are suitable for us e in the present method, preferred are cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion and combinations thereof. More preferably the samnpel type is selected form the group consisting of blood plasma, sputum and biological matter derived from bronchoscopy (including but not limited to bronchial lavage, bronchial alveolar lavage, bronchial brushing, bronchial abrasion) and all possible combinations thereof.

Once the nucleic acids have been extracted, the genomic double-stranded DNA is used in the analysis.

In a preferred embodiment, the DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting of MseI, BfaI, Csp6I, Tru1I, Tvu1I, Tru9I, Tvu9I, MaeI and XspI. Particularly preferred is the use of two or three such enzymes. Particularly preferred is the use of a combination of MseI, BfaI and Csp6I.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the *third step,* the DNA (or fragments thereof) is then digested with one or more methylation sensitive restriction enzymes. The digestion is carried out such that hydrolysis of the DNA at the restriction site is informative of the methylation status of a specific CpG dinucleotide of the gene or genomic sequence of PTGER4.

Preferably, the methylation-specific restriction enzyme is selected from the group consisting of *Bsi El, Hga I HinPl, Hpy99I, Ava I, Bce AI, Bsa HI, BisI, BstUI, BshI236I, AccII, BstFNI, McrBC, GlaI, MvnI, HpaII (HapII), HhaI, AciI, SmaI, HinPlI, HpyCH4IV, EagI* and mixtures of two or more of the above enzymes. Preferred is a mixture containing the restriction enzymes BstUI, HpaII, HipyCH4IV and HinP1I.

In the *fourth step,* which is optional but a preferred embodiment, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence at least 16 nucleotides in length that is complementary to, or hybridizes under moderately stringent or stringent conditions to a sequence selected from the group consisting of SEQ ID NO: 4, and complements thereof. Preferably said contiguous sequence is at least 16, 20 or 25 nucleotides in length. In an alternative embodiment said primers may be complementary to any adaptors linked to the fragments.

In the *fifth step* the amplificates are detected. The detection may be by any means standard in the art, for example, but not limited to, gel electrophoresis analysis, hybridisation analysis, incorporation of detectable tags within the PCR products, DNA array analysis, MALDI or ESI analysis. Preferably said detection is carried out by hybridisation to at least one nucleic acid or peptide nucleic acid comprising in each case a contiguous sequence at least 16 nucleotides in length that is complementary to, or hybridizes under moderately stringent or stringent conditions to a sequence selected from the group consisting of SEQ ID NO: 4, and complements thereof. Preferably said contiguous sequence is at least 16, 20 or 25 nucleotides in length.

Subsequent to the determination of the methylation state or level of the genomic nucleic acids the presence, absence of lung carcinoma, is deduced based upon the methylation state or level of at least one CpG dinucleotide sequence of SEQ ID NO: 4, or an average, or a value reflecting an average methylation state of a plurality of CpG dinucleotide sequences of SEQ ID NO: 4 wherein methylation is associated with the presence of lung carcinoma. Wherein said methylation is determined by quantitative means the cut-off point for determining said the presence of methylation is preferably zero (i.e. wherein a sample displays any degree of methylation it is determined as having a methylated status at the analysed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected form the group consisting of 0%-5%, 5%-10%, 10%-15%, 15%-20%, 20%-30% and 30%-50%. Particularly preferred are the cut-offs 10%, 15%, 25%, and 30%.

Upon determination of the methylation of the gene or genomic sequence of PTGER4 the presence or absence of lung carcinoma is determined, wherein hyper-methylation indicates the presence of lung carcinoma and hypo-methylation and /or over-expression indicates the absence of lung carcinoma within the subject. Particularly preferred is a lung carcinoma selected from the group consisting of lung adenocarcinoma; large cell lung cancer; squamous cell lung carcinoma; small cell lung carcinoma.

### FURTHER IMPROVEMENTS

The disclosed invention provides treated nucleic acids, derived from genomic SEQ ID NO: 4, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. In a preferred embodiment of the invention, the invention provides a non-naturally occurring modified nucleic acid comprising a sequence of at least 16 contiguous nucleotide bases in length of a sequence selected from the group consisting of SEQ ID NO: 4. In further preferred embodiments of the invention said nucleic acid is at least 50, 100, 150, 200, 250 or 500 base pairs in length of a segment of the nucleic acid sequence disclosed in SEQ ID NO: 19, 20, 43 and 44. Particularly preferred is a nucleic acid molecule that is not identical or complementary to all or a portion of the sequences SEQ ID NO: 19, 20, 43 and 44 but not SEQ ID NO: 4 or other naturally occurring DNA.

It is preferred that said sequence comprises at least one CpG, TpA or CpA dinucleotide and sequences complementary thereto. The sequences of SEQ ID NO: 19, 20, 43 and 44 provide non-naturally occurring modified versions of the nucleic acid according to SEQ ID NO: 4, wherein the modification of each genomic sequence results in the synthesis of a nucleic acid having a sequence that is unique and distinct from said genomic sequence as follows. For each sense strand genomic DNA, *e.g.*, SEQ ID NO: 4, four converted versions are disclosed. A first version wherein "C" is converted to "T," but "CpG" remains "CpG" (*i.e.*, corresponds to case where, for the genomic sequence, all "C" residues of CpG dinucleotide sequences are methylated and are thus not converted); a second version discloses the complement of the disclosed genomic DNA sequence (i.e. *antisense* strand), wherein "C" is converted to "T," but "CpG" remains "CpG" (*i.e.*, corresponds to case where, for all "C" residues of CpG dinucleotide sequences are methylated and are thus not converted). The 'upmethylated' converted sequences of SEQ ID NO: 4 correspond to SEQ ID NO: 19 or 20. A third chemically converted version of each genomic sequences is provided, wherein "C" is converted to "T" for all "C" residues, including those of "CpG" dinucleotide sequences (*i.e.*, corresponds to case where, for the genomic sequences, all "C" residues of CpG dinucleotide sequences are *un*methylated); a final chemically converted version of each sequence, discloses the complement of the disclosed genomic DNA sequence (i.e. *anti*sense strand), wherein "C" is converted to "T" for all "C" residues, including those of "CpG" dinucleotide sequences (*i.e*., corresponds to case where, for the complement (*antisense* strand) of each genomic sequence, all "C" residues of CpG dinucleotide sequences are *un*methylated). The 'downmethylated' converted sequences of SEQ ID NO: 4 corresponds to SEQ ID NO: 43 or 44.

Significantly, heretofore, the nucleic acid sequences and molecules according SEQ ID NO: 19, 20, 43 and 44 were not implicated in or connected with the detection or diagnosis of lung carcinoma. Particularly preferred is a lung cancer selected from the group consisting of lung adenocarcinoma; large cell lung cancer; squamous cell lung carcinoma; small cell lung carcinoma.

In an alternative preferred embodiment, the invention further discloses oligonucleotides or oligomers suitable for use in the methods of the invention for detecting the cytosine methylation state within genomic or treated (chemically modified) DNA, according to SEQ ID NO: 1 to SEQ ID NO: 60; SEQ ID NO: 79 to SEQ ID NO: 103. Said oligonucleotide or oligomer nucleic acids provide novel diagnostic means. Said oligonucleotide or oligomer comprising a nucleic acid sequence having a length of at least nine (9) nucleotides which is identical to, hybridizes, under moderately stringent or stringent conditions (as defined herein above), to a treated nucleic acid sequence according to SEQ ID NO: 19, 20, 43 and 44 and/or sequences complementary thereto, or to a genomic sequence according to SEQ ID NO: 4 and/or sequences complementary thereto.

Thus, the present invention discloses nucleic acid molecules (*e.g.*, oligonucleotides and peptide nucleic acid (PNA) molecules (PNA-oligomers)) that hybridize under moderately stringent and/or stringent hybridization conditions to all or a portion of the sequences SEQ ID NO: 1 to SEQ ID NO: 60; SEQ ID NO: 79 to SEQ ID NO: 103 or to the complements thereof. Particularly preferred is a nucleic acid molecule that hybridizes under moderately stringent and/or stringent hybridization conditions to all or a portion of the sequences SEQ ID NO: 19, 20, 43 and 44 but not SEQ ID NO: 4 or other human genomic DNA.

The identical or hybridizing portion of the hybridizing nucleic acids is typically at least 9, 16, 20, 25, 30 or 35 nucleotides in length. However, longer molecules have inventive utility, and are thus within the scope of the present invention.

Preferably, the hybridizing portion of the described hybridizing nucleic acids is at least 95%, or at least 98%, or 100% identical to the sequence, or to a portion thereof of SEQ ID NO: 1 to SEQ ID NO: 60; SEQ ID NO: 79 to SEQ ID NO: 103, or to the complements thereof.

Hybridizing nucleic acids of the type described herein can be used, for example, as a primer (*e.g.*, a PCR primer), or a diagnostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 4 (such as allelic variants and SNPs), rather than identical, it is useful to first establish the lowest temperature at which only homologous hybridization occurs with a particular concentration of salt (*e.g*., SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of described oligonucleotides of length X (in nucleotides), as indicated by polynucleotide positions with reference to, *e.g*., SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:
n to (n + (X-1));
where n=1, 2, 3,...(Y-(X-1));
where Y equals the length (nucleotides or base pairs) of SEQ ID NO: 1 (3905);
where X equals the common length (in nucleotides) of each oligonucleotide in the set (*e.g*., X=20 for a set of consecutively overlapping 20-mers); and
where the number (Z) of consecutively overlapping oligomers of length X for a given SEQ ID NO 1 of length Y is equal to Y- (X-1). For example Z= 3905 -19= 3886 for either sense or antisense sets of SEQ ID NO: 1, where X=20.

Preferably, the set is limited to those oligomers that comprise at least one CpG; TpG or CpA dinucleotide. Examples of described 20-mer oligonucleotides include the following set of 3905 oligomers (and the antisense set complementary thereto), indicated by polynucleotide positions with reference to SEQ ID NO: 1:
1-20, 2-21, 3-22, 4-23, 5-24, .............. and 3886- 3905

Preferably, the set is limited to those oligomers that comprise at least one CpG, TpG or CpA dinucleotide.

Likewise, examples of described 25-mer oligonucleotides include the following set of 3881 oligomers (and the antisense set complementary thereto), indicated by polynucleotide positions with reference to SEQ ID NO: 1:
1-25, 2-26, 3-27, 4-28, 5-29, ...... and 3881- 3905.

Preferably, the set is limited to those oligomers that comprise at least one CpG, TpG or CpA dinucleotide.

The present invention describes, for *each* of SEQ ID NO: 1 to SEQ ID NO: 60; SEQ ID NO: 79 to SEQ ID NO: 103 (sense and antisense), multiple consecutively overlapping sets of oligonucleotides or modified oligonucleotides of length X, where, *e.g*., X= 9, 10, 17, 20, 22, 23, 25, 27, 30 or 35 nucleotides.

The oligonucleotides or oligomers constitute effective tools useful to ascertain genetic and epigenetic parameters of the genomic sequence corresponding to SEQ ID NO: 1 to SEQ ID NO: 12; SEQ ID NO: 79 to SEQ ID NO: 83. Preferred sets of such oligonucleotides or modified oligonucleotides of length X are those consecutively overlapping sets of oligomers corresponding to SEQ ID NO: 1 to SEQ ID NO: 60; SEQ ID NO: 79 to SEQ ID NO: 103 (and to the complements thereof). Preferably, said oligomers comprise at least one CpG, TpG or CpA dinucleotide.

Particularly preferred oligonucleotides or oligomers are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, *e.g*., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers are typically used in 'sets,' which contain at least one oligomer for analysis of each of the CpG dinucleotides of a genomic sequence selected from the group consisting SEQ ID NO: 1 to SEQ ID NO: 12; SEQ ID NO: 79 to SEQ ID NO: 83 and sequences complementary thereto, or to the corresponding CpG, TpG or CpA dinucleotide within a sequence of the treated nucleic acids according to SEQ ID NO: 13 to SEQ ID NO: 60; SEQ ID NO: 84 to SEQ ID NO: 103 and sequences complementary thereto. However, it is anticipated that for economic or other factors it may be preferable to analyse a limited selection of the CpG dinucleotides within said sequences, and the content of the set of oligonucleotides is altered accordingly.

Therefore, described is a set of at least two (2) (oligonucleotides and/or PNA-oligomers) useful for detecting the cytosine methylation state in treated genomic DNA (SEQ ID NO: 13 to SEQ ID NO: 60; SEQ ID NO: 84 to SEQ ID NO: 103), or in genomic DNA (SEQ ID NO: 1 to SEQ ID NO: 12; SEQ ID NO: 79 to SEQ ID NO: 83 and sequences complementary thereto). These probes enable diagnosis and detection of. Particularly preferred is a lung cancer selected from the group consisting of lung adenocarcinoma; large cell lung cancer; squamous cell lung carcinoma; small cell lung carcinoma. The set of oligomers may also be used for detecting single nucleotide polymorphisms (SNPs) in treated genomic DNA (SEQ ID NO: 13 to SEQ ID NO: 60; SEQ ID NO: 84 to SEQ ID NO: 103), or in genomic DNA (SEQ ID NO: 1 to SEQ ID NO: 12; SEQ ID NO: 79 to SEQ ID NO: 83 and sequences complementary thereto).

In preferred embodiments, at least one, and more preferably all members of a set of oligonucleotides is bound to a solid phase.

In further embodiments, the present invention discloses a set of at least two (2) oligonucleotides that are used as 'primer' oligonucleotides for amplifying DNA sequences of one of SEQ ID NO: 1 to SEQ ID NO: 60; SEQ ID NO: 79 to SEQ ID NO: 103 and sequences complementary thereto, or segments thereof.

It is anticipated that the oligonucleotides may constitute all or part of an "array" or "DNA chip" (i.e., an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the prior art in oligomer array manufacturing can be gathered from a special edition of Nature Genetics (Nature Genetics Supplement, Volume 21, January 1999, and from the literature cited therein). Fluorescently labelled probes are often used for the scanning of immobilized DNA arrays. The simple attachment of Cy3 and Cy5 dyes to the 5'-OH of the specific probe are particularly suitable for fluorescence labels. The detection of the fluorescence of the hybridised probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e*., each analyte) can be uniquely bound by a unique label and thus detected (each label is directly counted, resulting in a digital read-out of each molecular species in the mixture).

It is particularly preferred that the oligomers are utilised for detecting, or for diagnosing lung carcinoma. Particularly preferred is a lung cancer selected from the group consisting of lung adenocarcinoma; large cell lung cancer; squamous cell lung carcinoma; small cell lung carcinoma.

### Kits

Moreover, a kit is described comprising: a means for determining the expression at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1 methylation. The means for determining the expression of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1 preferably comprise a bisulfite-containing reagent; one or a plurality of oligonucleotides consisting whose sequences in each case are identical, are complementary, or hybridise under stringent or highly stringent conditions to a 9 or more preferably 18 base long segment of a sequence selected from SEQ ID NO: 13 to SEQ ID NO: 60; SEQ ID NO: 84 to SEQ ID NO: 103; and optionally instructions for carrying out and evaluating the described method of methylation analysis. In one embodiment the base sequence of said oligonucleotides comprises at least one CpG, CpA or TpG dinucleotide.

In a further embodiment, said kit may further comprise standard reagents for performing a CpG position-specific methylation analysis, wherein said analysis comprises one or more of the following techniques: MS-SNuPE, MSP, MethyLight™, HeavyMethyl, COBRA, and nucleic acid sequencing. However, a kit can also contain only part of the aforementioned components.

In a preferred embodiment the kit may comprise additional bisulfite conversion reagents selected from the group consisting: DNA denaturation buffer; sulfonation buffer; DNA recovery reagents or kits (*e.g*., precipitation, ultrafiltration, affinity column); desulfonation buffer; and DNA recovery components.

In a further alternative embodiment, the kit may contain, packaged in separate containers, a polymerase and a reaction buffer optimised for primer extension mediated by the polymerase, such as PCR. In another embodiment the kit further comprising means for obtaining a biological sample of the patient. Preferred is a kit, which further comprises a container suitable for containing the means for determining methylation of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1 in the biological sample of the patient, and most preferably further comprises instructions for use and interpretation of the kit results. In a preferred embodiment the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 13 to SEQ ID NO: 60; SEQ ID NO: 84 to SEQ ID NO: 103; and optionally (d) instructions for use and interpretation of the kit results. In an alternative preferred embodiment the kit comprises: (a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridises to a pre-treated nucleic acid sequence according to one of SEQ ID NO: 13 to SEQ ID NO: 60; SEQ ID NO: 84 to SEQ ID NO: 103 and sequences complementary thereto; and optionally (d) instructions for use and interpretation of the kit results.

In an alternative embodiment the kit comprises: (a) a bisulfite, reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 13 to SEQ ID NO: 60; SEQ ID NO: 84 to SEQ ID NO: 103; (d) at least one oligonucleotides and/or PNA-oligomer having a length of at least 9 or 16 nucleotides which is identical to or hybridises to a pre-treated nucleic acid sequence according to one of SEQ ID NO: 13 to SEQ ID NO: 60; SEQ ID NO: 84 to SEQ ID NO: 103 and sequences complementary thereto; and optionally (e) instructions for use and interpretation of the kit results.

The kit may also contain other components such as buffers or solutions suitable for blocking, washing or coating, packaged in a separate container.

Described is a kit for use in determining the presence of and/or diagnosing lung carcinoma. Particularly preferred is a lung cancer selected from the group consisting of lung adenocarcinoma; large cell lung cancer; squamous cell lung carcinoma; small cell lung carcinoma. Said kit prefereably comprises: a means for measuring the level of transcription of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1 and a means for determining at least one gene or genomic sequence selected from the group consisting of PTGER4; RUNX1; EVX2;EVX-1; SHOX2; SEQ ID NO: 6; CN027; LRAT; IL-12RB1; TFAP2C; BCL2; EN2; PRDM14; SEQ ID NO: 81; ARID5A; VAX1 methylation.

Typical reagents (*e.g*., as might be found in a typical COBRA™-based kit) for COBRA™ analysis may include, but are not limited to: PCR primers for at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1; restriction enzyme and appropriate buffer; gene-hybridization oligo; control hybridization oligo; kinase labeling kit for oligo probe; and labeled nucleotides. Typical reagents (*e.g*., as might be found in a typical MethyLight™-based kit) for MethyLight™ analysis may include, but are not limited to: PCR primers for the bisulfite converted sequence of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1; bisulfite specific probes (e.g. TaqMan™ or Lightcycler™); optimized PCR buffers and deoxynucleotides; and Taq polymerase.

Typical reagents (*e.g*., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE^{™} primers for the bisulfite converted sequence of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1; reaction buffer (for the Ms-SNuPE reaction); and labelled nucleotides.

Typical reagents (e.g., as might be found in a typical MSP-based kit) for MSP analysis may include, but are not limited to: methylated and unmethylated PCR primers for the bisulfite converted sequence of at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1, optimized PCR buffers and deoxynucleotides, and specific probes.

Moreover, described is an alternative kit comprising a means for determining at least one gene or genomic sequence selected from the group consisting of PTGER4; RUNX1; EVX2; EVX-1; SHOX2; SEQ ID NO: 6; CN027; LRAT;IL-12RH1; TFAP2C; BCL2; EN2; PRDM14; SEQ ID NO: 81; ARID5A; VAX1 methylation, wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence selected from SEQ ID NO: 1 to SEQ ID NO: 12; SEQ ID NO: 79 to SEQ ID NO: 83; and optionally instructions for carrying out and evaluating the described method of methylation analysis. In one embodiment the base sequence of said oligonucleotides are identical, are complementary, or hybridise sunder stringent or highly stringent conditions to an at least 18 base long segment of a sequence selected from SEQ ID NO: 1 to SEQ ID NO: 12; SEQ ID NO: 79 to SEQ ID NO: 83.

In a further embodiment said kit may comprise one or a plurality of oligonucleotide probes for the analysis of the digest fragments, preferably said oligonucleotides are identical, are complementary, or hybridise under stringent or highly stringent conditions to an at least 16 base long segment of a sequence selected from SEQ ID NO: 1 to SEQ ID NO: 12; SEQ ID NO: 79 to SEQ ID NO: 83.

In a preferred embodiment the kit may comprise additional reagents selected from the group consisting: buffer (e.g. restriction enzyme, PCR, storage or washing buffers); DNA recovery reagents or kits (*e.g*., precipitation, ultrafiltration, affinity column) and DNA recovery components.

In a further alternative embodiment, the kit may contain, packaged in separate containers, a polymerase and a reaction buffer optimised for primer extension mediated by the polymerase, such as PCR. In another embodiment of the invention the kit further comprising means for obtaining a biological sample of the patient. In a preferred embodiment the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridise under stringent or highly stringent conditions to an at least 9 base long segment of a sequence selected from SEQ ID NO: 1 to SEQ ID NO: 12; SEQ ID NO: 79 to SEQ ID NO: 83; and optionally (d) instructions for use and interpretation of the kit results.

In an alternative preferred embodiment the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 12; SEQ ID NO: 79 to SEQ ID NO: 83; and optionally (d) instructions for use and interpretation of the kit results.

In an alternative embodiment the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 12; SEQ ID NO: 79 to SEQ ID NO: 83; (d) at least one set of oligonucleotides one or a plurality of nucleic acids or peptide nucleic acids which are identical, are complementary, or hybridise under stringent or highly stringent conditions to an at least 9 base long segment of a sequence selected from SEQ ID NO: 1 to SEQ ID NO: 12; SEQ ID NO: 79 to SEQ ID NO: 83 and optionally (e) instructions for use and interpretation of the kit results.

The kit may also contain other components such as buffers or solutions suitable for blocking, washing or coating, packaged in a separate container.

Described is a kit for use in providing a diagnosis of the presence or absence of lung carcinoma, in a subject by means of methylation-sensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for at least one gene or genomic sequence selected from the group consisting of PTGER4; RUNX1; EVX2; EVX-1; SHOX2; SEQ ID NO: 6; CN027; LRAT; IL-12RB1; TFAP2C; BCL2; EN2; PRDM14; SEQ ID NO: 81; ARID5A; VAX1 and comprises a sequence of at least 15 base pairs in length but no more than 200 bp of a sequence according to one of SEQ ID NO: 1 to SEQ ID NO: 12; SEQ ID NO: 79 to SEQ ID NO: 83. Preferably said sequence is at least 15 base pairs in length but no more than 80 bp of a sequence according to one of SEQ ID NO: 1 to SEQ ID NO: 12; SEQ ID NO: 79 to SEQ ID NO: 83. It is further preferred that said sequence is at least 20 base pairs in length but no more than 30 bp of a sequence according to one of SEQ ID NO: 1 to SEQ ID NO: 12; SEQ ID NO: 79 to SEQ ID NO: 83.

Said test kit preferably further comprises a restriction enzyme component comprising one or a plurality of methylation-sensitive restriction enzymes.

In a further embodiment said test kit is further characterized in that it comprises at least one methylation-specific restriction enzyme, and wherein the oligonucleotides comprise a restriction site of said at least one methylation specific restriction enzymes.

The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step.

Further described is a composition of matter useful for detecting, or for diagnosing lung carcinoma. Particularly preferred is a lung cancer selected from the group consisting of lung adenocarcinoma; large cell lung cancer; squamous cell lung carcinoma; small cell lung carcinoma.

Said composition preferably comprises at least one nucleic acid 18 base pairs in length of a segment of the nucleic acid sequence disclosed in SEQ ID NO: 13 to SEQ ID NO: 60; SEQ ID NO: 84 to SEQ ID NO: 103, and one or more substances taken from the group comprising: 1-5 mM Magnesium Chloride, 100-500 µM dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 13 to SEQ ID NO: 60; SEQ ID NO: 84 to SEQ ID NO: 103 and sequences complementary thereto. It is preferred that said composition of matter comprises a buffer solution appropriate for the stabilization of said nucleic acid in an aqueous solution and enabling polymerase based reactions within said solution. Suitable buffers are known in the art and commercially available.

Said at least one nucleic acid can be at least 50, 100, 150, 200, 250 or 500 base pairs in length of a segment of the nucleic acid sequence disclosed in SEQ ID NO: 13 to SEQ ID NO: 60; SEQ ID NO: 84 to SEQ ID NO: 103.

**Table 1**

| Gene | Genomic SEQ ID NO: | Pretreated methylated sequence (sense) SEQ ID NO: | Pretreated methylated strand (antisense) SEQ ID NO: | Pretreated unmethylated sequence (sense) SEQ ID NO: | Pretreated unmethylated sequence (antisense) SEQ ID NO: |
|---|---|---|---|---|---|
| RUNX1 | 1 | 13 | 14 | 37 | 38 |
| EVX2 | 2 | 15 | 16 | 39 | 40 |
| EVX-1 | 3 | 17 | 18 | 41 | 42 |
| PTGER4 | 4 | 19 | 20 | 43 | 44 |
| SHOX2 | 5 | 21 | 22 | 45 | 46 |
| No annotated gene, sequence located between TIM14 & SOX-2 genes | 6 | 23 | 24 | 47 | 48 |
| CN027 | 7 | 25 | 26 | 49 | 50 |
| LRAT | 8 | 27 | 28 | 51 | 52 |
| IL-12RB1 | 9 | 29 | 30 | 53 | 54 |
| TFAP2C | 10 | 31 | 32 | 55 | 56 |
| BCL2 | 11 | 33 | 34 | 57 | 58 |
| ARID5A | 12 | 35 | 36 | 59 | 60 |
| Homeobox protein engrailed-2 (Hu-En-2); EN2; HME2 | 79 | 84 | 85 | 94 | 95 |
| PR domain zinc finger protein 14 (PR domain-containing protein 14); pRDM14 | 80 | 86. | 87 | 96 | 97 |
| Chromosomal location (NCBI36) chromosome: 15 bp45139161 to 45139783 | 81 | 88 | 89 | 98 | 99 |
| AT rich interactive domain 5A; ARID5A | 82 | 90 | 91 | 100 | 101 |
| Ventral anterior homeobox 1; VAX1 | 83 | 92 | 93 | 102 | 103 |

**Table 2**

| Gene | Preferred disorder |
|---|---|
| EVX2 | Cancers, preferably lung |
| RUNX1 | Cancers, preferably lung, prostate and/or breast |
| PTGER4 | Cancers, preferably lung, prostate and/or breast |
| SHOX2 | Cancers, preferably lung, breast and/or bladder |
| none; upstream: TIM14 / downstream: SOX-2 | Cancers, preferably prostate |
| CN027 | Cancers, preferably lung and/or prostate |
| LRAT | Cancers, preferably colon |
| IL-12RB1 | Cancers, preferably prostate and/or breast |
| EVX-1 | Cancers |
| TFAP2C | Cancers |
| BCL2 | Cancers, preferably lung |
| Homeobox protein engrailed-2 (Hu-En-2); EN2; HME2 | Cancers, preferably lung |
| PR domain zinc finger protein 14 (PR domain-containing protein 14); PRDM14 | Cancers, preferably lung |
| Chromosomal location (NCBI36) chromosome: 15 bp45139161 to 45139783 | Cancers, preferably lung |
| AT rich interactive domain 5A; ARID5A | Cancers, preferably lung |
| Ventral anterior homeobox 1; VAX1 | Cancers, preferably lung |

**Table 3A**

| Gene/ Genomic region | Primer 1 SEQ ID NO: | Primer 2 SEQ ID NO: | Probe SEQ ID NO: |
|---|---|---|---|
| SEQ ID NO:6 | 64 | 65 | 66 |
| CN027 | 67 | 68 | 69 |
| LRAT | 70 | 71 | 72 |
| TFAP2C | 73 | 74 | 75 |
| EVX-1 | 76 | 77 | 78 |
| EVX2 | 79 | 80 | 81 |
| SHOX2 | 82 | 83 | 84 |
| ARID5A | 85 | 86 | 87 |
| RUNX1 | 88 | 89 | 90 |
| PTGER4 | 91 | 92 | 93 |
| BCL2 | 94 | 95 | 96 |
| IL-12RB1 | 97 | 98 | 99 |

**Table 3B**

| Target sequence (Genomic) | Oligo SEQ ID NO: | Sequence |
|---|---|---|
| SEQ ID NO: 79 Forward Primer | 104 | tatcgcggagattttcgagttttcgttg |
| SEQ ID NO: 79 Probe | 105 | ggggttgtnttcgggatt |
| SEQ ID NO: 79 Reverse Primer | 106 | gataaccctaaaacgcaactcgaa |
| SEQ ID NO: 80 Reverse Primer | 107 | cgtttcctaacgagcgtctt |
| SEQ ID NO: 80 Forward Primer | 108 | cgcgttcttcgcggttagtttcgt |
| SEQ ID NO: 80 Probe | 109 | cgacgttttcgcgtgg |
| SEQ ID NO: 81 Forward Primer | 110 | gttttgaaatttattagaataacgacgtt |
| SEQ ID NO: 81 Reverse Primer | 111 | ctttctaaaaataaccgaactatactacgac |
| SEQ ID NO:81 Probe | 112 | tacggacgttcgcgggtcgtt |
| SEQ ID NO: 82 Probe | 113 | agtaagttcgcgtagattcggttt |
| SEQ ID NO: 82 Reverse Primer | 114 | taaaacgacgaaacgaccgat |
| SEQ ID NO: 82 Forward Primer | 115 | tcgtcgttttcgggttgtcgagtg |
| SEQ ID NO: 83 Probe | 116 | aaggaagtggaataaatcgtcgta |
| SEQ ID NO: 83 Forward Primer | 117 | aggcgtttttgttttttcggaaattcgaaattc |
| SEQ ID NO: 83 Reverse Primer | 118 | ctacgactaataccgtaaacgccta |

**Table 4A**

| Tissue type | All lung cancers | Lung adenocarcinoma | Large cell lung cancer | Squamouscell lung carcinoma | Small cell lung carcinoma | Prostate carcinoma |
|---|---|---|---|---|---|---|
| PMR threshold (%) | >20 | >20 | >20 | >20 | >20 | >20 |
| N total | 61 | 21 | 12 | 21 | 7 | 10 |
| Gene | | | | | | |
| EVX2 | 76,8 | 52,4 | 75,0 | 90,5 | 100,0 | 60,0 |
| RUNX1 | 76,7 | 61,9 | 58,3 | 85,7 | 100,0 | 100,0 |
| PTGER4 | 66,1 | 66,7 | 66,7 | 61,9 | 71,4 | 100,0 |
| SHOX2 | 63,0 | 38,1 | 50,0 | 90,5 | 71,4 | 20,0 |
| SEQ IDNO: 6 | 68,2 | 38,1 | 33,3 | 95,2 | 100,0 | 100,0 |
| CN027 | 55,9 | 38,1 | 41,7 | 71,4 | 71,4 | 100,0 |
| LRAT | 41,7 | 42,9 | 33,3 | 42,9 | 42,9 | 30,0 |
| IL-12RB1 | 24,9 | 14,3 | 16,7 | 28,6 | 42,9 | 100,0 |
| EVX-1 | 47,4 | 38,1 | 33,3 | 57,1 | 57,1 | 100,0 |
| TFAP2C | 68,1 | 38,1 | 58,3 | 85,7 | 100,0 | 100,0 |
| BCL2 | 14,1 | 23,8 | 8,3 | 14,3 | 0,0 | 50,0 |

**Table 4B**

| Tissue type | Colorectal cancer | Breast carcinoma | Bladder carcinoma | Lung diseasesd (Not cancer) | Lung healthy | Blood |
|---|---|---|---|---|---|---|
| PMR threshold (%) | >20 | >20 | >20 | >5 | >5 | >0,2 |
| N (total) | 10 | 10 | 10 | 7 | 12 | 20 |
| Gene | | | | | | |
| EVX2 | 40,0 | 80,0 | 80,0 | 0,0 | 0,0 | 55,0 |
| RUNX1 | 20,0 | 80,0 | 60,0 | 0,0 | 0,0 | 0,0 |
| PTGER4 | 0,0 | 80,0 | 30,0 | 0,0 | 0,0 | 0,0 |
| SHOX2 | 60,0 | 80,0 | 70,0 | 0,0 | 0,0 | 10,0 |
| SEQ ID NO: 6 | 60,0 | 50,0 | 50,0 | 28,6 | 33,3 | 25,0 |
| CN027 | 90,0 | 70,0 | 70,0 | 0,0 | 8,3 | 25,0 |
| LRAT | 90,0 | 60,0 | 60,0 | 0.0 | 0,0 | 0,0 |
| IL-12RB1 | 0,0 | 80,0 | 30,0 | 85,7 | 100,0 | 0,0 |
| EVX-1 | 80,0 | 80,0 | 90,0 | 0,0 | 0,0 | 0,0 |
| TFAP2C | 100,0 | 100,0 | 60,0 | 14,3 | 0,0 | 25,0 |
| BCL2 | 0,0 | 10,0 | 60,0 | 0,0 | 0,0 | 0,0 |

**Table 4C**

| Tissue type | All lung cancers | Lung adenocarcinoma | Large cell lung cancer | Squamouscell lung carcinoma | Small cell lung carcinoma | Prostate carcinoma |
|---|---|---|---|---|---|---|
| PMR threshold (%) | >20 | >20 | >20 | >20 | >20 | >20 |
| N (total) | 61 | 21 | 12 | 21 | 7 | 10 |
| SEQ ID NO: | | | | | | |
| SEQ ID NO: 79 | 38 | 29 | 33 | 57 | 29 | 55 |
| SEQ ID NO: 80 | 42 | 29 | 16 | 66 | 43 | 11 |
| SEQ ID NO: 81 | 24 | 24 | 8 | 28 | 29 | 11 |
| SEQ ID NO: 82 | 58 | 57 | 50 | 62 | 57 | 66 |
| SEQ ID NO: 83 | 45 | 24 | 33 | 66 | 57 | 25 |

**Table 4D**

| Tissue type | Healthy Prostate | Colorectal cancer | Healthy Colon | Breast carcinoma | Healthy breast | Bladder carcinoma | Healthy Bladder | Lung diseasesd (Not cancer) | Lung healthy | Blood |
|---|---|---|---|---|---|---|---|---|---|---|
| PMR threshold (%) | >5 | >20 | >5 | >20 | >5 | >20 | >5 | >5 | >5 | >0,2 |
| N (total) | 11 | 10 | 9 | 10 | 12 | 10 | 10 | 7 | 12 | 20 |
| SEQ ID NO: | | | | | | | | | | |
| SEQ ID NO: 79 | 0 | 40 | 0 | 50 | 0 | 40 | 0 | 0 | 0 | 0 |
| SEQ ID NO: 80 | 0 | 50 | 0 | 40 | 0 | 30 | 0 | 0 | 0 | 0 |
| SEQ ID NO: 81 | 0 | 40 | 0 | 20 | 0 | 10 | 0 | 0 | 0 | 0 |
| SEQ ID NO: 82 | 12 | 10 | 22 | 60 | 0 | 90 | 70 | 0 | 0 | 0 |
| SEQ ID NO: 83 | 12 | 60 | 0 | 50 | 0 | 60 | 10 | 0 | 0 | 0 |

**Table 5**

| | PTGER4 | | RUNX1\| | |
|---|---|---|---|---|
| NSCLC | Sens | Spec | Sens | Spec |
| Bronchial Lavage | 52% | 91% | 39% | 91% |
| Blood Plasma | 69% | 91% | --- | --- |

### EXAMPLES

### Example 1

Analysis of methylation of the genes/sequences according to Table 1 in multiple cancer and control tissue samples was carried out by means of a Real-Time MSP assay using the components according to Table 3.

### Samples

| Tissue Type | No. of samples |
|---|---|
| Lung adenocarcinoma | 21 |
| Large cell lung cancer | 12 |
| Squamouscell lung carcinoma | 21 |
| Small cell lung carcinoma | 7 |
| Prostate carcinoma | 10 |
| Colorectal cancer | 10 |
| Breast carcinoma | 10 |
| Bladder carcinoma | 10 |
| Lung diseasesd (Not cancer) | 7 |
| Lung healthy | 12 |
| Blood/white blood cells | 20 |

### DNA extraction and bisulfite treatment

The DNA was isolated from the all samples according to a modified protocol based on that disclosed in the Qiagen Genomic DNA Handbook (August 2001) (pg 28-31, 44-47). The DNA in the eluate was quantified and the eulate was treated according to the following bisulfite reaction.

The eluate was mixed with 354 µl of bisulfite solution (5.89 mol/l) and 146 µl of dioxane containing a radical scavenger(6-hydroxy-2,5,7,8-tetramethylchromane 2-carboxylic acid, 98.6 mg in 2.5 ml of dioxane). The reaction mixture was denatured for 3 min at 99°C and subsequently incubated at the following temperature program for a total of 7 h min 50°C; one thermospike (99.9°C) for 3 min; 1.5 h 50°C; one thermospike (99°C) for 3 min; 3 h 50°C. The reaction mixture was subsequently purified by ultrafiltration using a Millipore Microcon ™ column. The purification was conducted essentially according to the manufacturer's instructions. For this purpose, the reaction mixture was mixed with 300 µl of water, loaded onto the ultrafiltration membrane, centrifuged for 15 min and subsequently washed with 1 x TE buffer. The DNA remains on the membrane in this treatment. Then desulfonation is performed. For this purpose, 0.2 mol/l NaOH was added and incubated for 10 min. A centrifugation (10 min) was then conducted, followed by a washing step with 1 x TE buffer. After this, the DNA was eluted. For this purpose, the membrane was mixed for 10 minutes with 75 µl of warm 1 x TE buffer (50°C). The membrane was turned over according to the manufacturer's instructions. Subsequently a repeated centrifugation was conducted, with which the DNA was removed from the membrane. 10 µl of the eluate was utilized for the Lightcycler Real Time PCR assay.

### Rference assay

The GSTP1 reference assay design makes it suitable for quantitating bisulfite converted DNA from different sources, including fresh/frozen samples, remote samples such as plasma or serum, and DNA obtained from archival specimen such as paraffin embedded material. The assay measures the total DNA independly from its methylation status, as long as no CpG is covered by the PCR oligonucleotides.

The following components were used in the reaction to amplify the control amplificate:
- 10 µl of template DNA
- 2 µl of FastStart LightCycler Mix for hybridization probes (Roche Diagnostics)
- 3.5 mmol/l MgC12 (Roche Diagnostics)
- 0.60 µmol/l forward primer (SEQ ID NO: 61, TIB-MolBiol)
- 0.60 µmol/l reverse primer (SEQ ID NO: 62, TIB-MolBiol)
- 0.2 µmol/l probe (SEQ ID NO: 63, TIB-MolBiol)

The following oligonucleotides were used in the reaction to amplify the control amplificate:
Primer1: GGAGTGGAGGAAATTGAGAT (SEQ ID NO: 61)
Primer2: CCACACAACAAATACTCAAAAC (SEQ ID NO: 62)
Probe: FAM-TGGGTGTTTGTAATTTTTGTTTTGTGTTAGGTT-TAMRA (SEQ ID NO: 63)

The assay was performed in the LightCycler 480 according to the following temperature-time-profile:
- Activation 10 min at 95°C
- 50 cycles: 10 sec at 95 °C
   30 sec at 56 °C
   10 sec at 72 °C

The detection was carried out during the annealing phase at 56 °C in channel for 530 nm with a target specific fluorescence probe (Seq ID-63).

### Methylation Specific Real Time PCR

The methylation specific real time PCR (MSP) reactions were performed on the bisulfite converted sample DNA. All MSP mastermixes were the Roche FastStart TaqMan Probe Master containing 300 nM ROX reference dye on the ABI7900 instrument. Each reaction contained 600 nM of the forward primer, 600 nM reverse primer, and 200 nM of the detection probe (see table with Seq-IDs of the markers). The reactions were performed in a final volume of 20 microL using the ABI7900 instrument with following temperature and time profile :
- Activation 10 min at 95 °C
- 50 cycles: 15 sec at 95°C
   60 sec at 60°C

### Data interpretation

### Calculation of DNA concentration.

The CP (crossing point values) as calculated by the ABI7900 instrument software using a specific threshold was used for each assay to determine DNA concentration. The DNA concentration was calculated by reference of the CP value of each well to a calibration standard. The calibration standard was prepared from methylated bisulfite converted human DNA containing 10, 5, 2.5, 1, 0.4, 0.1, and 0.05 ng per reaction. The calibration curves was used for both the methylation specific marker assays and the C3 reference assay.

### Percentage methylation

For each sample the detected percentage methylation was calculated as the measured concentration of DNA quantified using the methylation assays over the concentration of DNA in the sample as quantified by the C3 assay. The methylation ratios were calculated according to the PMR value method (Eads et al., Cancer Res. 2001 Apr 15; 61(8): 3410-8, PMID 11309301) against the total DNA quantified by the C3 reference PCR.

Detection of methylation was determined at multiple different threshold levels, see Tables 4A to 4D).

Results are provided in Table 4A to 4D indicating the %of samples of each tissue class with methylation above the PMR (% methylation) threshold.

### Example 2

Analysis of methylation of the genes PTGER4 and RUNX1 was confirmed in cancer and control body fluid samples (plasma, bronchial lavage).

### Samples

| Analysis Group | plasma | lavage |
|---|---|---|
| Lung adenocarcinoma | 50 | 50 |
| Benign lung disease | 50 | 50 |

DNA was extracted from plasma and bronchial lavage using MagnaPure (Roche Diagnostics)

For bronchial lavage (BL) samples the following preprocessing was performed:

1 ml of BL sample was centrifuged for 10 minutes at 8,000 x g to pellet sample. After removing 900 ul of supernatant the cells were resuspended in the remaining 100 ul of liquid. Then 130 ul of Bacteria Lysis Buffer and 20 ul of Proteinase K were added to the sampe, which was vortexed for 10 seconds. After a quick spin down to collect the sample at the bottom of the tube it was incubated for 10 minutes at 60 C and for 15 minutes at 90 C. Then the sample was cooled down for 60 seconds and collected at the bottom of the tube by briefly centrifuging it.

Bisulfite treatment and reference assays were carried out, substantially as above. Methylation analysis was carried out by means of a Real-Time PCR HM assay.

Results are provided in Table 5.

## Claims

1. A method for detecting lung carcinoma by detecting the presence or absence of CpG methylation within the PTGER4 gene and/or promoter or regulatory elements in a subject wherein hyper-methylation is indicative of the presence of lung carcinoma.

2. The method according to claim 1, comprising contacting genomic DNA isolated from a biological sample obtained from said subject with at least one reagent, or series of reagents that distinguishes between methylated and non-methylated CpG dinucleotides within at least one target region of the genomic DNA, wherein the target region comprises, or hybridizes under stringent conditions to a sequence of at least 16 contiguous nucleotides of SEQ ID NO: 4, wherein said contiguous nucleotides comprise at least one CpG dinucleotide sequence, whereby detecting said lung carcinoma is afforded.

3. The method for detecting cell proliferative disorders according to claim 1 or 2, comprising:
a) extracting or otherwise isolating genomic DNA from a biological sample obtained from the subject;
b) treating the genomic DNA of a), or a fragment thereof, with one or more reagents to convert cytosine bases that are unmethylated in the 5-position thereof to uracil or to another base that is detectably dissimilar to cytosine in terms of hybridization properties;
c) contacting the treated genomic DNA, or the treated fragment thereof, with an amplification enzyme and at least one primer comprising, a contiguous sequence of at least 9 nucleotides that is complementary to, or hybridizes under moderately stringent or stringent conditions to a sequence selected from the group consisting of SEQ ID NO: 19, 20, 43, and 44, and complements thereof, wherein the treated genomic DNA or the fragment thereof is either amplified to produce at least one amplificate, or is not amplified; and
d) determining, based on a presence or absence of, or on a property of said amplificate, the methylation state or level of at least one CpG dinucleotide of SEQ ID NO: 4, or an average, or a value reflecting an average methylation state or level of a plurality of CpG dinucleotides of SEQ ID NO: 4, whereby at least one of detecting and diagnosing cell proliferative disorders, is afforded.

4. The method according to claim 3, wherein treating the genomic DNA, or the fragment thereof in b), comprises use of a reagent selected from the group comprising of bisulfite, hydrogen sulfite, disulfite, and combinations thereof.

5. The method according to any of claims 1 to 4, wherein said biological sample obtained from the subject is selected from the group comprising cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion and combinations thereof.

6. A method for detecting cell proliferative disorders according to any of claims 1 to 3, comprising:
a) extracting or otherwise isolating genomic DNA from a biological sample obtained from the subject;
b) digesting the genomic DNA of a), or a fragment thereof, with one or more methylation sensitive restriction enzymes;
contacting the DNA restriction enzyme digest of b), with an amplification enzyme and at least two primers suitable for the amplification of a sequence comprising at least one CpG dinucleotide of SEQ ID NO: 4; and
c) determining, based on a presence or absence of an amplificate the methylation state or level of at least one CpG dinucleotide of SEQ ID NO: 4, whereby at least one of detecting and diagnosing cell proliferative disorders, is afforded.

7. Use of a method according to any of claims 1 to 6 in the diagnosis, prognosis and/or detection of lung carcinoma.

## Patentansprüche

1. Verfahren zum Nachweis von Lungenkarzinom durch Nachweisen der Anwesenheit oder Abwesenheit von CpG Methylierung innerhalb des Gens PTGER4 und/oder dessen Promotor oder regulatorischer Elemente in einem Subjekt, wobei Hypermethylierung die Anwesenheit von Lungenkarzinom anzeigt.

2. Verfahren nach Anspruch 1, umfassend in Kontakt bringen von genomischer DNA, die aus einer biologischen Probe isoliert wurde, die von dem Subjekt erhalten wurde, mit mindestens einem Reagenz, oder einer Reihe von Reagenzien, die zwischen methylierten und nicht-methylierten CpG Dinukleotiden innerhalb mindestens einer Zielregion der genomischen DNA unterscheidet, wobei die Zielregion unter stringenten Bedingungen an eine Sequenz von mindestens 16 aufeinander folgenden Nukleotiden of SEQ ID NO: 4 hybridisiert oder diese umfasst, wobei die aufeinander folgenden Nukleotide mindestens eine CpG Dinukleotidsequenz umfassen, wodurch das Nachweisen des Lungenkarzinoms bewirkt wird.

3. Verfahren zum Nachweis von proliferativen Störungen von Zellen nach Anspruch 1 oder 2, umfassend:
a) Extrahieren oder auf andere Weise Isolieren von genomischer DNA aus einer biologischen Probe, die von dem Subjekt erhalten wurde;
b) Behandeln der genomischen DNA von a), oder eines Fragments davon, mit einem oder mehreren Reagenzien, um Cytosinbasen, die in ihrer 5-Position nicht methyliert sind, zu Uracil oder in eine andere Base umzuwandeln, die im Hinblick auf ihre Hybridisierungseigenschaften von Cytosin nachweisbar verschieden ist;
c) in Kontakt bringen der behandelten genomischen DNA, oder dem behandelten Fragment davon, mit einem Amplifikationsenzym und mindestens einem Primer umfassend eine aufeinander folgende Sequenz von mindestens 9 Nukleotiden, die unter moderat stringenten oder stringenten Bedingungen an eine Sequenz ausgewählt aus der Gruppe bestehend aus SEQ ID NO: 19, 20, 43 und 44, und Komplementen davon hybridisiert oder dazu komplementär ist, wobei die behandelte genomische DNA oder das Fragment davon entweder amplifiziert wird, um mindestens ein Amplifikat herzustellen, oder nicht amplifiziert wird; und
d) Bestimmen, basierend auf einer Anwesenheit oder Abwesenheit von, oder einer Eigenschaft des Amplifikats, des Methylierungszustands oder -spiegels von mindestens einem CpG Dinukleotid von SEQ ID NO: 4, oder eines Durchschnitts, oder eines Wertes, der einen durchschnittlichen Methylierungszustand oder -spiegel einer Vielzahl von CpG Dinukleotiden von SEQ ID NO: 4 widerspiegelt, wodurch mindestens Eines von Nachweisen und Diagnostizieren von proliferativen Störungen von Zellen bewirkt wird.

4. Verfahren nach Anspruch 3, wobei das Behandeln der genomischen DNA, oder des Fragments davon in b) die Verwendung eines Reagenz ausgewählt aus der Gruppe bestehend aus of Bisulfit, Hydrogensulfit, Disulfit und Kombinationen davon umfasst.

5. Verfahren nach einem der Ansprüche 1 bis 4, wobei die biologische Probe, die von dem Subjekt erhalten wurde, ausgewählt ist aus der Gruppe umfassend Zellen oder Zelllinien, histologische Schnitte, Biopsien, in Paraffin eingebettetes Gewebe, Körperflüssigkeiten, Ejakulat, Urin, Blutplasma, Blutserum, Vollblut, isolierte Blutzellen, Sputum und biologisches Material abgeleitet von einer Bronchoskopie (einschließlich, aber nicht beschränkt auf, bronchiale Lavage, bronchiale alveolare Lavage, bronchiales Bürsten, bronchiale Abrasion und Kombinationen davon.

6. Verfahren zum Nachweis von proliferativen Störungen von Zellen nach einem der Ansprüche 1 bis 3, umfassend:
a) Extrahieren oder auf andere Weise Isolieren von genomischer DNA aus einer biologischen Probe, die von dem Subjekt erhalten wurde;
b) Verdauen der genomischen DNA von a), oder eines Fragments davon, mit einem oder mehreren Methylierungs-sensitiven Restriktionsenzymen;
in Kontakt bringen des DNA Restriktionsverdaus von b), mit einem Amplifikationsenzym und mindestens zwei Primern, die für die Amplifikation einer Sequenz geeignet sind, die mindestens ein CpG Dinukleotid von SEQ ID NO: 4 umfasst; und
c) Bestimmen, basierend auf einer Anwesenheit oder Abwesenheit eines Amplifikats, des Methylierungszustands oder -spiegels von mindestens einem CpG Dinukleotid von SEQ ID NO: 4, wodurch mindestens Eines von Nachweisen und Diagnostizieren von proliferativen Störungen von Zellen bewirkt wird.

7. Verwendung eines Verfahrens nach einem der Ansprüche 1 bis 6 in der Diagnose, Prognose und/oder dem Nachweis von Lungenkarzinom.

## Revendications

1. Procédé de détection d'un carcinome pulmonaire en détectant la présence éventuelle de méthylation de CpG dans le gène PTGER4 et/ou le promoteur ou les éléments régulateurs chez un sujet où une hyper-méthylation indique la présence d'un carcinome pulmonaire.

2. Procédé selon la revendication 1, comprenant le fait de mettre en contact un ADN génomique isolé d'un échantillon biologique obtenu dudit sujet avec au moins un réactif, ou avec une série de réactifs qui fait la distinction entre des dinucléotides CpG méthylés et non-méthylés dans au moins une région cible de l'ADN génomique, où la région cible comprend, ou s'hybride sous des conditions stringentes à une séquence d'au moins 16 nucléotides contigus de la SEQ ID NO : 4, où lesdits nucléotides contigus comprennent au moins une séquence de nucléotides CpG, moyennant quoi la détection dudit carcinome pulmonaire est possible.

3. Procédé de détection de désordres prolifératifs cellulaires selon la revendication 1 ou 2, comprenant le fait :
a) d'extraire ou sinon d'isoler un ADN génomique d'un échantillon biologique obtenu auprès du sujet ;
b) de traiter l'ADN génomique de a), ou l'un de ses fragments, avec un ou plusieurs réactifs pour convertir des bases de cytosine qui sont non-méthylées dans leur position 5 en uracile ou en une autre base qui est distincte de manière détectable à la cytosine en termes de propriétés d'hybridation ;
c) de mettre en contact l'ADN génomique traité, ou l'un de ses fragments traité, avec une enzyme d'amplification et au moins une amorce comprenant, une séquence contigüe d'au moins 9 nucléotides qui sont complémentaires à une séquence sélectionnée du groupe composé des SEQ ID NO : 19, 20, 43, et 44, et leurs compléments, ou qui s'hybride sous des conditions stringentes ou modérément stringentes à ces séquences, où l'ADN génomique traité ou son fragment est soit amplifié pour produire au moins un amplificateur, ou n'est pas amplifié ; et
d) de déterminer, sur la base de la présence ou de l'absence dudit amplificateur, ou sur d'une propriété de celui-ci, l'état ou le niveau de méthylation d'au moins un dinucléotide CpG de la SEQ ID NO : 4, ou une moyenne, ou une valeur reflétant l'état ou le niveau de méthylation moyen(ne) d'une pluralité de dinucléotides CpG de la SEQ ID NO : 4, moyennant quoi au moins la détection ou le diagnostic des désordres prolifératifs cellulaire, est possible.

4. Procédé selon la revendication 3, dans lequel le fait de traiter l'ADN génomique, ou de son fragment dans b), comprend l'utilisation d'un réactif sélectionné parmi le groupe comprenant du bisulfite, ou du sulfite d'hydrogène, du disulfite, et leurs combinaisons.

5. Procédé selon l'une des revendications 1 à 4, dans lequel ledit échantillon biologique obtenu auprès du sujet est sélectionné parmi le groupe se composant de cellules ou de lignes cellulaires, de lames histologiques, de biopsies, de tissu incorporé dans la paraffine, de liquides organiques, d'éjaculations, d'urine, de plasma sanguin, de sérum sanguin, du sang total, de cellules sanguines isolées, de crachat, et de matières biologiques dérivées de la bronchoscopie (comportant sans s'y limiter un lavage bronchique, un lavage alvéolaire bronchique, un brossage bronchique, une abrasion bronchique et leurs combinaisons).

6. Procédé de détection de désordres prolifératifs cellulaires selon l'une des revendications 1 à 3, comprenant le fait :
a) d'extraire ou autrement d'isoler un ADN génomique d'un échantillon biologique obtenu auprès du sujet ;
b) de faire digérer l'ADN génomique de a), ou son fragment, avec un ou plusieurs enzymes de restriction sensibles à la méthylation ;
de mettre en contact le digesté de l'enzyme de restriction d'ADN de b), avec une enzyme d'amplification et au moins deux amorces appropriées à l'amplification d'une séquence comprenant au moins un dinucléotide CpG de la SEQ ID : 4 ; et
c) de déterminer, sur la base de la présence ou de l'absence d'un amplificateur l'état ou le niveau de méthylation d'au moins un dinucléotide CpG de la SEQ ID : 4, moyennant quoi au moins la détection ou le diagnostic de désordres prolifératifs des cellules, est possible.

7. Utilisation d'un procédé selon l'une des revendications 1 à 6 dans le diagnostic, le pronostic et/ou la détection d'un carcinome pulmonaire.


### Sequence listing

<110> Epigenomics AG
<120> METHODS AND NUCLEIC ACIDS FOR ANALYSES OF CELL PROLIFERATIVE DISORDERS.
<13> E31013PCT
<160> 118
<210> 1
   <211> 3905
   <212> DNA
   <213> Homo Sapiens
<400> 1
   ttcaaaaaag cagatccgaa gatgtttaaa gtaataatgg tgctgttaga atggtgctcc      60
   ctctccctcc caggggatga ctttgaagga cactacaata acagatgtgt aaattctgaa     120
   atatatggct aaaatgaggt ttattatttt ctagtcacca cttagacatc ttttagtcat     180
   ttgtttatga cagtttataa atgagaaaga agtccattta aaatatctcc atgaggacct     240
   caaggtaaat aataggaatc gatcattcat caatctggcc tggccccggc ccactgaaga     300
   aagttccttc ttgagtgcat tcaaagctct catggtccag ccctgttcaa cctgagcagc     360
   atcctatctg ctcctccccc agacgttccg atgccctgca ccagattcaa gagagagcca     420
   agttccccaa caggctatgc acgtggctat taacacccag cacacgctat ccctggccag     480
   ctctctcctt cttccctaag aattaaacat tctttggcct gagttttcct cctttatgtc     540
   accataaagt tgagttattc attattaggg tctatacatg agtagccaaa atatacgggt     600
   ctctatattt aggtcaaggg gccttcagca ggagaacatg aataattagg agtgaggcca     660
   tgagacagag gaagaaaagc tcataggaat gggaagacag cctctcagat gagaggtgtg     720
   cggagtgatt gctgtgagga attgctcaat aggaaagtta gccaacctga ggacgtttct     780
   aggggaggaa aagggaaact catatggttc tctgctaatt cttaaaaata aggtagattc     840
   ttactcatct agggtgcttg ctcgttatga acacaggaag attaaggaat gagtatggac     900
   tgtttctact ctatgttttc ggacaagaag aataataaaa aagcactggg tagctggaac     960
   aactgcagag gccccaacaa tgatggagac tttctggtgc accgtggcac aggggtctct    1020
   tccaaaggga ctcaatcaac cccaacaggc atcatcttct gtgactcaag atgtcctttt    1080
   taggagtgct tgtacttttt aatgctcaga gaagttcgta ttactgattc gggaacactg    1140
   agtttttcag ctcctgtaaa actattttca ggtttatttt caagtacatt ctttacctcg    1200
   gggggtcaaa tcttttggtt tccagtgaat atcgtggtta tacctgtggt tacccactga    1260
   cttctcatct gcctagctta tctgataaca ggcaaatggg gaaagaggct ttaatacccc    1320
   tcaaaagggg aagattgata attagttatt caccgcatag aatcaggcca ggtcaactct    1380
   ctccaagaag aaaagcaatc tggatattgc actttggggt cccttttcat tttggcatta    1440
   caggcctcat gaggatgtag tctttctgta tttctttctt tctttttaaa ttgttattta    1500
   tttatttatt ttaaccaggt caaagcatta catactgcag aagagaaagc aacttcagct    1560
   tctcttaaac ggtgtcattt tcatgacagc tcgtaaattt tggcctccta tgggtccagg    1620
   atctgcgcgc atccgcccgg ggacttgttg gtggatccat ccctcatggc ggagcagcaa    1680
   ggggatcctt tagaaaaagc aatgggcgaa gtaactgaaa gagcgacgca gaaagcaaca    1740
   gccagaaacg gcggggacgc gagcggccca gacaggaagg gaggcggtgg cgcagctctg    1800
   gtgcgcagcg cgccgcagcg acggaacttc tgcaaaagct gcctgcccgc gcgttatcag    1860
   cggcgcgcag gcctgtggtt ttctcgctct cgcaaccctg ctttaactgc cggtttattt    1920
   ttcgacaaac aggatgcctc catctgaggc tgtcagcatc ccaggctctt tgaaagaaaa    1980
   gaagtagagc ggccccaccc tagaggcaag gacggggtct gtgtcaagag gcttcccaga    2040
   gaagtgaaaa ctctgcaggt gcagccgctg ggagagcatc aagaagggca gggtggaggg    2100
   gcagggggcg aagggagggg gtgaagcccg caccctaccc ccacatgaaa ctgattccac    2160
   taccccatct ctgcaagcgt ccagaggcag agaggccaac atttcgggga cagcttggag    2220
   gcgggagatt taggcagggc tccttaaact tttatgtgca tgaaaatcag gccaatcacg    2280
   gggctcttga gcaaatgggg acgatgattc agcaggtctg ggctgaggcc tcagattctg    2340
   cacttctaac aagttcccag gtggtagtga tgctgccagt ccaaagacca cactgtgaat    2400
   agcaaggcgt cgtcgggaca gtctgcttta gagtaaaacc taatagatgt gcaaatattt    2460
   aaaccaagct gtatttattc ctgaggccct ggtttaactg gagccacctt gctaatggaa    2520
   aatggaaaat gaaattcctt taggttccaa agggaaggaa agtttgaaat ggttaaggta    2580
   tttaaaaaat cctagttggg agtggccttg gggattcggg agagttgaag ttaggttctg    2640
   agccaagaga tgggggagga agggtgggat gtggcaaata aatataaaca cacacacaca    2700
   aaccccagcc caaccacagg ttctttccta tcaatgaaac atttgcatct tgcaggtaga 2760
   actcacacgt gtccacattt ttcagaagtt gaatggtttt gtgtaatggt acagaatttg 2820
   gagcccatat ttgaatattc tagatccaat gttatctcag actggcttta ggatcttgtg 2880
   gcctacattt aaaaatgcat gtttctgagt catgtttaat tagtagtagt atctgagaac 2940
   tgagagacta gactaagaaa tccatggtat atgaataaaa tctccagaca gcagctagaa 3000
   catagaggtc cctccagagg cagtgccagt tgtcagctgt aaaaataggt tctgctcagt 3060
   tttgcttagg cctgaagttt cctgaagatt tcatttagac ttcatcaaaa ccagtcaaag 3120
   acttcctgtt acagaaaggt tcttaaagta tggctgggac tacagggcta ttaagatgca 3180
   gattcttggg cctctctgcc tcactcatca tggtttctca gggtgggtgc cgggaacctg 3240
   cattttaaca aacgctttga ggattcttat gtactgccaa gtttaggagt ccctggtctg 3300
   caggtttaag ttcaaccttc tcactgcagc attcaaagac ctggcttcaa ttttgctgtt 3360
   cacaggcgag cctcagtgac ttcctcactg ccctctgagc actcctggag ccacctggct 3420
   gcctcattca tttcttctcc tctgtctttc cccagccctg gaataaaatg acttccatct 3480
   tcctctccga actctcttca gatcatggct tcccactcag caagcagtta ctttcctggt 3540
   ctgccactgc tgtggtatta atgataggcc actcgattct cattttgttg actgaatatt 3600
   tctcatgctt aggctttgcc tccctgacca gacagtaagt tctctgaagc catttcatgc 3660
   tatataatct ttatgttcaa gactactttc taacgcagtg tcatgtccac atggatcatt 3720
   cagtaaattt accgatggct tatctatcac caacatgttt tacagagtgt tattaagcag 3780
   ggacactctc tatgggtcta ggatcagaag aagctgggaa gagaaatggg gtagtggtga 3840
   gagtgtcatg agaatctggt tatacagtca tcaaagacac agaggaagtg ggaaaaaggg 3900
   agtgt                                                             3905
<210> 2
   <211> 5338
   <212> DNA
   <213> Homo Sapiens
<400> 2
   ggcaacctct tttgccatca ctttttgcat aaaatatgca agatgcttcc attccatcag      60
   cactgaaacc aaagaccaaa agagagaaag gtgttggacc gaaaaggagg cggcgcaacg     120
   gccacccctt ccagcacctc ggctttgtcc ttcccgggga aggcggccac atccctaccc     180
   gccttgctcc tgaacgtagt aaacaatctc acaaacaacc accgctgccc acgctctcca     240
   tccgtcctcc cggccttatc agacctccgt tctcccgcac tcttcgggca gggtccctaa     300
   taagctcagg ctgaaagaac gtttgccacc tcccccaccc tcgttgaaag aaaaggaaga     360
   aaaacagcag cagcgagaaa cctccggggc gactcctccc ccgcccccaa gcaccagcgc     420
   acagcatccc cctctgtctt tgttgtggtt ctccgttgct tcgggccacg ccgttcagcc     480
   aagcaacccg gacctgagag tgcacagcca ggactagctt agggggcgag gggttggtct     540
   ttgggaaacc aagcgctcag gacagaggtg gaaagtgggt cccgggagcc agaaaagaga     600
   gagaagggca gacggctggg tggcaaatac aaaaatagaa ataatttagg gggatgcccg     660
   ccaggctttt gcgcctgctc cttctccccc aattcggagc aggttccctt cggcctcccg     720
   cgccccgggg cgccccctgg cggcagcggc agcagcgggc aacgcgcgga gggctcaggg     780
   ggcgcacagg ggactcccgg gcacactcag agaggcgggc gcggccccct ggcggtggcg     840
   acgtagttat ctggtgagcg gagcctcgtc cctctggtcc ggcgggctca cggccgtctt     900
   actaagcacc gcggccgagt agggcaggaa gccgctctcg gaacgcggcg ccgccgcgct     960
   gcagccgaag tccgagcctc ccccggcccc ggcgcccccg ccgccgccgc caccaccacc    1020
   accgccgccg ccaccgccac cccgggaacc cagggctgcg gcagctgctg ccgcggctgc    1080
   cgccgccgac tgactgctgt ggcaactgag gcacgagcag ggtgcagagc cgccgctggg    1140
   gggcgcgccg gccgccgccg ccgaggaggc cgcagccgct gcggctgccg cggctgccgc    1200
   ggcagaggcc gcgctgttga gccccgcggc ggccgcggga gcctggtaga gaccagggtg    1260
   gcggaagcta cacagcagct ccggccgaga gtaggggtgc gagagggcgc ggaaggtgtc    1320
   cagtggccgg atggaagtag cgaagggcga cgaagccgcg gccgccgcgc ctgaggctgc    1380
   agccgcggcc gccgccgccg tgacgcccac gtgcgggtag tagtgcagcg gcacgtgcga    1440
   gtggaagggg tagggcaggc ttccggtggc ggccgcgtgc gtcatcatgt aggtgtagaa    1500
   gctggggtcg gctgggtgcg gccaggacat ggccaggcgc tgccgcttgt ccttcatgcg    1560
   ccggttctgg aaccacacct gcggggagag acgcgccgca gcctgggtta gggagcgccc    1620
   cgtgttccca gctcctgtcc caggacctct gccccttccg gacctctgaa tggcttggtc    1680
   tacttctctc cgaccaagcc caaccccgag taccctgtgg tctcccagct gggaaagtgt    1740
   ggacggcagt gtgtggaccg ccgtgggcac accgtcctca acgaagaggg tcctctcccc    1800
   cgcgtccggc tgctgctgct cctcaggctt ttattccctt acttcttgct gcactttttt    1860
   gtcccaatcc aacctttcct ctccctcccg ccacccacca gtgccggtct cgctgagcac    1920
   ccgtctctca atcccaggat ttgtacgggg attctgggca gcctttgaag agaggctgcc    1980
   gctcagcttt tctgagaggt cgccgcgcga agtcttgagc cctccgaact gcaaggacct    2040
   gcccctaggg gcacgggtcc gattttgata ttgaagggat gattttgttg gaatcgtttg    2100
   ccttaaatga gtgggtagag caatgtctcc ataaacgggg aaggggacgt ttccacccct    2160
   cccaacacta tctaataaag acatcattcg tcacaactct aaattaaaga aagcccgatc    2220
   aaaccagagg gagacttcca cactcctccc ctaccccgtg gagatttttt cttttttctg    2280
   cagtgtcgaa cgctccatga agggctcacc aaatcgccta acctcccggc tatctctccc    2340
   agacgtataa taaaattaat aacctaaagt tatatataaa taaggacaat ttcgttgcat    2400
   ttttccccgc aggaggttgc ccttttttcg ttgcccaaga ggaaaatgtt caggaaacta    2460
   ctgtctcaaa ccaatcgatt ttaaagatac agtatccttt tctccgtgta acgatttatg    2520
   cggaaaataa atctccaagc tcaagagcaa atgaaaagtt tcacctctgg ttcctgcttg    2580
   aggaacaaag accaactggg cttgccgcct aggggaaagt ggggccgtgg gtatgggcga    2640
   gggggcatct ggccaggcgt tgggcacaat ggagcagggg cgagtgcttt cagcattgga    2700
   gtcaccattc gggggccttc ttagatccgt caggccggac aaccgttcgg attcggtggc    2760
   cgggaaataa ataagccaat tcctttggtg actacccccc gcggatttcc agacccttag    2820
   ctaaatctag ccacccagaa aggggaaagg ggaaaaagaa acaatcaacc cagatgcccc    2880
   cggggaggcc agagcaggca tgcactggaa ttgatacctt gatggtggtt tcgggcaggt    2940
   tgagtgccgc ggccagctcg caccggcggg gccgcgacac atagttctcc cggtagaact    3000
   ccttctccag gcgcgcgatc tgctcgcggg tgaacgccgt acggtagcgc cgcacttgat    3060
   ccgcgccaga gccggagcca cccagcgccg cgctcccgcc gctgcctccg ctgcctccat    3120
   gcaggcttcc gaggcctgag cccgacgccg acgtcgtggt gccggcagcc gagccgctct    3180
   ctgcgtaccc tggcaaacaa acgaccaaca gcgcatgagt ggctgtagga ccaacagccc    3240
   ggcgctggcg ctgcgcgcgg atcggggaag ccccgtcagg aaggagagtc gctgccggaa    3300
   ttgatggggt ctgtcatgct tacaaattgc tgccgttaat ggaatcaata aagtttgggg    3360
   agcccttcat aagcaaataa tagaaacgga attaggagat ttctttttta ataattagaa    3420
   attttcaacc aaggaggaaa agtggccgag ggaaaatgcc tacctctggg cgcagtttgg    3480
   gaagctctgg gtttcccatg gtctggagac cgcagggcag ctttttatac ggcctctaac    3540
   ctttatctga gtcttcgggg tttcaaatat tttaagagtt ccaacacagc agtagctcaa    3600
   acccaagcca atagggggtg aaatatactt ttcaactctt tctcccgctg acctaaacag    3660
   agacgccggc gaggcttcct ccgacttacc cagaagaatg ccccctccag ccctgagggc    3720
   acaggggcag gggagttcag agtagttgcc cagggtacct ttcccaaaag caactcggct    3780
   gctgactagg ggtctaccgg ctcgctcggg ctcttagaca cgagcgcaga aacattttct    3840
   cggactcagg agcgagggcg ggtgggcctg gtgttccagg ctccgcaggc ctgagcctgg    3900
   cgggaaagct caaggagcag aactggaagg cacggtccca gacatgcgtc ccgcccccgc    3960
   ccgtgctagc tcggtgtagc ttgcctgtgg agggtctgag aggggaaaag gcaccgggaa    4020
   aggctggcgg gggccgcgga ggagcaaaga ggatgggact ggagagcgcg gtgcggccgg    4080
   cgcggttacc tttgccattg ttttccttaa gctgagcggc gccgaggccc ccgggggagc    4140
   gaagcgcgga gcagcccacc tccacgtcgc tgctcatgtc ggcctcagcg gccgcctctg    4200
   aataatggcc cggcttcttg cggctctcgg cggcggagga gatttcggag gagacggtgc    4260
   tttcgctgcc cgtgtgctgc aggttgaaca aagtgtctat ttcgaatttg cccttggcgg    4320
   ggagttctcc cagagcgctg tgcagggggg cagacggcag gcgcgggctt aggcgagccg    4380
   ggtgctgcga attttccagg gcctcgagca cagcattgcc agccgagttg gacaaattgg    4440
   agaatctctt gcccgccgta gggctgtgca gccctctctc catcagaatc atctcttttc    4500
   ttattctttc catcatctca gctttcttaa aaatgtcaca gtggccctgc tgtcccgtcc    4560
   taatgatagg ctgcgcctag ggctaattct cattcagccc cagcgaacgc ctctaaatat    4620
   tattatcgct ttcggaggga ggcggaaaaa ttgtgggatg ccagagcgag ggaatgactg    4680
   gtcaaaatga cccatacatc cttccgatcc cgagatgtca ttcatcaaaa agtagcgccc    4740
   gctcgtcatt aaggtacgaa tgacgctgtt cgaataatca tttattgtaa caggtttata    4800
   agcaaataaa tacaccctcc tgtcagtggg taatgaaggc agcttcagag cagacaaata    4860
   gatccaggta ggaggcgaga agagacaagt gaggaggaag gctccggtcc tttgcccgct    4920
   ccgagccagt tctgctcgcc cgggggtttg gcctcggggg tgaaattgac agtctgatta    4980
   atagagcgcg ccgcggcaca tttccccctt catttagggg catcattacg ctctcagttt    5040
   gctaggttgc cccttaggtc ctaatcatgc agcgccttct tcatttttcc ttggacacag    5100
   atacgtatta aataatagat aatggtttga ctttccagag taggtcctcg ggaaggtttg    5160
   gttttttttt tttttttttt tttttttttt accatttaca agctgggggc ataagtgggg    5220
   ggagataagg gggaggatgc aggaagagtt gcgtaaggag aagaccttgc gcctctccta    5280
   tctggattac ttatctttcg gatttcggaa ccaaatatgt attttattta acaaatag      5338
<210> 3
   <211> 5332
   <212> DNA
   <213> Homo Sapiens
<400> 3
   ctagtttcaa accctgcacc cgcactctcc agacaggaag tccctctccc tcgaccaggg 60
   attgagggag aagagattca gaatgcatct ccttgcccta ggcctttcgg gggggacatg 120
   gccttagaca gtcaccccga aattgggctc aggtcctcac agtaagtcga gtgtcccccc 180
   acatccccaa ccccggccct ttcactaaag tttaaaagat tcttttgttc ctcaggcttc 240
   tcccgcttgg ttgcttccat tattccccaa ctgcggaaac accagccacc atatgtccgc 300
   cggagaataa gcgtgagtgt cgcggtggac agttgccgcc aaaggtgtcc agacggatgg 360
   cattctcaaa gtagggatct tagcatcctc tccatacatc atcacccaag aatggtagag 420
   gaaagagtag gagtggggtt acagatagga gagaaaggag gttgaggcca caaaattggc 480
   atttctctaa ttatcaaccc cctcctccac catcctgcca ccatttgtta tggcaaagtt 540
   ctctttctgt gccccttccc taataaagaa atcctcagtc agcctccacc tactcctttt 600
   gatttgcagg aagccaaact gccagcagat tgagctttct aagggatacc tttgggatat 660
   gttggaattc tgatttgttc aagatgatga cggtcatcag tacaaatcag aaacaatgcc 720
   actcttcagg gtgtcatttg taagtgcaat aacacggatt catttgcaaa acccagctaa 780
   actgcgtctg gataagtgac tagatccaaa cgacaaattt cttccatcac tcagctcctc 840
   ccaagctctg cgggggcagc acagaagact gctttagggt gaaactaagg ctcagctcac 900
   catttgtgcc agcttgaggg accctggaaa tgttgactct gagaaaaaga caaaacaaaa 960
   caaaacaaaa caaaacccaa tcacaaaaca tatcctactg tctacaaagc aaacagaaac 1020
   acaaagttgt ttccaaaatc accttttcag caaataagta tgtgatattc tgagcaggag 1080
   caggagggag agaaagactg ccacttaaaa aaacatgaaa gcattaagta ataatttaaa 1140
   acacggctgt ttagaaaaat atttatattt attgcagctg ctcaattggc ctcgttaaag 1200
   tcggcaagca tttaaattgt gttacaatct catttaaatc ccgttccgtt ccagcaggct 1260
   gaagagcttg aatagaccaa tcacttcata atgatgatga atgagaaatt aattcagata 1320
   caagcaagac aatttaggcc ttcatctgtt taaatagcct tccaatatta ttcgccatcg 1380
   cgggtcacag attgaatcaa ttgtccaatc ttgtgaaagt catatccttg cgtcttcatc 1440
   gagattattt atagcgcagt gagcgctgct gaaaggtata cgctgttaac agggacaatt 1500
   acttaaaggg aagcgtttgt aaaatggaaa acaaatgccg gggttggtaa caaatgggat 1560
   caaaagcagc cattccaatc tggctccccg agggaaagga gcgggcgtgg ccctgtagga 1620
   ttaggggcgc ttctgacctc cccaagaccc aggtgaggcc gagggtcccc ggcgcgccag 1680
   cctgcgccgt ggctgtggct gcggtggcga ctcgggccgg gcctcgctct cgccggcttc 1740
   aggttcccgc ctccctcgcg caggcagcgg gcgcgtgtgg cccgggctgg gcaagccgag 1800
   gaacagcgag cccccggacg ctgactgcag gacgtcccag tttgtgcccg ggtctccgtc 1860
   cctccccgta cggggctcgt acccccgggc ctgggtctga cccacagggc gctgaggcct 1920
   ttgtagctga agtcggaagg cctcgttgcg agcgcggcac aggttgctgg tagcttctgg 1980
   actctggagg cttggccttc cttctaagcc gatggcgggg aaagaacctc gtttccacag 2040
   cttccccgac ccccgccgct tgccatttgg ggacgggaag cgcgcccggg tcgcttcacg 2100
   tccctctggg ccggagccct ttccatggct ggctcctctg gggcccttgg ggcctgtgag 2160
   cagcgtctac ttccctcaga gaagaatcct ttccttcccc catcgaagtg tccctttctg 2220
   tatcctgaaa taacccctcc tgggtgaggc cagttcccct ctgtcgccct cctcccgcag 2280
   gcgtccggga gcctcgtgag gaccccgtgc agttgagtcc aggcgacagg tgcctcccca 2340
   ggtgtctact tgccctccct caacctgttt agggaaagac cagaacgatg tgcgcgggga 2400
   ggtggttttg tttctcccga aactggcgct cttgggtaaa accgcgtgcc gcgtctacgc 2460
   gggctctgtt agtgtgcgtg tcccagaaga gtgggccttc taggcggcac tctttggaga 2520
   agtaagtgag gtgaactcag aacagagaag cgggaacgat cgtttgatgt gccgagccgg 2580
   aaaaagagag gagagggaga ggctcccagc gagcgcggag acagaggagc ccacgcggca 2640
   cagcacgagc cctactcttg cccgcgtaga ggtgtgtgtg tgtgtgtgtg tgtgtgtgtg 2700
   tgtgtgtgtg tgtgtgtgtt ttcctctgta tttctgtgtt tgaaacaaaa gcccaactaa 2760
   cggattcctg gtgctctgag gactcactcc cgggagcgac tttgatgtat tgctgtccaa 2820
   ttagtgcctt taattggtgt cctcggagac aggcaggccc ggagctggga cagagcctcc 2880
   tctcgcctct cctgcacact ggaaaggtaa aatttataac acactgtggc caccccttgg 2940
   gagagcgaaa gagagtggga ggcagggaag aaagggagag atgcagctgt ttgcagagga 3000
   cggagcctcc agccccgggg gtcgtgggga agggcccaga ccaccgggtt cgactcagtt 3060
   ttctgtgtaa gagggttcgg gccggctgag tcgctgagga ttatgaattt gctcgcagaa 3120
   aggcctaggg agctctcatt gtctgtacaa acccacccca tgtggtctac catctcgtta 3180
   ataatggctg tttgttaaca aggatgcgga ggttaaataa tccgggtgga agattaaaca 3240
   cgtctcatca aggcgcttgg aagccgcgcg gtgcagagac ctgcgctgga gccgcctggc 3300
   tcactggagc ctggagggag atgggagcac agtctgcaag gagccccggg gtagaagagg 3360
   aggcaaggtc tagtctccct cttcagggtg tcctgctatg ccaggacagg tctaactctt 3420
   ccacatgtca aacagaaaat ccacatagaa gtcagcccca atgaggtgtg gaggtcaaac 3480
   cctcggagta gttggctgga gggcagacat ggagcatctg ggtctgaacc caaaggcccc 3540
   cttttgagga ggggtggcca tcccctgcct cccagtcctt acatgtcatg catctggcaa 3600
   tttgtgccta catgggatta gcattggatt agaagacaga agatgtggtt tctagtctgg 3660
   gttctgcccc taaatcactg tagaattttt attacattat tctcccagct tcatttttct 3720
   actgtgtaag agaaaggaga tggggcttga tgtagatgat gtacagcatt tcttcttttg 3780
   tccagcttcc caccagagtt agggaactga acaccttata agatgctctg tctcccctaa    3840
   accttgcagg atgcagtcaa aaagaatttt atttagcaaa tatctgaggg cctactctgc    3900
   accagtcatg gtgcagtatg tattttttaa ctgagagcag aatatcagtt catattcccc    3960
   tgttattctc aaatataatg ttttataaca aaagatgaaa gagaaagaac ttcaaagaga    4020
   atagaatagg atgggtatgt atgtacaggg tagagaaact atggaggtaa aggtaaaggc    4080
   attgacaaaa gctgtgaaga acttgttgaa ctggcaagga agtcagtgga taaatactgc    4140
   agaagtagct ccttaacaga gatactatgt aggagagtag agagaaggtc ccctcctgaa    4200
   tcctggtgat ttggcagaga acaaaagggt aagggttgca gaatttgact aggaggccca    4260
   agttgtatgc atctaatatt gccaattttt ggtttatata ttctttcagt agttggaatt    4320
   tctctctctc tctctctctc tctttttctt tttgagatgg ggtcttgctc tgttgctcag    4380
   gctggagtgc agtggcccca tcaaggctca ctgcagcctt aaactcccag gctcaagtga    4440
   tcctctcatc tcagtctccc gagtagcaag ggccacaggc atatgccacc atgcctggat    4500
   aatttttttt tttggttgag gggtacagat ggggtcttgc tgtgttaccc aggctggtct    4560
   caagcttcta ggctcaatga accctcccac cctggtgtcc caaagtgttg agattacagg    4620
   aatgagccac tgtgcccggc ctaggaagtt tactctttag ctaattttga aacattactt    4680
   tctgaatcca ccaatcagga aactcggctg ctaggctggg actcttcaag cttctgggtg    4740
   atgggcattt agcctcaagg aaggcaatga tgcttaaatc actaaaccat gtctcagttt    4800
   ctgtgagtca ggaatctagt gagcttagct gtgtgtccct ggctccaggc tattccactg    4860
   gggttgtggt cttatctaaa gactcagctg ggtgaggata tgttccgggc tcactcatgt    4920
   ggttgtttgg caggattcag ttccttgctg gtgggctatt tggctgaagg cctcagttcc    4980
   ttcttggtgg ttggccagag gtttctctgt tccttaccat atgagcctct ctatagggta    5040
   gcttataaag tggcagctgt ctttcctcag actgagtaag caagagtgag agtggagggg    5100
   ggagaaagca cccaagacag aagctacact cttttggtca tctaatcttg gaagtgactt    5160
   tccatcacta tgccatatta tatttgttag aagtgagtca ctagatccag cccacactaa    5220
   aggaaagggg attacagaag gcatcaaatc caggaggcag ggatcactgg ggactgtctt    5280
   agaggctgct gcccagaccc cttttatata acagctctga ggcttatatg gt            5332
<210> 4
   <211> 4215
   <212> DNA
   <213> Homo Sapiens
<400> 4
   ggtgcccgac caagcgcagc ccgcagtctg ggcactgcca actgactcca actcctttta     60
   tggtgagagg atggattctt cgttatttcc ccgcccaatc tggtacccac ccacccaccc    120
   acctaccacg tccgctgggc gcacccaagt ctaaccccgg ggcgcacgcg ctagcgcaga    180
   caccgtattt cttctccttt ctcggccaac cctaggtaga atcctaaaac aactgccctc    240
   tcttccacga tctagatgtt gcggcccgcg gacaggaggt tcaagaaata gtacactccg    300
   agcggcaggc agcgaggcgg aaacggtcgc cggtttcagt ggtggcccca ctggaagccg    360
   agttcaggag cggctaagcg gtcgccgggg aaagcaccgg ggcttcccag ggtcccctcc    420
   gagttcccac tccgcacctc cgagggcgtg aaaaccacgg gagccgcccc accccgcgcg    480
   cccagccccg ccccagccca gacaccgccc cccgccagtc ttccctgcgg cgcccaggga    540
   ggacgcgctc cgcccccttc caatccggcc aatgggcgcc cgggcagcgc gcggtttgcc    600
   tccgcctccg ccagggaaac ttggaggagg agaaaagttt gtacagaggg tggaaaggcg    660
   agagcggagc tccaagcccg gcagcccgag aggaagatga acagccccag gccagagcct    720
   ctggcagagt ggaccccgag ccgcccccag gtagccagga gcggcctcag cggcagccgc    780
   aaactccagt agccgcccgt gctgcccgtg gctggggcgg agggcagcca gagctgggga    840
   ccaaggctcc gcgccacctg cgcgcacagc ctcacacctg aacgctgtcc tcccgcagac    900
   gagaccggcg ggcactgcaa agctgggact cgtctttgaa ggaaaaaaaa tagcgagtaa    960
   gaaatccagc accattcttc actgacccat cccgctgcac ctcttgtttc ccaagttttt   1020
   gaaagctggc aactctgacc tcggtgtcca aaaatcgaca gccactgaga ccggctttga   1080
   gaagccgaag atttggcagt ttccagactg agcaggacaa ggtgaaagca ggttggaggc   1140
   gggtccagga catctgaggg ctgaccctgg gggctcgtga ggctgccacc gctgctgccg   1200
   ctacaggtga gatggcgttg ggctgacgtt ggggtcaacg ggtagagaac gcagggatgc   1260
   ggccctcgcc gaagagagcc aagaagggaa gagcgcgctc tccaaattgc ttttgtaact   1320
   tgttttcagt gagcatttta ttgattcaga atctatcgag aatagcacta gcgagctact   1380
   tttcccttga gatgggtctt attcatcttg gcaatggagt gagttggatt gtggggagga   1440
   agaggaatgg gaaaatcagt ttataaatat taatgtcagc aagagtgtgc tgttggcagg   1500
   acgtatcgcg agcctggaga ttttggtggc cgcagttggt aagtggctac aatccagaaa   1560
   gtaggatcga gttgctcccc ttgtcttatc agtgtatcgt ttctcgggcg cgggtctaac   1620
   accttacaag tggtaatttc cgctcacggc agctttgtct ctcttctacc atccccagac   1680
   ccagccttgc actccaaggc tgcgcaccgc cagccactat catgtccact cccggggtca   1740
   attcgtccgc ctccttgagc cccgaccggc tgaacagccc agtgaccatc ccggcggtga    1800
   tgttcatctt cggggtggtg ggcaacctgg tggccatcgt ggtgctgtgc aagtcgcgca    1860
   aggagcagaa ggagacgacc ttctacacgc tggtatgtgg gctggctgtc accgacctgt    1920
   tgggcacttt gttggtgagc ccggtgacca tcgccacgta catgaagggc caatggcccg    1980
   ggggccagcc gctgtgcgag tacagcacct tcattctgct cttcttcagc ctgtccggcc    2040
   tcagcatcat ctgcgccatg agtgtcgagc gctacctggc catcaaccat gcctatttct    2100
   acagccacta cgtggacaag cgattggcgg gcctcacgct ctttgcagtc tatgcgtcca    2160
   acgtgctctt ttgcgcgctg cccaacatgg gtctcggtag ctcgcggctg cagtacccag    2220
   acacctggtg cttcatcgac tggaccacca acgtgacggc gcacgccgcc tactcctaca    2280
   tgtacgcggg cttcagctcc ttcctcattc tcgccaccgt cctctgcaac gtgcttgtgt    2340
   gcggcgcgct gctccgcatg caccgccagt tcatgcgccg cacctcgctg ggcaccgagc    2400
   agcaccacgc ggccgcggcc gcctcggttg cctcccgggg ccaccccgct gcctccccag    2460
   ccttgccgcg cctcagcgac tttcggcgcc gccggagctt ccgccgcatc gcgggcgccg    2520
   agatccagat ggtcatctta ctcattgcca cctccctggt ggtgctcatc tgctccatcc    2580
   cgctcgtggt gagtgaccgg ggctggggcc ctactcggcc tttttctcgc atccacctcc    2640
   cgcgtccatt ccccgctccc tgctttccct ctgagtcctt ggcagtgaac gtgtcgcctt    2700
   taggtcgggg ctgggattcc cacactgttt ctcagagcag gcccaaccct ctttgaagtc    2760
   ccaaccctaa cgagatttag caggtgcttt gcccctacat cccccagttt atgttcccgg    2820
   aagcctgggt ttctttctcc accgagacag cccttacccc ttgctgcctg acactggccg     2880
   agtcttccaa gaaaaccccc cgccccctct gttagacgtg gaggggagcc tgctgtagtg     2940
   tgacttagcc cattcctccg tactgtgaac tgtgaactgc aaaacctgaa atgtcaggga    3000
   tggtgatctg ttcgttgtaa accattttta aggaaagtgg ggtttttggt gttttgatgt    3060
   atgcaaggtt tcttgaattt cttcccccgt ggattgcgta gaattttaaa gctagaagag    3120
   atatcaagag gtgttttagt ctaactccct cacttcactg agggagtgaa tgatacgctt    3180
   aaaataatcc agccgaacct acacagtcaa gctaggctcc ctgctcttta ccatggtgtg    3240
   ccctgggagg gcagcttaaa gcacacaaat tctaattcat atacagggca gggtccagac    3300
   tcagtttctt tcattctgtc tagaccagga aagcaaagtt tctcagggtg actaattatg    3360
   gacaggctga tatacatagg gtatgggatt tgaattctcg acaactgtgc gaggtacagt    3420
   accattttag agaaaccgag gtttagagat ggctgtcttt gtttcttcaa gccaaagctc    3480
   tacagcttgt aatggacctt aggccgtctg aggccagacc cagctcatgt gttattacat    3540
   cacgtctccc tcgtagaaca tttctcattt tggcgtttgt aaagcgtggg ttggagtttt    3600
   ggtacacttc atttttagtt catcttttaa gaattgcctt tggattaact tttaaaatat    3660
   gataaacaga aaggctcaaa ccctacctag ttttctactt tgcctgtggc taaaatggaa    3720
   tgcaccagaa gcagatgggc agaataatgc aatactttat ctagagtttc tcttgaaagc    3780
   aactagttat gagttcttcc atcttccaaa acactgcatt cctggaaatt ctaaactacc    3840
   aactcaaaaa gagataactg ctttttctta tgcttgttgg gaacaagaga atataaaatg    3900
   taatgggcta ttattccttc cttttcctta ttgatactgt ccagggatct ttggtgtaaa    3960
   aatatatctt tgataaaagt gactgtaaat ggaaggaact tagagggggc tcagagccag    4020
   aatacagtag gttcttacca atttgtataa acagaagtaa atataatagg ccataagagc    4080
   ctttaagcca ttccaggacc caatataatt tacagagtta agtcacactt gagccacata    4140
   aatatatttt actgtctgta atggcaacct agaataaagt gagattttgg aagaaacaac    4200
   actgctcctg atggg                                                     4215
<210> 5
   <211> 5473
   <212> DNA
   <213> Homo Sapiens
<400> 5
   gtaatgttaa ttagcttcat cttaaagaaa aaccaaacac cagagcaaat aatgactcct     60
   ttgcggagaa ggagttgtaa gggttgggga gaggtaatta gtgtcataaa agcctcacta    120
   caaagaagtt tagaatttct ctgttctgct gtgcagcaga gtgaaacaca gtagcagaat    180
   aattgtgcac cattatcatg ataatctaat ttgcttcttt agaaaaagcc actgtttgca    240
   gttaggatag taggaaaaca tctagaaaat aactaaaatg taaataaaag tatgaaacaa    300
   caataaagtt cacttcttaa gagaaaaaaa gaagagcact cagcgaaagg agaatgaagt    360
   atatataaag caagtaactc ttaggtactt tgttctcctg gatgttacct gcttaaactt    420
   gacaactttt acaggcagta tgacagattt tttaaaattc aaataaagct tgcttttgta    480
   ttccactttc aataataaag catattgatt gactcacaaa ttaaagtttt taaaacccca    540
   ttaaagtaaa aatactgctt cagagctttc attggtatgt ttttgaaagc aagtgtcata    600
   gtcaatgtaa atcttcaaaa atttctctgc tatttagtac aacagagggt ggattttgtg    660
   ccagggtggc atcctttgtg taattcacat ggcctgaggc ttcttgcctt tttttcccct    720
   agaagagttg gaattaagat actaataatc ataactgtaa taattatcat catgttttta    780
   aggggttgct gagcctctgg agatctcagt gcacagagcg aagcaggaga ggcttaaaaa 840
   ggaaggctct gctgctgatg ctgtgatgac caaggcagcc tattattact tttcctcttc 900
   aacgtgactg ctctgttttt ttttttcctg atcctgttac aagatgtggg gaagcccctt 960
   aagaattgtg catagttcgc tacagatggt ggtctctgtg cccagagacc gaaagagtat 1020
   ctgttacagc gatactttgt aacagctgca gatgtgcggg cctaaaactg ctccatgtgt 1080
   tcaaaccaat aataactatc attttattct attttattat ctgagcggaa atacaatctg 1140
   aaacatttaa atttgttccc cctaaagcta gaaggatatg tgtatgtgaa tgtacacaat 1200
   ttgctccttt aaatgccccc aaagcctatt tttgctatta catttgcctc cacaaccaat 1260
   aaagaaaatc tgtttcagtc ctaacttcat tgggtatggc acgtgctgga gggcaggctt 1320
   tggaaatata aatactaata tatgtggttt gttttaaaat ctaatgtatt tagggtcagt 1380
   ttcgttgcct tggttaaatg aatgaacaaa gagtgaccaa aatgcgttta tttctccgct 1440
   atttcacaca cagtttgaga tcgaccgcta acagcagact gtaaatttct gcacttaaca 1500
   gttactaatt ttctttatag tctgcacatc tataaaccta attgggatat ccgcttgaca 1560
   aacctagacc ctgcagagca gactttcaag gaaatccaac tccagagaac aaaatactct 1620
   ttggaacttt taaggcatcc agagataaaa cgttttctgt gggatttttt tttaagcctt 1680
   ataaaagtag gcccattagg attaaaccac attaaagcca gtgcgaggca aggggggtgt 1740
   ggtttataga aactctggga gaaagcccag cacagctttc ttcttctggg aacatatgtt 1800
   aggatggaaa aaaaaaaaag gaggggagtg ctataggact aagatctact ttggaaaata 1860
   agacctctag taaagatatc tctttccttc tcatcttaca ccagacacta gaagcaccac 1920
   ctcccgagtg tgtcccccaa gcctctttcc aaccccaggc tctctctgct ccctgggccc 1980
   tcgacctcct ggcagctggg tacctgcact cgggcctccg acaggcccag tcgctggctc 2040
   agttcctctc gcatgaaggc gtcgggatag tgggtctcgt caaaaagcct ctccagctca 2100
   ttgagttgtt ccagggtgaa attggtccga cttcgcctct gcttgatttt ggtctggcct 2160
   tcgtcctcca tccctttcgc atcctctttg cgatctttca gctccgggga cactggaggg 2220
   ggcaccccag cggggccaca cgtgcatcca cacgaacaca cacacacacg cacacacaca 2280
   cggacgaaaa cagcacagca aatcctgtta ccagatttgt cgcaaaaaag aatagagaat 2340
   ccttccccct cgtggaatcc tggtccggct tctcaaccca cccggaggaa gaatatcccg 2400
   gagaagcgca aaggtcaagt ctgagcggcc gcctggggca aggtgggcag tcatccacca 2460
   cctccttcac aaacgtacct cgactcaccg cctcactatt caccccccag tcaccggcct 2520
   tccccagcaa acaccaaatg gtttccactc actttcgcac tcacttgctc cgcagcactc 2580
   acagaaatca aaggcgcagc gccaggcctc tcacagagca aagtctggac ataagggcgc 2640
   ggcaaggcca cccactggcc tctcacaccg gccattccca gaaggctggc tcgttctcag 2700
   gccagctctc accaccgctg gctctctgcc taccgcaaac ttgctggtct aatttaggaa 2760
   caattgggcc gaaaggtatc agcgagagca acagaccccg gtgttgtgcc gcacagggag 2820
   ccgcatccgc agacgcccct cgctgcccct gggctcgggc caaaccctgc ataaggtccc 2880
   ctggacagcc aggtaatctc cgtcccgcct gcccgaccgg ggtcgcacga gcacaggcgc 2940
   ccacgccatg ttggctgccc aaagggctcg ccgcccaagc cgggccagaa ggcaggaggc 3000
   ggaaaaccag cctccggtgg cgggcgaaag caaccgctct ttctgttctc tcttcgccct 3060
   ccctcgtgga aacgcagact cgaccctaaa cgcttaaccc acagagatca acaggttcaa 3120
   gcggaatatt cgcgatcctc ggtttctatt ggttgctcaa agccttttca tgcaaccagc 3180
   agctcggatg tttaataaaa tatgaatttt tctgctctgg gtttgcacac atccgttgcc 3240
   actttcctgt ccttctcctc cctttaaaaa caggagctct tctagtgtga gcatatacag 3300
   ccaccatata tatatccccc tggaaccttt atcctctgcg cccgaatcag agactccggg 3360
   ttttaagaag tcaaagctga gtttagatag tcgaggctgt ttgcagttct tcccagcgca 3420
   ggatccaggc cagcatgggt gttaaaaatc atttccggaa gagccggtct gcagagaaat 3480
   taatacttgc cctgagcttg gaaatcaaca atcccaggaa ctgcgcccgc cgggggttca 3540
   gacctgaacc tcgccgtcag gcgcgctggc gaacctgtag ttcgggcctg tagttgtgga 3600
   ctcatggccg ggaggctgca ctgggcacct tgacggtaac tccaccactt tacaaattta 3660
   ggaagacctg gtgctggttt ggaggaagaa gagtcgctag atctcggccc tgccgggcat 3720
   tctagctacg gtgatcttaa atattgccgt attttctagt tctcagagtt agttttgtgg 3780
   acatttgtcc tttctttagg aaggaacatt atagtaaaca ttatgggcca atttaatagc 3840
   aaagtatttt tactgatatt gtattgaaat ttaaatatga aaacacaccc ttcaaattct 3900
   gccaatgagg tgggaaggct ttgtctatta catgtcatct acacagtgga tgccactcaa 3960
   agttttgggg cttcgaggag atgtccaaat ttcaagttga tccatagact taataaaatc 4020
   tctatttttg tccccaaatt tccaattact gatctaaaca ggaacagacc gtgtcataaa 4080
   caacttaatt gcttgcttaa atatgtgatt ccacgcatgc cacatatttt cttaattgga 4140
   aaaatatggc agtacaccag ttactgcagg caccaagtgc caaatcaatt ttattgttga 4200
   actcctttga attggtatag aacgctctgc cattgagata aagacgtttc ctaacactga 4260
   gctagcacca aaggattttt gctctaagag aaccgagtac tgggtgattg aaaacactaa 4320
   aggaattgtg taaatataga tccccacctc ccccgccgcc cccccccccc gcccccaaca 4380
   caccaagaaa ccgaaacgcc tcgcccggga gaagcagcgt agcctggacc tcagctttcg 4440
   ttggcttcct tctaccttga aagagaatgg gaagttttgg cccctaagat ccaaagattc 4500
   aggggctctg cggagttcga ggggtcttgg cggccccaaa cacctaggcg accggagggt 4560
   taagctttcc acctctgccc cttctccctc ccggcaaact ctgcgctaga ggctagcttt     4620
   agtcccgccg tctggttcag caccccctcc tgcagcccgg ccccgcgaac ttccacgtcc     4680
   gcctgcgggc catccgggct gcgggcccat cctcccgctg gtgaaaaatg catttcggtg     4740
   gaatgggaac atctgggaag ggcgcgcaca aaccccacac gcttcccccc acgccacccc     4800
   cacaacacac taggacccca cccatgaccg agcataccac cggaccccca cccttccact     4860
   atcactctag ctgacaaagc tggggtccag ggccctctcc gtgtccctct cccctccccg     4920
   ctgggcctcg gagtcctctc ccgcccgaga ggagtccgga aggcgggaag gggaaccgct     4980
   gccgggggtc agtcaggtcg ttaccctccg tcagtcgcgg gctgcccggc tccctgcttc     5040
   tctcggcggc gcccatgtcc agctcccgga cgggagagcg ccctcctcca gctcctccgc     5100
   ctgctcctcc tcctcctaca cctcctccgc ctcctccgcc gccgcctccg cctcctccgc     5160
   cgccgcctcc gccggccgcc cggactgccg ggctgctgcg gtcgtcgcgg cccgcctcgg     5220
   tgcagccggt cggctccttg gccccgcgca gcggcccgct ctccagcacc tcccggtacg     5280
   tgatcgcctc cttcttctcc ttcactttct ggtcaaaaga cttggagacg aacgccgtaa     5340
   gttcttccat cgccgccgca cgtcagcccg gcgctcaacc tctgccagca gagccccgct     5400
   ctttttttcc ttcttctttt tttactgctc cagccccccc aataataaca catcaatggg     5460
   acaggaggtg ggg                                                        5473
<210> 6
   <211> 5328
   <212> DNA
   <213> Homo Sapiens
<400> 6
   ttgacaagag aaaaggtgtt gagggcaggt cgcgctgtct tatttacagc ccactgcttg      60
   ctgtagagtc tttgttagga ctggaaagct aatttagtgc tgatcaaaag caccacagag     120
   ttaaaggact ggtaatgact gctaatttca tttcagcctc atgcatcccc aactgctgta     180
   attaaagaca cgctatttaa atacaggtga agggtttttt ttttttaagt tgggttgggg      240
   gggggtgggt gggtggatta caattttatt tagatcttct actctacttg ggtgttatgt     300
   agtcccaaat tagattttat tattattatt atttagagcc gctccttgtc atttatacct     360
   ggctttgcag aatcggaagt gtgaaatgtg tcactcacat aaaagcccct gtttaaagaa     420
   agggtgggac gggggagtgc agaggagtaa ctatttaggg gtgatttaaa tatttaggtc     480
   tccttttttt ctccttcaaa ttaatagaaa ggccttcgat atagttaaaa gtatttgaaa     540
   ctgggggcaa ggttaacagg ccaaagggat cagtgtcagc tttttaagta cttctaaaaa     600
   gatcatgtga acagatggaa aaatatattt ttaattaaat aatttccccc acactatagc     660
   tgcagtgttg gctcagaaac cgtgtaattt ctcatccgat aggagtttct ttatgagaac     720
   atcaaaggat aaaaagctct gcattttcgt agttggttaa aattgataac caccccccca    780
   tgcgcgcgcg cgcacacaca catacacaca tatacacaca cacacaccaa cacacacaca     840
   cacacacaca cacacacaca cacacacaca tatccccaaa aaagaattta gcctttaatc     900
   tctttttatc cacgaagact aaagctctgt actcgacccc gcgagcggct ctcgccttcc     960
   ggggctggaa cccgactcag atgtgaagcg gcactgagcc gctgcgcagg ttcgtgccca    1020
   gcgccccctg ttggacacag taggagctgc gcagcgccag tcccagaaat cgctggaatc    1080
   ctgctctggg gttagagaaa actcttttat tgccctccaa aaaagaaacg ggatgcgaaa    1140
   acaaattttt atatccttga aaaataatat aatattaact caataaaaaa ctaaagaggc    1200
   gatactacaa ttttaattca ataaaaaata aagactgtcc tcctcacatc ccagccatcc    1260
   cccacccggt ttggttttgt aactcttttg aaaggttctg cgatggtaaa acaggtaaag    1320
   aaaagtgacg aaaaacaaag catttttctg tagccgtgga gaatcaagca aagtatactt    1380
   cctccgtttt gtccacattt caaaaaatta aacggaagat gctgctgctt gctgttttga    1440
   cgttgtcagc agcgtcttga atgccctcag aggtcctgca agtatcgtaa ctgagccttc    1500
   gagtaaacga aataacttgc ttttatgtgt ttatttccag agtataatta aaagaccagt    1560
   ctcttctttg agtgtttccc atcccctaga ccaatcctgg tcaaagtttt tccttcccag    1620
   atactggcta agaaacacat actggctaag aaacaggtgc ctggtatctt ttttctttca    1680
   tcttagccgt aggatttcgg ggaaggtgtg ccaatggtag ggaaggtttt ctttgaaatt    1740
   cagcatttcg ttgaccagga atccaaatta tcctagggaa gggggagtat ttttacactt    1800
   ccaaaaaact acctgtctac ataataaaga gctttgcctt ttttattatt attatttctt    1860
   cttcttcttt ttggtcagca agtgagggca aaagtagcct ttgaattatt tcagaaagag    1920
   aaactgtaaa aatggcacat cctctgaatt taaaacaata gggaaatgtg ggtttgctta    1980
   attgtttaaa atcaaataga aataattact gtgctaaatg aacaagatgt tttaagattt    2040
   acatcttcag ggttatggtt attgatttta actttaccct taagaagttt agagatcgcg    2100
   tttttaaagt ttgcttattg acggaaatcg gcttaatttt tcataaaaga atggcttttt    2160
   aaaatattga atactatggc tatgcaaggt ttatgatacc tttatgttta ttcttcccac    2220
   ctcccaaaat tagcgaattc gagataacat ttcaagggga attgctattt agtgcgatga    2280
   gagatgactt ctccatctgg ttccagaatt agatctcggc ttaggttttt tagtaagtag    2340
   gttcagatgc aatggaagga gagaagaatc aagccttttg ataaaatctc taccccgaat 2400
   tctatggaaa agacaggcaa gcgggattcc agccacagca cccccacctt ttgctctacc 2460
   aaaaggcttg tccccattac agaaataata tagtcggtgc tagttttgtg aacccgcgcc 2520
   aaccgataag gtaagaaacg gtgcgctgac agccgaggtc cgaaggctgc tcagtggttc 2580
   ccgcagcccg tggggtaggg aggccaaagc cagcaaaatg ttggaggcga cgccagaaag 2640
   agagtatatg cctgctattc ggaagtgatg tcgaacgtgc aatagcagag tcctggagat 2700
   ttccaccccg ttccgcaccc agggatccca gcctgtgggg tagcaggttg ttgggtgaag 2760
   gcaggaggga gaagccgcac ctggctgagg gcgggtgggc gtgccgggag cgagtgccca 2820
   actttctagg gcccatggag aaagcataac ccctagttga ggaaagagag ctaaacaagt 2880
   gggtggccca ggcctgggaa tgccacccag ctctcccgac gcagtgttat ttctgtggtc 2940
   ccagggggcc ccagcacctg cccgaatccc actccccaag gccgccagca ggaaggcagc 3000
   ccctccatct ttgagggctg aggagggggc agcgagtgac ttgattcctt agtagcaggg 3060
   aaaaccgcag cggctggcaa cgggccgcgt ttctccgcgg cggcaaagcg ccctttaggg 3120
   gcggggcggg catttttggt ctcctgtccc agcgtccagg cctgagcccg cgcgggttgg 3180
   ctgggcgccc tgctttgtaa gtaaccaatc ttgctggatt ctgaggacta tccaggcgga 3240
   tgggcccact tagcttaggc ccactgagac cctgccaact cctcagaccc ttttcccctt 3300
   catccctcac acctttcatg atgaacccag gaaagaaccc gaactctgaa aaacagtaca 3360
   tgtggtttga agtttgatag ttatttgggt gtgagggaca tgactctatt aataaagact 3420
   aagattcaag gctgagctct catttattaa atttgctctt tcactctgtt ttcaaactct 3480
   ggagatcttt cagtttcgtt gctccaaccc tgacacagac aaaggcgcca gggagaaagc 3540
   ccaggcgggc ttgaagcagt tgaggtatag actgatactc atctctccat cactcagacg 3600
   ggcagataag cactgccaga ctgggggtcc taatccaggg cccagcggct acgtgggttg 3660
   ctgcggggtg ttgaaaccac aagtacaact tgacaccatt gagcccacgg tgcttgtcaa 3720
   taatctgtga attaggtgca gagattgtca ctgtagatca taacagtttc tgaaggactc 3780
   attacagcct atcgtgtcaa tgatcttcaa attaaggagg ggaggtgggg cgagacgaaa 3840
   aaggagtggg gagagccagc aaaaagggaa gaagggtggg agggaagata aaagtcagag 3900
   ccccagctcc gaaggagcct ttaaagatta tcaggccagc gatctgagaa ttccattggg 3960
   atgggtttgg aaacccaact ccatctgagc caggactgga agaggggcgc tgggggcaag 4020
   cagaagtggg cccctgggaa ggccgacttc tcaggcattt cttttgaggc cggaatagct 4080
   tttgggagag ggttctagat gtcaatggga ggccttgagg atgccagccc gcccaggatg 4140
   ggtgtacact ttgatttaga gcactttcca gggcaagccg cagctgcaga tctttgaaaa 4200
   aagtggtaat agtttcgttt agggatgatg gtgattgtag gatgactttt tttttccaat 4260
   ggtttgcttt aacttctttt catgccacag gtgaaagctt tcttcctttt cctctgatag 4320
   tattcctgtc tgtccctcac atgttacgta gatctaattt ctttccccaa cttgtttttt 4380
   tgggtgccaa acacatcagc cctctgagat tttatcactt ttgtggtttg tattgagtcc 4440
   aactgtaatt ctggaaaggg aacttgtgga taacacagta gcagtatgtt ccataagatt 4500
   ccagaagaaa gaagactaaa ttccatttat gcggtgtggc aggggccagg ctaagggaaa 4560
   gaggttttcc ctgcttggta aattgtcctt gcagtgaaag ggtagcccag ccttcactgg 4620
   agtgtcattt aatactggct agagaaaggt taaggaaagt gtaggtttgt ttttcccaca 4680
   aaagttgtgg tcacaactaa acgaagtaat cacagttgga gaatttaaaa caactgtttt 4740
   ttctctaaaa tgagggtaat aaccctagcc actggaaaat tgggattcta aggaaaacct 4800
   tttcctttgg tataattaat tgcataagat agtgtggtca atcagagaca tatgtattta 4860
   gactcaagca atgatttaca ggtgtaaaag gtccagtggc tttttgggct acctggagtt 4920
   cctactgatc attttgcctt ttaaaaaatt ttcttctaat tttaaaaata aaagtcatag 4980
   atttaatgca actgaaaacc actgatggca gctcttcagg ttttcagttt atgtttttaa 5040
   agggaattta tatttaaaat aattaaatca tttaatggtc acactacatt ttctcctgga 5100
   caacagctgt agtgcaggga tagaatctta acttacatta aaattgcttg ataattcaga 5160
   aatggaaatg aaattgtgaa ttaatttctc cagaaaatat ttgagctaga atataagaag 5220
   gatgttcata tataatactg taagtaacat gatgattatt atatgcagct tgaaagacct 5280
   acacaaatga ttatatacta cttctgttgc tataatttaa agtaaggg 5328
<210> 7
   <211> 4324
   <212> DNA
   <213> Homo Sapiens
<400> 7
   gtcccccagt atcaccatcc taaaggcact tccacttcct ctatcatcag ggaaggagtg      60
   tgcagttctg atttagctcc tcagtggagt aaagggaatt tagaggaagg gggatttctg     120
   cagaaattgt cagttacaca agtaagttcc tcttggttcc ccgtttcatc aaaagtgaaa     180
   aactgttcac aggagtgccc atgtcccctg tttccctatg catcactaga gccaagaggg     240
   gactccgcca gcaaggcacc ctctgctctg tcccgcagat cacagtcccc gactttcacc     300
   ccattgtttg tgcaatgtgc agaactggag cccaaagcag cgggctcagg atcactctcc 360
   aggcccggcg ggcccataga ctccttcctt ggctgggaag aggggctttc gtgaaagcag 420
   agcttgacaa gccccagtac tgtcatggtc aagatcacca acactactac tgctgtgctc 480
   acaaatatga agaccacggc agaggaggag tcgaaagcct gaggagtggc agaggaagtc 540
   gtagaattaa acttggaaat cacgctccct gatggggtga tagtggcctt tgactcggct 600
   tgaagggaca tttgaagggt agacatcgtg ctctgtgatc cccatcgagg aatctcagga 660
   atagatgtta ctgaattgtc ttgttcaggg acaagtggtg tctctcccag cttctcgtcg 720
   accctgattg gccatgttct ctgcggcacg gggctggttg cagtggccgg cgggcgcctg 780
   gtgggcaccc cggtcccccc aagggtcggc tgtccttccc cactggtcac acaagagcgg 840
   ccgtccttcc ccagctcgaa gcccgtagca cattcgcagg caaagcctcc caagtcgtct 900
   aggcagttag ggagctctgc gcatttgcca gcacggaggt acctcccggg gcagggacac 960
   aacacatcgc ccgagagttt gtcccagcga gcgccgattt cgtccgcgat gcaagtaact 1020
   gagatcggga gctgtccccg gcagagcgca ctcacctcgg tcccaggtgg actgaagtcc 1080
   agagcggcgc tgtgcagctg gaagggcgcg cgatagctca agttagaggc ggccccgggg 1140
   cgcggcgcag gacacaagac ctcaaactgg tacttgcaca ggtagccgtt ggcgcgcagg 1200
   tggcatcgca tctccttcca gcctgcgggc tcgaccccac cggtggcctg gagtaccgcg 1260
   catctccgcg cggtgcagga gcgttggggc tcctccaccc actgcagcgt gtcgctttcg 1320
   agaccgccgg ggtcggagga cagccaggag aaaccccgca aaggctcgtt ctccagggtg 1380
   cagtgggaac gcctgcgctc cagtgcgacc cagaacagca ggtctttgga gccccctccg 1440
   ggccctgggc ctgcccgcag gagcgcgagc acagcgcgca gctcggcgcc cgcacgcacg 1500
   gtgctgagcg ccccacctcg caggatgcag gcctcctcgg ccgcctgccg cttcatggta 1560
   gcgtggtgca ggctgtagca ggcccccgag gccgagcagc cagcacggtc ggcagtgggg 1620
   tgttcgccgc cgcccggccc gggccagagc gcctgccaga ggaggcacag ggcgaacgcc 1680
   ggcctcattc tctgaggccc cgcacgcaga gctgctccca acttggatct gtcccgctcg 1740
   aggacgcaga gctgtgtctg gagcgcggtc accgccctct acagctggct tccccaaccc 1800
   ggttccccct cccttcccct ccgccctcgc tcccgacgga ccccccaact gccaagaatc 1860
   cccccgcggg cccagcggcc aacagtttcc cttaggccgc gacaggaaat gtgtgcggag 1920
   cgctgccagg cccggtgcag ccctgagtta ttctggggtc aggaacacag ctggcgccgg 1980
   gtacagggga cgcagtcgac tagaaggtca aacaccctgc ttagagcaag aggagaaatt 2040
   cgccactggg atgaagtgtg tggtttttct ccgttcaggg agtttcatca ggaccgcaga 2100
   acccataaca cgtaagcgaa cataggcagg aaggacttga gaaaggaact ggcttatgat 2160
   tataagacca cgcccgtcta ttgtgtttct gtccggagct gtggagttag cgtcagaata 2220
   attcgtagac ggctccagtg tttggggggc ctgtgcctgt gggggaaaaa tatgttgtga 2280
   aacaacttag caactgcttt tgccaccaga ggcagcacta tttaaccgca tatctctcta 2340
   tgggtctttg cttctctatt atgtcaagag atttcaggag tcctaactac taaagaattt 2400
   ggagttgttt gggtatttta aatgggaaat tctgcttctt ccatcagcac ttgctttaga 2460
   aatttatttt tcacttccac gtgtttctct ttgagttaga ccatattgat tcattacaga 2520
   aaggaaatgt atttctagga ctaaatgaaa aataatgacc atagatctta acagttaggt 2580
   tgttagcttt ccttgccact gtattggttc aaaaattctt caagtttata ggttatacat 2640
   aaatgagttc gtggccttat aaatattcga gaacaactgc cctatgtgtg attttagcct 2700
   tgaactattt tagtatcatt ttctcctatg attgagtggc agcatttata atagtaagtt 2760
   gtaaacatct cctttaagga aaattagagt tccttaagaa attaaaatgt ttattccatt 2820
   ataatgaata ttgtatactg tcaaaaaata tcttgtaatt tgaaagatgg gtgagccaat 2880
   ttctttagaa ttccagtggg tgttttgttt taatataaac atttcaactc agtgggaagt 2940
   ggcaaggaat taaagatgaa taaagaagta aaggttaaaa actgtactgt tatgaaagga 3000
   tttcttgtta gcaacttaga caattcagta tcagtttaga aaacgtacat tctatcttgg 3060
   ttcataatgg ccaaatacgt tgccatcaga aatacataga gagtttcagc taagccctct 3120
   tagagatgtc tgatgctttt aaatatcttt aatcagaatg aaagttgcca ttccatggat 3180
   aatgcatata agctcacttt gttttactgc attcaggttg atgaggggtg ggagggaagg 3240
   tttcaggatc acatctgtat gcttccatct tatttttacc ataactctca ttcttctttc 3300
   ctcctggaaa ttccatgtat cttaacaaac aacagtaagc ctatgagtca tcatcatact 3360
   taccttgctt ttgctgagta tggagctttg cactaggatt tctctcctgc cctatttccc 3420
   aatttaaaga acattcctta gtccttcctt cagatgtctg agaatcacct taaacgtggc 3480
   tttgcgttta aattaggtaa cttagtgtct tataagaaag aaacatgaaa atccagaaat 3540
   caaattgaat ggtttttaaa aaatacttag tcacttgggc cagctctcta tagaccattg 3600
   caatcttatg actgtgtttg taaatgtgtc catgtagtgc aatattttta ataaaatctt 3660
   tttcaaactt aagtagtcaa tggaaatttc ttagatagct ttaaaaatcg tttgggatga 3720
   gatgaggtgt ctacagtaag aaagaatgaa tagctggaaa gcttaagtaa atacatgatc 3780
   tcactccctg acattctgtc taggggagtg ggattatagc agagaacaca ttggccataa 3840
   cgactatatg agagtactga ccatgaccat gaccatatca tggaatgaca caagagttag 3900
   aaaaaattag ctcaaagtag gacgtaaata gttttgtaat tagtttgaaa tagacagttt 3960
   atctgaccaa attagaaatg gaggaaagtt tctatttttt aataatattt attaagataa 4020
   taatatagaa ccattttacc cccactggat tccaaatgcc taaaaatctt ataataccaa 4080
   atgtgaatct attggctctt ttacatgtta cagattagag caaccacttt ggaaaaattt 4140
   gacacttttt ttcactaaaa ttgaatattt gcaaactcta gcacttccat gataaggtat 4200
   aaaactaaga gaaatttttg cacatgtaca acagaagata tgtaaaaaat gtttatagtg 4260
   acactattca tattaacaaa acgttgaatc aatcaaaata tccaaaaaca gtataaaaat 4320
   aaac 4324
<210> 8
   <211> 5683
   <212> DNA
   <213> Homo Sapiens
<400> 8
   ataatgctaa atgtaaattc tttgttttgt tcaatgttct tattttatgg ttggcaaaca 60
   aaaacaacaa aacagcattg acaacaacaa cagaaacaac caactgaagc tgcagctttt 120
   aatctttcta aagacaatag gctaaagtga ggtcctcata ataagatgct agtcaccagg 180
   aaggaagcca gggaaaatct cctcccaggg ggtaattgat ccctattggc ctaacactgc 240
   atgtagcact gagttaggag gctccatgga gaagtgtctg cttcttttcc tctgctgtca 300
   gaacaaagtt cacaaacaac tttgttttcc ccacaccttt ggagttagct agttagggcc 360
   cctatttatg tccaggggac cagacctgga catttgtaaa agaactgaat atagatggat 420
   agctaagaaa aaaacaaatg ttaagattat cattatttat agcccaatga tttagtaatc 480
   taaaaaaatc caagaacaac aaaataatcc ctaagaagga caagaatttt aaaacatgca 540
   gtttagaaaa ccaaatttct caggcctaga gctcaagtaa aagaaaatct gagataatga 600
   agtctggaac actgggcctg cattctgctc ctgactctgt gcccttgggt aagacattga 660
   acttccccac cccagattca tcctacaccc taaggagggt gtaggatttt gtagccttcc 720
   tagtgatggc atactattaa tcagaacctt aaccagctct attctcacta tcccagtaaa 780
   atgatgttaa aaattagacc tcgtgacatg agagaagagg ggaaattaat tacagattac 840
   aaagaaattg tgggatctaa gtttagggtg aggtgtttta ttgaacctct agggttggtc 900
   atagcacctg ccatatagca ggtattcaat aaatgatgtg taatgggtgg ttagactgat 960
   ggaaaggcag gccttcccag tgacggtaaa ggacctgggg caaaagagac ctgtggttga 1020
   gtcctgatcc ccacacatta gcacactgac tagacgaagt tataagcttc aatttcttcc 1080
   tgtgtaaaat ggagataata cctcataggg ttatgaggat taaataagaa aatgcttagt 1140
   tcagtgccta atacacagga agcacaccgt aaatattagt tattattatt actaatactg 1200
   tgatcatatc ttccatccaa agacttctct gaggagcagg gggcatccag agataaaaag 1260
   cctgcaggtg ggaaatctgt tagccttcta gggcgttagg tttctgtgga actctgcacc 1320
   tcttccctgt cctagttctt aagaacagaa actctccagg gacctctggt gaggtagccg 1380
   tgggaagatg aggtgcagaa gtaagctggg acctgtgagc ctcaatttcg gcctcttctg 1440
   cgctgagacc caagcggatc ttgcttggcc tgtatgcgtt actgggggaa atggacgtgg 1500
   gcctgagcgc ggcaggtgcg agggcgctgc cccggggccg accaccctgc ggggacactg 1560
   tagctgtcat tccttcttct gcaggcgggt aggggaagcg gtggccaaag tgggagtcga 1620
   ccgctcagca cagtctgtct gagtgttgac caggaaagtc caggctcttt ctaaatctcg 1680
   ccgccagacc tggtgacgca ttcgcatgta tttaaggcgt ttgcacgcag aacgttatca 1740
   cagaatgtag ccacctttct taacggtccg ggaaaccaga ggtctctcca gctactcagg 1800
   gtagaggaat ttctcctatc tccatgtgac atcttctgat ttagaagaac taatgttaga 1860
   tttctcttgg gcctttccac ctacagctat agtcttccct ttgtttagct aaaattgagg 1920
   caggtaggaa aatattattg ggggcataag cctattagtg tgtaaacgta tttttatgaa 1980
   gtgtgcctcc agggagccat taaaaactga cctctcaacc acagaaatag atgagatttt 2040
   gagaacattg agaagctgcc ttttgcaaag taaatttgca atggtccttg acgaaggggg 2100
   gtcgggggcg gggagaagtc cagccgagag aggagctcat tccacgctat atttttgcag 2160
   ttgaaaagct gcctaatcat cgctaaccgc ttcccgcata agagttctgg gaagacttca 2220
   gaaacaaggc aaatgaagac ttttcactgc ctccttcggg ctgtcgcttc cggaagccga 2280
   agtcctagca cgcagagcag caggagaggg ttactttcag gcaattccac tgagcaaaat 2340
   aaatcactta atggcataac gttctggctt aaaaaattgg aatttatcag aggcaaaaat 2400
   atccttcaag aaactatgga cactccgcgc cctattcatt tccatggcag cagagtatct 2460
   gcatcttgag ccacctatac agattcatgc ctcgtatcgc tctcacctcc tttctttttg 2520
   aagtaaagcc ctttcccaag aaggcggcca gaaagtggac cccaccgggg gaaaaagaaa 2580
   aatgaaacgc aaatcagctt ggcactgctt gcgtcttcca aaacgcggtg ggacaaggct 2640
   attgagtcta tagctaattc tttcatgtat ataaaatgta tacatatgta tatattttta 2700
   tatacataaa agaattcata tatatgtata tagctatgtg gagccctgaa gcaattctcc 2760
   atgcttttgt ctccctcaag ttccccaggt ggaggcagtc ataagcatta taagccgcct 2820
   tagtgaccac cagggacgga aaccgttaat tatcacgttt cctttcatct ccaggggccc 2880
   tttggcccgt gacacaagag gcttcggtat tggcgctttc ccagaactgg cccagaggag 2940
   ccagttcaga gtgtgaggtc gggtctgcat tgaacgtaca caccgaggtc tatcagactc 3000
   ccccgatttt agcgaagggt gctgactgct gtgctgctag aggctagcaa gctccctgtg 3060
   cgcagctgat gagtttcagc aactcgccac ctgggcgctt ttctttaaat tttgggagta 3120
   aactgggaaa ataaaaaaat ctccacgtcc actggctctc tccccttctc caacttcctc 3180
   tttcgactcg tttgtgggag ttttctcctc tttgctggga ctataatgtg atgcgcaatc 3240
   gtttgtgaat gaacaaaagt caccggcaag cagggagacg gggacagatc gctgacggca 3300
   gattgagggt ggcagcaaag gcccggcctc caaggataat ggggagccgt tttccctcac 3360
   gcctggtctc tatggccccc ttcgtcttcc aggtaaaatg aatgttcctt catccatcat 3420
   ccgcagagta ccctcaggcg tgcgtagaat ctgctgatga aacctattag cgccgactgg 3480
   gcagctttgt ggagccaccc gaggctctcc attgtggcct ttgtctgcag aatttaagca 3540
   tttacataat gcattagcac ggaactcagc acccggtggg gacatcgcgt gccaagcctg 3600
   gcgcggccaa cgcttcagcg gctccctcac ccggcagctc cctaggacca ccctcgagga 3660
   ggcattggag tcgggctgca ggcgcacggg caaagaactt agcatctcat ccaagtactt 3720
   cgccttcctt ggccgtctcc gggaggttat gcttaaaaac ataaaaataa aaataaaaat 3780
   aaaaataaag ggaggcggac aaagtttcgg tgggtgaact gaagctgggt ccatgtgacc 3840
   ctgaagccgg agaaataaac ttaacatgaa tcttgctttc ctggcgggcg ttgggacccc 3900
   gccgtttttc atgccaaccg ttggaagctt cgtactcaac ggccacaggt gcctaggagc 3960
   gcagagaggc ctcgggttca aatcaccggc gcgcagggac tggactcgcg ggtagcgacc 4020
   cccccaaccc cccccccccc gccctacaca cacaccctcg cgccggctga aagcatggag 4080
   gattcagggc atttgaaaaa agaggggctg ggcgcggtgg ctcacgcctg taatcccagc 4140
   ggtttgggag gtccagaagg gcggatcact tgaggtcagg agttcgagac cagcctggcc 4200
   aacaccagcc tggccaacat ggtgagaccc cgtgtctact aaaaatacaa aaattagcca 4260
   ggcgtggtgc ctgtaatatc agctacttgg gaggctgagg cacgagaatc gcttgaacct 4320
   gggaggcgga ggttgcagtg agcccagatc gcgccaccgc cctcctgctc tgggtgatag 4380
   agcaaggcac tgtctcaaaa caaaacaaaa caaaacgaaa gattcggtca ggaaagaatc 4440
   tgcaggcatt cgaggcgctc gcactttgca aagtaaatgc aatctcttta ttaagccgaa 4500
   gtccctcata tctatccttt tagaggaagg tggtccaact cagaaatctc tcccaagagg 4560
   actttccacc gaagactacc gcgaagtgcc aggaactcgc cccagtcccg acaggtgcag 4620
   gacctttcgt gccgccacac cttgggactc tacctcccta aataggccac ttaaaagcca 4680
   gtagtgcaac cgggatcccg cggcgataaa gaatcactgt gcagaaccct ggagctggga 4740
   gtcggcccgc cccccctcca aagaaaccgg gatcccgcgt cctccccgcc gctagcgcag 4800
   cgcgccagcg gcgcccaatc agtgagcttt ccgggtctgt gacggccttc ggctccgccc 4860
   cctcgacggc cataaaaagt cgcagcgaag cctgcacctc cgagcaccgc gcgcggccct 4920
   gcccccggca cggcccccag gtgcgctcct tctccggctg cttgtagcac tggtctcact 4980
   gtccccgccg tcagccaccg gttccttatc cgtctcattc cccattgtgg cttggctgag 5040
   ccggtcgcca ggcctcgctg tcctcctttg ccttcctctc tcctcagcgg ccgtactttg 5100
   cgccgtacct cacctggcct gcaggtgagc agcagcgcag cacccctgcc cggcgagctt 5160
   aacttgccca gcccggcccc tgccggagtg gcaccggcac ctctccaaga cgccctcttc 5220
   cctgcaggat gaagaacccc atgctggagg tggtgtcttt actactggag aagctgctcc 5280
   tcatctccaa cttcacgctc tttagttcgg gcgccgcggg cgaagacaaa gggaggaaca 5340
   gtttttatga aaccagctct ttccaccgag gcgacgtgct ggaggtgccc cggacccacc 5400
   tgacccacta tggcatctac ctaggagaca accgtgttgc ccacatgatg cccgacatcc 5460
   tgttggccct gacagacgac atggggcgca cgcagaaggt ggtctccaac aagcgtctca 5520
   tcctgggcgt tattgtcaaa gtggccagca tccgcgtgga cacagtggag gacttcgcct 5580
   acggagctaa catcctggtc aatcacctgg acgagtccct ccagaaaaag gcactgctca 5640
   acgaggaggt ggcgcggagg gctgaaaagc tgctgggctt tac 5683
<210> 9
   <211> 2347
   <212> DNA
   <213> Homo Sapiens
<400> 9
   ctgaggctta agccagggag gtcgagactg cagtgagctc taattgcgcc actgcactcc 60
   agcctgggcc acagagcaag accctgctca aaaaataaca agaaaggtgg aggaaatagg 120
   tagagatcag aggaaagtca tggccccctc ctgaccatcc tgagaccctc ccaactccac 180
   caattcctgg agagtccctc acttgcctta tttcacagat tgggaaactg agtccagggg 240
   cactcagacc acctgaggcc ccagctggag atgaagagat ggattcaaac caccccttgt 300
   ggagtatagt tttgcccctt ccagagtgga gaccgtgcct cagggcttcc cccacccgca 360
   ccaaggttgg ccctctcagg cctcatcttt cccacctgca aaatgggctt ttgtgagtat 420
   gaaggagggc ttggcttgca gtgagcgctg tccacccgct ggccagtgtg attagtcatt 480
   gttgttgttc cccagtggcc tctgcctggc agctgctgga aaaacatcga gggagggaga 540
   tacagggctg gggaggtgaa aagctggaca cagacgcccc tagccccttg ggatcaggaa 600
   cagatggagg ccgggtgccc tgaagtggag cagggaggac tgcctggagg agggacactt 660
   gagctagaat gtatagaatt acagggcatc tccggcagag gccacagcag aggcaaaagt 720
   ccagaggagg tgctttgggg accaccaaga tggacacagt gggtctgggt ttctgtgggg 780
   tgatgggctg gggcctcgag cgccaggcta aagggcctgg ctttctccga gggcggtagg 840
   gagccatggc aggtgtgtga gctggagggg taccccagac aaaggtgaag tggtatttct 900
   ccccttattg aagtcacaac ctgacactcc ctctgaagtt ccccacatcc caccttcccc 960
   ttgccacatc ttggcaagcc cacctccttc cctccgtctc taggcctgcc aagaggccct 1020
   gtgggcccct tatggcccca cccagctgcc ccagaagctc gtctgagata agttcccagg 1080
   gctgccccac tttcaggtgc cccacagact cgggggcggg tgaagcagga aggatcgagt 1140
   cacgagggcc ccagaaaaca tgacgggagg aaccacagca cttccccacc tcctcagctt 1200
   ccgccttctc agggagagag accttcagcc cgaggaggag ggccgagctt ccctccatgt 1260
   ggcaagagac cctaacgtcg ctgaggatca tctaaagagg gtgttgaagg atgcatagga 1320
   gttagccagg agacaaccca tttgttaatt taacaaactc ataggcatca ctcatcaaca 1380
   agaacaattt tcatcattca atcaggactt tctaatgcct ctgagggatc atttgaggat 1440
   gtttaattat tatttttatt tgagacaggg tctcactctg ttacccaggc tggagtgcag 1500
   tgacacaatc atagctcact gcagcctcaa cctcccagtt tcaagctatc cttctacctc 1560
   agcctctgat atagctgaga cacaggccaa tgccaccatg atgcctggct tttctttttt 1620
   tttttttttt ttttactttt gagacagagt cttgctcagt cacccaggct ggagtgcagt 1680
   ggtgcgattt cagctcacta taacctccgc ctcctgagtt taagcaattc tcctgcctca 1740
   gcctcctgag tagctgggat tacagacatg caccaccaca cccagctaat ttttgtattt 1800
   gtagtagaga tggggtttca ccatgttgtc caggctggtc tcaaatgcct gaccttaagt 1860
   gatctgctgg cctcggcctc ccaaagggct gggattaccg gcgtaagcca ccgcccctcg 1920
   accatccagc tttttttttt tttttttcag tttttggtag aaatgtggtc tcatgcctgt 1980
   atcccagcac tttgggaagg ccgaggtggg cagatcactt gaggtcagga attcgagact 2040
   agcctgacca acatggagaa accccgtctc tactaaaaat acaaaattag ccgggcatgg 2100
   tggctcctgt ctgtaatccc agctacttgg gaggctgagg caagagaatc acttgaacct 2160
   ggtaagcggg gtttgcggtg agccaagatc gtgccattgc actccagcct gggcaacaag 2220
   agtgaaattc tgtctcaaaa aaataaaata aaataaaaat acaaaattag ccaggcatgg 2280
   tggcacgcgc ctgtaatcct agctactcag gaggctgagg caggagaatc gcttgaacct 2340
   ggtaggc 2347
<210> 10
   <211> 6709
   <212> DNA
   <213> Homo Sapiens
<400> 10
   actcttaaac tcctcaactc acagagggtt ggagagctgg caccatgagc ttccagttcc      60
   tacgggtgag aaaagaggtg tctgtgtgga gggcaggtgc gaccccgggg gctagcagag     120
   aggagaggct gcgggaggac tcgctgcgaa gggcgagggg tggcgctggg tgggacgggc     180
   gcctggggcc gtgcagggtg gctcctgggt gctgcccggg ctgcctgtcg cccagtactg     240
   gcgcaggaag acgggtccgc gcagcgtctg gcaacagtgg cctgctccac gcctggaggc     300
   gaccaagttc tgagttaaag gcgccgggct tagccgggaa gtgtatggcg acccagaaca     360
   cggagcggag aaggcctcag gggacacgag gctaccgcct tagtcctcgg gcctgcgctc     420
   cgagcggtta gggtgcgtac ggatgggctc cgggatgtta gtggagaggt gacaggagtc     480
   taccatccca cggccaagcg tgtgaggcct ccggcgggcg ggggtccccc ccggggcagg      540
   gcagggggag agtcaggcat gtctctaacg ggctccctag tagaaccgct aggaaagtgg     600
   ccttcaaccc taggcacctt tgtggctttc aggtagttgg gcgactaaaa attcactttt     660
   gcctgctgtg aaatgggaca ggtggttgtc aggttagaga ctgtgcccac gaagtctcgt     720
   ttggcgggcc gggactgacg tggacccggg ccctcctcgg aagcttggga acctgtgagc     780
   ccctcagctc ctcgcctcac tttgcctgtt tgaaaaaagg agttgtttga gaggaagagt     840
   taaggctcaa aaggttctaa acgaagagca tgcggcctga cgcggagcgc aaagaagcga     900
   gcagtgaact gaatctggtg ggactgagac cagaggcgtc tgtatgcggc ggccgcttcg     960
   cgccctatta aagcaatctg ttatttacga taatcatttt tgtaattatt ttggagtggc    1020
   tgtgacttga tgtctgtggt gccccagggc atgcccttac tggacataga tacttcaggg    1080
   tgccctttga cgccagtgtc ggtccagtgg gtgccagtgc cgcgaaggga gggtggcggg    1140
   cgtgaatgcc ccctcctcct gggtttgccg caggccccgg cctttccacc ttgcttgatc    1200
   ctggcgggtc tgccacttac tgaggtccct accggcgctc acgttgtccc gagaggcggg    1260
   acaaacgtcc ggcgcgtggc gcggagtctg cgaaagcgct cgggggcctc catcttctgc    1320
   tcgggagtcc cctccctggg gcgggaagag gagtcgcaga tctgcccgaa tcttgctcta    1380
   cccgcctggc cgccttcttc cgtctggggt gggggcaggg gtggcggatg actcaggcct    1440
   ggggccggga gccctgtttc caaaggcacc acctcgcgcc tctttggaag tggcgcgctg    1500
   ttggggcgct cccgctgttg cagaggcctg gtttgcggtt tcataggggc gtgggtccag    1560
   ggcttccagg gagtaagtga gtttaggctc cctagggggc ggtagagggg aacccccgac   1620
   aaccttccat cctcttctcc aggcggctgc gaggcctccg gggttccaga ggggtcccct    1680
   tctgccggtg ccggctgtct tgagcgcggc cacgcagtcc gcgtcttcac gtgggcaagg    1740
   ggcggggacg ttggagggag gctgtcttgg atgctggagc cttcggttcc cggctctgct    1800
   gagcagaaaa gttgaagtca aggaagggtt gaggcttcct cacaagggcg cgcgcgtgcc    1860
   tgggtgccgg cccagaagcc gagaatgtgc gtattgggga agggtctccc gtgccgagaa    1920
   gacagaggat ttgttctagc tgccttccgt acagagggcg cggaggttgc gctccagttc    1980
   gaacgcttac ccattggaaa gagggcagcg ccggggtcca gggaagctcc ttgggaatga    2040
   atggcctttg ccaagcggtt ccggatcctc tgggtccttt gggcccacgg cacggtgctg    2100
   cgcgagccct cagtgcccat cggctccctt cgcctcctgc gtagacgctc ccaggcgggg    2160
   aggcatatcg gttcctccgg gcagctttgg ctagtgttgc tgtgggaaag gagagccagg    2220
   gcctgggatg ggggatgagc accttcttgc ccattccggg ccccagcgtg caggaggtaa     2280
   acttgccagc acagacaaga cagcttgttc aagctgcacc tcaggccggg tcagagaata    2340
   aaaccgaggg ctagaaggcc cagaatgtcg gacagcccag cggcacccgt cagggagtcc    2400
   caggcgcccg aaagaggcgc cgcacctctg gcgagtctag gacccatctt cctggacctg    2460
   tgctctggag tgcctgcggg cctgggtcta atttctgctt ctggcaggtg tccccctccc    2520
   ccgccgctaa taccaggagc gctgcttctg cggtaactta ttttaccccc agaagcctgt    2580
   tttgggaccg aagtgtcagg gtcctgtgtg tctttttatg cactgttctc cttagtgcaa    2640
   agccctgcag atatgcttgg ccgaaaagtc tcagtggttt caacttccag attttgttcc     2700
   cgcgtccggt cggaaaatca cctgggattt ggctgctgtc taaggccggg ggaaagtttc    2760
   ccttggagga cgcctattat tattattttc ttttaggccc acctgggtct aaataaatgc    2820
   taaagttcaa acgaacccga gaaggaacag cgaggcagtg gatgccgcca ttccgtggat    2880
   gggagagtca tgtctactgc aggtgcggac accgtactga taggcattga ttattgcaaa    2940
   atcgtattca gtatcaaata gaaatagtct tgtcctctaa tccccacaga atgtttaaat    3000
   ccagcccagg gagccagttg ggcgtcccca acaaatacgc cctcgagaat tagcgatgtt    3060
   ccccttacaa tgaaagtcaa ttgctgcata ataactgggc caaagaattt cgttagttta    3120
   aattttaaaa attagtattg cactttgctt tcaagtttgg gtggcaagta ccgaagggga    3180
   gagggaagca gcgtttgtgt ggaaggaagt ttcgttttct gtctaaacat gtgggacctg    3240
   ggtgtctacc tctccagtcg agagctctct gcaggctctc tcccaacttc ctgcacctcc    3300
   tcaccctcac cttacccgtg gcgctcgggt tttattcctg cccggcgctt ctttcccagg    3360
   gcgagtagag gcttctgagt gaccggcccg ccagaggcag ctgcagagcc ggcgttccgc    3420
   agggcagggc agggccgggc cgctgggctg gtccagtgcg cgcgggttcc ccctggcctc    3480
   agccagccgc gggagatgcc acttcgggcg acagcggcga gacgccgctg tccgcaactc    3540
   ctgggcgggt aggaccgtct gctgcctctg cggcccttgg ggcagactcc ccaggagtcc    3600
   cttcctctct cctcccgccc gggggccggg accgcgctgt gcccacggag ggaactgggc    3660
   gtctctggcg cactggggct ccagctgcat gatcccagct cagaccttaa gcctcagtgg    3720
   ggtgtaggtg ggatgggatg tgtttttagg agatgaggag accccggttg ttgagtcgct    3780
   ccccaaatgc gcagtttctt aactccaggt gacatccgtc ttttggagga acgagtgaag    3840
   gccgtttagg cgaaagaggg gtgggtttca gtatttggat cccttccccc agctctgcca    3900
   atctcaggtg tgtttacggt ggaggtgaca gaaggaaggc gcgtcggggg acccaggcct    3960
   cgcaggtctt cggctgtcaa cgaggcaccg cccactgact ccgcgcttcg tccctcacag   4020
   cgtggggctc ggcgctgcgc caggcccggg tgcggggcgg agctaggctg ggactggctg    4080
   ggggccgccc cgcccggcgc ctgggcctcc gcgccggccc ccggggagga gttatgataa    4140
   tttccttctc attaaggcgc tcgggtcccc cggctatcgc caggacacac tgttcgggcg    4200
   cggctttccc cgtccgcgga gcggtcttga cactcgcggc ggcagcatct acgctcgcag    4260
   agccgccgat gcgtgtccag tgacccggac agcaaggccc gcgcgcggcg ggggcggcgg    4320
   cagacgcctg gtcaccgtga ccccgatttt ggatttaccg cttgggggct ggggggatcc    4380
   tggatttaac tggcgactgt tttgggggac gccggacgcc atgttgtgga aaataaccga    4440
   taatgtcaag tacgaagagg actgcgaggt gagctggggc tccggggtgc agccccgccc    4500
   cgccgaggac agtccgggag gcaggggcca ctggaccgag gtcggggacg agggcatagg    4560
   agccctggcc tctcggtggg acgggatcca cttctccgga caccccttag tccatttctg    4620
   cggggcccct ttcctgtata gggccttttc ctaaatagcc tcccctcggg aacgaggcct     4680
   ggaaagagaa tggcaaatcc cacccacaca gcttaccggg cgctttccta agcggcaaac    4740
   agccctgctt cccgttttct cggaaaagtg ccccgtctgc aaccctcaga cgtggctttt    4800
   acgcacgaag tctctgtcca aaggggtcag atgaggctac ctgctagcga caccttggcg    4860
   gacactcctc caaggccggc ttgtcacctg ccccgctacc tcctcgggga agcacgaaaa    4920
   caaccgaagt ttggctttca aaagaagtgg gggctgggga gtggtggtgg gggtgcccga    4980
   ggccagatgg ggccgtaaga gctcgcgagt gctccagcct gtcggacagg ttgagctaga    5040
   gtttcggaga gtgggaggga agaaggaggc ggcgagcggg aagaggaaga agacgcaggc    5100
   agcgctaggc gagctcaggg gcgccgggag cgcggagctc gggctacgga ctcgcgggag    5160
   ttcactgcgc ctccgggccc tggagggctg cccctgcccg caggcccggg cgcttccgcc    5220
   aggaggcgac agcgccatgt tcctccaggt tcccggcgcc ccgagacccc tgggcagatg    5280
   gggacgcatc ctttagaaac tgagtcgggc cgcccagggg cgagggaacg tgcgcgggca 5340
   aggctgccca atttccaggg ttcttcatgc cccctctgcg ccccgacgtg cgagaacact 5400
   gcccttggca gtgcagggcg cccccactta ttactcccct cggctgggcc cggccagcag 5460
   ggagggccgc cctgtgcgcg cgctccctct tcacttccca gggcggcgca gggtggcggg 5520
   ccctgctttc cgagcgccgc ccgctcggag gtctttgtcc cgagggcgtg gaagggaggg 5580
   tcccgggggc gggggaggtg cgcatttccc gcgccgcgca ctctatccgc gcctgccgcg 5640
   ctgccacctc cagcagtccc tgcgtcatgg gcgggctcca cgagatagct ctgcaccggg 5700
   cgtccggctc cttcgccccg ggctctggct ccttcgcccc gggctccggc cacggacttt 5760
   tctggcccaa gacccagggt tcggacttgg cgcctccaag cgcctcgggc ttgggagcag 5820
   cgcctagacc ttcgccgccg ggctttgaga actcgttccc ccaggtcttt caccagactc 5880
   tcctccctcc ccgcactctt tgcttacaac gaaatcctcg gggcgcattg cccccgggta 5940
   ccttccgacc ctgggcaagc cccgccgggc ggggtgcggt tggtcccccg gggccctctg 6000
   cgtagcccgg cgatgccggc cagttcgcag tagcggggtt tcgcactaac ggggtctcct 6060
   gttttttttt ttccctccag gatcgccacg acgggagcag caatgggaat ccgcgggtcc 6120
   cccacctctc ctccgccggg cagcacctct acagccccgc gccacccctc tcccacactg 6180
   gagtcgccga atatcagccg ccaccctact ttccccctcc ctaccagcag ctggcctact 6240
   cccagtcggc cgacccctac tcgcatctgg gggaagcgta cgccgccgcc atcaaccccc 6300
   tgcaccagcc ggcgcccaca ggcagccagc agcaggcctg gcccggccgc cagagccagg 6360
   agggagcggg gctgccctcg caccacgggc gcccggccgg cctactgccc cacctctccg 6420
   ggctggaggc gggcgcggtg agcgcccgca gggatgccta ccgccgctcc gacctgctgc 6480
   tgccccacgc acacgccctg gatgccgcgg gcctggccga gaacctgggg ctccacgaca 6540
   tgcctcacca gatggacgag gtgcaggtga gcggcgctgc ggctcctgac cggacctgtt 6600
   caccctacgg ccttctgccc ccaccccgca ctcctctagg ctcccccgaa cttaagggaa 6660
   ttttgtcctc tctcccccag aatgtcgacg accagcacct gttgctgca 6709
<210> 11
   <211> 2296
   <212> DNA
   <213> Homo Sapiens
<400> 11
   gtcactgcac cctgccaaca tttttcaaaa gtaaaaaata aattaaaaaa aaagtcttta      60
   gtggattgct gagtcccctc tgacaggagg taccaaatgt gcttttcctg aggacagggg     120
   aagagttcac tattcagcca tacctttgca tattttacaa aattcacttc taccaaggat     180
   gcctctccac aaaatgagga gtagcacaaa gaacacctaa gaaaccaggc ctgacccagg     240
   gcctctgatg ctggggcagg ttccccgcgt gctaatatcc caggggaccc tgttgcagtt     300
   ctctgaatac cggctgtgag ctttccaact tcagctgagt cccagcatcc tcctcacacc     360
   acttatcctc cttgcctgtc cctggcgcgt ttagacaact ggaatctttg ctgacatttt     420
   aaatgggctg gctacttctc cagcgtggag gcacctagca caaggtgtga tagatcagta     480
   tttgttgatg agtaacaaag ttattctggc aaattggact tttcccctga aacgttttgg     540
   cttttacatt tttaatgatg ggtcaagaaa cagcccccaa gaagaagggg cggagatagg     600
   gggtaggacc ctgcctgaag cttccaagct gactctgttt gcagtcactt aattcactag     660
   ggagggctgg atctgcctcc ctgtggtgga ctctccgcat ctccagaaag tgcaggggtg     720
   gagaattaac tcagcccccc tcctccccat aagagccccg ccaggtcagc ctgcatgagg     780
   agggcacgtg cacacctcca aaaggactgg tgcagccact tccctaagct cacagtccca     840
   gtgtgagctc agggcccaga ctcggggcct ctggtgtttc aggcgtgagc caagtcccct     900
   gagcaagagg cctcatgata aatcagttgg agcctggggg ccatgaaagc attggttatg     960
   gcagaaacta ccagctcgtc accgtttatg ctgctttgct taaaaatgac accgcgtaag    1020
   ggattttgcc aattcccaac ctcgggagat cacccaccac ctggctgtgg tccgccgctg    1080
   aacaggaaag cactccgaga tgccaagaga ctaaaaggct cgtaactgtt gtttagaaat    1140
   gaaaatggcc ctaaattata accagagatg gaagcgcgtc tcaggggagg gcggggcgct    1200
   gtgtaatcga gcctcccatg cgcaaggcgg atacaaacgg tttatggatg tgggcgaagg    1260
   ttctcagagc aaagaatgtc catttccatc agattaaaat acagtttggg aaaacaatta    1320
   cctgcctgag atcctgggaa agtaaaactt cctggtttct ttgccaggaa gaacgattgt    1380
   aaaacaaggg ctgagaatct cccaaacaag attttagaat tttatccaaa cagctgcagt    1440
   tcacctttga ggagccccag actcacttgg gtctgatgga tgttgcaaca ccaagtttga    1500
   ggtctcccca gcctggtgcc aagagtgggc cccaggggac aaacagagaa cagttgctct    1560
   cttaatcgga gtagaaaaca aaactccctc cccttcccac ccccaacctc cctgctttgt    1620
   tgtgaaattg tcactgcgcc tttgaggagt gattaaaatc ccagccatga ccaatttaaa    1680
   gggcaggttc ccaggcaggg aagggaaagg ggttcaaggc aaaaaaaaaa aaaaaaagtc    1740
   agttgaagtg agtttatgga ggatttgggg atcacaaaag tcatgggact tgagtcaatt    1800
   aaaattaatg gaaaagcacc aacatgcaat ttaaaaaata ataataaaat aaagcagtaa    1860
   ggatatttct ggcttgctct ctgcttccat tcttgagcct gcttgggaat cttggagcag    1920
   acttcctgaa gcacagcatg agtgcccaat ggctgtttta acctattttc ctgcccttca    1980
   cacacaaaga ttctgcaatg gagagtggga atgaatggtc cacccacccc tgaacatttc    2040
   ctgtgagttg ggaatgcgct cagtaaggct gctggagtgg agggaaaaga aaaggtgtct    2100
   gagttctcta gacatgggca gaaaaggaga gaatttgaga caggctgaga gaaagctgct    2160
   ccggttggca gcattgccag attctcagac acaccgtaca ttccagcccc cgaaaaggcc    2220
   atcctgagtc cctctagggc ccctggggct ctcatctcat cccaaatatt tggaactgtc    2280
   tgtgcctgct ataata                                                    2296
<210> 12
   <211> 4247
   <212> DNA
   <213> Homo Sapiens
<400> 12
   ctccaaaaaa actaaaacac aaaacaacaa taattactaa aaatcctacc tccgctcctc     60
   taaataaaaa acctattcca aaaactccaa tctaaaaacc taaaaacgaa aaacgcgcac    120
   tacaattccc aaaaatcccc gaactcaaaa acgatccccg ccccccgcct tctccgcgcg    180
   acgaaaccga acttaccaat aaaaacccga aataaaacga acaaaaacga aacgaaacga    240
   aactacgaaa aaataaccga cgaaccgcgc cgcgaaccaa tatctcaaaa aacgcgaaat    300
   ccgaacaacc gcgcgctaaa aatctcgaaa cgaacgccgc gaaacctctc cgaaccataa    360
   ataaacgact ctacgacgcg acccgaacaa aaactcgacg accctacaaa aaaattcaaa    420
   ataccgacgc cgaatcccct ccaaccctcg taacaatcga tacgctatat aaaacgaaaa    480
   aaaacccgaa ataaatttcg aaacgcgaac caaaaaaaac gaacccgacc cgcgacttca    540
   acgctccccg accgacgcga aaaaaataaa actaaacgtc cgcgcgaact tcgaaaaaaa    600
   aaaaaacaaa aaattttaac gaccaaaaac ttcccgaacg tttatctcga aactcttttc    660
   gccgtctctt acctaaaata taccgaaccc gatacgactc tatcgtccca cttcctacta    720
   cgatttccac ctcgaactaa actcaactaa tttattataa caactatcga caaccgcccc    780
   tccgccccgt aacatcccgc aaatttcaaa accgcgacgc cgccgaacac aaactccgaa    840
   tccaaaacgc gcgacccttt cctctacgac cgccgtttcc taaaaaaaac tacgcgaata    900
   taaaaaactt actcaatacg ctttataaaa tccactattc ctctttcgac cgcgaactac    960
   gccgataatc cgaaaaaccc gacttctcgc cccctacccc ttccttctct tcccttttct   1020
   cttttcctct cctatctctc cgcccacccg ccgcaacccc acaaaaaaaa aatcgcttcc   1080
   ctaattactc tcctctccta tcccttatat atccccttat cttccctcga tctacaaaaa   1140
   aaacgaaata acgactaaaa aacgtcaaaa aaaaaaaaca aaaacgtaaa ctacgaactt   1200
   acaaacgata cccgacttcg cgctcgacga aaactacgaa ccaacccgaa cccgcgctcc   1260
   gtccctccgc gatatctaaa aatcgcccga actaaaatcg cgtcaccctc cgcccaacgc   1320
   cgacgtaata aaacttacgc gatacaaaac ccccgaaaac ccaaaaaatc ccaaaacgct   1380
   acgaatccaa tatcccttta tttacaaaat cttaaaccaa taaaaaatac tccgcgaacc   1440
   ccaaaaaaaa acaacaaata cctcttaccc cacccgaact cctctaataa aaacccacac   1500
   gcacgataac gtcactacgc ctacaaacaa cgctaaaaca caaaaaaaat aaaaaatcga   1560
   aactcgaaaa cttcaccccg caacaaaact caaaacccaa tccaaacctt aaaaacccgc   1620
   ctaaaaccta aaacttaata taacaaaaat aaaaaaaacc tccgataatc tacttctaca   1680
   attaaaaaac gtcaactaac acctctaaac ctctaccccg taaataaaat aaaaacacac   1740
   gaaaccatct aaaaaactct cactatccgt caccaccaaa tataacccaa actccgtaaa   1800
   aaaaaacaat cccatcctaa aactaaaact tttaaaacat cccaaaaccc gataaaaaaa   1860
   acaaaaaaaa taaaacttaa aaattaataa cctcacattc aatatccccc gcacctcctt   1920
   ccctttctat accctctaat aactcttata acctcaacta cccccaaacc ctatctctct   1980
   cgccctcccc caaactaaaa tactaaaatt aaactataac ttaaactaca cctataccct   2040
   attaaaaaat aaaaatccca aacccatcgc gtttcttctc actactaact ccaactcaac   2100
   acttaacaaa cccccctccc caaactacta aaacaaactc gcgtaaactc gacctaaccg   2160
   ccgctcccga actaccgaat aaaccgaccg tttcgccgct tcaaaaacta tctttatttt   2220
   actccaactc aaacttaaaa aaacttaaac caacaacctc aaccgaaaaa acgaaacgac   2280
   gtctactaat tattattaaa actactatta ccattattat aataattaac gcaatattcc   2340
   caatcgataa cttttcataa aaaaaaatat cacttccttc ccatcacacc caaaataaat   2400
   aaaacgaaca acaaaaaaat aaaaaaaaaa aaaaaaaaaa aaatatatac ctccaacaaa   2460
   cccttaaact taaaaactat ataaccttat actaatcact acccctctct aaacaacaaa   2520
   aatctataaa tccattacgc tcatcctaca cccaaaaaac taatatttcc gaaacaccta   2580
   ctaaacaacg aaatacctta aaaaaaaaac aaaacaaaca aacctctaat cttctcacta   2640
   aacctaacta taatttataa aaaaaacctc ctttctcttc tcactttttt ctactaaaaa   2700
   cattaaacac attaaatcta acacgcccat cttataaata aaaaaaaaaa aatacaaaac   2760
   caataaccaa cttaataaaa aatacaacca tacaatacta aaacccctta ccccatctat   2820
   cctcttaacc cactttcatc tacttccaac ttaatatcta acaaaacaaa aacccactaa     2880
   cttccccgca tccacccact acctaacccc ataactacct tctaatccta actaaacccc     2940
   gtaaaacatc ttaaatttat cttcaataac tctcaaccac cgcccgacta ccttcacctt     3000
   cctaccttta catacctacc tttacttcgc caaattaatt aaaccctaac caaaactaac     3060
   cccctactct ctcaaataac aaataacaat tttttaaata aaatctcaat aaatatcaac     3120
   aaatctttcc tccttcctca aaattataaa aataacaccc cttattcaaa aataataaat     3180
   cctctaaacc ctccccgacc caaaccaaaa aaattaattc ataaaatata aaaaccatac     3240
   cccttcccga ccctccccct ctccccgcta ctacccaact ctctaaccaa ccccctccca     3300
   tccctaaacc taaatacctt aacaatcaac acaaacctac tcttattaac aaaataacaa     3360
   aaacgatata ctaaaaaact acacatctac ttaaaaaaaa aaaacaaatt tacatcctct     3420
   aaacacaaat aaaaaactta aaatctccct actcccatta aaaaaaaaaa aatctctaac     3480
   tctcaaaaat caacgtctct caaaaatcaa actaccctaa ttctaaatcc caactctacc     3540
   ataaataaac tctataaccc caaacaaatt actaaacctc tctaaacctc aaacattata     3600
   tctattatat ctaaataact aaaaaaattc aacaatacaa tattatataa aatacctaac     3660
   ccacaccaac caaaacacta atttaaatcg ataaaacata taacttaaaa acaaataaac     3720
   ctataaaaac atcctctact aaaaacaata acaaactaac atcttcaata ctaaaatttt     3780
   aaaatctaaa aattaaactt aactcaaaca aaaactataa aattaaaaaa aaatcaaact     3840
   cataacacaa aaaaaacctc aaacaatcct cctacccaac accctaccgc accccctctc     3900
   tacttaataa aaaactaaat cactaaaaac ccaaactcat cttctaacgc aaccacctaa     3960
   cctacctcga ccctcttccc aacaataaaa taaaaaaatc gcaaaaaaac ttttataaaa     4020
   taaactacta ttatcaaaat ttttctaaca ccattttcta actacacaaa actcaaaaaa     4080
   caaactaaaa ataaacctcc ccaaaaaaac acaaaaactt tttaaaaaaa aacttctaaa     4140
   aactacaaaa aaaaacctaa attataaaaa cacataatcc taaaaaaaaa aatacaatac     4200
   tataaaaata ttatttctcc tcaaaataat ttctaaattt aaataca                   4247
<210> 13
   <211> 3905
   <212> DNA
   <213> Artificial Sequence
<220>
   <223> chemically treated genomic DNA (Homo sapiens)
<400> 13
   tttaaaaaag tagattcgaa gatgtttaaa gtaataatgg tgttgttaga atggtgtttt     60
   tttttttttt taggggatga ttttgaagga tattataata atagatgtgt aaattttgaa    120
   atatatggtt aaaatgaggt ttattatttt ttagttatta tttagatatt ttttagttat    180
   ttgtttatga tagtttataa atgagaaaga agtttattta aaatattttt atgaggattt    240
   taaggtaaat aataggaatc gattatttat taatttggtt tggtttcggt ttattgaaga    300
   aagttttttt ttgagtgtat ttaaagtttt tatggtttag ttttgtttaa tttgagtagt    360
   attttatttg tttttttttt agacgtttcg atgttttgta ttagatttaa gagagagtta    420
   agttttttaa taggttatgt acgtggttat taatatttag tatacgttat ttttggttag    480
   tttttttttt tttttttaag aattaaatat tttttggttt gagttttttt tttttatgtt    540
   attataaagt tgagttattt attattaggg tttatatatg agtagttaaa atatacgggt    600
   ttttatattt aggttaaggg gtttttagta ggagaatatg aataattagg agtgaggtta    660
   tgagatagag gaagaaaagt ttataggaat gggaagatag ttttttagat gagaggtgtg   720
   cggagtgatt gttgtgagga attgtttaat aggaaagtta gttaatttga ggacgttttt    780
   aggggaggaa aagggaaatt tatatggttt tttgttaatt tttaaaaata aggtagattt    840
   ttatttattt agggtgtttg ttcgttatga atataggaag attaaggaat gagtatggat    900
   tgtttttatt ttatgttttc ggataagaag aataataaaa aagtattggg tagttggaat    960
   aattgtagag gttttaataa tgatggagat tttttggtgt atcgtggtat aggggttttt   1020
   tttaaaggga tttaattaat tttaataggt attatttttt gtgatttaag atgttttttt   1080
   taggagtgtt tgtatttttt aatgtttaga gaagttcgta ttattgattc gggaatattg   1140
   agttttttag tttttgtaaa attattttta ggtttatttt taagtatatt ttttatttcg   1200
   gggggttaaa ttttttggtt tttagtgaat atcgtggtta tatttgtggt tatttattga   1260
   ttttttattt gtttagttta tttgataata ggtaaatggg gaaagaggtt ttaatatttt   1320
   ttaaaagggg aagattgata attagttatt tatcgtatag aattaggtta ggttaatttt   1380
   ttttaagaag aaaagtaatt tggatattgt attttggggt ttttttttat tttggtatta   1440
   taggttttat gaggatgtag tttttttgta tttttttttt tttttttaaa ttgttattta  1500
   tttatttatt ttaattaggt taaagtatta tatattgtag aagagaaagt aattttagtt   1560
   ttttttaaac ggtgttattt ttatgatagt tcgtaaattt tggtttttta tgggtttagg  1620
   atttgcgcgt attcgttcgg ggatttgttg gtggatttat tttttatggc ggagtagtaa   1680
   ggggattttt tagaaaaagt aatgggcgaa gtaattgaaa gagcgacgta gaaagtaata    1740
   gttagaaacg gcggggacgc gagcggttta gataggaagg gaggcggtgg cgtagttttg    1800
   gtgcgtagcg cgtcgtagcg acggaatttt tgtaaaagtt gtttgttcgc gcgttattag    1860
   cggcgcgtag gtttgtggtt ttttcgtttt cgtaattttg ttttaattgt cggtttattt    1920
   ttcgataaat aggatgtttt tatttgaggt tgttagtatt ttaggttttt tgaaagaaaa    1980
   gaagtagagc ggttttattt tagaggtaag gacggggttt gtgttaagag gttttttaga    2040
   gaagtgaaaa ttttgtaggt gtagtcgttg ggagagtatt aagaagggta gggtggaggg    2100
   gtagggggcg aagggagggg gtgaagttcg tattttattt ttatatgaaa ttgattttat    2160
   tattttattt ttgtaagcgt ttagaggtag agaggttaat atttcgggga tagtttggag    2220
   gcgggagatt taggtagggt tttttaaatt tttatgtgta tgaaaattag gttaattacg    2280
   gggtttttga gtaaatgggg acgatgattt agtaggtttg ggttgaggtt ttagattttg    2340
   tatttttaat aagtttttag gtggtagtga tgttgttagt ttaaagatta tattgtgaat    2400
   agtaaggcgt cgtcgggata gtttgtttta gagtaaaatt taatagatgt gtaaatattt    2460
   aaattaagtt gtatttattt ttgaggtttt ggtttaattg gagttatttt gttaatggaa    2520
   aatggaaaat gaaatttttt taggttttaa agggaaggaa agtttgaaat ggttaaggta    2580
   tttaaaaaat tttagttggg agtggttttg gggattcggg agagttgaag ttaggttttg    2640
   agttaagaga tgggggagga agggtgggat gtggtaaata aatataaata tatatatata    2700
   aattttagtt taattatagg ttttttttta ttaatgaaat atttgtattt tgtaggtaga    2760
   atttatacgt gtttatattt tttagaagtt gaatggtttt gtgtaatggt atagaatttg    2820
   gagtttatat ttgaatattt tagatttaat gttattttag attggtttta ggattttgtg    2880
   gtttatattt aaaaatgtat gtttttgagt tatgtttaat tagtagtagt atttgagaat    2940
   tgagagatta gattaagaaa tttatggtat atgaataaaa tttttagata gtagttagaa    3000
   tatagaggtt tttttagagg tagtgttagt tgttagttgt aaaaataggt tttgtttagt    3060
   tttgtttagg tttgaagttt tttgaagatt ttatttagat tttattaaaa ttagttaaag    3120
   attttttgtt atagaaaggt ttttaaagta tggttgggat tatagggtta ttaagatgta    3180
   gatttttggg tttttttgtt ttatttatta tggtttttta gggtgggtgt cgggaatttg    3240
   tattttaata aacgttttga ggatttttat gtattgttaa gtttaggagt ttttggtttg    3300
   taggtttaag tttaattttt ttattgtagt atttaaagat ttggttttaa ttttgttgtt    3360
   tataggcgag ttttagtgat ttttttattg ttttttgagt atttttggag ttatttggtt    3420
   gttttattta tttttttttt tttgtttttt tttagttttg gaataaaatg atttttattt    3480
   tttttttcga atttttttta gattatggtt ttttatttag taagtagtta tttttttggt    3540
   ttgttattgt tgtggtatta atgataggtt attcgatttt tattttgttg attgaatatt    3600
   ttttatgttt aggttttgtt tttttgatta gatagtaagt tttttgaagt tattttatgt    3660
   tatataattt ttatgtttaa gattattttt taacgtagtg ttatgtttat atggattatt    3720
   tagtaaattt atcgatggtt tatttattat taatatgttt tatagagtgt tattaagtag    3780
   ggatattttt tatgggttta ggattagaag aagttgggaa gagaaatggg gtagtggtga    3840
   gagtgttatg agaatttggt tatatagtta ttaaagatat agaggaagtg ggaaaaaggg    3900
   agtgt                                                                3905
<210> 14
   <211> 3905
   <212> DNA
   <213> Artificial Sequence
<220>
   <223> chemically treated genomic DNA (Homo sapiens)
<400> 14
   atattttttt ttttttattt tttttgtgtt tttgatgatt gtataattag atttttatga 60
   tatttttatt attattttat tttttttttt agtttttttt gattttagat ttatagagag 120
   tgtttttgtt taataatatt ttgtaaaata tgttggtgat agataagtta tcggtaaatt 180
   tattgaatga tttatgtgga tatgatattg cgttagaaag tagttttgaa tataaagatt 240
   atatagtatg aaatggtttt agagaattta ttgtttggtt agggaggtaa agtttaagta 300
   tgagaaatat ttagttaata aaatgagaat cgagtggttt attattaata ttatagtagt 360
   ggtagattag gaaagtaatt gtttgttgag tgggaagtta tgatttgaag agagttcgga 420
   gaggaagatg gaagttattt tattttaggg ttggggaaag atagaggaga agaaatgaat 480
   gaggtagtta ggtggtttta ggagtgttta gagggtagtg aggaagttat tgaggttcgt 540
   ttgtgaatag taaaattgaa gttaggtttt tgaatgttgt agtgagaagg ttgaatttaa 600
   atttgtagat tagggatttt taaatttggt agtatataag aatttttaaa gcgtttgtta 660
   aaatgtaggt tttcggtatt tattttgaga aattatgatg agtgaggtag agaggtttaa 720
   gaatttgtat tttaatagtt ttgtagtttt agttatattt taagaatttt tttgtaatag 780
   gaagtttttg attggttttg atgaagttta aatgaaattt ttaggaaatt ttaggtttaa 840
   gtaaaattga gtagaattta tttttatagt tgataattgg tattgttttt ggagggattt     900
   ttatgtttta gttgttgttt ggagatttta tttatatatt atggattttt tagtttagtt     960
   ttttagtttt tagatattat tattaattaa atatgattta gaaatatgta tttttaaatg    1020
   taggttataa gattttaaag ttagtttgag ataatattgg atttagaata tttaaatatg    1080
   ggttttaaat tttgtattat tatataaaat tatttaattt ttgaaaaatg tggatacgtg    1140
   tgagttttat ttgtaagatg taaatgtttt attgatagga aagaatttgt ggttgggttg    1200
   gggtttgtgt gtgtgtgttt atatttattt gttatatttt attttttttt ttttattttt    1260
   tggtttagaa tttaatttta attttttcga atttttaagg ttatttttaa ttaggatttt    1320
   ttaaatattt taattatttt aaattttttt tttttttgga atttaaagga attttatttt    1380
   ttatttttta ttagtaaggt ggttttagtt aaattagggt tttaggaata aatatagttt   1440
   ggtttaaata tttgtatatt tattaggttt tattttaaag tagattgttt cgacgacgtt    1500
   ttgttattta tagtgtggtt tttggattgg tagtattatt attatttggg aatttgttag    1560
   aagtgtagaa tttgaggttt tagtttagat ttgttgaatt atcgttttta tttgtttaag    1620
   agtttcgtga ttggtttgat ttttatgtat ataaaagttt aaggagtttt gtttaaattt    1680
   ttcgttttta agttgttttc gaaatgttgg tttttttgtt tttggacgtt tgtagagatg    1740
   gggtagtgga attagtttta tgtgggggta gggtgcgggt tttatttttt tttttcgttt    1800
   tttgtttttt tattttgttt tttttgatgt ttttttagcg gttgtatttg tagagttttt    1860
   atttttttgg gaagtttttt gatatagatt tcgtttttgt ttttagggtg gggtcgtttt    1920
   attttttttt ttttaaagag tttgggatgt tgatagtttt agatggaggt attttgtttg    1980
   tcgaaaaata aatcggtagt taaagtaggg ttgcgagagc gagaaaatta taggtttgcg    2040
   cgtcgttgat aacgcgcggg taggtagttt ttgtagaagt ttcgtcgttg cggcgcgttg    2100
   cgtattagag ttgcgttatc gttttttttt ttgtttgggt cgttcgcgtt ttcgtcgttt    2160
   ttggttgttg ttttttgcgt cgttttttta gttatttcgt ttattgtttt ttttaaagga   2220
   tttttttgtt gtttcgttat gagggatgga tttattaata agtttcgggg cggatgcgcg   2280
   tagattttgg atttatagga ggttaaaatt tacgagttgt tatgaaaatg atatcgttta    2340
   agagaagttg aagttgtttt tttttttgta gtatgtaatg ttttgatttg gttaaaataa    2400
   ataaataaat aataatttaa aaagaaagaa agaaatatag aaagattata tttttatgag    2460
   gtttgtaatg ttaaaatgaa aagggatttt aaagtgtaat atttagattg tttttttttt    2520
   tggagagagt tgatttggtt tgattttatg cggtgaataa ttaattatta attttttttt    2580
   tttgaggggt attaaagttt ttttttttat ttgtttgtta ttagataagt taggtagatg    2640
   agaagttagt gggtaattat aggtataatt acgatattta ttggaaatta aaagatttga    2700
   tttttcgagg taaagaatgt atttgaaaat aaatttgaaa atagttttat aggagttgaa    2760
   aaatttagtg ttttcgaatt agtaatacga atttttttga gtattaaaaa gtataagtat    2820
   ttttaaaaag gatattttga gttatagaag atgatgtttg ttggggttga ttgagttttt    2880
   ttggaagaga tttttgtgtt acggtgtatt agaaagtttt tattattgtt ggggtttttg    2940
   tagttgtttt agttatttag tgttttttta ttattttttt tgttcgaaaa tatagagtag    3000
   aaatagttta tatttatttt ttaatttttt tgtgtttata acgagtaagt attttagatg    3060
   agtaagaatt tattttattt ttaagaatta gtagagaatt atatgagttt tttttttttt    3120
   tttttagaaa cgtttttagg ttggttaatt tttttattga gtaatttttt atagtaatta    3180
   tttcgtatat tttttatttg agaggttgtt tttttatttt tatgagtttt tttttttttg    3240
   ttttatggtt ttatttttaa ttatttatgt ttttttgttg aaggtttttt gatttaaata   3300
   tagagattcg tatattttgg ttatttatgt atagatttta ataatgaata atttaatttt    3360
   atggtgatat aaaggaggaa aatttaggtt aaagaatgtt taatttttag ggaagaagga   3420
   gagagttggt tagggatagc gtgtgttggg tgttaatagt tacgtgtata gtttgttggg    3480
   gaatttggtt tttttttgaa tttggtgtag ggtatcggaa cgtttggggg aggagtagat    3540
   aggatgttgt ttaggttgaa tagggttgga ttatgagagt tttgaatgta tttaagaagg    3600
   aatttttttt agtgggtcgg ggttaggtta gattgatgaa tgatcgattt ttattattta    3660
   ttttgaggtt tttatggaga tattttaaat ggattttttt tttatttata aattgttata    3720
   aataaatgat taaaagatgt ttaagtggtg attagaaaat aataaatttt attttagtta    3780
   tatattttag aatttatata tttgttattg tagtgttttt taaagttatt ttttgggagg    3840
   gagagggagt attattttaa tagtattatt attattttaa atattttcgg atttgttttt    3900
   ttgaa                                                                3905
<210> 15
   <211> 5338
   <212> DNA
   <213> Artificial Sequence
<220>
   <223> chemically treated genomic DNA (Homo sapiens)
<400> 15
   ggtaattttt tttgttatta ttttttgtat aaaatatgta agatgttttt attttattag     60
   tattgaaatt aaagattaaa agagagaaag gtgttggatc gaaaaggagg cggcgtaacg  120
   gttatttttt ttagtatttc ggttttgttt ttttcgggga aggcggttat atttttattc  180
   gttttgtttt tgaacgtagt aaataatttt ataaataatt atcgttgttt acgtttttta  240
   ttcgtttttt cggttttatt agattttcgt tttttcgtat ttttcgggta gggtttttaa  300
   taagtttagg ttgaaagaac gtttgttatt ttttttattt tcgttgaaag aaaaggaaga  360
   aaaatagtag tagcgagaaa ttttcggggc gatttttttt tcgtttttaa gtattagcgt  420
   atagtatttt tttttgtttt tgttgtggtt tttcgttgtt tcgggttacg tcgtttagtt  480
   aagtaattcg gatttgagag tgtatagtta ggattagttt agggggcgag gggttggttt  540
   ttgggaaatt aagcgtttag gatagaggtg gaaagtgggt ttcgggagtt agaaaagaga  600
   gagaagggta gacggttggg tggtaaatat aaaaatagaa ataatttagg gggatgttcg  660
   ttaggttttt gcgtttgttt tttttttttt aattcggagt aggttttttt cggtttttcg  720
   cgtttcgggg cgttttttgg cggtagcggt agtagcgggt aacgcgcgga gggtttaggg  780
   ggcgtatagg ggattttcgg gtatatttag agaggcgggc gcggtttttt ggcggtggcg  840
   acgtagttat ttggtgagcg gagtttcgtt tttttggttc ggcgggttta cggtcgtttt  900
   attaagtatc gcggtcgagt agggtaggaa gtcgttttcg gaacgcggcg tcgtcgcgtt  960
   gtagtcgaag ttcgagtttt tttcggtttc ggcgttttcg tcgtcgtcgt tattattatt  1020
   atcgtcgtcg ttatcgttat ttcgggaatt tagggttgcg gtagttgttg tcgcggttgt  1080
   cgtcgtcgat tgattgttgt ggtaattgag gtacgagtag ggtgtagagt cgtcgttggg  1140
   gggcgcgtcg gtcgtcgtcg tcgaggaggt cgtagtcgtt gcggttgtcg cggttgtcgc  1200
   ggtagaggtc gcgttgttga gtttcgcggc ggtcgcggga gtttggtaga gattagggtg  1260
   gcggaagtta tatagtagtt tcggtcgaga gtaggggtgc gagagggcgc ggaaggtgtt  1320
   tagtggtcgg atggaagtag cgaagggcga cgaagtcgcg gtcgtcgcgt ttgaggttgt  1380
   agtcgcggtc gtcgtcgtcg tgacgtttac gtgcgggtag tagtgtagcg gtacgtgcga  1440
   gtggaagggg tagggtaggt tttcggtggc ggtcgcgtgc gttattatgt aggtgtagaa  1500
   gttggggtcg gttgggtgcg gttaggatat ggttaggcgt tgtcgtttgt tttttatgcg  1560
   tcggttttgg aattatattt gcggggagag acgcgtcgta gtttgggtta gggagcgttt  1620
   cgtgttttta gtttttgttt taggattttt gtttttttcg gatttttgaa tggtttggtt  1680
   tatttttttt cgattaagtt taatttcgag tattttgtgg ttttttagtt gggaaagtgt  1740
   ggacggtagt gtgtggatcg tcgtgggtat atcgttttta acgaagaggg tttttttttt  1800
   cgcgttcggt tgttgttgtt ttttaggttt ttattttttt attttttgtt gtattttttt  1860
   gttttaattt aatttttttt ttttttttcg ttatttatta gtgtcggttt cgttgagtat  1920
   tcgtttttta attttaggat ttgtacgggg attttgggta gtttttgaag agaggttgtc  1980
   gtttagtttt tttgagaggt cgtcgcgcga agttttgagt ttttcgaatt gtaaggattt  2040
   gtttttaggg gtacgggttc gattttgata ttgaagggat gattttgttg gaatcgtttg  2100
   ttttaaatga gtgggtagag taatgttttt ataaacgggg aaggggacgt ttttattttt  2160
   tttaatatta tttaataaag atattattcg ttataatttt aaattaaaga aagttcgatt  2220
   aaattagagg gagattttta tatttttttt ttatttcgtg gagatttttt tttttttttg  2280
   tagtgtcgaa cgttttatga agggtttatt aaatcgttta atttttcggt tatttttttt  2340
   agacgtataa taaaattaat aatttaaagt tatatataaa taaggataat ttcgttgtat  2400
   tttttttcgt aggaggttgt ttttttttcg ttgtttaaga ggaaaatgtt taggaaatta  2460
   ttgttttaaa ttaatcgatt ttaaagatat agtatttttt ttttcgtgta acgatttatg  2520
   cggaaaataa atttttaagt ttaagagtaa atgaaaagtt ttatttttgg tttttgtttg  2580
   aggaataaag attaattggg tttgtcgttt aggggaaagt ggggtcgtgg gtatgggcga  2640
   gggggtattt ggttaggcgt tgggtataat ggagtagggg cgagtgtttt tagtattgga  2700
   gttattattc gggggttttt ttagattcgt taggtcggat aatcgttcgg attcggtggt  2760
   cgggaaataa ataagttaat ttttttggtg attatttttc gcggattttt agatttttag  2820
   ttaaatttag ttatttagaa aggggaaagg ggaaaaagaa ataattaatt tagatgtttt  2880
   cggggaggtt agagtaggta tgtattggaa ttgatatttt gatggtggtt tcgggtaggt  2940
   tgagtgtcgc ggttagttcg tatcggcggg gtcgcgatat atagtttttt cggtagaatt  3000
   ttttttttag gcgcgcgatt tgttcgcggg tgaacgtcgt acggtagcgt cgtatttgat  3060
   tcgcgttaga gtcggagtta tttagcgtcg cgttttcgtc gttgttttcg ttgtttttat  3120
   gtaggttttc gaggtttgag ttcgacgtcg acgtcgtggt gtcggtagtc gagtcgtttt  3180
   ttgcgtattt tggtaaataa acgattaata gcgtatgagt ggttgtagga ttaatagttc  3240
   ggcgttggcg ttgcgcgcgg atcggggaag tttcgttagg aaggagagtc gttgtcggaa  3300
   ttgatggggt ttgttatgtt tataaattgt tgtcgttaat ggaattaata aagtttgggg  3360
   agttttttat aagtaaataa tagaaacgga attaggagat ttttttttta ataattagaa  3420
   atttttaatt aaggaggaaa agtggtcgag ggaaaatgtt tatttttggg cgtagtttgg  3480
   gaagttttgg gttttttatg gtttggagat cgtagggtag ttttttatac ggtttttaat  3540
   ttttatttga gttttcgggg ttttaaatat tttaagagtt ttaatatagt agtagtttaa  3600
   atttaagtta atagggggtg aaatatattt tttaattttt tttttcgttg atttaaatag  3660
   agacgtcggc gaggtttttt tcgatttatt tagaagaatg ttttttttag ttttgagggt  3720
   ataggggtag gggagtttag agtagttgtt tagggtattt tttttaaaag taattcggtt  3780
   gttgattagg ggtttatcgg ttcgttcggg tttttagata cgagcgtaga aatatttttt 3840
   cggatttagg agcgagggcg ggtgggtttg gtgttttagg tttcgtaggt ttgagtttgg 3900
   cgggaaagtt taaggagtag aattggaagg tacggtttta gatatgcgtt tcgttttcgt 3960
   tcgtgttagt tcggtgtagt ttgtttgtgg agggtttgag aggggaaaag gtatcgggaa 4020
   aggttggcgg gggtcgcgga ggagtaaaga ggatgggatt ggagagcgcg gtgcggtcgg 4080
   cgcggttatt tttgttattg ttttttttaa gttgagcggc gtcgaggttt tcgggggagc 4140
   gaagcgcgga gtagtttatt tttacgtcgt tgtttatgtc ggttttagcg gtcgtttttg 4200
   aataatggtt cggttttttg cggttttcgg cggcggagga gatttcggag gagacggtgt 4260
   tttcgttgtt cgtgtgttgt aggttgaata aagtgtttat ttcgaatttg tttttggcgg 4320
   ggagtttttt tagagcgttg tgtagggggg tagacggtag gcgcgggttt aggcgagtcg 4380
   ggtgttgcga attttttagg gtttcgagta tagtattgtt agtcgagttg gataaattgg 4440
   agaatttttt gttcgtcgta gggttgtgta gttttttttt tattagaatt attttttttt 4500
   ttattttttt tattatttta gtttttttaa aaatgttata gtggttttgt tgtttcgttt 4560
   taatgatagg ttgcgtttag ggttaatttt tatttagttt tagcgaacgt ttttaaatat 4620
   tattatcgtt ttcggaggga ggcggaaaaa ttgtgggatg ttagagcgag ggaatgattg 4680
   gttaaaatga tttatatatt ttttcgattt cgagatgtta tttattaaaa agtagcgttc 4740
   gttcgttatt aaggtacgaa tgacgttgtt cgaataatta tttattgtaa taggtttata 4800
   agtaaataaa tatatttttt tgttagtggg taatgaaggt agttttagag tagataaata 4860
   gatttaggta ggaggcgaga agagataagt gaggaggaag gtttcggttt tttgttcgtt 4920
   tcgagttagt tttgttcgtt cgggggtttg gtttcggggg tgaaattgat agtttgatta 4980
   atagagcgcg tcgcggtata tttttttttt tatttagggg tattattacg tttttagttt 5040
   gttaggttgt tttttaggtt ttaattatgt agcgtttttt ttattttttt ttggatatag 5100
   atacgtatta aataatagat aatggtttga ttttttagag taggttttcg ggaaggtttg 5160
   gttttttttt tttttttttt tttttttttt attatttata agttgggggt ataagtgggg 5220
   ggagataagg gggaggatgt aggaagagtt gcgtaaggag aagattttgc gtttttttta 5280
   tttggattat ttatttttcg gatttcggaa ttaaatatgt attttattta ataaatag 5338
<210> 16
   <211> 5338
   <212> DNA
   <213> Artificial Sequence
<220>
   <223> chemically treated genomic DNA (Homo sapiens)
<400> 16
   ttatttgtta aataaaatat atatttggtt tcgaaattcg aaagataagt aatttagata      60
   ggagaggcgt aaggtttttt ttttacgtaa ttttttttgt attttttttt ttattttttt     120
   ttatttatgt ttttagtttg taaatggtaa aaaaaaaaaa aaaaaaaaaa aaaaaaatta     180
   aattttttcg aggatttatt ttggaaagtt aaattattat ttattattta atacgtattt     240
   gtgtttaagg aaaaatgaag aaggcgttgt atgattagga tttaaggggt aatttagtaa     300
   attgagagcg taatgatgtt tttaaatgaa gggggaaatg tgtcgcggcg cgttttatta     360
   attagattgt taattttatt ttcgaggtta aattttcggg cgagtagaat tggttcggag     420
   cgggtaaagg atcggagttt ttttttttat ttgttttttt tcgtttttta tttggattta     480
   tttgtttgtt ttgaagttgt ttttattatt tattgatagg agggtgtatt tatttgttta     540
   taaatttgtt ataataaatg attattcgaa tagcgttatt cgtattttaa tgacgagcgg     600
   gcgttatttt ttgatgaatg atatttcggg atcggaagga tgtatgggtt attttgatta     660
   gttatttttt cgttttggta ttttataatt ttttcgtttt ttttcgaaag cgataataat     720
   atttagaggc gttcgttggg gttgaatgag aattagtttt aggcgtagtt tattattagg     780
   acgggatagt agggttattg tgatattttt aagaaagttg agatgatgga aagaataaga     840
   aaagagatga ttttgatgga gagagggttg tatagtttta cggcgggtaa gagatttttt     900
   aatttgttta attcggttgg taatgttgtg ttcgaggttt tggaaaattc gtagtattcg     960
   gttcgtttaa gttcgcgttt gtcgtttgtt tttttgtata gcgttttggg agaatttttc    1020
   gttaagggta aattcgaaat agatattttg tttaatttgt agtatacggg tagcgaaagt    1080
   atcgtttttt tcgaaatttt tttcgtcgtc gagagtcgta agaagtcggg ttattattta    1140
   gaggcggtcg ttgaggtcga tatgagtagc gacgtggagg tgggttgttt cgcgtttcgt    1200
   tttttcgggg gtttcggcgt cgtttagttt aaggaaaata atggtaaagg taatcgcgtc    1260
   ggtcgtatcg cgttttttag ttttattttt tttgtttttt cgcggttttc gttagttttt    1320
   ttcggtgttt tttttttttt tagatttttt ataggtaagt tatatcgagt tagtacgggc    1380
   gggggcggga cgtatgtttg ggatcgtgtt ttttagtttt gttttttgag ttttttcgtt    1440
   aggtttaggt ttgcggagtt tggaatatta ggtttattcg ttttcgtttt tgagttcgag    1500
   aaaatgtttt tgcgttcgtg tttaagagtt cgagcgagtc ggtagatttt tagttagtag    1560
   tcgagttgtt tttgggaaag gtattttggg taattatttt gaattttttt gcttttgtgt 1620
   ttttagggtt ggagggggta tttttttggg taagtcggag gaagtttcgt cggcgttttt 1680
   gtttaggtta gcgggagaaa gagttgaaaa gtatatttta ttttttattg gtttgggttt 1740
   gagttattgt tgtgttggaa tttttaaaat atttgaaatt tcgaagattt agataaaggt 1800
   tagaggtcgt ataaaaagtt gttttgcggt ttttagatta tgggaaattt agagtttttt 1860
   aaattgcgtt tagaggtagg tatttttttt cggttatttt ttttttttgg ttgaaaattt 1920
   ttaattatta aaaaagaaat tttttaattt cgtttttatt atttgtttat gaagggtttt 1980
   ttaaatttta ttgattttat taacggtagt aatttgtaag tatgatagat tttattaatt 2040
   tcggtagcga tttttttttt tgacggggtt ttttcgattc gcgcgtagcg ttagcgtcgg 2100
   gttgttggtt ttatagttat ttatgcgttg ttggtcgttt gtttgttagg gtacgtagag 2160
   agcggttcgg ttgtcggtat tacgacgtcg gcgtcgggtt taggtttcgg aagtttgtat 2220
   ggaggtagcg gaggtagcgg cgggagcgcg gcgttgggtg gtttcggttt tggcgcggat 2280
   taagtgcggc gttatcgtac ggcgtttatt cgcgagtaga tcgcgcgttt ggagaaggag 2340
   ttttatcggg agaattatgt gtcgcggttt cgtcggtgcg agttggtcgc ggtatttaat 2400
   ttgttcgaaa ttattattaa ggtattaatt ttagtgtatg tttgttttgg ttttttcggg 2460
   ggtatttggg ttgattgttt tttttttttt tttttttttt ttgggtggtt agatttagtt 2520
   aagggtttgg aaattcgcgg ggggtagtta ttaaaggaat tggtttattt atttttcggt 2580
   tatcgaattc gaacggttgt tcggtttgac ggatttaaga aggttttcga atggtgattt 2640
   taatgttgaa agtattcgtt tttgttttat tgtgtttaac gtttggttag atgttttttc 2700
   gtttatattt acggttttat ttttttttag gcggtaagtt tagttggttt ttgtttttta 2760
   agtaggaatt agaggtgaaa ttttttattt gtttttgagt ttggagattt atttttcgta 2820
   taaatcgtta tacggagaaa aggatattgt atttttaaaa tcgattggtt tgagatagta 2880
   gttttttgaa tatttttttt ttgggtaacg aaaaaagggt aattttttgc ggggaaaaat 2940
   gtaacgaaat tgtttttatt tatatataat tttaggttat taattttatt atacgtttgg 3000
   gagagatagt cgggaggtta ggcgatttgg tgagtttttt atggagcgtt cgatattgta 3060
   gaaaaaagaa aaaattttta cggggtaggg gaggagtgtg gaagtttttt tttggtttga 3120
   tcgggttttt tttaatttag agttgtgacg aatgatgttt ttattagata gtgttgggag 3180
   gggtggaaac gttttttttt tcgtttatgg agatattgtt ttatttattt atttaaggta 3240
   aacgatttta ataaaattat ttttttaata ttaaaatcgg attcgtgttt ttaggggtag 3300
   gtttttgtag ttcggagggt ttaagatttc gcgcggcgat tttttagaaa agttgagcgg 3360
   tagttttttt ttaaaggttg tttagaattt tcgtataaat tttgggattg agagacgggt 3420
   gtttagcgag atcggtattg gtgggtggcg ggagggagag gaaaggttgg attgggataa 3480
   aaaagtgtag taagaagtaa gggaataaaa gtttgaggag tagtagtagt cggacgcggg 3540
   ggagaggatt tttttcgttg aggacggtgt gtttacggcg gtttatatat tgtcgtttat 3600
   atttttttag ttgggagatt atagggtatt cggggttggg tttggtcgga gagaagtaga 3660
   ttaagttatt tagaggttcg gaaggggtag aggttttggg ataggagttg ggaatacggg 3720
   gcgtttttta atttaggttg cggcgcgttt tttttcgtag gtgtggtttt agaatcggcg 3780
   tatgaaggat aagcggtagc gtttggttat gttttggtcg tatttagtcg attttagttt 3840
   ttatatttat atgatgacgt acgcggtcgt tatcggaagt ttgttttatt ttttttattc 3900
   gtacgtgtcg ttgtattatt attcgtacgt gggcgttacg gcggcggcgg tcgcggttgt 3960
   agttttaggc gcggcggtcg cggtttcgtc gtttttcgtt atttttattc ggttattgga 4020
   tatttttcgc gttttttcgt atttttattt tcggtcggag ttgttgtgta gttttcgtta 4080
   ttttggtttt tattaggttt tcgcggtcgt cgcggggttt aatagcgcgg tttttgtcgc 4140
   ggtagtcgcg gtagtcgtag cggttgcggt tttttcggcg gcggcggtcg gcgcgttttt 4200
   tagcggcggt tttgtatttt gttcgtgttt tagttgttat agtagttagt cggcggcggt 4260
   agtcgcggta gtagttgtcg tagttttggg ttttcggggt ggcggtggcg gcggcggtgg 4320
   tggtggtggc ggcggcggcg ggggcgtcgg ggtcggggga ggttcggatt tcggttgtag 4380
   cgcggcggcg tcgcgtttcg agagcggttt tttgttttat tcggtcgcgg tgtttagtaa 4440
   gacggtcgtg agttcgtcgg attagaggga cgaggtttcg tttattagat aattacgtcg 4500
   ttatcgttag ggggtcgcgt tcgttttttt gagtgtgttc gggagttttt tgtgcgtttt 4560
   ttgagttttt cgcgcgttgt tcgttgttgt cgttgtcgtt agggggcgtt tcggggcgcg 4620
   ggaggtcgaa gggaatttgt ttcgaattgg gggagaagga gtaggcgtaa aagtttggcg 4680
   ggtatttttt taaattattt ttatttttgt atttgttatt tagtcgtttg tttttttttt 4740
   ttttttttgg ttttcgggat ttatttttta tttttgtttt gagcgtttgg ttttttaaag 4800
   attaattttt cgttttttaa gttagttttg gttgtgtatt tttaggttcg ggttgtttgg 4860
   ttgaacggcg tggttcgaag taacggagaa ttataataaa gatagagggg gatgttgtgc 4920
   gttggtgttt gggggcgggg gaggagtcgt ttcggaggtt tttcgttgtt gttgtttttt 4980
   tttttttttt tttaacgagg gtgggggagg tggtaaacgt ttttttagtt tgagtttatt 5040
   agggattttg ttcgaagagt gcgggagaac ggaggtttga taaggtcggg aggacggatg 5100
   gagagcgtgg gtagcggtgg ttgtttgtga gattgtttat tacgtttagg agtaaggcgg 5160
   gtagggatgt ggtcgttttt ttcgggaagg ataaagtcga ggtgttggaa ggggtggtcg 5220
   ttgcgtcgtt tttttttcgg tttaatattt tttttttttt tggtttttgg ttttagtgtt 5280
   gatggaatgg aagtattttg tatattttat gtaaaaagtg atggtaaaag aggttgtt 5338
<210> 17
   <211> 5332
   <212> DNA
   <213> Artificial Sequence
<220>
   <223> chemically treated genomic DNA (Homo sapiens)
<400> 17
   ttagttttaa attttgtatt cgtatttttt agataggaag tttttttttt tcgattaggg 60
   attgagggag aagagattta gaatgtattt ttttgtttta ggtttttcgg gggggatatg 120
   gttttagata gttatttcga aattgggttt aggtttttat agtaagtcga gtgttttttt 180
   atatttttaa tttcggtttt tttattaaag tttaaaagat ttttttgttt tttaggtttt 240
   tttcgtttgg ttgtttttat tattttttaa ttgcggaaat attagttatt atatgttcgt 300
   cggagaataa gcgtgagtgt cgcggtggat agttgtcgtt aaaggtgttt agacggatgg 360
   tatttttaaa gtagggattt tagtattttt tttatatatt attatttaag aatggtagag 420
   gaaagagtag gagtggggtt atagatagga gagaaaggag gttgaggtta taaaattggt 480
   atttttttaa ttattaattt ttttttttat tattttgtta ttatttgtta tggtaaagtt 540
   ttttttttgt gttttttttt taataaagaa atttttagtt agtttttatt tatttttttt 600
   gatttgtagg aagttaaatt gttagtagat tgagtttttt aagggatatt tttgggatat 660
   gttggaattt tgatttgttt aagatgatga cggttattag tataaattag aaataatgtt 720
   attttttagg gtgttatttg taagtgtaat aatacggatt tatttgtaaa atttagttaa 780
   attgcgtttg gataagtgat tagatttaaa cgataaattt tttttattat ttagtttttt 840
   ttaagttttg cgggggtagt atagaagatt gttttagggt gaaattaagg tttagtttat 900
   tatttgtgtt agtttgaggg attttggaaa tgttgatttt gagaaaaaga taaaataaaa 960
   taaaataaaa taaaatttaa ttataaaata tattttattg tttataaagt aaatagaaat 1020
   ataaagttgt ttttaaaatt atttttttag taaataagta tgtgatattt tgagtaggag 1080
   taggagggag agaaagattg ttatttaaaa aaatatgaaa gtattaagta ataatttaaa 1140
   atacggttgt ttagaaaaat atttatattt attgtagttg tttaattggt ttcgttaaag 1200
   tcggtaagta tttaaattgt gttataattt tatttaaatt tcgtttcgtt ttagtaggtt 1260
   gaagagtttg aatagattaa ttattttata atgatgatga atgagaaatt aatttagata 1320
   taagtaagat aatttaggtt tttatttgtt taaatagttt tttaatatta ttcgttatcg 1380
   cgggttatag attgaattaa ttgtttaatt ttgtgaaagt tatatttttg cgtttttatc 1440
   gagattattt atagcgtagt gagcgttgtt gaaaggtata cgttgttaat agggataatt 1500
   atttaaaggg aagcgtttgt aaaatggaaa ataaatgtcg gggttggtaa taaatgggat 1560
   taaaagtagt tattttaatt tggtttttcg agggaaagga gcgggcgtgg ttttgtagga 1620
   ttaggggcgt ttttgatttt tttaagattt aggtgaggtc gagggttttc ggcgcgttag 1680
   tttgcgtcgt ggttgtggtt gcggtggcga ttcgggtcgg gtttcgtttt cgtcggtttt 1740
   aggttttcgt ttttttcgcg taggtagcgg gcgcgtgtgg ttcgggttgg gtaagtcgag 1800
   gaatagcgag ttttcggacg ttgattgtag gacgttttag tttgtgttcg ggttttcgtt 1860
   ttttttcgta cggggttcgt attttcgggt ttgggtttga tttatagggc gttgaggttt 1920
   ttgtagttga agtcggaagg tttcgttgcg agcgcggtat aggttgttgg tagtttttgg 1980
   attttggagg tttggttttt tttttaagtc gatggcgggg aaagaatttc gtttttatag 2040
   ttttttcgat tttcgtcgtt tgttatttgg ggacgggaag cgcgttcggg tcgttttacg 2100
   tttttttggg tcggagtttt ttttatggtt ggtttttttg ggggtttttg ggtttgtgag 2160
   tagcgtttat tttttttaga gaagaatttt tttttttttt tatcgaagtg tttttttttg 2220
   tattttgaaa taattttttt tgggtgaggt tagttttttt ttgtcgtttt tttttcgtag 2280
   gcgttcggga gtttcgtgag gatttcgtgt agttgagttt aggcgatagg tgttttttta 2340
   ggtgtttatt tgtttttttt taatttgttt agggaaagat tagaacgatg tgcgcgggga 2400
   ggtggttttg tttttttcga aattggcgtt tttgggtaaa atcgcgtgtc gcgtttacgc 2460
   gggttttgtt agtgtgcgtg ttttagaaga gtgggttttt taggcggtat tttttggaga 2520
   agtaagtgag gtgaatttag aatagagaag cgggaacgat cgtttgatgt gtcgagtcgg 2580
   aaaaagagag gagagggaga ggtttttagc gagcgcggag atagaggagt ttacgcggta 2640
   tagtacgagt tttatttttg ttcgcgtaga ggtgtgtgtg tgtgtgtgtg tgtgtgtgtg 2700
   tgtgtgtgtg tgtgtgtgtt tttttttgta tttttgtgtt tgaaataaaa gtttaattaa 2760
   cggatttttg gtgttttgag gatttatttt cgggagcgat tttgatgtat tgttgtttaa 2820
   ttagtgtttt taattggtgt tttcggagat aggtaggttc ggagttggga tagagttttt 2880
   tttcgttttt tttgtatatt ggaaaggtaa aatttataat atattgtggt tattttttgg 2940
   gagagcgaaa gagagtggga ggtagggaag aaagggagag atgtagttgt ttgtagagga 3000
   cggagttttt agtttcgggg gtcgtgggga agggtttaga ttatcgggtt cgatttagtt 3060
   ttttgtgtaa gagggttcgg gtcggttgag tcgttgagga ttatgaattt gttcgtagaa 3120
   aggtttaggg agtttttatt gtttgtataa atttatttta tgtggtttat tatttcgtta 3180
   ataatggttg tttgttaata aggatgcgga ggttaaataa ttcgggtgga agattaaata 3240
   cgttttatta aggcgtttgg aagtcgcgcg gtgtagagat ttgcgttgga gtcgtttggt 3300
   ttattggagt ttggagggag atgggagtat agtttgtaag gagtttcggg gtagaagagg 3360
   aggtaaggtt tagttttttt ttttagggtg ttttgttatg ttaggatagg tttaattttt 3420
   ttatatgtta aatagaaaat ttatatagaa gttagtttta atgaggtgtg gaggttaaat 3480
   tttcggagta gttggttgga gggtagatat ggagtatttg gggtttgaat taaaggtttt 3540
   tttttgagga ggggtggtta ttttttgttt tttagttttt atatgttatg tatttggtaa 3600
   tttgtgttta tatgggatta gtattggatt agaagataga agatgtggtt tttagtttgg 3660
   gttttgtttt taaattattg tagaattttt attatattat ttttttagtt ttattttttt 3720
   attgtgtaag agaaaggaga tggggtttga tgtagatgat gtatagtatt tttttttttg 3780
   tttagttttt tattagagtt agggaattga atattttata agatgttttg ttttttttaa 3840
   attttgtagg atgtagttaa aaagaatttt atttagtaaa tatttgaggg tttattttgt 3900
   attagttatg gtgtagtatg tattttttaa ttgagagtag aatattagtt tatatttttt 3960
   tgttattttt aaatataatg ttttataata aaagatgaaa gagaaagaat tttaaagaga 4020
   atagaatagg atgggtatgt atgtataggg tagagaaatt atggaggtaa aggtaaaggt 4080
   attgataaaa gttgtgaaga atttgttgaa ttggtaagga agttagtgga taaatattgt 4140
   agaagtagtt ttttaataga gatattatgt aggagagtag agagaaggtt tttttttgaa 4200
   ttttggtgat ttggtagaga ataaaagggt aagggttgta gaatttgatt aggaggttta 4260
   agttgtatgt atttaatatt gttaattttt ggtttatata tttttttagt agttggaatt 4320
   tttttttttt tttttttttt tttttttttt tttgagatgg ggttttgttt tgttgtttag 4380
   gttggagtgt agtggtttta ttaaggttta ttgtagtttt aaatttttag gtttaagtga 4440
   tttttttatt ttagtttttc gagtagtaag ggttataggt atatgttatt atgtttggat 4500
   aatttttttt tttggttgag gggtatagat ggggttttgt tgtgttattt aggttggttt 4560
   taagttttta ggtttaatga atttttttat tttggtgttt taaagtgttg agattatagg 4620
   aatgagttat tgtgttcggt ttaggaagtt tattttttag ttaattttga aatattattt 4680
   tttgaattta ttaattagga aattcggttg ttaggttggg attttttaag tttttgggtg 4740
   atgggtattt agttttaagg aaggtaatga tgtttaaatt attaaattat gttttagttt 4800
   ttgtgagtta ggaatttagt gagtttagtt gtgtgttttt ggttttaggt tattttattg 4860
   gggttgtggt tttatttaaa gatttagttg ggtgaggata tgtttcgggt ttatttatgt 4920
   ggttgtttgg taggatttag ttttttgttg gtgggttatt tggttgaagg ttttagtttt 4980
   tttttggtgg ttggttagag gtttttttgt tttttattat atgagttttt ttatagggta 5040
   gtttataaag tggtagttgt ttttttttag attgagtaag taagagtgag agtggagggg 5100
   ggagaaagta tttaagatag aagttatatt tttttggtta tttaattttg gaagtgattt 5160
   tttattatta tgttatatta tatttgttag aagtgagtta ttagatttag tttatattaa 5220
   aggaaagggg attatagaag gtattaaatt taggaggtag ggattattgg ggattgtttt 5280
   agaggttgtt gtttagattt tttttatata atagttttga ggtttatatg gt 5332
<210> 18
   <211> 5332
   <212> DNA
   <213> Artificial Sequence
<220>
   <223> chemically treated genomic DNA (Homo sapiens)
<400> 18
   attatataag ttttagagtt gttatataaa aggggtttgg gtagtagttt ttaagatagt 60
   ttttagtgat ttttgttttt tggatttgat gttttttgta attttttttt ttttagtgtg 120
   ggttggattt agtgatttat ttttaataaa tataatatgg tatagtgatg gaaagttatt 180
   tttaagatta gatgattaaa agagtgtagt ttttgttttg ggtgtttttt ttttttttta 240
   tttttatttt tgtttattta gtttgaggaa agatagttgt tattttataa gttattttat 300
   agagaggttt atatggtaag gaatagagaa atttttggtt aattattaag aaggaattga 360
   ggtttttagt taaatagttt attagtaagg aattgaattt tgttaaataa ttatatgagt 420
   gagttcggaa tatattttta tttagttgag tttttagata agattataat tttagtggaa 480
   tagtttggag ttagggatat atagttaagt ttattagatt tttgatttat agaaattgag 540
   atatggttta gtgatttaag tattattgtt ttttttgagg ttaaatgttt attatttaga 600
   agtttgaaga gttttagttt agtagtcgag ttttttgatt ggtggattta gaaagtaatg 660
   ttttaaaatt agttaaagag taaatttttt aggtcgggta tagtggttta tttttgtaat 720
   tttaatattt tgggatatta gggtgggagg gtttattgag tttagaagtt tgagattagt 780
   ttgggtaata tagtaagatt ttatttgtat tttttaatta aaaaaaaaaa ttatttaggt 840
   atggtggtat atgtttgtgg tttttgttat tcgggagatt gagatgagag gattatttga 900
   gtttgggagt ttaaggttgt agtgagtttt gatggggtta ttgtatttta gtttgagtaa 960
   tagagtaaga ttttatttta aaaagaaaaa gagagagaga gagagagaga gaaattttaa 1020
   ttattgaaag aatatataaa ttaaaaattg gtaatattag atgtatataa tttgggtttt 1080
   ttagttaaat tttgtaattt ttattttttt gttttttgtt aaattattag gatttaggag 1140
   gggatttttt ttttattttt ttatatagta tttttgttaa ggagttattt ttgtagtatt 1200
   tatttattga tttttttgtt agtttaataa gttttttata gtttttgtta atgtttttat 1260
   ttttattttt atagtttttt tattttgtat atatatattt attttatttt attttttttg 1320
   aagttttttt ttttttattt tttgttataa aatattatat ttgagaataa taggggaata 1380
   tgaattgata ttttgttttt agttaaaaaa tatatattgt attatgattg gtgtagagta 1440
   ggtttttaga tatttgttaa ataaaatttt ttttgattgt attttgtaag gtttagggga 1500
   gatagagtat tttataaggt gtttagtttt ttaattttgg tgggaagttg gataaaagaa    1560
   gaaatgttgt atattattta tattaagttt tatttttttt tttttatata gtagaaaaat    1620
   gaagttggga gaataatgta ataaaaattt tatagtgatt taggggtaga atttagatta 1680
   gaaattatat tttttgtttt ttaatttaat gttaatttta tgtaggtata aattgttaga 1740
   tgtatgatat gtaaggattg ggaggtaggg gatggttatt tttttttaaa aggggttttt 1800
   tggtttagat tttagatgtt ttatgtttgt tttttagtta attatttcga gggtttgatt 1860
   tttatatttt attggggttg atttttatgt ggattttttg tttgatatgt ggaagagtta 1920
   gatttgtttt ggtatagtag gatattttga agagggagat tagattttgt tttttttttt 1980
   atttcggggt tttttgtaga ttgtgttttt attttttttt aggttttagt gagttaggcg 2040
   gttttagcgt aggtttttgt atcgcgcggt ttttaagcgt tttgatgaga cgtgtttaat 2100
   tttttattcg gattatttaa ttttcgtatt tttgttaata aatagttatt attaacgaga 2160
   tggtagatta tatggggtgg gtttgtatag ataatgagag ttttttaggt ttttttgcga 2220
   gtaaatttat aatttttagc gatttagtcg gttcgaattt ttttatatag aaaattgagt 2280
   cgaattcggt ggtttgggtt tttttttacg attttcgggg ttggaggttt cgttttttgt 2340
   aaatagttgt attttttttt tttttttttg ttttttattt tttttcgttt ttttaagggg 2400
   tggttatagt gtgttataaa ttttattttt ttagtgtgta ggagaggcga gaggaggttt 2460
   tgttttagtt tcgggtttgt ttgttttcga ggatattaat taaaggtatt aattggatag 2520
   taatatatta aagtcgtttt cgggagtgag tttttagagt attaggaatt cgttagttgg 2580
   gtttttgttt taaatataga aatatagagg aaaatatata tatatatata tatatatata 2640
   tatatatata tatatatata tttttacgcg ggtaagagta gggttcgtgt tgtgtcgcgt 2700
   gggttttttt gttttcgcgt tcgttgggag tttttttttt tttttttttt tttcggttcg 2760
   gtatattaaa cgatcgtttt cgtttttttg ttttgagttt attttattta tttttttaaa 2820
   gagtgtcgtt tagaaggttt atttttttgg gatacgtata ttaatagagt tcgcgtagac 2880
   gcggtacgcg gttttattta agagcgttag tttcgggaga aataaaatta ttttttcgcg 2940
   tatatcgttt tggttttttt ttaaataggt tgagggaggg taagtagata tttggggagg 3000
   tatttgtcgt ttggatttaa ttgtacgggg tttttacgag gttttcggac gtttgcggga 3060
   ggagggcgat agaggggaat tggttttatt taggaggggt tattttagga tatagaaagg 3120
   gatatttcga tgggggaagg aaaggatttt tttttgaggg aagtagacgt tgtttatagg 3180
   tttaagggtt tttagaggag ttagttatgg aaagggtttc ggtttagagg gacgtgaagc 3240
   gattcgggcg cgtttttcgt ttttaaatgg taagcggcgg gggtcgggga agttgtggaa 3300
   acgaggtttt tttttcgtta tcggtttaga aggaaggtta agtttttaga gtttagaagt 3360
   tattagtaat ttgtgtcgcg ttcgtaacga ggtttttcga ttttagttat aaaggtttta 3420
   gcgttttgtg ggttagattt aggttcgggg gtacgagttt cgtacgggga gggacggaga 3480
   ttcgggtata aattgggacg ttttgtagtt agcgttcggg ggttcgttgt ttttcggttt 3540
   gtttagttcg ggttatacgc gttcgttgtt tgcgcgaggg aggcgggaat ttgaagtcgg 3600
   cgagagcgag gttcggttcg agtcgttatc gtagttatag ttacggcgta ggttggcgcg 3660
   tcggggattt tcggttttat ttgggttttg gggaggttag aagcgttttt aattttatag 3720
   ggttacgttc gttttttttt ttcggggagt tagattggaa tggttgtttt tgattttatt 3780
   tgttattaat ttcggtattt gttttttatt ttataaacgt ttttttttaa gtaattgttt 3840
   ttgttaatag cgtatatttt ttagtagcgt ttattgcgtt ataaataatt tcgatgaaga 3900
   cgtaaggata tgatttttat aagattggat aattgattta atttgtgatt cgcgatggcg 3960
   aataatattg gaaggttatt taaatagatg aaggtttaaa ttgttttgtt tgtatttgaa 4020
   ttaatttttt atttattatt attatgaagt gattggttta tttaagtttt ttagtttgtt 4080
   ggaacggaac gggatttaaa tgagattgta atataattta aatgtttgtc gattttaacg 4140
   aggttaattg agtagttgta ataaatataa atattttttt aaatagtcgt gttttaaatt 4200
   attatttaat gtttttatgt ttttttaagt ggtagttttt tttttttttt tgtttttgtt 4260
   tagaatatta tatatttatt tgttgaaaag gtgattttgg aaataatttt gtgtttttgt 4320
   ttgttttgta gatagtagga tatgttttgt gattgggttt tgttttgttt tgttttgttt 4380
   tgtttttttt ttagagttaa tatttttagg gttttttaag ttggtataaa tggtgagttg 4440
   agttttagtt ttattttaaa gtagtttttt gtgttgtttt cgtagagttt gggaggagtt 4500
   gagtgatgga agaaatttgt cgtttggatt tagttattta tttagacgta gtttagttgg 4560
   gttttgtaaa tgaattcgtg ttattgtatt tataaatgat attttgaaga gtggtattgt 4620
   ttttgatttg tattgatgat cgttattatt ttgaataaat tagaatttta atatatttta 4680
   aaggtatttt ttagaaagtt taatttgttg gtagtttggt tttttgtaaa ttaaaaggag    4740
   taggtggagg ttgattgagg atttttttat tagggaaggg gtatagaaag agaattttgt    4800
   tataataaat ggtggtagga tggtggagga gggggttgat aattagagaa atgttaattt    4860
   tgtggtttta attttttttt ttttttattt gtaattttat ttttattttt ttttttatta    4920
   tttttgggtg atgatgtatg gagaggatgt taagattttt attttgagaa tgttattcgt    4980
   ttggatattt ttggcggtaa ttgtttatcg cgatatttac gtttattttt cggcggatat    5040
   atggtggttg gtgttttcgt agttggggaa taatggaagt aattaagcgg gagaagtttg    5100
   aggaataaaa gaatttttta aattttagtg aaagggtcgg ggttggggat gtggggggat    5160
   attcgattta ttgtgaggat ttgagtttaa tttcggggtg attgtttaag gttatgtttt    5220
   tttcgaaagg tttagggtaa ggagatgtat tttgaatttt ttttttttta atttttggtc    5280
   gagggagagg gattttttgt ttggagagtg cgggtgtagg gtttgaaatt ag            5332
<210> 19
   <211> 4215
   <212> DNA
   <213> Artificial Sequence
<220>
   <223> chemically treated genomic DNA (Homo sapiens)
<400> 19
   ggtgttcgat taagcgtagt tcgtagtttg ggtattgtta attgatttta atttttttta 60
   tggtgagagg atggattttt cgttattttt tcgtttaatt tggtatttat ttatttattt 120
   atttattacg ttcgttgggc gtatttaagt ttaatttcgg ggcgtacgcg ttagcgtaga 180
   tatcgtattt tttttttttt ttcggttaat tttaggtaga attttaaaat aattgttttt 240
   ttttttacga tttagatgtt gcggttcgcg gataggaggt ttaagaaata gtatatttcg 300
   agcggtaggt agcgaggcgg aaacggtcgt cggttttagt ggtggtttta ttggaagtcg 360
   agtttaggag cggttaagcg gtcgtcgggg aaagtatcgg ggttttttag ggtttttttc 420
   gagtttttat ttcgtatttt cgagggcgtg aaaattacgg gagtcgtttt atttcgcgcg 480
   tttagtttcg ttttagttta gatatcgttt ttcgttagtt ttttttgcgg cgtttaggga 540
   ggacgcgttt cgtttttttt taattcggtt aatgggcgtt cgggtagcgc gcggtttgtt 600
   ttcgttttcg ttagggaaat ttggaggagg agaaaagttt gtatagaggg tggaaaggcg 660
   agagcggagt tttaagttcg gtagttcgag aggaagatga atagttttag gttagagttt 720
   ttggtagagt ggatttcgag tcgtttttag gtagttagga gcggttttag cggtagtcgt 780
   aaattttagt agtcgttcgt gttgttcgtg gttggggcgg agggtagtta gagttgggga 840
   ttaaggtttc gcgttatttg cgcgtatagt tttatatttg aacgttgttt tttcgtagac 900
   gagatcggcg ggtattgtaa agttgggatt cgtttttgaa ggaaaaaaaa tagcgagtaa 960
   gaaatttagt attatttttt attgatttat ttcgttgtat tttttgtttt ttaagttttt 1020
   gaaagttggt aattttgatt tcggtgttta aaaatcgata gttattgaga tcggttttga 1080
   gaagtcgaag atttggtagt ttttagattg agtaggataa ggtgaaagta ggttggaggc 1140
   gggtttagga tatttgaggg ttgattttgg gggttcgtga ggttgttatc gttgttgtcg 1200
   ttataggtga gatggcgttg ggttgacgtt ggggttaacg ggtagagaac gtagggatgc 1260
   ggttttcgtc gaagagagtt aagaagggaa gagcgcgttt tttaaattgt ttttgtaatt 1320
   tgtttttagt gagtatttta ttgatttaga atttatcgag aatagtatta gcgagttatt 1380
   ttttttttga gatgggtttt atttattttg gtaatggagt gagttggatt gtggggagga 1440
   agaggaatgg gaaaattagt ttataaatat taatgttagt aagagtgtgt tgttggtagg 1500
   acgtatcgcg agtttggaga ttttggtggt cgtagttggt aagtggttat aatttagaaa 1560
   gtaggatcga gttgtttttt ttgttttatt agtgtatcgt ttttcgggcg cgggtttaat 1620
   attttataag tggtaatttt cgtttacggt agttttgttt tttttttatt atttttagat 1680
   ttagttttgt attttaaggt tgcgtatcgt tagttattat tatgtttatt ttcggggtta 1740
   attcgttcgt ttttttgagt ttcgatcggt tgaatagttt agtgattatt tcggcggtga 1800
   tgtttatttt cggggtggtg ggtaatttgg tggttatcgt ggtgttgtgt aagtcgcgta 1860
   aggagtagaa ggagacgatt ttttatacgt tggtatgtgg gttggttgtt atcgatttgt 1920
   tgggtatttt gttggtgagt tcggtgatta tcgttacgta tatgaagggt taatggttcg 1980
   ggggttagtc gttgtgcgag tatagtattt ttattttgtt tttttttagt ttgttcggtt 2040
   ttagtattat ttgcgttatg agtgtcgagc gttatttggt tattaattat gtttattttt 2100
   atagttatta cgtggataag cgattggcgg gttttacgtt ttttgtagtt tatgcgttta 2160
   acgtgttttt ttgcgcgttg tttaatatgg gtttcggtag ttcgcggttg tagtatttag 2220
   atatttggtg ttttatcgat tggattatta acgtgacggc gtacgtcgtt tatttttata 2280
   tgtacgcggg ttttagtttt ttttttattt tcgttatcgt tttttgtaac gtgtttgtgt 2340
   gcggcgcgtt gtttcgtatg tatcgttagt ttatgcgtcg tatttcgttg ggtatcgagt 2400
   agtattacgc ggtcgcggtc gtttcggttg tttttcgggg ttatttcgtt gtttttttag 2460
   ttttgtcgcg ttttagcgat tttcggcgtc gtcggagttt tcgtcgtatc gcgggcgtcg 2520
   agatttagat ggttatttta tttattgtta tttttttggt ggtgtttatt tgttttattt 2580
   cgttcgtggt gagtgatcgg ggttggggtt ttattcggtt tttttttcgt atttattttt 2640
   cgcgtttatt tttcgttttt tgtttttttt ttgagttttt ggtagtgaac gtgtcgtttt 2700
   taggtcgggg ttgggatttt tatattgttt tttagagtag gtttaatttt ttttgaagtt 2760
   ttaattttaa cgagatttag taggtgtttt gtttttatat tttttagttt atgttttcgg 2820
   aagtttgggt tttttttttt atcgagatag tttttatttt ttgttgtttg atattggtcg 2880
   agttttttaa gaaaattttt cgtttttttt gttagacgtg gaggggagtt tgttgtagtg 2940
   tgatttagtt tattttttcg tattgtgaat tgtgaattgt aaaatttgaa atgttaggga 3000
   tggtgatttg ttcgttgtaa attattttta aggaaagtgg ggtttttggt gttttgatgt 3060
   atgtaaggtt ttttgaattt tttttttcgt ggattgcgta gaattttaaa gttagaagag 3120
   atattaagag gtgttttagt ttaatttttt tattttattg agggagtgaa tgatacgttt 3180
   aaaataattt agtcgaattt atatagttaa gttaggtttt ttgtttttta ttatggtgtg 3240
   ttttgggagg gtagtttaaa gtatataaat tttaatttat atatagggta gggtttagat 3300
   ttagtttttt ttattttgtt tagattagga aagtaaagtt ttttagggtg attaattatg 3360
   gataggttga tatatatagg gtatgggatt tgaattttcg ataattgtgc gaggtatagt 3420
   attattttag agaaatcgag gtttagagat ggttgttttt gtttttttaa gttaaagttt 3480
   tatagtttgt aatggatttt aggtcgtttg aggttagatt tagtttatgt gttattatat 3540
   tacgtttttt tcgtagaata ttttttattt tggcgtttgt aaagcgtggg ttggagtttt 3600
   ggtatatttt atttttagtt tattttttaa gaattgtttt tggattaatt tttaaaatat 3660
   gataaataga aaggtttaaa ttttatttag ttttttattt tgtttgtggt taaaatggaa 3720
   tgtattagaa gtagatgggt agaataatgt aatattttat ttagagtttt ttttgaaagt 3780
   aattagttat gagttttttt attttttaaa atattgtatt tttggaaatt ttaaattatt 3840
   aatttaaaaa gagataattg ttttttttta tgtttgttgg gaataagaga atataaaatg 3900
   taatgggtta ttattttttt ttttttttta ttgatattgt ttagggattt ttggtgtaaa 3960
   aatatatttt tgataaaagt gattgtaaat ggaaggaatt tagagggggt ttagagttag 4020
   aatatagtag gtttttatta atttgtataa atagaagtaa atataatagg ttataagagt 4080
   ttttaagtta ttttaggatt taatataatt tatagagtta agttatattt gagttatata 4140
   aatatatttt attgtttgta atggtaattt agaataaagt gagattttgg aagaaataat 4200
   attgtttttg atggg 4215
<210> 20
   <211> 4215
   <212> DNA
   <213> Artificial Sequence
<220>
   <223> chemically treated genomic DNA (Homo sapiens)
<400> 20
   tttattagga gtagtgttgt tttttttaaa attttatttt attttaggtt gttattatag     60
   atagtaaaat atatttatgt ggtttaagtg tgatttaatt ttgtaaatta tattgggttt    120
   tggaatggtt taaaggtttt tatggtttat tatatttatt tttgtttata taaattggta    180
   agaatttatt gtattttggt tttgagtttt ttttaagttt tttttattta tagttatttt    240
   tattaaagat atatttttat attaaagatt tttggatagt attaataagg aaaaggaagg    300
   aataatagtt tattatattt tatatttttt tgtttttaat aagtataaga aaaagtagtt    360
   attttttttt gagttggtag tttagaattt ttaggaatgt agtgttttgg aagatggaag    420
   aatttataat tagttgtttt taagagaaat tttagataaa gtattgtatt attttgttta    480
   tttgtttttg gtgtatttta ttttagttat aggtaaagta gaaaattagg tagggtttga    540
   gtttttttgt ttattatatt ttaaaagtta atttaaaggt aatttttaaa agatgaatta    600
   aaaatgaagt gtattaaaat tttaatttac gttttataaa cgttaaaatg agaaatgttt    660
   tacgagggag acgtgatgta ataatatatg agttgggttt ggttttagac ggtttaaggt    720
   ttattataag ttgtagagtt ttggtttgaa gaaataaaga tagttatttt taaatttcgg    780
   tttttttaaa atggtattgt atttcgtata gttgtcgaga atttaaattt tatattttat    840
   gtatattagt ttgtttataa ttagttattt tgagaaattt tgtttttttg gtttagatag    900
   aatgaaagaa attgagtttg gattttgttt tgtatatgaa ttagaatttg tgtgttttaa    960
   gttgtttttt tagggtatat tatggtaaag agtagggagt ttagtttgat tgtgtaggtt   1020
   cggttggatt attttaagcg tattatttat ttttttagtg aagtgaggga gttagattaa   1080
   aatatttttt gatatttttt ttagttttaa aattttacgt aatttacggg ggaagaaatt   1140
   taagaaattt tgtatatatt aaaatattaa aaattttatt ttttttaaaa atggtttata   1200
   acgaatagat tattattttt gatattttag gttttgtagt ttatagttta tagtacggag   1260
   gaatgggtta agttatatta tagtaggttt ttttttacgt ttaatagagg gggcgggggg   1320
   tttttttgga agattcggtt agtgttaggt agtaaggggt aagggttgtt tcggtggaga 1380
   aagaaattta ggttttcggg aatataaatt gggggatgta ggggtaaagt atttgttaaa 1440
   tttcgttagg gttgggattt taaagagggt tgggtttgtt ttgagaaata gtgtgggaat 1500
   tttagtttcg atttaaaggc gatacgttta ttgttaagga tttagaggga aagtagggag 1560
   cggggaatgg acgcgggagg tggatgcgag aaaaaggtcg agtagggttt tagtttcggt 1620
   tatttattac gagcgggatg gagtagatga gtattattag ggaggtggta atgagtaaga 1680
   tgattatttg gatttcggcg ttcgcgatgc ggcggaagtt tcggcggcgt cgaaagtcgt 1740
   tgaggcgcgg taaggttggg gaggtagcgg ggtggtttcg ggaggtaatc gaggcggtcg 1800
   cggtcgcgtg gtgttgttcg gtgtttagcg aggtgcggcg tatgaattgg cggtgtatgc 1860
   ggagtagcgc gtcgtatata agtacgttgt agaggacggt ggcgagaatg aggaaggagt 1920
   tgaagttcgc gtatatgtag gagtaggcgg cgtgcgtcgt tacgttggtg gtttagtcga 1980
   tgaagtatta ggtgtttggg tattgtagtc gcgagttatc gagatttatg ttgggtagcg 2040
   cgtaaaagag tacgttggac gtatagattg taaagagcgt gaggttcgtt aatcgtttgt 2100
   ttacgtagtg gttgtagaaa taggtatggt tgatggttag gtagcgttcg atatttatgg 2160
   cgtagatgat gttgaggtcg gataggttga agaagagtag aatgaaggtg ttgtattcgt 2220
   atagcggttg gttttcgggt tattggtttt ttatgtacgt ggcgatggtt atcgggttta 2280
   ttaataaagt gtttaatagg tcggtgatag ttagtttata tattagcgtg tagaaggtcg 2340
   tttttttttg ttttttgcgc gatttgtata gtattacgat ggttattagg ttgtttatta 2400
   tttcgaagat gaatattatc gtcgggatgg ttattgggtt gtttagtcgg tcggggttta 2460
   aggaggcgga cgaattgatt tcgggagtgg atatgatagt ggttggcggt gcgtagtttt 2520
   ggagtgtaag gttgggtttg gggatggtag aagagagata aagttgtcgt gagcggaaat 2580
   tattatttgt aaggtgttag attcgcgttc gagaaacgat atattgataa gataagggga 2640
   gtaattcgat tttatttttt ggattgtagt tatttattaa ttgcggttat taaaattttt 2700
   aggttcgcga tacgttttgt taatagtata tttttgttga tattaatatt tataaattga 2760
   tttttttatt tttttttttt tttataattt aatttatttt attgttaaga tgaataagat 2820
   ttattttaag ggaaaagtag ttcgttagtg ttattttcga tagattttga attaataaaa 2880
   tgtttattga aaataagtta taaaagtaat ttggagagcg cgtttttttt tttttggttt 2940
   ttttcggcga gggtcgtatt tttgcgtttt ttattcgttg attttaacgt tagtttaacg 3000
   ttattttatt tgtagcggta gtagcggtgg tagttttacg agtttttagg gttagttttt 3060
   agatgttttg gattcgtttt taatttgttt ttattttgtt ttgtttagtt tggaaattgt 3120
   taaattttcg gttttttaaa gtcggtttta gtggttgtcg atttttggat atcgaggtta 3180
   gagttgttag tttttaaaaa tttgggaaat aagaggtgta gcgggatggg ttagtgaaga 3240
   atggtgttgg attttttatt cgttattttt ttttttttaa agacgagttt tagttttgta 3300
   gtgttcgtcg gtttcgtttg cgggaggata gcgtttaggt gtgaggttgt gcgcgtaggt 3360
   ggcgcggagt tttggttttt agttttggtt gtttttcgtt ttagttacgg gtagtacggg 3420
   cggttattgg agtttgcggt tgtcgttgag gtcgtttttg gttatttggg ggcggttcgg 3480
   ggtttatttt gttagaggtt ttggtttggg gttgtttatt tttttttcgg gttgtcgggt 3540
   ttggagtttc gttttcgttt ttttattttt tgtataaatt tttttttttt tttaagtttt 3600
   tttggcggag gcggaggtaa atcgcgcgtt gttcgggcgt ttattggtcg gattggaagg 3660
   gggcggagcg cgtttttttt gggcgtcgta gggaagattg gcggggggcg gtgtttgggt 3720
   tggggcgggg ttgggcgcgc ggggtggggc ggttttcgtg gtttttacgt tttcggaggt 3780
   gcggagtggg aattcggagg ggattttggg aagtttcggt gtttttttcg gcgatcgttt 3840
   agtcgttttt gaattcggtt tttagtgggg ttattattga aatcggcgat cgttttcgtt 3900
   tcgttgtttg tcgttcggag tgtattattt tttgaatttt ttgttcgcgg gtcgtaatat 3960
   ttagatcgtg gaagagaggg tagttgtttt aggattttat ttagggttgg tcgagaaagg 4020
   agaagaaata cggtgtttgc gttagcgcgt gcgtttcggg gttagatttg ggtgcgttta 4080
   gcggacgtgg taggtgggtg ggtgggtggg tattagattg ggcggggaaa taacgaagaa 4140
   tttatttttt tattataaaa ggagttggag ttagttggta gtgtttagat tgcgggttgc 4200
   gtttggtcgg gtatt 4215
<210> 21
   <211> 5473
   <212> DNA
   <213> Artificial Sequence
<220>
   <223> chemically treated genomic DNA (Homo sapiens)
<400> 21
   gtaatgttaa ttagttttat tttaaagaaa aattaaatat tagagtaaat aatgattttt      60
   ttgcggagaa ggagttgtaa gggttgggga gaggtaatta gtgttataaa agttttatta     120
   taaagaagtt tagaattttt ttgttttgtt gtgtagtaga gtgaaatata gtagtagaat     180
   aattgtgtat tattattatg ataatttaat ttgttttttt agaaaaagtt attgtttgta 240
   gttaggatag taggaaaata tttagaaaat aattaaaatg taaataaaag tatgaaataa 300
   taataaagtt tattttttaa gagaaaaaaa gaagagtatt tagcgaaagg agaatgaagt 360
   atatataaag taagtaattt ttaggtattt tgtttttttg gatgttattt gtttaaattt 420
   gataattttt ataggtagta tgatagattt tttaaaattt aaataaagtt tgtttttgta 480
   ttttattttt aataataaag tatattgatt gatttataaa ttaaagtttt taaaatttta 540
   ttaaagtaaa aatattgttt tagagttttt attggtatgt ttttgaaagt aagtgttata 600
   gttaatgtaa atttttaaaa atttttttgt tatttagtat aatagagggt ggattttgtg 660
   ttagggtggt attttttgtg taatttatat ggtttgaggt tttttgtttt tttttttttt 720
   agaagagttg gaattaagat attaataatt ataattgtaa taattattat tatgttttta 780
   aggggttgtt gagtttttgg agattttagt gtatagagcg aagtaggaga ggtttaaaaa 840
   ggaaggtttt gttgttgatg ttgtgatgat taaggtagtt tattattatt tttttttttt 900
   aacgtgattg ttttgttttt tttttttttg attttgttat aagatgtggg gaagtttttt 960
   aagaattgtg tatagttcgt tatagatggt ggtttttgtg tttagagatc gaaagagtat 1020
   ttgttatagc gatattttgt aatagttgta gatgtgcggg tttaaaattg ttttatgtgt 1080
   ttaaattaat aataattatt attttatttt attttattat ttgagcggaa atataatttg 1140
   aaatatttaa atttgttttt tttaaagtta gaaggatatg tgtatgtgaa tgtatataat 1200
   ttgttttttt aaatgttttt aaagtttatt tttgttatta tatttgtttt tataattaat 1260
   aaagaaaatt tgttttagtt ttaattttat tgggtatggt acgtgttgga gggtaggttt 1320
   tggaaatata aatattaata tatgtggttt gttttaaaat ttaatgtatt tagggttagt 1380
   ttcgttgttt tggttaaatg aatgaataaa gagtgattaa aatgcgttta ttttttcgtt 1440
   attttatata tagtttgaga tcgatcgtta atagtagatt gtaaattttt gtatttaata 1500
   gttattaatt ttttttatag tttgtatatt tataaattta attgggatat tcgtttgata 1560
   aatttagatt ttgtagagta gatttttaag gaaatttaat tttagagaat aaaatatttt 1620
   ttggaatttt taaggtattt agagataaaa cgttttttgt gggatttttt tttaagtttt 1680
   ataaaagtag gtttattagg attaaattat attaaagtta gtgcgaggta aggggggtgt 1740
   ggtttataga aattttggga gaaagtttag tatagttttt tttttttggg aatatatgtt 1800
   aggatggaaa aaaaaaaaag gaggggagtg ttataggatt aagatttatt ttggaaaata 1860
   agatttttag taaagatatt tttttttttt ttattttata ttagatatta gaagtattat 1920
   ttttcgagtg tgttttttaa gttttttttt aattttaggt tttttttgtt ttttgggttt 1980
   tcgatttttt ggtagttggg tatttgtatt cgggttttcg ataggtttag tcgttggttt 2040
   agtttttttc gtatgaaggc gtcgggatag tgggtttcgt taaaaagttt ttttagttta 2100
   ttgagttgtt ttagggtgaa attggttcga tttcgttttt gtttgatttt ggtttggttt 2160
   tcgtttttta tttttttcgt attttttttg cgatttttta gtttcgggga tattggaggg 2220
   ggtattttag cggggttata cgtgtattta tacgaatata tatatatacg tatatatata 2280
   cggacgaaaa tagtatagta aattttgtta ttagatttgt cgtaaaaaag aatagagaat 2340
   tttttttttt cgtggaattt tggttcggtt ttttaattta ttcggaggaa gaatatttcg 2400
   gagaagcgta aaggttaagt ttgagcggtc gtttggggta aggtgggtag ttatttatta 2460
   ttttttttat aaacgtattt cgatttatcg ttttattatt tattttttag ttatcggttt 2520
   tttttagtaa atattaaatg gtttttattt attttcgtat ttatttgttt cgtagtattt 2580
   atagaaatta aaggcgtagc gttaggtttt ttatagagta aagtttggat ataagggcgc 2640
   ggtaaggtta tttattggtt ttttatatcg gttattttta gaaggttggt tcgtttttag 2700
   gttagttttt attatcgttg gttttttgtt tatcgtaaat ttgttggttt aatttaggaa 2760
   taattgggtc gaaaggtatt agcgagagta atagatttcg gtgttgtgtc gtatagggag 2820
   tcgtattcgt agacgttttt cgttgttttt gggttcgggt taaattttgt ataaggtttt 2880
   ttggatagtt aggtaatttt cgtttcgttt gttcgatcgg ggtcgtacga gtataggcgt 2940
   ttacgttatg ttggttgttt aaagggttcg tcgtttaagt cgggttagaa ggtaggaggc 3000
   ggaaaattag ttttcggtgg cgggcgaaag taatcgtttt ttttgttttt ttttcgtttt 3060
   ttttcgtgga aacgtagatt cgattttaaa cgtttaattt atagagatta ataggtttaa 3120
   gcggaatatt cgcgattttc ggtttttatt ggttgtttaa agttttttta tgtaattagt 3180
   agttcggatg tttaataaaa tatgaatttt tttgttttgg gtttgtatat attcgttgtt 3240
   atttttttgt tttttttttt tttttaaaaa taggagtttt tttagtgtga gtatatatag 3300
   ttattatata tatatttttt tggaattttt attttttgcg ttcgaattag agatttcggg 3360
   ttttaagaag ttaaagttga gtttagatag tcgaggttgt ttgtagtttt ttttagcgta 3420
   ggatttaggt tagtatgggt gttaaaaatt attttcggaa gagtcggttt gtagagaaat 3480
   taatatttgt tttgagtttg gaaattaata attttaggaa ttgcgttcgt cgggggttta 3540
   gatttgaatt tcgtcgttag gcgcgttggc gaatttgtag ttcgggtttg tagttgtgga 3600
   tttatggtcg ggaggttgta ttgggtattt tgacggtaat tttattattt tataaattta 3660
   ggaagatttg gtgttggttt ggaggaagaa gagtcgttag atttcggttt tgtcgggtat 3720
   tttagttacg gtgattttaa atattgtcgt attttttagt ttttagagtt agttttgtgg 3780
   atatttgttt tttttttagg aaggaatatt atagtaaata ttatgggtta atttaatagt 3840
   aaagtatttt tattgatatt gtattgaaat ttaaatatga aaatatattt tttaaatttt 3900
   gttaatgagg tgggaaggtt ttgtttatta tatgttattt atatagtgga tgttatttaa 3960
   agttttgggg tttcgaggag atgtttaaat tttaagttga tttatagatt taataaaatt 4020
   tttatttttg tttttaaatt tttaattatt gatttaaata ggaatagatc gtgttataaa 4080
   taatttaatt gtttgtttaa atatgtgatt ttacgtatgt tatatatttt tttaattgga 4140
   aaaatatggt agtatattag ttattgtagg tattaagtgt taaattaatt ttattgttga 4200
   atttttttga attggtatag aacgttttgt tattgagata aagacgtttt ttaatattga 4260
   gttagtatta aaggattttt gttttaagag aatcgagtat tgggtgattg aaaatattaa 4320
   aggaattgtg taaatataga tttttatttt tttcgtcgtt tttttttttc gtttttaata 4380
   tattaagaaa tcgaaacgtt tcgttcggga gaagtagcgt agtttggatt ttagttttcg 4440
   ttggtttttt tttattttga aagagaatgg gaagttttgg tttttaagat ttaaagattt 4500
   aggggttttg cggagttcga ggggttttgg cggttttaaa tatttaggcg atcggagggt 4560
   taagtttttt atttttgttt tttttttttt tcggtaaatt ttgcgttaga ggttagtttt 4620
   agtttcgtcg tttggtttag tatttttttt tgtagttcgg tttcgcgaat ttttacgttc 4680
   gtttgcgggt tattcgggtt gcgggtttat tttttcgttg gtgaaaaatg tatttcggtg 4740
   gaatgggaat atttgggaag ggcgcgtata aattttatac gttttttttt acgttatttt 4800
   tataatatat taggatttta tttatgatcg agtatattat cggattttta tttttttatt 4860
   attattttag ttgataaagt tggggtttag ggtttttttc gtgttttttt ttttttttcg 4920
   ttgggtttcg gagttttttt tcgttcgaga ggagttcgga aggcgggaag gggaatcgtt 4980
   gtcgggggtt agttaggtcg ttatttttcg ttagtcgcgg gttgttcggt tttttgtttt 5040
   tttcggcggc gtttatgttt agttttcgga cgggagagcg ttttttttta gttttttcgt 5100
   ttgttttttt tttttttata tttttttcgt ttttttcgtc gtcgttttcg tttttttcgt 5160
   cgtcgttttc gtcggtcgtt cggattgtcg ggttgttgcg gtcgtcgcgg ttcgtttcgg 5220
   tgtagtcggt cggttttttg gtttcgcgta gcggttcgtt ttttagtatt tttcggtacg 5280
   tgatcgtttt tttttttttt tttatttttt ggttaaaaga tttggagacg aacgtcgtaa 5340
   gtttttttat cgtcgtcgta cgttagttcg gcgtttaatt tttgttagta gagtttcgtt 5400
   tttttttttt tttttttttt tttattgttt tagttttttt aataataata tattaatggg 5460
   ataggaggtg ggg 5473
<210> 22
   <211> 5473
   <212> DNA
   <213> Artificial Sequence
<220>
   <223> chemically treated genomic DNA (Homo sapiens)
<400> 22
   ttttattttt tgttttattg atgtgttatt attggggggg ttggagtagt aaaaaaagaa 60
   gaaggaaaaa aagagcgggg ttttgttggt agaggttgag cgtcgggttg acgtgcggcg 120
   gcgatggaag aatttacggc gttcgttttt aagttttttg attagaaagt gaaggagaag 180
   aaggaggcga ttacgtatcg ggaggtgttg gagagcgggt cgttgcgcgg ggttaaggag 240
   tcgatcggtt gtatcgaggc gggtcgcgac gatcgtagta gttcggtagt tcgggcggtc 300
   ggcggaggcg gcggcggagg aggcggaggc ggcggcggag gaggcggagg aggtgtagga 360
   ggaggaggag taggcggagg agttggagga gggcgttttt tcgttcggga gttggatatg 420
   ggcgtcgtcg agagaagtag ggagtcgggt agttcgcgat tgacggaggg taacgatttg 480
   attgattttc ggtagcggtt ttttttttcg tttttcggat ttttttcggg cgggagagga 540
   tttcgaggtt tagcggggag gggagaggga tacggagagg gttttggatt ttagttttgt 600
   tagttagagt gatagtggaa gggtgggggt tcggtggtat gttcggttat gggtggggtt 660
   ttagtgtgtt gtgggggtgg cgtgggggga agcgtgtggg gtttgtgcgc gtttttttta 720
   gatgttttta ttttatcgaa atgtattttt tattagcggg aggatgggtt cgtagttcgg 780
   atggttcgta ggcggacgtg gaagttcgcg gggtcgggtt gtaggagggg gtgttgaatt 840
   agacggcggg attaaagtta gtttttagcg tagagtttgt cgggagggag aaggggtaga 900
   ggtggaaagt ttaatttttc ggtcgtttag gtgtttgggg tcgttaagat ttttcgaatt 960
   tcgtagagtt tttgaatttt tggattttag gggttaaaat tttttatttt tttttaaggt 1020
   agaaggaagt taacgaaagt tgaggtttag gttacgttgt ttttttcggg cgaggcgttt 1080
   cggttttttg gtgtgttggg ggcggggggg gggggcggcg ggggaggtgg ggatttatat 1140
   ttatataatt tttttagtgt ttttaattat ttagtattcg gtttttttag agtaaaaatt 1200
   ttttggtgtt agtttagtgt taggaaacgt ttttatttta atggtagagc gttttatatt 1260
   aatttaaagg agtttaataa taaaattgat ttggtatttg gtgtttgtag taattggtgt 1320
   attgttatat ttttttaatt aagaaaatat gtggtatgcg tggaattata tatttaagta 1380
   agtaattaag ttgtttatga tacggtttgt ttttgtttag attagtaatt ggaaatttgg 1440
   ggataaaaat agagatttta ttaagtttat ggattaattt gaaatttgga tattttttcg 1500
   aagttttaaa attttgagtg gtatttattg tgtagatgat atgtaataga taaagttttt 1560
   ttattttatt ggtagaattt gaagggtgtg tttttatatt taaattttaa tataatatta 1620
   gtaaaaatat tttgttatta aattggttta taatgtttat tataatgttt ttttttaaag 1680
   aaaggataaa tgtttataaa attaattttg agaattagaa aatacggtaa tatttaagat 1740
   tatcgtagtt agaatgttcg gtagggtcga gatttagcga tttttttttt tttaaattag 1800
   tattaggttt ttttaaattt gtaaagtggt ggagttatcg ttaaggtgtt tagtgtagtt 1860
   tttcggttat gagtttataa ttataggttc gaattatagg ttcgttagcg cgtttgacgg 1920
   cgaggtttag gtttgaattt tcggcgggcg tagtttttgg gattgttgat ttttaagttt 1980
   agggtaagta ttaatttttt tgtagatcgg ttttttcgga aatgattttt aatatttatg 2040
   ttggtttgga ttttgcgttg ggaagaattg taaatagttt cgattattta aatttagttt 2100
   tgatttttta aaattcggag tttttgattc gggcgtagag gataaaggtt ttagggggat 2160
   atatatatgg tggttgtata tgtttatatt agaagagttt ttgtttttaa agggaggaga 2220
   aggataggaa agtggtaacg gatgtgtgta aatttagagt agaaaaattt atattttatt 2280
   aaatattcga gttgttggtt gtatgaaaag gttttgagta attaatagaa atcgaggatc 2340
   gcgaatattt cgtttgaatt tgttgatttt tgtgggttaa gcgtttaggg tcgagtttgc 2400
   gtttttacga gggagggcga agagagaata gaaagagcgg ttgttttcgt tcgttatcgg 2460
   aggttggttt ttcgtttttt gttttttggt tcggtttggg cggcgagttt tttgggtagt 2520
   taatatggcg tgggcgtttg tgttcgtgcg atttcggtcg ggtaggcggg acggagatta 2580
   tttggttgtt taggggattt tatgtagggt ttggttcgag tttaggggta gcgaggggcg 2640
   tttgcggatg cggttttttg tgcggtataa tatcggggtt tgttgttttc gttgatattt 2700
   ttcggtttaa ttgtttttaa attagattag taagtttgcg gtaggtagag agttagcggt 2760
   ggtgagagtt ggtttgagaa cgagttagtt ttttgggaat ggtcggtgtg agaggttagt 2820
   gggtggtttt gtcgcgtttt tatgtttaga ttttgttttg tgagaggttt ggcgttgcgt 2880
   ttttgatttt tgtgagtgtt gcggagtaag tgagtgcgaa agtgagtgga aattatttgg 2940
   tgtttgttgg ggaaggtcgg tgattggggg gtgaatagtg aggcggtgag tcgaggtacg 3000
   tttgtgaagg aggtggtgga tgattgttta ttttgtttta ggcggtcgtt tagatttgat 3060
   ttttgcgttt tttcgggata tttttttttc gggtgggttg agaagtcgga ttaggatttt 3120
   acgaggggga aggatttttt attttttttt gcgataaatt tggtaatagg atttgttgtg 3180
   ttgttttcgt tcgtgtgtgt gtgcgtgtgt gtgtgtgttc gtgtggatgt acgtgtggtt 3240
   tcgttggggt gtttttttta gtgttttcgg agttgaaaga tcgtaaagag gatgcgaaag 3300
   ggatggagga cgaaggttag attaaaatta agtagaggcg aagtcggatt aattttattt 3360
   tggaataatt taatgagttg gagaggtttt ttgacgagat ttattatttc gacgttttta 3420
   tgcgagagga attgagttag cgattgggtt tgtcggaggt tcgagtgtag gtatttagtt 3480
   gttaggaggt cgagggttta gggagtagag agagtttggg gttggaaaga ggtttggggg 3540
   atatattcgg gaggtggtgt ttttagtgtt tggtgtaaga tgagaaggaa agagatattt 3600
   ttattagagg ttttattttt taaagtagat tttagtttta tagtattttt tttttttttt 3660
   ttttttttat tttaatatat gtttttagaa gaagaaagtt gtgttgggtt tttttttaga 3720
   gtttttataa attatatttt ttttgtttcg tattggtttt aatgtggttt aattttaatg 3780
   ggtttatttt tataaggttt aaaaaaaaat tttatagaaa acgttttatt tttggatgtt 3840
   ttaaaagttt taaagagtat tttgtttttt ggagttggat ttttttgaaa gtttgttttg 3900
   tagggtttag gtttgttaag cggatatttt aattaggttt atagatgtgt agattataaa 3960
   gaaaattagt aattgttaag tgtagaaatt tatagtttgt tgttagcggt cgattttaaa 4020
   ttgtgtgtga aatagcggag aaataaacgt atttggttta ttttttgttt atttatttaa 4080
   ttaaggtaac gaaattgatt ttaaatatat tagattttaa aataaattat atatattagt 4140
   atttatattt ttaaagtttg ttttttagta cgtgttatat ttaatgaagt taggattgaa 4200
   atagattttt tttattggtt gtggaggtaa atgtaatagt aaaaataggt tttgggggta 4260
   tttaaaggag taaattgtgt atatttatat atatatattt ttttagtttt agggggaata 4320
   aatttaaatg ttttagattg tattttcgtt tagataataa aatagaataa aatgatagtt 4380
   attattggtt tgaatatatg gagtagtttt aggttcgtat atttgtagtt gttataaagt 4440
   atcgttgtaa tagatatttt ttcggttttt gggtatagag attattattt gtagcgaatt 4500
   atgtataatt tttaaggggt ttttttatat tttgtaatag gattaggaaa aaaaaaaata 4560
   gagtagttac gttgaagagg aaaagtaata ataggttgtt ttggttatta tagtattagt 4620
   agtagagttt ttttttttaa gttttttttg tttcgttttg tgtattgaga tttttagagg 4680
   tttagtaatt ttttaaaaat atgatgataa ttattatagt tatgattatt agtattttaa 4740
   ttttaatttt tttaggggaa aaaaaggtaa gaagttttag gttatgtgaa ttatataaag 4800
   gatgttattt tggtataaaa tttatttttt gttgtattaa atagtagaga aatttttgaa 4860
   gatttatatt gattatgata tttgttttta aaaatatatt aatgaaagtt ttgaagtagt 4920
   atttttattt taatggggtt ttaaaaattt taatttgtga gttaattaat atgttttatt 4980
   attgaaagtg gaatataaaa gtaagtttta tttgaatttt aaaaaatttg ttatattgtt 5040
   tgtaaaagtt gttaagttta agtaggtaat atttaggaga ataaagtatt taagagttat 5100
   ttgttttata tatattttat ttttttttcg ttgagtgttt tttttttttt tttttaagaa 5160
   gtgaatttta ttgttgtttt atatttttat ttatatttta gttatttttt agatgttttt 5220
   ttattatttt aattgtaaat agtggttttt tttaaagaag taaattagat tattatgata 5280
   atggtgtata attattttgt tattgtgttt tattttgttg tatagtagaa tagagaaatt 5340
   ttaaattttt ttgtagtgag gtttttatga tattaattat ttttttttaa tttttataat     5400
   ttttttttcg taaaggagtt attatttgtt ttggtgtttg gttttttttt aagatgaagt     5460
   taattaatat tat                                                         5473
<210> 23
   <211> 5328
   <212> DNA
   <213> Artificial Sequence
<220>
   <223> chemically treated genomic DNA (Homo sapiens)
<400> 23
   ttgataagag aaaaggtgtt gagggtaggt cgcgttgttt tatttatagt ttattgtttg 60
   ttgtagagtt tttgttagga ttggaaagtt aatttagtgt tgattaaaag tattatagag 120
   ttaaaggatt ggtaatgatt gttaatttta ttttagtttt atgtattttt aattgttgta 180
   attaaagata cgttatttaa atataggtga agggtttttt ttttttaagt tgggttgggg 240
   gggggtgggt gggtggatta taattttatt tagatttttt attttatttg ggtgttatgt 300
   agttttaaat tagattttat tattattatt atttagagtc gttttttgtt atttatattt 360
   ggttttgtag aatcggaagt gtgaaatgtg ttatttatat aaaagttttt gtttaaagaa 420
   agggtgggac gggggagtgt agaggagtaa ttatttaggg gtgatttaaa tatttaggtt 480
   tttttttttt tttttttaaa ttaatagaaa ggttttcgat atagttaaaa gtatttgaaa 540
   ttgggggtaa ggttaatagg ttaaagggat tagtgttagt tttttaagta tttttaaaaa 600
   gattatgtga atagatggaa aaatatattt ttaattaaat aatttttttt atattatagt 660
   tgtagtgttg gtttagaaat cgtgtaattt tttattcgat aggagttttt ttatgagaat 720
   attaaaggat aaaaagtttt gtattttcgt agttggttaa aattgataat tattttttta 780
   tgcgcgcgcg cgtatatata tatatatata tatatatata tatatattaa tatatatata 840
   tatatatata tatatatata tatatatata tatttttaaa aaagaattta gtttttaatt 900
   tttttttatt tacgaagatt aaagttttgt attcgatttc gcgagcggtt ttcgtttttc 960
   ggggttggaa ttcgatttag atgtgaagcg gtattgagtc gttgcgtagg ttcgtgttta 1020
   gcgttttttg ttggatatag taggagttgc gtagcgttag ttttagaaat cgttggaatt 1080
   ttgttttggg gttagagaaa atttttttat tgttttttaa aaaagaaacg ggatgcgaaa 1140
   ataaattttt atatttttga aaaataatat aatattaatt taataaaaaa ttaaagaggc 1200
   gatattataa ttttaattta ataaaaaata aagattgttt tttttatatt ttagttattt 1260
   tttattcggt ttggttttgt aatttttttg aaaggttttg cgatggtaaa ataggtaaag 1320
   aaaagtgacg aaaaataaag tatttttttg tagtcgtgga gaattaagta aagtatattt 1380
   ttttcgtttt gtttatattt taaaaaatta aacggaagat gttgttgttt gttgttttga 1440
   cgttgttagt agcgttttga atgtttttag aggttttgta agtatcgtaa ttgagttttc 1500
   gagtaaacga aataatttgt ttttatgtgt ttatttttag agtataatta aaagattagt 1560
   tttttttttg agtgtttttt attttttaga ttaattttgg ttaaagtttt ttttttttag 1620
   atattggtta agaaatatat attggttaag aaataggtgt ttggtatttt ttttttttta 1680
   ttttagtcgt aggatttcgg ggaaggtgtg ttaatggtag ggaaggtttt ttttgaaatt 1740
   tagtatttcg ttgattagga atttaaatta ttttagggaa gggggagtat ttttatattt 1800
   ttaaaaaatt atttgtttat ataataaaga gttttgtttt ttttattatt attatttttt 1860
   tttttttttt ttggttagta agtgagggta aaagtagttt ttgaattatt ttagaaagag 1920
   aaattgtaaa aatggtatat tttttgaatt taaaataata gggaaatgtg ggtttgttta 1980
   attgtttaaa attaaataga aataattatt gtgttaaatg aataagatgt tttaagattt 2040
   atatttttag ggttatggtt attgatttta attttatttt taagaagttt agagatcgcg 2100
   tttttaaagt ttgtttattg acggaaatcg gtttaatttt ttataaaaga atggtttttt 2160
   aaaatattga atattatggt tatgtaaggt ttatgatatt tttatgttta ttttttttat 2220
   tttttaaaat tagcgaattc gagataatat tttaagggga attgttattt agtgcgatga 2280
   gagatgattt ttttatttgg ttttagaatt agatttcggt ttaggttttt tagtaagtag 2340
   gtttagatgt aatggaagga gagaagaatt aagttttttg ataaaatttt tatttcgaat 2400
   tttatggaaa agataggtaa gcgggatttt agttatagta tttttatttt ttgttttatt 2460
   aaaaggtttg tttttattat agaaataata tagtcggtgt tagttttgtg aattcgcgtt 2520
   aatcgataag gtaagaaacg gtgcgttgat agtcgaggtt cgaaggttgt ttagtggttt 2580
   tcgtagttcg tggggtaggg aggttaaagt tagtaaaatg ttggaggcga cgttagaaag 2640
   agagtatatg tttgttattc ggaagtgatg tcgaacgtgt aatagtagag ttttggagat 2700
   ttttatttcg tttcgtattt agggatttta gtttgtgggg tagtaggttg ttgggtgaag 2760
   gtaggaggga gaagtcgtat ttggttgagg gcgggtgggc gtgtcgggag cgagtgttta 2820
   atttttaggg gtttatggag aaagtataat ttttagttga ggaaagagag ttaaataagt 2880
   gggtggttta ggtttgggaa tgttatttag ttttttcgac gtagtgttat ttttgtggtt 2940
   ttagggggtt ttagtatttg ttcgaatttt attttttaag gtcgttagta ggaaggtagt     3000
   ttttttattt ttgagggttg aggagggggt agcgagtgat ttgatttttt agtagtaggg     3060
   aaaatcgtag cggttggtaa cgggtcgcgt tttttcgcgg cggtaaagcg ttttttaggg     3120
   gcggggcggg tatttttggt tttttgtttt agcgtttagg tttgagttcg cgcgggttgg     3180
   ttgggcgttt tgttttgtaa gtaattaatt ttgttggatt ttgaggatta tttaggcgga     3240
   tgggtttatt tagtttaggt ttattgagat tttgttaatt ttttagattt tttttttttt     3300
   tattttttat attttttatg atgaatttag gaaagaattc gaattttgaa aaatagtata     3360
   tgtggtttga agtttgatag ttatttgggt gtgagggata tgattttatt aataaagatt     3420
   aagatttaag gttgagtttt tatttattaa atttgttttt ttattttgtt tttaaatttt     3480
   ggagattttt tagtttcgtt gttttaattt tgatatagat aaaggcgtta gggagaaagt     3540
   ttaggcgggt ttgaagtagt tgaggtatag attgatattt atttttttat tatttagacg     3600
   ggtagataag tattgttaga ttgggggttt taatttaggg tttagcggtt acgtgggttg     3660
   ttgcggggtg ttgaaattat aagtataatt tgatattatt gagtttacgg tgtttgttaa     3720
   taatttgtga attaggtgta gagattgtta ttgtagatta taatagtttt tgaaggattt     3780
   attatagttt atcgtgttaa tgatttttaa attaaggagg ggaggtgggg cgagacgaaa     3840
   aaggagtggg gagagttagt aaaaagggaa gaagggtggg agggaagata aaagttagag     3900
   ttttagtttc gaaggagttt ttaaagatta ttaggttagc gatttgagaa ttttattggg     3960
   atgggtttgg aaatttaatt ttatttgagt taggattgga agaggggcgt tgggggtaag     4020
   tagaagtggg tttttgggaa ggtcgatttt ttaggtattt tttttgaggt cggaatagtt     4080
   tttgggagag ggttttagat gttaatggga ggttttgagg atgttagttc gtttaggatg     4140
   ggtgtatatt ttgatttaga gtatttttta gggtaagtcg tagttgtaga tttttgaaaa     4200
   aagtggtaat agtttcgttt agggatgatg gtgattgtag gatgattttt tttttttaat     4260
   ggtttgtttt aatttttttt tatgttatag gtgaaagttt tttttttttt tttttgatag     4320
   tatttttgtt tgttttttat atgttacgta gatttaattt ttttttttaa tttgtttttt     4380
   tgggtgttaa atatattagt tttttgagat tttattattt ttgtggtttg tattgagttt     4440
   aattgtaatt ttggaaaggg aatttgtgga taatatagta gtagtatgtt ttataagatt     4500
   ttagaagaaa gaagattaaa ttttatttat gcggtgtggt aggggttagg ttaagggaaa     4560
   gaggtttttt ttgtttggta aattgttttt gtagtgaaag ggtagtttag tttttattgg     4620
   agtgttattt aatattggtt agagaaaggt taaggaaagt gtaggtttgt tttttttata     4680
   aaagttgtgg ttataattaa acgaagtaat tatagttgga gaatttaaaa taattgtttt     4740
   ttttttaaaa tgagggtaat aattttagtt attggaaaat tgggatttta aggaaaattt     4800
   ttttttttgg tataattaat tgtataagat agtgtggtta attagagata tatgtattta     4860
   gatttaagta atgatttata ggtgtaaaag gtttagtggt tttttgggtt atttggagtt     4920
   tttattgatt attttgtttt ttaaaaaatt tttttttaat tttaaaaata aaagttatag     4980
   atttaatgta attgaaaatt attgatggta gttttttagg tttttagttt atgtttttaa     5040
   agggaattta tatttaaaat aattaaatta tttaatggtt atattatatt tttttttgga     5100
   taatagttgt agtgtaggga tagaatttta atttatatta aaattgtttg ataatttaga     5160
   aatggaaatg aaattgtgaa ttaatttttt tagaaaatat ttgagttaga atataagaag     5220
   gatgtttata tataatattg taagtaatat gatgattatt atatgtagtt tgaaagattt     5280
   atataaatga ttatatatta tttttgttgt tataatttaa agtaaggg                5328
<210> 24
   <211> 5328
   <212> DNA
   <213> Artificial Sequence
<220>
   <223> chemically treated genomic DNA (Homo sapiens)
<400> 24
   tttttatttt aaattatagt aatagaagta gtatataatt atttgtgtag gttttttaag      60
   ttgtatataa taattattat gttatttata gtattatata tgaatatttt ttttatattt     120
   tagtttaaat attttttgga gaaattaatt tataatttta tttttatttt tgaattatta     180
   agtaatttta atgtaagtta agattttatt tttgtattat agttgttgtt taggagaaaa     240
   tgtagtgtga ttattaaatg atttaattat tttaaatata aatttttttt aaaaatataa     300
   attgaaaatt tgaagagttg ttattagtgg tttttagttg tattaaattt atgattttta     360
   tttttaaaat tagaagaaaa ttttttaaaa ggtaaaatga ttagtaggaa ttttaggtag     420
   tttaaaaagt tattggattt tttatatttg taaattattg tttgagttta aatatatatg     480
   tttttgattg attatattat tttatgtaat taattatatt aaaggaaaag gtttttttta     540
   gaattttaat tttttagtgg ttagggttat tatttttatt ttagagaaaa aatagttgtt     600
   ttaaattttt taattgtgat tatttcgttt agttgtgatt ataatttttg tgggaaaaat     660
   aaatttatat tttttttaat ttttttttag ttagtattaa atgatatttt agtgaaggtt     720
   gggttatttt tttattgtaa ggataattta ttaagtaggg aaaatttttt ttttttagtt 780
   tggtttttgt tatatcgtat aaatggaatt tagttttttt ttttttggaa ttttatggaa 840
   tatattgtta ttgtgttatt tataagtttt ttttttagaa ttatagttgg atttaatata 900
   aattataaaa gtgataaaat tttagagggt tgatgtgttt ggtatttaaa aaaataagtt 960
   ggggaaagaa attagattta cgtaatatgt gagggataga taggaatatt attagaggaa 1020
   aaggaagaaa gtttttattt gtggtatgaa aagaagttaa agtaaattat tggaaaaaaa 1080
   aagttatttt ataattatta ttatttttaa acgaaattat tattattttt tttaaagatt 1140
   tgtagttgcg gtttgttttg gaaagtgttt taaattaaag tgtatattta ttttgggcgg 1200
   gttggtattt ttaaggtttt ttattgatat ttagaatttt tttttaaaag ttatttcggt 1260
   tttaaaagaa atgtttgaga agtcggtttt tttaggggtt tatttttgtt tgtttttagc 1320
   gttttttttt tagttttggt ttagatggag ttgggttttt aaatttattt taatggaatt 1380
   tttagatcgt tggtttgata atttttaaag gttttttcgg agttggggtt ttgattttta 1440
   tttttttttt tatttttttt ttttttttgt tggttttttt tatttttttt tcgtttcgtt 1500
   ttattttttt tttttaattt gaagattatt gatacgatag gttgtaatga gttttttaga 1560
   aattgttatg atttatagtg ataatttttg tatttaattt atagattatt gataagtatc 1620
   gtgggtttaa tggtgttaag ttgtatttgt ggttttaata tttcgtagta atttacgtag 1680
   tcgttgggtt ttggattagg atttttagtt tggtagtgtt tatttgttcg tttgagtgat 1740
   ggagagatga gtattagttt atattttaat tgttttaagt tcgtttgggt tttttttttg 1800
   gcgtttttgt ttgtgttagg gttggagtaa cgaaattgaa agatttttag agtttgaaaa 1860
   tagagtgaaa gagtaaattt aataaatgag agtttagttt tgaattttag tttttattaa 1920
   tagagttatg ttttttatat ttaaataatt attaaatttt aaattatatg tattgttttt 1980
   tagagttcgg gttttttttt gggtttatta tgaaaggtgt gagggatgaa ggggaaaagg 2040
   gtttgaggag ttggtagggt tttagtgggt ttaagttaag tgggtttatt cgtttggata 2100
   gtttttagaa tttagtaaga ttggttattt ataaagtagg gcgtttagtt aattcgcgcg 2160
   ggtttaggtt tggacgttgg gataggagat taaaaatgtt cgtttcgttt ttaaagggcg 2220
   ttttgtcgtc gcggagaaac gcggttcgtt gttagtcgtt gcggtttttt ttgttattaa 2280
   ggaattaagt tattcgttgt ttttttttta gtttttaaag atggaggggt tgtttttttg 2340
   ttggcggttt tggggagtgg gattcgggta ggtgttgggg ttttttggga ttatagaaat 2400
   aatattgcgt cgggagagtt gggtggtatt tttaggtttg ggttatttat ttgtttagtt 2460
   tttttttttt aattaggggt tatgtttttt ttatgggttt tagaaagttg ggtattcgtt 2520
   ttcggtacgt ttattcgttt ttagttaggt gcggtttttt ttttttgttt ttatttaata 2580
   atttgttatt ttataggttg ggatttttgg gtgcggaacg gggtggaaat ttttaggatt 2640
   ttgttattgt acgttcgata ttattttcga atagtaggta tatatttttt ttttggcgtc 2700
   gtttttaata ttttgttggt tttggttttt ttattttacg ggttgcggga attattgagt 2760
   agttttcgga tttcggttgt tagcgtatcg ttttttattt tatcggttgg cgcgggttta 2820
   taaaattagt atcgattata ttatttttgt aatggggata agttttttgg tagagtaaaa 2880
   ggtgggggtg ttgtggttgg aatttcgttt gtttgttttt tttatagaat tcggggtaga 2940
   gattttatta aaaggtttga tttttttttt tttttattgt atttgaattt atttattaaa 3000
   aaatttaagt cgagatttaa ttttggaatt agatggagaa gttatttttt atcgtattaa 3060
   atagtaattt tttttgaaat gttatttcga attcgttaat tttgggaggt gggaagaata 3120
   aatataaagg tattataaat tttgtatagt tatagtattt aatattttaa aaagttattt 3180
   ttttatgaaa aattaagtcg attttcgtta ataagtaaat tttaaaaacg cgatttttaa 3240
   attttttaag ggtaaagtta aaattaataa ttataatttt gaagatgtaa attttaaaat 3300
   attttgttta tttagtatag taattatttt tatttgattt taaataatta agtaaattta 3360
   tattttttta ttgttttaaa tttagaggat gtgttatttt tatagttttt ttttttgaaa 3420
   taatttaaag gttatttttg tttttatttg ttgattaaaa agaagaagaa gaaataataa 3480
   taataaaaaa ggtaaagttt tttattatgt agataggtag ttttttggaa gtgtaaaaat 3540
   attttttttt ttttaggata atttggattt ttggttaacg aaatgttgaa ttttaaagaa 3600
   aatttttttt attattggta tatttttttc gaaattttac ggttaagatg aaagaaaaaa 3660
   gatattaggt atttgttttt tagttagtat gtgtttttta gttagtattt gggaaggaaa 3720
   aattttgatt aggattggtt taggggatgg gaaatattta aagaagagat tggtttttta 3780
   attatatttt ggaaataaat atataaaagt aagttatttc gtttattcga aggtttagtt 3840
   acgatatttg taggattttt gagggtattt aagacgttgt tgataacgtt aaaatagtaa 3900
   gtagtagtat ttttcgttta attttttgaa atgtggataa aacggaggaa gtatattttg 3960
   tttgattttt tacggttata gaaaaatgtt ttgtttttcg ttattttttt ttatttgttt 4020
   tattatcgta gaatttttta aaagagttat aaaattaaat cgggtggggg atggttggga 4080
   tgtgaggagg atagttttta ttttttattg aattaaaatt gtagtatcgt ttttttagtt 4140
   ttttattgag ttaatattat attatttttt aaggatataa aaatttgttt tcgtatttcg 4200
   tttttttttt ggagggtaat aaaagagttt tttttaattt tagagtagga ttttagcgat 4260
   ttttgggatt ggcgttgcgt agtttttatt gtgtttaata gggggcgttg ggtacgaatt 4320
   tgcgtagcgg tttagtgtcg ttttatattt gagtcgggtt ttagtttcgg aaggcgagag 4380
   tcgttcgcgg ggtcgagtat agagttttag ttttcgtgga taaaaagaga ttaaaggtta 4440
   aatttttttt tggggatatg tgtgtgtgtg tgtgtgtgtg tgtgtgtgtg tgtgtgtgtt 4500
   ggtgtgtgtg tgtgtatatg tgtgtatgtg tgtgtgcgcg cgcgcgtatg ggggggtggt     4560
   tattaatttt aattaattac gaaaatgtag agttttttat tttttgatgt ttttataaag     4620
   aaatttttat cggatgagaa attatacggt ttttgagtta atattgtagt tatagtgtgg     4680
   gggaaattat ttaattaaaa atatattttt ttatttgttt atatgatttt tttagaagta     4740
   tttaaaaagt tgatattgat ttttttggtt tgttaatttt gtttttagtt ttaaatattt     4800
   ttaattatat cgaaggtttt tttattaatt tgaaggagaa aaaaaggaga tttaaatatt     4860
   taaattattt ttaaatagtt atttttttgt attttttcgt tttatttttt ttttaaatag     4920
   gggtttttat gtgagtgata tattttatat tttcgatttt gtaaagttag gtataaatga     4980
   taaggagcgg ttttaaataa taataataat aaaatttaat ttgggattat ataatattta     5040
   agtagagtag aagatttaaa taaaattgta atttatttat ttattttttt ttaatttaat     5100
   ttaaaaaaaa aaaatttttt atttgtattt aaatagcgtg tttttaatta tagtagttgg     5160
   ggatgtatga ggttgaaatg aaattagtag ttattattag ttttttaatt ttgtggtgtt     5220
   tttgattagt attaaattag ttttttagtt ttaataaaga ttttatagta agtagtgggt     5280
   tgtaaataag atagcgcgat ttgtttttaa tatttttttt tttgttaa                 5328
<210> 25
   <211> 4324
   <212> DNA
   <213> Artificial Sequence
<220>
   <223> chemically treated genomic DNA (Homo sapiens)
<400> 25
   gttttttagt attattattt taaaggtatt tttatttttt ttattattag ggaaggagtg       60
   tgtagttttg atttagtttt ttagtggagt aaagggaatt tagaggaagg gggatttttg     120
   tagaaattgt tagttatata agtaagtttt ttttggtttt tcgttttatt aaaagtgaaa     180
   aattgtttat aggagtgttt atgttttttg tttttttatg tattattaga gttaagaggg     240
   gatttcgtta gtaaggtatt ttttgttttg tttcgtagat tatagttttc gatttttatt     300
   ttattgtttg tgtaatgtgt agaattggag tttaaagtag cgggtttagg attatttttt      360
   aggttcggcg ggtttataga tttttttttt ggttgggaag aggggttttc gtgaaagtag     420
   agtttgataa gttttagtat tgttatggtt aagattatta atattattat tgttgtgttt      480
   ataaatatga agattacggt agaggaggag tcgaaagttt gaggagtggt agaggaagtc     540
   gtagaattaa atttggaaat tacgtttttt gatggggtga tagtggtttt tgattcggtt     600
   tgaagggata tttgaagggt agatatcgtg ttttgtgatt tttatcgagg aattttagga     660
   atagatgtta ttgaattgtt ttgtttaggg ataagtggtg ttttttttag tttttcgtcg     720
   attttgattg gttatgtttt ttgcggtacg gggttggttg tagtggtcgg cgggcgtttg     780
   gtgggtattt cggttttttt aagggtcggt tgtttttttt tattggttat ataagagcgg     840
   tcgttttttt ttagttcgaa gttcgtagta tattcgtagg taaagttttt taagtcgttt     900
   aggtagttag ggagttttgc gtatttgtta gtacggaggt atttttcggg gtagggatat     960
   aatatatcgt tcgagagttt gttttagcga gcgtcgattt cgttcgcgat gtaagtaatt    1020
   gagatcggga gttgttttcg gtagagcgta tttatttcgg ttttaggtgg attgaagttt    1080
   agagcggcgt tgtgtagttg gaagggcgcg cgatagttta agttagaggc ggtttcgggg    1140
   cgcggcgtag gatataagat tttaaattgg tatttgtata ggtagtcgtt ggcgcgtagg    1200
   tggtatcgta ttttttttta gtttgcgggt tcgattttat cggtggtttg gagtatcgcg    1260
   tattttcgcg cggtgtagga gcgttggggt ttttttattt attgtagcgt gtcgttttcg    1320
   agatcgtcgg ggtcggagga tagttaggag aaatttcgta aaggttcgtt ttttagggtg    1380
   tagtgggaac gtttgcgttt tagtgcgatt tagaatagta ggtttttgga gtttttttcg   1440
   ggttttgggt ttgttcgtag gagcgcgagt atagcgcgta gttcggcgtt cgtacgtacg    1500
   gtgttgagcg ttttatttcg taggatgtag gttttttcgg tcgtttgtcg ttttatggta   1560
   gcgtggtgta ggttgtagta ggttttcgag gtcgagtagt tagtacggtc ggtagtgggg    1620
   tgttcgtcgt cgttcggttc gggttagagc gtttgttaga ggaggtatag ggcgaacgtc    1680
   ggttttattt tttgaggttt cgtacgtaga gttgttttta atttggattt gtttcgttcg    1740
   aggacgtaga gttgtgtttg gagcgcggtt atcgtttttt atagttggtt tttttaattc    1800
   ggtttttttt tttttttttt tcgttttcgt tttcgacgga ttttttaatt gttaagaatt    1860
   ttttcgcggg tttagcggtt aatagttttt tttaggtcgc gataggaaat gtgtgcggag    1920
   cgttgttagg ttcggtgtag ttttgagtta ttttggggtt aggaatatag ttggcgtcgg    1980
   gtatagggga cgtagtcgat tagaaggtta aatattttgt ttagagtaag aggagaaatt   2040
   cgttattggg atgaagtgtg tggttttttt tcgtttaggg agttttatta ggatcgtaga   2100
   atttataata cgtaagcgaa tataggtagg aaggatttga gaaaggaatt ggtttatgat    2160
   tataagatta cgttcgttta ttgtgttttt gttcggagtt gtggagttag cgttagaata    2220
   attcgtagac ggttttagtg tttggggggt ttgtgtttgt gggggaaaaa tatgttgtga   2280
   aataatttag taattgtttt tgttattaga ggtagtatta tttaatcgta tattttttta    2340
   tgggtttttg tttttttatt atgttaagag attttaggag ttttaattat taaagaattt    2400
   ggagttgttt gggtatttta aatgggaaat tttgtttttt ttattagtat ttgttttaga    2460
   aatttatttt ttatttttac gtgttttttt ttgagttaga ttatattgat ttattataga    2520
   aaggaaatgt atttttagga ttaaatgaaa aataatgatt atagatttta atagttaggt    2580
   tgttagtttt ttttgttatt gtattggttt aaaaattttt taagtttata ggttatatat    2640
   aaatgagttc gtggttttat aaatattcga gaataattgt tttatgtgtg attttagttt    2700
   tgaattattt tagtattatt tttttttatg attgagtggt agtatttata atagtaagtt    2760
   gtaaatattt tttttaagga aaattagagt tttttaagaa attaaaatgt ttattttatt    2820
   ataatgaata ttgtatattg ttaaaaaata ttttgtaatt tgaaagatgg gtgagttaat    2880
   ttttttagaa ttttagtggg tgttttgttt taatataaat attttaattt agtgggaagt    2940
   ggtaaggaat taaagatgaa taaagaagta aaggttaaaa attgtattgt tatgaaagga    3000
   ttttttgtta gtaatttaga taatttagta ttagtttaga aaacgtatat tttattttgg    3060
   tttataatgg ttaaatacgt tgttattaga aatatataga gagttttagt taagtttttt    3120
   tagagatgtt tgatgttttt aaatattttt aattagaatg aaagttgtta ttttatggat    3180
   aatgtatata agtttatttt gttttattgt atttaggttg atgaggggtg ggagggaagg    3240
   ttttaggatt atatttgtat gtttttattt tatttttatt ataattttta tttttttttt    3300
   tttttggaaa ttttatgtat tttaataaat aatagtaagt ttatgagtta ttattatatt    3360
   tattttgttt ttgttgagta tggagttttg tattaggatt ttttttttgt tttatttttt    3420
   aatttaaaga atatttttta gttttttttt tagatgtttg agaattattt taaacgtggt    3480
   tttgcgttta aattaggtaa tttagtgttt tataagaaag aaatatgaaa atttagaaat    3540
   taaattgaat ggtttttaaa aaatatttag ttatttgggt tagtttttta tagattattg    3600
   taattttatg attgtgtttg taaatgtgtt tatgtagtgt aatattttta ataaaatttt    3660
   ttttaaattt aagtagttaa tggaaatttt ttagatagtt ttaaaaatcg tttgggatga    3720
   gatgaggtgt ttatagtaag aaagaatgaa tagttggaaa gtttaagtaa atatatgatt    3780
   ttattttttg atattttgtt taggggagtg ggattatagt agagaatata ttggttataa    3840
   cgattatatg agagtattga ttatgattat gattatatta tggaatgata taagagttag    3900
   aaaaaattag tttaaagtag gacgtaaata gttttgtaat tagtttgaaa tagatagttt    3960
   atttgattaa attagaaatg gaggaaagtt tttatttttt aataatattt attaagataa    4020
   taatatagaa ttattttatt tttattggat tttaaatgtt taaaaatttt ataatattaa    4080
   atgtgaattt attggttttt ttatatgtta tagattagag taattatttt ggaaaaattt    4140
   gatatttttt tttattaaaa ttgaatattt gtaaatttta gtatttttat gataaggtat    4200
   aaaattaaga gaaatttttg tatatgtata atagaagata tgtaaaaaat gtttatagtg    4260
   atattattta tattaataaa acgttgaatt aattaaaata tttaaaaata gtataaaaat    4320
   aaat                                                                4324
<210> 26
   <211> 4324
   <212> DNA
   <213> Artificial Sequence
<220>
   <223> chemically treated genomic DNA (Homo sapiens)
<400> 26
   gtttattttt atattgtttt tggatatttt gattgattta acgttttgtt aatatgaata      60
   gtgttattat aaatattttt tatatatttt ttgttgtata tgtgtaaaaa ttttttttag     120
   ttttatattt tattatggaa gtgttagagt ttgtaaatat ttaattttag tgaaaaaaag     180
   tgttaaattt ttttaaagtg gttgttttaa tttgtaatat gtaaaagagt taatagattt     240
   atatttggta ttataagatt tttaggtatt tggaatttag tgggggtaaa atggttttat     300
   attattattt taataaatat tattaaaaaa tagaaatttt tttttatttt taatttggtt     360
   agataaattg tttattttaa attaattata aaattattta cgttttattt tgagttaatt     420
   ttttttaatt tttgtgttat tttatgatat ggttatggtt atggttagta tttttatata     480
   gtcgttatgg ttaatgtgtt ttttgttata attttatttt tttagataga atgttaggga     540
   gtgagattat gtatttattt aagtttttta gttatttatt tttttttatt gtagatattt     600
   tattttattt taaacgattt ttaaagttat ttaagaaatt tttattgatt atttaagttt     660
   gaaaaagatt ttattaaaaa tattgtatta tatggatata tttataaata tagttataag     720
   attgtaatgg tttatagaga gttggtttaa gtgattaagt atttttaaaa aattatttaa     780
   tttgattttt ggatttttat gttttttttt tataagatat taagttattt aatttaaacg     840
   taaagttacg tttaaggtga tttttagata tttgaaggaa ggattaagga atgtttttta     900
   aattgggaaa tagggtagga gagaaatttt agtgtaaagt tttatattta gtaaaagtaa     960
   ggtaagtatg atgatgattt ataggtttat tgttgtttgt taagatatat ggaattttta    1020
   ggaggaaaga agaatgagag ttatggtaaa aataagatgg aagtatatag atgtgatttt     1080
   gaaatttttt tttttatttt ttattaattt gaatgtagta aaataaagtg agtttatatg      1140
   tattatttat ggaatggtaa tttttatttt gattaaagat atttaaaagt attagatatt     1200
   tttaagaggg tttagttgaa attttttatg tatttttgat ggtaacgtat ttggttatta     1260
   tgaattaaga tagaatgtac gttttttaaa ttgatattga attgtttaag ttgttaataa     1320
   gaaatttttt tataatagta tagtttttaa tttttatttt tttatttatt tttaattttt    1380
   tgttattttt tattgagttg aaatgtttat attaaaataa aatatttatt ggaattttaa    1440
   agaaattggt ttatttattt tttaaattat aagatatttt ttgatagtat ataatattta    1500
   ttataatgga ataaatattt taatttttta aggaatttta atttttttta aaggagatgt      1560
   ttataattta ttattataaa tgttgttatt taattatagg agaaaatgat attaaaatag    1620
   tttaaggtta aaattatata tagggtagtt gttttcgaat atttataagg ttacgaattt    1680
   atttatgtat aatttataaa tttgaagaat ttttgaatta atatagtggt aaggaaagtt    1740
   aataatttaa ttgttaagat ttatggttat tattttttat ttagttttag aaatatattt    1800
   tttttttgta atgaattaat atggtttaat ttaaagagaa atacgtggaa gtgaaaaata     1860
   aatttttaaa gtaagtgttg atggaagaag tagaattttt tatttaaaat atttaaataa     1920
   ttttaaattt tttagtagtt aggatttttg aaattttttg atataataga gaagtaaaga      1980
   tttatagaga gatatgcggt taaatagtgt tgtttttggt ggtaaaagta gttgttaagt    2040
   tgttttataa tatatttttt ttttataggt ataggttttt taaatattgg agtcgtttac     2100
   gaattatttt gacgttaatt ttatagtttc ggatagaaat ataatagacg ggcgtggttt    2160
   tataattata agttagtttt ttttttaagt tttttttgtt tatgttcgtt tacgtgttat      2220
   gggttttgcg gttttgatga aattttttga acggagaaaa attatatatt ttattttagt     2280
   ggcgaatttt tttttttgtt ttaagtaggg tgtttgattt tttagtcgat tgcgtttttt    2340
   gtattcggcg ttagttgtgt ttttgatttt agaataattt agggttgtat cgggtttggt    2400
   agcgtttcgt atatattttt tgtcgcggtt taagggaaat tgttggtcgt tgggttcgcg    2460
   gggggatttt tggtagttgg ggggttcgtc gggagcgagg gcggagggga agggaggggg     2520
   aatcgggttg gggaagttag ttgtagaggg cggtgatcgc gttttagata tagttttgcg    2580
   ttttcgagcg ggatagattt aagttgggag tagttttgcg tgcggggttt tagagaatga    2640
   ggtcggcgtt cgttttgtgt tttttttggt aggcgttttg gttcgggtcg ggcggcggcg     2700
   aatattttat tgtcgatcgt gttggttgtt cggtttcggg ggtttgttat agtttgtatt    2760
   acgttattat gaagcggtag gcggtcgagg aggtttgtat tttgcgaggt ggggcgttta    2820
   gtatcgtgcg tgcgggcgtc gagttgcgcg ttgtgttcgc gtttttgcgg gtaggtttag    2880
   ggttcggagg gggttttaaa gatttgttgt tttgggtcgt attggagcgt aggcgttttt    2940
   attgtatttt ggagaacgag tttttgcggg gttttttttg gttgtttttc gatttcggcg     3000
   gtttcgaaag cgatacgttg tagtgggtgg aggagtttta acgtttttgt atcgcgcgga    3060
   gatgcgcggt attttaggtt atcggtgggg tcgagttcgt aggttggaag gagatgcgat    3120
   gttatttgcg cgttaacggt tatttgtgta agtattagtt tgaggttttg tgttttgcgt    3180
   cgcgtttcgg ggtcgttttt aatttgagtt atcgcgcgtt tttttagttg tatagcgtcg    3240
   ttttggattt tagtttattt gggatcgagg tgagtgcgtt ttgtcgggga tagttttcga    3300
   ttttagttat ttgtatcgcg gacgaaatcg gcgttcgttg ggataaattt tcgggcgatg    3360
   tgttgtgttt ttgtttcggg aggtattttc gtgttggtaa atgcgtagag ttttttaatt   3420
   gtttagacga tttgggaggt tttgtttgcg aatgtgttac gggtttcgag ttggggaagg    3480
   acggtcgttt ttgtgtgatt agtggggaag gatagtcgat ttttgggggg atcggggtgt    3540
   ttattaggcg ttcgtcggtt attgtaatta gtttcgtgtc gtagagaata tggttaatta    3600
   gggtcgacga gaagttggga gagatattat ttgtttttga ataagataat ttagtaatat    3660
   ttatttttga gatttttcga tggggattat agagtacgat gtttattttt taaatgtttt     3720
   tttaagtcga gttaaaggtt attattattt tattagggag cgtgattttt aagtttaatt    3780
   ttacgatttt ttttgttatt ttttaggttt tcgatttttt ttttgtcgtg gtttttatat    3840
   ttgtgagtat agtagtagta gtgttggtga ttttgattat gatagtattg ggtttgttta    3900
   agttttgttt ttacgaaagt tttttttttt agttaaggaa ggagtttatg ggttcgtcgg    3960
   gtttggagag tgattttgag ttcgttgttt tgggttttag ttttgtatat tgtataaata    4020
   atggggtgaa agtcggggat tgtgatttgc gggatagagt agagggtgtt ttgttggcgg    4080
   agtttttttt tggttttagt gatgtatagg gaaatagggg atatgggtat ttttgtgaat     4140
   agttttttat ttttgatgaa acggggaatt aagaggaatt tatttgtgta attgataatt     4200
   tttgtagaaa tttttttttt tttaaatttt ttttatttta ttgaggagtt aaattagaat    4260
   tgtatatttt ttttttgatg atagaggaag tggaagtgtt tttaggatgg tgatattggg    4320
   ggat                                                                 4324
<210> 27
   <211> 5683
   <212> DNA
   <213> Artificial Sequence
<220>
   <223> chemically treated genomic DNA (Homo sapiens)
<400> 27
   ataatgttaa atgtaaattt tttgttttgt ttaatgtttt tattttatgg ttggtaaata     60
   aaaataataa aatagtattg ataataataa tagaaataat taattgaagt tgtagttttt    120
   aattttttta aagataatag gttaaagtga ggtttttata ataagatgtt agttattagg    180
   aaggaagtta gggaaaattt ttttttaggg ggtaattgat ttttattggt ttaatattgt    240
   atgtagtatt gagttaggag gttttatgga gaagtgtttg tttttttttt tttgttgtta    300
   gaataaagtt tataaataat tttgtttttt ttatattttt ggagttagtt agttagggtt    360
   tttatttatg tttaggggat tagatttgga tatttgtaaa agaattgaat atagatggat    420
   agttaagaaa aaaataaatg ttaagattat tattatttat agtttaatga tttagtaatt    480
   taaaaaaatt taagaataat aaaataattt ttaagaagga taagaatttt aaaatatgta    540
   gtttagaaaa ttaaattttt taggtttaga gtttaagtaa aagaaaattt gagataatga    600
   agtttggaat attgggtttg tattttgttt ttgattttgt gtttttgggt aagatattga    660
   atttttttat tttagattta ttttatattt taaggagggt gtaggatttt gtagtttttt    720
   tagtgatggt atattattaa ttagaatttt aattagtttt atttttatta ttttagtaaa    780
   atgatgttaa aaattagatt tcgtgatatg agagaagagg ggaaattaat tatagattat    840
   aaagaaattg tgggatttaa gtttagggtg aggtgtttta ttgaattttt agggttggtt    900
   atagtatttg ttatatagta ggtatttaat aaatgatgtg taatgggtgg ttagattgat    960
   ggaaaggtag gtttttttag tgacggtaaa ggatttgggg taaaagagat ttgtggttga   1020
   gttttgattt ttatatatta gtatattgat tagacgaagt tataagtttt aatttttttt   1080
   tgtgtaaaat ggagataata ttttataggg ttatgaggat taaataagaa aatgtttagt   1140
   ttagtgttta atatatagga agtatatcgt aaatattagt tattattatt attaatattg   1200
   tgattatatt ttttatttaa agattttttt gaggagtagg gggtatttag agataaaaag   1260
   tttgtaggtg ggaaatttgt tagtttttta gggcgttagg tttttgtgga attttgtatt   1320
   ttttttttgt tttagttttt aagaatagaa attttttagg gatttttggt gaggtagtcg   1380
   tgggaagatg aggtgtagaa gtaagttggg atttgtgagt tttaatttcg gttttttttg   1440
   cgttgagatt taagcggatt ttgtttggtt tgtatgcgtt attgggggaa atggacgtgg   1500
   gtttgagcgc ggtaggtgcg agggcgttgt ttcggggtcg attattttgc ggggatattg   1560
   tagttgttat tttttttttt gtaggcgggt aggggaagcg gtggttaaag tgggagtcga   1620
   tcgtttagta tagtttgttt gagtgttgat taggaaagtt taggtttttt ttaaatttcg   1680
   tcgttagatt tggtgacgta ttcgtatgta tttaaggcgt ttgtacgtag aacgttatta   1740
   tagaatgtag ttattttttt taacggttcg ggaaattaga ggttttttta gttatttagg   1800
   gtagaggaat tttttttatt tttatgtgat attttttgat ttagaagaat taatgttaga   1860
   ttttttttgg gtttttttat ttatagttat agtttttttt ttgtttagtt aaaattgagg   1920
   taggtaggaa aatattattg ggggtataag tttattagtg tgtaaacgta tttttatgaa   1980
   gtgtgttttt agggagttat taaaaattga ttttttaatt atagaaatag atgagatttt   2040
   gagaatattg agaagttgtt ttttgtaaag taaatttgta atggtttttg acgaaggggg   2100
   gtcgggggcg gggagaagtt tagtcgagag aggagtttat tttacgttat atttttgtag   2160
   ttgaaaagtt gtttaattat cgttaatcgt ttttcgtata agagttttgg gaagatttta   2220
   gaaataaggt aaatgaagat tttttattgt ttttttcggg ttgtcgtttt cggaagtcga   2280
   agttttagta cgtagagtag taggagaggg ttatttttag gtaattttat tgagtaaaat   2340
   aaattattta atggtataac gttttggttt aaaaaattgg aatttattag aggtaaaaat   2400
   attttttaag aaattatgga tatttcgcgt tttatttatt tttatggtag tagagtattt   2460
   gtattttgag ttatttatat agatttatgt ttcgtatcgt ttttattttt tttttttttg   2520
   aagtaaagtt tttttttaag aaggcggtta gaaagtggat tttatcgggg gaaaaagaaa   2580
   aatgaaacgt aaattagttt ggtattgttt gcgtttttta aaacgcggtg ggataaggtt   2640
   attgagttta tagttaattt ttttatgtat ataaaatgta tatatatgta tatattttta   2700
   tatatataaa agaatttata tatatgtata tagttatgtg gagttttgaa gtaatttttt   2760
   atgtttttgt tttttttaag ttttttaggt ggaggtagtt ataagtatta taagtcgttt   2820
   tagtgattat tagggacgga aatcgttaat tattacgttt ttttttattt ttaggggttt   2880
   tttggttcgt gatataagag gtttcggtat tggcgttttt ttagaattgg tttagaggag   2940
   ttagtttaga gtgtgaggtc gggtttgtat tgaacgtata tatcgaggtt tattagattt   3000
   tttcgatttt agcgaagggt gttgattgtt gtgttgttag aggttagtaa gttttttgtg   3060
   cgtagttgat gagttttagt aattcgttat ttgggcgttt ttttttaaat tttgggagta   3120
   aattgggaaa ataaaaaaat ttttacgttt attggttttt tttttttttt taattttttt   3180
   tttcgattcg tttgtgggag tttttttttt tttgttggga ttataatgtg atgcgtaatc   3240
   gtttgtgaat gaataaaagt tatcggtaag tagggagacg gggatagatc gttgacggta   3300
   gattgagggt ggtagtaaag gttcggtttt taaggataat ggggagtcgt ttttttttac   3360
   gtttggtttt tatggttttt ttcgtttttt aggtaaaatg aatgtttttt tatttattat   3420
   tcgtagagta tttttaggcg tgcgtagaat ttgttgatga aatttattag cgtcgattgg   3480
   gtagttttgt ggagttattc gaggtttttt attgtggttt ttgtttgtag aatttaagta   3540
   tttatataat gtattagtac ggaatttagt attcggtggg gatatcgcgt gttaagtttg     3600
   gcgcggttaa cgttttagcg gtttttttat tcggtagttt tttaggatta ttttcgagga     3660
   ggtattggag tcgggttgta ggcgtacggg taaagaattt agtattttat ttaagtattt     3720
   cgtttttttt ggtcgttttc gggaggttat gtttaaaaat ataaaaataa aaataaaaat     3780
   aaaaataaag ggaggcggat aaagtttcgg tgggtgaatt gaagttgggt ttatgtgatt     3840
   ttgaagtcgg agaaataaat ttaatatgaa ttttgttttt ttggcgggcg ttgggatttc     3900
   gtcgtttttt atgttaatcg ttggaagttt cgtatttaac ggttataggt gtttaggagc     3960
   gtagagaggt ttcgggttta aattatcggc gcgtagggat tggattcgcg ggtagcgatt     4020
   tttttaattt tttttttttc gttttatata tatattttcg cgtcggttga aagtatggag     4080
   gatttagggt atttgaaaaa agaggggttg ggcgcggtgg tttacgtttg taattttagc     4140
   ggtttgggag gtttagaagg gcggattatt tgaggttagg agttcgagat tagtttggtt     4200
   aatattagtt tggttaatat ggtgagattt cgtgtttatt aaaaatataa aaattagtta     4260
   ggcgtggtgt ttgtaatatt agttatttgg gaggttgagg tacgagaatc gtttgaattt     4320
   gggaggcgga ggttgtagtg agtttagatc gcgttatcgt ttttttgttt tgggtgatag     4380
   agtaaggtat tgttttaaaa taaaataaaa taaaacgaaa gattcggtta ggaaagaatt     4440
   tgtaggtatt cgaggcgttc gtattttgta aagtaaatgt aattttttta ttaagtcgaa     4500
   gttttttata tttatttttt tagaggaagg tggtttaatt tagaaatttt ttttaagagg     4560
   attttttatc gaagattatc gcgaagtgtt aggaattcgt tttagtttcg ataggtgtag     4620
   gatttttcgt gtcgttatat tttgggattt tattttttta aataggttat ttaaaagtta     4680
   gtagtgtaat cgggatttcg cggcgataaa gaattattgt gtagaatttt ggagttggga     4740
   gtcggttcgt ttttttttta aagaaatcgg gatttcgcgt ttttttcgtc gttagcgtag     4800
   cgcgttagcg gcgtttaatt agtgagtttt tcgggtttgt gacggttttc ggtttcgttt     4860
   tttcgacggt tataaaaagt cgtagcgaag tttgtatttt cgagtatcgc gcgcggtttt     4920
   gttttcggta cggtttttag gtgcgttttt ttttcggttg tttgtagtat tggttttatt     4980
   gttttcgtcg ttagttatcg gttttttatt cgttttattt tttattgtgg tttggttgag     5040
   tcggtcgtta ggtttcgttg tttttttttg tttttttttt tttttagcgg tcgtattttg     5100
   cgtcgtattt tatttggttt gtaggtgagt agtagcgtag tatttttgtt cggcgagttt     5160
   aatttgttta gttcggtttt tgtcggagtg gtatcggtat ttttttaaga cgtttttttt     5220
   tttgtaggat gaagaatttt atgttggagg tggtgttttt attattggag aagttgtttt     5280
   ttatttttaa ttttacgttt tttagttcgg gcgtcgcggg cgaagataaa gggaggaata     5340
   gtttttatga aattagtttt ttttatcgag gcgacgtgtt ggaggtgttt cggatttatt     5400
   tgatttatta tggtatttat ttaggagata atcgtgttgt ttatatgatg ttcgatattt     5460
   tgttggtttt gatagacgat atggggcgta cgtagaaggt ggtttttaat aagcgtttta     5520
   ttttgggcgt tattgttaaa gtggttagta ttcgcgtgga tatagtggag gatttcgttt     5580
   acggagttaa tattttggtt aattatttgg acgagttttt ttagaaaaag gtattgttta     5640
   acgaggaggt ggcgcggagg gttgaaaagt tgttgggttt tat                       5683
<210> 28
   <211> 5683
   <212> DNA
   <213> Artificial Sequence
<220>
   <223> chemically treated genomic DNA (Homo sapiens)
<400> 28
   gtaaagttta gtagtttttt agtttttcgc gttatttttt cgttgagtag tgtttttttt      60
   tggagggatt cgtttaggtg attgattagg atgttagttt cgtaggcgaa gttttttatt    120
   gtgtttacgc ggatgttggt tattttgata ataacgttta ggatgagacg tttgttggag     180
   attatttttt gcgtgcgttt tatgtcgttt gttagggtta ataggatgtc gggtattatg     240
   tgggtaatac ggttgttttt taggtagatg ttatagtggg ttaggtgggt tcggggtatt     300
   tttagtacgt cgtttcggtg gaaagagttg gttttataaa aattgttttt ttttttgttt     360
   tcgttcgcgg cgttcgaatt aaagagcgtg aagttggaga tgaggagtag tttttttagt     420
   agtaaagata ttatttttag tatggggttt tttattttgt agggaagagg gcgttttgga     480
   gaggtgtcgg tgttatttcg gtaggggtcg ggttgggtaa gttaagttcg tcgggtaggg     540
   gtgttgcgtt gttgtttatt tgtaggttag gtgaggtacg gcgtaaagta cggtcgttga     600
   ggagagagga aggtaaagga ggatagcgag gtttggcgat cggtttagtt aagttataat     660
   ggggaatgag acggataagg aatcggtggt tgacggcggg gatagtgaga ttagtgttat     720
   aagtagtcgg agaaggagcg tatttggggg tcgtgtcggg ggtagggtcg cgcgcggtgt     780
   tcggaggtgt aggtttcgtt gcgatttttt atggtcgtcg agggggcgga gtcgaaggtc     840
   gttatagatt cggaaagttt attgattggg cgtcgttggc gcgttgcgtt agcggcgggg     900
   aggacgcggg atttcggttt ttttgggagg ggggcgggtc gatttttagt tttagggttt     960
   tgtatagtga ttttttatcg tcgcgggatt tcggttgtat tattggtttt taagtggttt 1020
   atttagggag gtagagtttt aaggtgtggc ggtacgaaag gttttgtatt tgtcgggatt 1080
   ggggcgagtt tttggtattt cgcggtagtt ttcggtggaa agtttttttg ggagagattt 1140
   ttgagttgga ttattttttt ttaaaaggat agatatgagg gatttcggtt taataaagag 1200
   attgtattta ttttgtaaag tgcgagcgtt tcgaatgttt gtagattttt ttttgatcga 1260
   atttttcgtt ttgttttgtt ttgttttgag atagtgtttt gttttattat ttagagtagg 1320
   agggcggtgg cgcgatttgg gtttattgta attttcgttt tttaggttta agcgattttc 1380
   gtgttttagt tttttaagta gttgatatta taggtattac gtttggttaa tttttgtatt 1440
   tttagtagat acggggtttt attatgttgg ttaggttggt gttggttagg ttggtttcga 1500
   atttttgatt ttaagtgatt cgtttttttg gattttttaa atcgttggga ttataggcgt 1560
   gagttatcgc gtttagtttt ttttttttta aatgttttga attttttatg tttttagtcg 1620
   gcgcgagggt gtgtgtgtag ggcggggggg ggggggttgg gggggtcgtt attcgcgagt 1680
   ttagtttttg cgcgtcggtg atttgaattc gaggtttttt tgcgttttta ggtatttgtg 1740
   gtcgttgagt acgaagtttt taacggttgg tatgaaaaac ggcggggttt taacgttcgt 1800
   taggaaagta agatttatgt taagtttatt ttttcggttt tagggttata tggatttagt 1860
   tttagtttat ttatcgaaat tttgttcgtt tttttttatt tttattttta tttttatttt 1920
   tatgttttta agtataattt ttcggagacg gttaaggaag gcgaagtatt tggatgagat 1980
   gttaagtttt ttgttcgtgc gtttgtagtt cgattttaat gttttttcga gggtggtttt 2040
   agggagttgt cgggtgaggg agtcgttgaa gcgttggtcg cgttaggttt ggtacgcgat 2100
   gtttttatcg ggtgttgagt ttcgtgttaa tgtattatgt aaatgtttaa attttgtaga 2160
   taaaggttat aatggagagt ttcgggtggt tttataaagt tgtttagtcg gcgttaatag 2220
   gttttattag tagattttac gtacgtttga gggtattttg cggatgatgg atgaaggaat 2280
   atttatttta tttggaagac gaagggggtt atagagatta ggcgtgaggg aaaacggttt 2340
   tttattattt ttggaggtcg ggtttttgtt gttattttta atttgtcgtt agcgatttgt 2400
   tttcgttttt ttgtttgtcg gtgatttttg tttatttata aacgattgcg tattatatta 2460
   tagttttagt aaagaggaga aaatttttat aaacgagtcg aaagaggaag ttggagaagg 2520
   ggagagagtt agtggacgtg gagatttttt tattttttta gtttattttt aaaatttaaa 2580
   gaaaagcgtt taggtggcga gttgttgaaa tttattagtt gcgtataggg agtttgttag 2640
   tttttagtag tatagtagtt agtatttttc gttaaaatcg ggggagtttg atagatttcg 2700
   gtgtgtacgt ttaatgtaga ttcgatttta tattttgaat tggttttttt gggttagttt 2760
   tgggaaagcg ttaatatcga agttttttgt gttacgggtt aaagggtttt tggagatgaa 2820
   aggaaacgtg ataattaacg gttttcgttt ttggtggtta ttaaggcggt ttataatgtt 2880
   tatgattgtt tttatttggg gaatttgagg gagataaaag tatggagaat tgttttaggg 2940
   ttttatatag ttatatatat atatatgaat ttttttatgt atataaaaat atatatatat 3000
   gtatatattt tatatatatg aaagaattag ttatagattt aatagttttg ttttatcgcg 3060
   ttttggaaga cgtaagtagt gttaagttga tttgcgtttt attttttttt ttttttcggt 3120
   ggggtttatt ttttggtcgt ttttttggga aagggtttta ttttaaaaag aaaggaggtg 3180
   agagcgatac gaggtatgaa tttgtatagg tggtttaaga tgtagatatt ttgttgttat 3240
   ggaaatgaat agggcgcgga gtgtttatag ttttttgaag gatatttttg tttttgataa 3300
   attttaattt tttaagttag aacgttatgt tattaagtga tttattttgt ttagtggaat 3360
   tgtttgaaag taattttttt ttgttgtttt gcgtgttagg atttcggttt tcggaagcga 3420
   tagttcgaag gaggtagtga aaagttttta tttgttttgt ttttgaagtt tttttagaat 3480
   ttttatgcgg gaagcggtta gcgatgatta ggtagttttt taattgtaaa aatatagcgt 3540
   ggaatgagtt tttttttcgg ttggattttt tttcgttttc gatttttttt cgttaaggat 3600
   tattgtaaat ttattttgta aaaggtagtt ttttaatgtt tttaaaattt tatttatttt 3660
   tgtggttgag aggttagttt ttaatggttt tttggaggta tattttataa aaatacgttt 3720
   atatattaat aggtttatgt ttttaataat atttttttat ttgttttaat tttagttaaa 3780
   taaagggaag attatagttg taggtggaaa ggtttaagag aaatttaata ttagtttttt 3840
   taaattagaa gatgttatat ggagatagga gaaatttttt tattttgagt agttggagag 3900
   atttttggtt tttcggatcg ttaagaaagg tggttatatt ttgtgataac gttttgcgtg 3960
   taaacgtttt aaatatatgc gaatgcgtta ttaggtttgg cggcgagatt tagaaagagt 4020
   ttggattttt ttggttaata tttagataga ttgtgttgag cggtcgattt ttattttggt 4080
   tatcgttttt tttattcgtt tgtagaagaa ggaatgatag ttatagtgtt ttcgtagggt 4140
   ggtcggtttc ggggtagcgt tttcgtattt gtcgcgttta ggtttacgtt tatttttttt 4200
   agtaacgtat ataggttaag taagattcgt ttgggtttta gcgtagaaga ggtcgaaatt 4260
   gaggtttata ggttttagtt tatttttgta ttttattttt ttacggttat tttattagag 4320
   gtttttggag agtttttgtt tttaagaatt aggataggga agaggtgtag agttttatag 4380
   aaatttaacg ttttagaagg ttaatagatt ttttatttgt aggtttttta tttttggatg 4440
   ttttttgttt tttagagaag tttttggatg gaagatatga ttatagtatt agtaataata 4500
   ataattaata tttacggtgt gttttttgtg tattaggtat tgaattaagt atttttttat 4560
   ttaattttta taattttatg aggtattatt tttattttat ataggaagaa attgaagttt 4620
   ataatttcgt ttagttagtg tgttaatgtg tggggattag gatttaatta taggtttttt 4680
   ttgttttagg ttttttatcg ttattgggaa ggtttgtttt tttattagtt taattattta 4740
   ttatatatta tttattgaat atttgttata tggtaggtgt tatgattaat tttagaggtt 4800
   taataaaata ttttatttta aatttagatt ttataatttt tttgtaattt gtaattaatt 4860
   tttttttttt ttttatgtta cgaggtttaa tttttaatat tattttattg ggatagtgag 4920
   aatagagttg gttaaggttt tgattaatag tatgttatta ttaggaaggt tataaaattt 4980
   tatatttttt ttagggtgta ggatgaattt ggggtgggga agtttaatgt tttatttaag 5040
   ggtatagagt taggagtaga atgtaggttt agtgttttag attttattat tttagatttt 5100
   tttttatttg agttttaggt ttgagaaatt tggtttttta aattgtatgt tttaaaattt 5160
   ttgttttttt tagggattat tttgttgttt ttggattttt ttagattatt aaattattgg 5220
   gttataaata atgataattt taatatttgt ttttttttta gttatttatt tatatttagt 5280
   ttttttataa atgtttaggt ttggtttttt ggatataaat aggggtttta attagttaat 5340
   tttaaaggtg tggggaaaat aaagttgttt gtgaattttg ttttgatagt agaggaaaag 5400
   aagtagatat ttttttatgg agttttttaa tttagtgtta tatgtagtgt taggttaata 5460
   gggattaatt attttttggg aggagatttt ttttggtttt ttttttggtg attagtattt 5520
   tattatgagg attttatttt agtttattgt ttttagaaag attaaaagtt gtagttttag 5580
   ttggttgttt ttgttgttgt tgttaatgtt gttttgttgt ttttgtttgt taattataaa 5640
   ataagaatat tgaataaaat aaagaattta tatttagtat tat 5683
<210> 29
   <211> 2347
   <212> DNA
   <213> Artificial Sequence
<220>
   <223> chemically treated genomic DNA (Homo sapiens)
<400> 29
   ttgaggttta agttagggag gtcgagattg tagtgagttt taattgcgtt attgtatttt      60
   agtttgggtt atagagtaag attttgttta aaaaataata agaaaggtgg aggaaatagg     120
   tagagattag aggaaagtta tggttttttt ttgattattt tgagattttt ttaattttat      180
   taatttttgg agagtttttt atttgtttta ttttatagat tgggaaattg agtttagggg      240
   tatttagatt atttgaggtt ttagttggag atgaagagat ggatttaaat tattttttgt     300
   ggagtatagt tttgtttttt ttagagtgga gatcgtgttt tagggttttt tttattcgta     360
   ttaaggttgg tttttttagg ttttattttt tttatttgta aaatgggttt ttgtgagtat     420
   gaaggagggt ttggtttgta gtgagcgttg tttattcgtt ggttagtgtg attagttatt     480
   gttgttgttt tttagtggtt tttgtttggt agttgttgga aaaatatcga gggagggaga     540
   tatagggttg gggaggtgaa aagttggata tagacgtttt tagttttttg ggattaggaa    600
   tagatggagg tcgggtgttt tgaagtggag tagggaggat tgtttggagg agggatattt     660
   gagttagaat gtatagaatt atagggtatt ttcggtagag gttatagtag aggtaaaagt     720
   ttagaggagg tgttttgggg attattaaga tggatatagt gggtttgggt ttttgtgggg     780
   tgatgggttg gggtttcgag cgttaggtta aagggtttgg tttttttcga gggcggtagg     840
   gagttatggt aggtgtgtga gttggagggg tattttagat aaaggtgaag tggtattttt     900
   ttttttattg aagttataat ttgatatttt ttttgaagtt ttttatattt tatttttttt     960
   ttgttatatt ttggtaagtt tatttttttt ttttcgtttt taggtttgtt aagaggtttt    1020
   gtgggttttt tatggtttta tttagttgtt ttagaagttc gtttgagata agtttttagg    1080
   gttgttttat ttttaggtgt tttatagatt cgggggcggg tgaagtagga aggatcgagt    1140
   tacgagggtt ttagaaaata tgacgggagg aattatagta tttttttatt tttttagttt     1200
   tcgttttttt agggagagag atttttagtt cgaggaggag ggtcgagttt ttttttatgt     1260
   ggtaagagat tttaacgtcg ttgaggatta tttaaagagg gtgttgaagg atgtatagga    1320
   gttagttagg agataattta tttgttaatt taataaattt ataggtatta tttattaata    1380
   agaataattt ttattattta attaggattt tttaatgttt ttgagggatt atttgaggat    1440
   gtttaattat tatttttatt tgagataggg ttttattttg ttatttaggt tggagtgtag    1500
   tgatataatt atagtttatt gtagttttaa ttttttagtt ttaagttatt tttttatttt    1560
   agtttttgat atagttgaga tataggttaa tgttattatg atgtttggtt tttttttttt    1620
   tttttttttt ttttattttt gagatagagt tttgtttagt tatttaggtt ggagtgtagt    1680
   ggtgcgattt tagtttatta taattttcgt tttttgagtt taagtaattt ttttgtttta    1740
   gttttttgag tagttgggat tatagatatg tattattata tttagttaat ttttgtattt    1800
   gtagtagaga tggggtttta ttatgttgtt taggttggtt ttaaatgttt gattttaagt    1860
   gatttgttgg tttcggtttt ttaaagggtt gggattatcg gcgtaagtta tcgtttttcg    1920
   attatttagt tttttttttt ttttttttag tttttggtag aaatgtggtt ttatgtttgt     1980
   attttagtat tttgggaagg tcgaggtggg tagattattt gaggttagga attcgagatt    2040
   agtttgatta atatggagaa atttcgtttt tattaaaaat ataaaattag tcgggtatgg    2100
   tggtttttgt ttgtaatttt agttatttgg gaggttgagg taagagaatt atttgaattt    2160
   ggtaagcggg gtttgcggtg agttaagatc gtgttattgt attttagttt gggtaataag    2220
   agtgaaattt tgttttaaaa aaataaaata aaataaaaat ataaaattag ttaggtatgg    2280
   tggtacgcgt ttgtaatttt agttatttag gaggttgagg taggagaatc gtttgaattt    2340
   ggtaggt                                                              2347
<210> 30
   <211> 2347
   <212> DNA
   <213> Artificial Sequence
<220>
   <223> chemically treated genomic DNA (Homo sapiens)
<400> 30
   gtttattagg tttaagcgat ttttttgttt tagttttttg agtagttagg attataggcg     60
   cgtgttatta tgtttggtta attttgtatt tttattttat tttatttttt tgagatagaa    120
   ttttattttt gttgtttagg ttggagtgta atggtacgat tttggtttat cgtaaatttc    180
   gtttattagg tttaagtgat ttttttgttt tagtttttta agtagttggg attatagata    240
   ggagttatta tgttcggtta attttgtatt tttagtagag acggggtttt tttatgttgg    300
   ttaggttagt ttcgaatttt tgattttaag tgatttgttt atttcggttt ttttaaagtg    360
   ttgggatata ggtatgagat tatattttta ttaaaaattg aaaaaaaaaa aaaaaaagtt    420
   ggatggtcga ggggcggtgg tttacgtcgg taattttagt tttttgggag gtcgaggtta    480
   gtagattatt taaggttagg tatttgagat tagtttggat aatatggtga aattttattt    540
   ttattataaa tataaaaatt agttgggtgt ggtggtgtat gtttgtaatt ttagttattt    600
   aggaggttga ggtaggagaa ttgtttaaat ttaggaggcg gaggttatag tgagttgaaa    660
   tcgtattatt gtattttagt ttgggtgatt gagtaagatt ttgttttaaa agtaaaaaaa    720
   aaaaaaaaaa aaagaaaagt taggtattat ggtggtattg gtttgtgttt tagttatatt    780
   agaggttgag gtagaaggat agtttgaaat tgggaggttg aggttgtagt gagttatgat    840
   tgtgttattg tattttagtt tgggtaatag agtgagattt tgttttaaat aaaaataata    900
   attaaatatt tttaaatgat tttttagagg tattagaaag ttttgattga atgatgaaaa    960
   ttgtttttgt tgatgagtga tgtttatgag tttgttaaat taataaatgg gttgtttttt   1020
   ggttaatttt tatgtatttt ttaatatttt ttttagatga tttttagcga cgttagggtt   1080
   ttttgttata tggagggaag ttcggttttt tttttcgggt tgaaggtttt tttttttgag  1140
   aaggcggaag ttgaggaggt ggggaagtgt tgtggttttt ttcgttatgt tttttggggt   1200
   tttcgtgatt cgattttttt tgttttattc gttttcgagt ttgtggggta tttgaaagtg   1260
   gggtagtttt gggaatttat tttagacgag tttttggggt agttgggtgg ggttataagg   1320
   ggtttatagg gttttttggt aggtttagag acggagggaa ggaggtgggt ttgttaagat  1380
   gtggtaaggg gaaggtggga tgtggggaat tttagaggga gtgttaggtt gtgattttaa   1440
   taaggggaga aatattattt tatttttgtt tggggtattt ttttagttta tatatttgtt   1500
   atggtttttt atcgttttcg gagaaagtta ggttttttag tttggcgttc gaggttttag   1560
   tttattattt tatagaaatt tagatttatt gtgtttattt tggtggtttt taaagtattt   1620
   tttttggatt tttgtttttg ttgtggtttt tgtcggagat gttttgtaat tttatatatt   1680
   ttagtttaag tgtttttttt ttaggtagtt ttttttgttt tattttaggg tattcggttt   1740
   ttatttgttt ttgattttaa ggggttaggg gcgtttgtgt ttagtttttt atttttttag   1800
   ttttgtattt ttttttttcg atgttttttt agtagttgtt aggtagaggt tattggggaa   1860
   taataataat gattaattat attggttagc gggtggatag cgtttattgt aagttaagtt   1920
   tttttttata tttataaaag tttattttgt aggtgggaaa gatgaggttt gagagggtta   1980
   attttggtgc gggtggggga agttttgagg tacggttttt attttggaag gggtaaaatt     2040
   atattttata aggggtggtt tgaatttatt tttttatttt tagttggggt tttaggtggt   2100
   ttgagtgttt ttggatttag ttttttaatt tgtgaaataa ggtaagtgag ggatttttta  2160
   ggaattggtg gagttgggag ggttttagga tggttaggag gggttatgaa tttttttttg   2220
   atttttattt atttttttta ttttttttgt tattttttga gtagggtttt gttttgtggt   2280
   ttaggttgga gtgtagtggc gtaattagag tttattgtag tttcgatttt tttggtttaa   2340
   gttttag                                                              2347
<210> 31
   <211> 6709
   <212> DNA
   <213> Artificial Sequence
<220>
   <223> chemically treated genomic DNA (Homo sapiens)
<400> 31
   atttttaaat tttttaattt atagagggtt ggagagttgg tattatgagt ttttagtttt 60
   tacgggtgag aaaagaggtg tttgtgtgga gggtaggtgc gatttcgggg gttagtagag 120
   aggagaggtt gcgggaggat tcgttgcgaa gggcgagggg tggcgttggg tgggacgggc 180
   gtttggggtc gtgtagggtg gtttttgggt gttgttcggg ttgtttgtcg tttagtattg 240
   gcgtaggaag acgggttcgc gtagcgtttg gtaatagtgg tttgttttac gtttggaggc 300
   gattaagttt tgagttaaag gcgtcgggtt tagtcgggaa gtgtatggcg atttagaata 360
   cggagcggag aaggttttag gggatacgag gttatcgttt tagttttcgg gtttgcgttt 420
   cgagcggtta gggtgcgtac ggatgggttt cgggatgtta gtggagaggt gataggagtt 480
   tattatttta cggttaagcg tgtgaggttt tcggcgggcg gggttttttt tcggggtagg 540
   gtagggggag agttaggtat gtttttaacg ggttttttag tagaatcgtt aggaaagtgg 600
   tttttaattt taggtatttt tgtggttttt aggtagttgg gcgattaaaa atttattttt 660
   gtttgttgtg aaatgggata ggtggttgtt aggttagaga ttgtgtttac gaagtttcgt 720
   ttggcgggtc gggattgacg tggattcggg tttttttcgg aagtttggga atttgtgagt 780
   tttttagttt ttcgttttat tttgtttgtt tgaaaaaagg agttgtttga gaggaagagt 840
   taaggtttaa aaggttttaa acgaagagta tgcggtttga cgcggagcgt aaagaagcga 900
   gtagtgaatt gaatttggtg ggattgagat tagaggcgtt tgtatgcggc ggtcgtttcg 960
   cgttttatta aagtaatttg ttatttacga taattatttt tgtaattatt ttggagtggt 1020
   tgtgatttga tgtttgtggt gttttagggt atgtttttat tggatataga tattttaggg 1080
   tgttttttga cgttagtgtc ggtttagtgg gtgttagtgt cgcgaaggga gggtggcggg 1140
   cgtgaatgtt tttttttttt gggtttgtcg taggtttcgg tttttttatt ttgtttgatt 1200
   ttggcgggtt tgttatttat tgaggttttt atcggcgttt acgttgtttc gagaggcggg 1260
   ataaacgttc ggcgcgtggc gcggagtttg cgaaagcgtt cgggggtttt tattttttgt 1320
   tcgggagttt tttttttggg gcgggaagag gagtcgtaga tttgttcgaa ttttgtttta 1380
   ttcgtttggt cgtttttttt cgtttggggt gggggtaggg gtggcggatg atttaggttt 1440
   ggggtcggga gttttgtttt taaaggtatt atttcgcgtt tttttggaag tggcgcgttg 1500
   ttggggcgtt ttcgttgttg tagaggtttg gtttgcggtt ttataggggc gtgggtttag 1560
   ggtttttagg gagtaagtga gtttaggttt tttagggggc ggtagagggg aattttcgat 1620
   aattttttat tttttttttt aggcggttgc gaggttttcg gggttttaga ggggtttttt 1680
   tttgtcggtg tcggttgttt tgagcgcggt tacgtagttc gcgtttttac gtgggtaagg 1740
   ggcggggacg ttggagggag gttgttttgg atgttggagt tttcggtttt cggttttgtt 1800
   gagtagaaaa gttgaagtta aggaagggtt gaggtttttt tataagggcg cgcgcgtgtt 1860
   tgggtgtcgg tttagaagtc gagaatgtgc gtattgggga agggtttttc gtgtcgagaa 1920
   gatagaggat ttgttttagt tgtttttcgt atagagggcg cggaggttgc gttttagttc 1980
   gaacgtttat ttattggaaa gagggtagcg tcggggttta gggaagtttt ttgggaatga 2040
   atggtttttg ttaagcggtt tcggattttt tgggtttttt gggtttacgg tacggtgttg 2100
   cgcgagtttt tagtgtttat cggttttttt cgttttttgc gtagacgttt ttaggcgggg 2160
   aggtatatcg gttttttcgg gtagttttgg ttagtgttgt tgtgggaaag gagagttagg 2220
   gtttgggatg ggggatgagt atttttttgt ttatttcggg ttttagcgtg taggaggtaa 2280
   atttgttagt atagataaga tagtttgttt aagttgtatt ttaggtcggg ttagagaata 2340
   aaatcgaggg ttagaaggtt tagaatgtcg gatagtttag cggtattcgt tagggagttt 2400
   taggcgttcg aaagaggcgt cgtatttttg gcgagtttag gatttatttt tttggatttg 2460
   tgttttggag tgtttgcggg tttgggttta atttttgttt ttggtaggtg tttttttttt 2520
   tcgtcgttaa tattaggagc gttgtttttg cggtaattta ttttattttt agaagtttgt 2580
   tttgggatcg aagtgttagg gttttgtgtg tttttttatg tattgttttt tttagtgtaa 2640
   agttttgtag atatgtttgg tcgaaaagtt ttagtggttt taatttttag attttgtttt 2700
   cgcgttcggt cggaaaatta tttgggattt ggttgttgtt taaggtcggg ggaaagtttt 2760
   ttttggagga cgtttattat tattattttt ttttaggttt atttgggttt aaataaatgt 2820
   taaagtttaa acgaattcga gaaggaatag cgaggtagtg gatgtcgtta tttcgtggat 2880
   gggagagtta tgtttattgt aggtgcggat atcgtattga taggtattga ttattgtaaa 2940
   atcgtattta gtattaaata gaaatagttt tgttttttaa tttttataga atgtttaaat 3000
   ttagtttagg gagttagttg gggcgtttta ataaatacgt tttcgagaat tagcgatgtt 3060
   ttttttataa tgaaagttaa ttgttgtata ataattgggt taaagaattt cgttagttta 3120
   aattttaaaa attagtattg tattttgttt ttaagtttgg gtggtaagta tcgaagggga 3180
   gagggaagta gcgtttgtgt ggaaggaagt ttcgtttttt gtttaaatat gtgggatttg 3240
   ggtgtttatt tttttagtcg agagtttttt gtaggttttt ttttaatttt ttgtattttt 3300
   ttatttttat tttattcgtg gcgttcgggt tttatttttg ttcggcgttt tttttttagg 3360
   gcgagtagag gtttttgagt gatcggttcg ttagaggtag ttgtagagtc ggcgtttcgt 3420
   agggtagggt agggtcgggt cgttgggttg gtttagtgcg cgcgggtttt ttttggtttt 3480
   agttagtcgc gggagatgtt atttcgggcg atagcggcga gacgtcgttg ttcgtaattt 3540
   ttgggcgggt aggatcgttt gttgtttttg cggtttttgg ggtagatttt ttaggagttt 3600
   tttttttttt tttttcgttc gggggtcggg atcgcgttgt gtttacggag ggaattgggc    3660
   gtttttggcg tattggggtt ttagttgtat gattttagtt tagattttaa gttttagtgg    3720
   ggtgtaggtg ggatgggatg tgtttttagg agatgaggag atttcggttg ttgagtcgtt    3780
   ttttaaatgc gtagtttttt aattttaggt gatattcgtt ttttggagga acgagtgaag    3840
   gtcgtttagg cgaaagaggg gtgggtttta gtatttggat tttttttttt agttttgtta    3900
   attttaggtg tgtttacggt ggaggtgata gaaggaaggc gcgtcggggg atttaggttt    3960
   cgtaggtttt cggttgttaa cgaggtatcg tttattgatt tcgcgtttcg ttttttatag    4020
   cgtggggttc ggcgttgcgt taggttcggg tgcggggcgg agttaggttg ggattggttg    4080
   ggggtcgttt cgttcggcgt ttgggttttc gcgtcggttt tcggggagga gttatgataa    4140
   tttttttttt attaaggcgt tcgggttttt cggttatcgt taggatatat tgttcgggcg    4200
   cggttttttt cgttcgcgga gcggttttga tattcgcggc ggtagtattt acgttcgtag    4260
   agtcgtcgat gcgtgtttag tgattcggat agtaaggttc gcgcgcggcg ggggcggcgg    4320
   tagacgtttg gttatcgtga tttcgatttt ggatttatcg tttgggggtt ggggggattt    4380
   tggatttaat tggcgattgt tttgggggac gtcggacgtt atgttgtgga aaataatcga    4440
   taatgttaag tacgaagagg attgcgaggt gagttggggt ttcggggtgt agtttcgttt    4500
   cgtcgaggat agttcgggag gtaggggtta ttggatcgag gtcggggacg agggtatagg    4560
   agttttggtt tttcggtggg acgggattta ttttttcgga tattttttag tttatttttg    4620
   cggggttttt tttttgtata gggttttttt ttaaatagtt ttttttcggg aacgaggttt    4680
   ggaaagagaa tggtaaattt tatttatata gtttatcggg cgttttttta agcggtaaat    4740
   agttttgttt ttcgtttttt cggaaaagtg tttcgtttgt aatttttaga cgtggttttt    4800
   acgtacgaag tttttgttta aaggggttag atgaggttat ttgttagcga tattttggcg    4860
   gatatttttt taaggtcggt ttgttatttg tttcgttatt ttttcgggga agtacgaaaa    4920
   taatcgaagt ttggttttta aaagaagtgg gggttgggga gtggtggtgg gggtgttcga    4980
   ggttagatgg ggtcgtaaga gttcgcgagt gttttagttt gtcggatagg ttgagttaga    5040
   gtttcggaga gtgggaggga agaaggaggc ggcgagcggg aagaggaaga agacgtaggt    5100
   agcgttaggc gagtttaggg gcgtcgggag cgcggagttc gggttacgga ttcgcgggag    5160
   tttattgcgt tttcgggttt tggagggttg tttttgttcg taggttcggg cgttttcgtt    5220
   aggaggcgat agcgttatgt ttttttaggt tttcggcgtt tcgagatttt tgggtagatg    5280
   gggacgtatt ttttagaaat tgagtcgggt cgtttagggg cgagggaacg tgcgcgggta    5340
   aggttgttta atttttaggg ttttttatgt tttttttgcg tttcgacgtg cgagaatatt    5400
   gtttttggta gtgtagggcg tttttattta ttattttttt cggttgggtt cggttagtag    5460
   ggagggtcgt tttgtgcgcg cgtttttttt ttatttttta gggcggcgta gggtggcggg    5520
   ttttgttttt cgagcgtcgt tcgttcggag gtttttgttt cgagggcgtg gaagggaggg    5580
   tttcgggggc gggggaggtg cgtatttttc gcgtcgcgta ttttattcgc gtttgtcgcg    5640
   ttgttatttt tagtagtttt tgcgttatgg gcgggtttta cgagatagtt ttgtatcggg    5700
   cgttcggttt tttcgtttcg ggttttggtt ttttcgtttc gggtttcggt tacggatttt    5760
   tttggtttaa gatttagggt tcggatttgg cgtttttaag cgtttcgggt ttgggagtag    5820
   cgtttagatt ttcgtcgtcg ggttttgaga attcgttttt ttaggttttt tattagattt    5880
   tttttttttt tcgtattttt tgtttataac gaaattttcg gggcgtattg ttttcgggta    5940
   tttttcgatt ttgggtaagt ttcgtcgggc ggggtgcggt tggtttttcg gggttttttg    6000
   cgtagttcgg cgatgtcggt tagttcgtag tagcggggtt tcgtattaac ggggtttttt    6060
   gttttttttt ttttttttag gatcgttacg acgggagtag taatgggaat tcgcgggttt    6120
   tttatttttt tttcgtcggg tagtattttt atagtttcgc gttatttttt ttttatattg    6180
   gagtcgtcga atattagtcg ttattttatt tttttttttt ttattagtag ttggtttatt    6240
   tttagtcggt cgatttttat tcgtatttgg gggaagcgta cgtcgtcgtt attaattttt    6300
   tgtattagtc ggcgtttata ggtagttagt agtaggtttg gttcggtcgt tagagttagg    6360
   agggagcggg gttgttttcg tattacgggc gttcggtcgg tttattgttt tattttttcg    6420
   ggttggaggc gggcgcggtg agcgttcgta gggatgttta tcgtcgtttc gatttgttgt    6480
   tgttttacgt atacgttttg gatgtcgcgg gtttggtcga gaatttgggg ttttacgata    6540
   tgttttatta gatggacgag gtgtaggtga gcggcgttgc ggtttttgat cggatttgtt    6600
   tattttacgg ttttttgttt ttatttcgta tttttttagg ttttttcgaa tttaagggaa    6660
   ttttgttttt ttttttttag aatgtcgacg attagtattt gttgttgta               6709
<210> 32
   <211> 6709
   <212> DNA
   <213> Artificial Sequence
<220>
   <223> chemically treated genomic DNA (Homo sapiens)
<400> 32
   tgtagtaata ggtgttggtc gtcgatattt tgggggagag aggataaaat ttttttaagt 60
   tcgggggagt ttagaggagt gcggggtggg ggtagaaggt cgtagggtga ataggttcgg 120
   ttaggagtcg tagcgtcgtt tatttgtatt tcgtttattt ggtgaggtat gtcgtggagt 180
   tttaggtttt cggttaggtt cgcggtattt agggcgtgtg cgtggggtag tagtaggtcg 240
   gagcggcggt aggtattttt gcgggcgttt atcgcgttcg tttttagttc ggagaggtgg 300
   ggtagtaggt cggtcgggcg ttcgtggtgc gagggtagtt tcgttttttt ttggttttgg 360
   cggtcgggtt aggtttgttg ttggttgttt gtgggcgtcg gttggtgtag ggggttgatg 420
   gcggcggcgt acgttttttt tagatgcgag taggggtcgg tcgattggga gtaggttagt 480
   tgttggtagg gagggggaaa gtagggtggc ggttgatatt cggcgatttt agtgtgggag 540
   aggggtggcg cggggttgta gaggtgttgt tcggcggagg agaggtgggg gattcgcgga 600
   tttttattgt tgttttcgtc gtggcgattt tggagggaaa aaaaaaaata ggagatttcg 660
   ttagtgcgaa atttcgttat tgcgaattgg tcggtatcgt cgggttacgt agagggtttc 720
   gggggattaa tcgtatttcg ttcggcgggg tttgtttagg gtcggaaggt attcgggggt 780
   aatgcgtttc gaggatttcg ttgtaagtaa agagtgcggg gagggaggag agtttggtga 840
   aagatttggg ggaacgagtt tttaaagttc ggcggcgaag gtttaggcgt tgtttttaag 900
   ttcgaggcgt ttggaggcgt taagttcgaa ttttgggttt tgggttagaa aagttcgtgg 960
   tcggagttcg gggcgaagga gttagagttc ggggcgaagg agtcggacgt tcggtgtaga 1020
   gttatttcgt ggagttcgtt tatgacgtag ggattgttgg aggtggtagc gcggtaggcg 1080
   cggatagagt gcgcggcgcg ggaaatgcgt atttttttcg ttttcgggat tttttttttt 1140
   acgttttcgg gataaagatt ttcgagcggg cggcgttcgg aaagtagggt tcgttatttt 1200
   gcgtcgtttt gggaagtgaa gagggagcgc gcgtataggg cggttttttt tgttggtcgg 1260
   gtttagtcga ggggagtaat aagtgggggc gttttgtatt gttaagggta gtgttttcgt 1320
   acgtcggggc gtagaggggg tatgaagaat tttggaaatt gggtagtttt gttcgcgtac 1380
   gttttttcgt ttttgggcgg ttcgatttag tttttaaagg atgcgttttt atttgtttag 1440
   gggtttcggg gcgtcgggaa tttggaggaa tatggcgttg tcgttttttg gcggaagcgt 1500
   tcgggtttgc gggtaggggt agttttttag ggttcggagg cgtagtgaat tttcgcgagt 1560
   tcgtagttcg agtttcgcgt tttcggcgtt tttgagttcg tttagcgttg tttgcgtttt 1620
   tttttttttt tcgttcgtcg tttttttttt tttttttatt tttcgaaatt ttagtttaat 1680
   ttgttcgata ggttggagta ttcgcgagtt tttacggttt tatttggttt cgggtatttt 1740
   tattattatt ttttagtttt tatttttttt gaaagttaaa tttcggttgt tttcgtgttt 1800
   tttcgaggag gtagcggggt aggtgataag tcggttttgg aggagtgttc gttaaggtgt 1860
   cgttagtagg tagttttatt tgattttttt ggatagagat ttcgtgcgta aaagttacgt 1920
   ttgagggttg tagacggggt attttttcga gaaaacggga agtagggttg tttgtcgttt 1980
   aggaaagcgt tcggtaagtt gtgtgggtgg gatttgttat ttttttttta ggtttcgttt 2040
   tcgaggggag gttatttagg aaaaggtttt atataggaaa ggggtttcgt agaaatggat 2100
   taaggggtgt tcggagaagt ggatttcgtt ttatcgagag gttagggttt ttatgttttc 2160
   gttttcgatt tcggtttagt ggtttttgtt tttcggattg ttttcggcgg ggcggggttg 2220
   tatttcggag ttttagttta tttcgtagtt tttttcgtat ttgatattat cggttatttt 2280
   ttataatatg gcgttcggcg ttttttaaaa tagtcgttag ttaaatttag gattttttta 2340
   gtttttaagc ggtaaattta aaatcggggt tacggtgatt aggcgtttgt cgtcgttttc 2400
   gtcgcgcgcg ggttttgttg ttcgggttat tggatacgta tcggcggttt tgcgagcgta 2460
   gatgttgtcg tcgcgagtgt taagatcgtt tcgcggacgg ggaaagtcgc gttcgaatag 2520
   tgtgttttgg cgatagtcgg gggattcgag cgttttaatg agaaggaaat tattataatt 2580
   ttttttcggg ggtcggcgcg gaggtttagg cgtcgggcgg ggcggttttt agttagtttt 2640
   agtttagttt cgtttcgtat tcgggtttgg cgtagcgtcg agttttacgt tgtgagggac 2700
   gaagcgcgga gttagtgggc ggtgtttcgt tgatagtcga agatttgcga ggtttgggtt 2760
   tttcgacgcg tttttttttt gttattttta tcgtaaatat atttgagatt ggtagagttg 2820
   ggggaaggga tttaaatatt gaaatttatt tttttttcgt ttaaacggtt tttattcgtt 2880
   tttttaaaag acggatgtta tttggagtta agaaattgcg tatttgggga gcgatttaat 2940
   aatcggggtt tttttatttt ttaaaaatat attttatttt atttatattt tattgaggtt 3000
   taaggtttga gttgggatta tgtagttgga gttttagtgc gttagagacg tttagttttt 3060
   ttcgtgggta tagcgcggtt tcggttttcg ggcgggagga gagaggaagg gatttttggg 3120
   gagtttgttt taagggtcgt agaggtagta gacggtttta ttcgtttagg agttgcggat 3180
   agcggcgttt cgtcgttgtc gttcgaagtg gtatttttcg cggttggttg aggttagggg 3240
   gaattcgcgc gtattggatt agtttagcgg ttcggttttg ttttgttttg cggaacgtcg 3300
   gttttgtagt tgtttttggc gggtcggtta tttagaagtt tttattcgtt ttgggaaaga 3360
   agcgtcgggt aggaataaaa ttcgagcgtt acgggtaagg tgagggtgag gaggtgtagg 3420
   aagttgggag agagtttgta gagagttttc gattggagag gtagatattt aggttttata 3480
   tgtttagata gaaaacgaaa ttttttttta tataaacgtt gttttttttt ttttttcggt 3540
   atttgttatt taaatttgaa agtaaagtgt aatattaatt tttaaaattt aaattaacga 3600
   aattttttgg tttagttatt atgtagtaat tgatttttat tgtaagggga atatcgttaa 3660
   ttttcgaggg cgtatttgtt gggacgtttt aattggtttt ttgggttgga tttaaatatt 3720
   ttgtggggat tagaggataa gattattttt atttgatatt gaatacgatt ttgtaataat 3780
   taatgtttat tagtacggtg ttcgtatttg tagtagatat gattttttta tttacggaat   3840
   ggcggtattt attgtttcgt tgtttttttt cgggttcgtt tgaattttag tatttattta    3900
   gatttaggtg ggtttaaaag aaaataataa taataggcgt tttttaaggg aaattttttt   3960
   tcggttttag atagtagtta aattttaggt gatttttcga tcggacgcgg gaataaaatt   4020
   tggaagttga aattattgag atttttcggt taagtatatt tgtagggttt tgtattaagg   4080
   agaatagtgt ataaaaagat atataggatt ttgatatttc ggttttaaaa taggtttttg   4140
   ggggtaaaat aagttatcgt agaagtagcg tttttggtat tagcggcggg ggagggggat   4200
   atttgttaga agtagaaatt agatttaggt tcgtaggtat tttagagtat aggtttagga   4260
   agatgggttt tagattcgtt agaggtgcgg cgtttttttc gggcgtttgg gattttttga  4320
   cgggtgtcgt tgggttgttc gatattttgg gttttttagt tttcggtttt attttttgat   4380
   tcggtttgag gtgtagtttg aataagttgt tttgtttgtg ttggtaagtt tattttttgt  4440
   acgttggggt tcggaatggg taagaaggtg tttatttttt attttaggtt ttggtttttt    4500
   ttttttatag taatattagt taaagttgtt cggaggaatc gatatgtttt ttcgtttggg    4560
   agcgtttacg taggaggcga agggagtcga tgggtattga gggttcgcgt agtatcgtgt   4620
   cgtgggttta aaggatttag aggattcgga atcgtttggt aaaggttatt tatttttaag   4680
   gagttttttt ggatttcggc gttgtttttt ttttaatggg taagcgttcg aattggagcg   4740
   taattttcgc gttttttgta cggaaggtag ttagaataaa ttttttgttt tttcggtacg     4800
   ggagattttt ttttaatacg tatattttcg gtttttgggt cggtatttag gtacgcgcgc    4860
   gtttttgtga ggaagtttta attttttttt gattttaatt tttttgttta gtagagtcgg     4920
   gaatcgaagg ttttagtatt taagatagtt tttttttaac gttttcgttt tttgtttacg   4980
   tgaagacgcg gattgcgtgg tcgcgtttaa gatagtcggt atcggtagaa ggggattttt   5040
   ttggaatttc ggaggtttcg tagtcgtttg gagaagagga tggaaggttg tcgggggttt  5100
   ttttttatcg ttttttaggg agtttaaatt tatttatttt ttggaagttt tggatttacg      5160
   tttttatgaa atcgtaaatt aggtttttgt aatagcggga gcgttttaat agcgcgttat   5220
   ttttaaagag gcgcgaggtg gtgtttttgg aaatagggtt ttcggtttta ggtttgagtt   5280
   attcgttatt tttgttttta ttttagacgg aagaaggcgg ttaggcgggt agagtaagat    5340
   tcgggtagat ttgcgatttt ttttttcgtt ttagggaggg gattttcgag tagaagatgg    5400
   aggttttcga gcgttttcgt agatttcgcg ttacgcgtcg gacgtttgtt tcgtttttcg   5460
   ggataacgtg agcgtcggta gggattttag taagtggtag attcgttagg attaagtaag   5520
   gtggaaaggt cggggtttgc ggtaaattta ggaggagggg gtatttacgt tcgttatttt   5580
   tttttcgcgg tattggtatt tattggatcg atattggcgt taaagggtat tttgaagtat   5640
   ttatgtttag taagggtatg ttttggggta ttatagatat taagttatag ttattttaaa   5700
   ataattataa aaatgattat cgtaaataat agattgtttt aatagggcgc gaagcggtcg   5760
   tcgtatatag acgtttttgg ttttagtttt attagattta gtttattgtt cgtttttttg   5820
   cgtttcgcgt taggtcgtat gtttttcgtt tagaattttt tgagttttaa tttttttttt   5880
   taaataattt ttttttttaa ataggtaaag tgaggcgagg agttgagggg tttataggtt     5940
   tttaagtttt cgaggagggt tcgggtttac gttagtttcg gttcgttaaa cgagatttcg   6000
   tgggtatagt ttttaatttg ataattattt gttttatttt atagtaggta aaagtgaatt   6060
   tttagtcgtt taattatttg aaagttataa aggtgtttag ggttgaaggt tattttttta   6120
   gcggttttat tagggagttc gttagagata tgtttgattt tttttttgtt ttgtttcggg    6180
   ggggattttc gttcgtcgga ggttttatac gtttggtcgt gggatggtag atttttgtta   6240
   tttttttatt aatatttcgg agtttattcg tacgtatttt aatcgttcgg agcgtaggtt  6300
   cgaggattaa ggcggtagtt tcgtgttttt tgaggttttt ttcgtttcgt gttttgggtc   6360
   gttatatatt tttcggttaa gttcggcgtt tttaatttag aatttggtcg tttttaggcg   6420
   tggagtaggt tattgttgtt agacgttgcg cggattcgtt tttttgcgtt agtattgggc   6480
   gataggtagt tcgggtagta tttaggagtt attttgtacg gttttaggcg ttcgttttat   6540
   ttagcgttat ttttcgtttt tcgtagcgag ttttttcgta gttttttttt tttgttagtt   6600
   ttcggggtcg tatttgtttt ttatatagat attttttttt ttattcgtag gaattggaag   6660
   tttatggtgt tagtttttta attttttgtg agttgaggag tttaagagt                6709
<210> 33
   <211> 2296
   <212> DNA
   <213> Artificial Sequence
<220>
   <223> chemically treated genomic DNA (Homo sapiens)
<400> 33
   gttattgtat tttgttaata ttttttaaaa gtaaaaaata aattaaaaaa aaagttttta      60
   gtggattgtt gagttttttt tgataggagg tattaaatgt gttttttttg aggatagggg      120
   aagagtttat tatttagtta tatttttgta tattttataa aatttatttt tattaaggat      180
   gtttttttat aaaatgagga gtagtataaa gaatatttaa gaaattaggt ttgatttagg      240
   gtttttgatg ttggggtagg tttttcgcgt gttaatattt taggggattt tgttgtagtt      300
   ttttgaatat cggttgtgag ttttttaatt ttagttgagt tttagtattt tttttatatt      360
   atttattttt tttgtttgtt tttggcgcgt ttagataatt ggaatttttg ttgatatttt      420
   aaatgggttg gttatttttt tagcgtggag gtatttagta taaggtgtga tagattagta      480
   tttgttgatg agtaataaag ttattttggt aaattggatt ttttttttga aacgttttgg      540
   tttttatatt tttaatgatg ggttaagaaa tagtttttaa gaagaagggg cggagatagg      600
   gggtaggatt ttgtttgaag tttttaagtt gattttgttt gtagttattt aatttattag      660
   ggagggttgg atttgttttt ttgtggtgga tttttcgtat ttttagaaag tgtaggggtg      720
   gagaattaat ttagtttttt ttttttttat aagagtttcg ttaggttagt ttgtatgagg      780
   agggtacgtg tatattttta aaaggattgg tgtagttatt tttttaagtt tatagtttta      840
   gtgtgagttt agggtttaga ttcggggttt ttggtgtttt aggcgtgagt taagtttttt      900
   gagtaagagg ttttatgata aattagttgg agtttggggg ttatgaaagt attggttatg      960
   gtagaaatta ttagttcgtt atcgtttatg ttgttttgtt taaaaatgat atcgcgtaag      1020
   ggattttgtt aatttttaat ttcgggagat tatttattat ttggttgtgg ttcgtcgttg      1080
   aataggaaag tatttcgaga tgttaagaga ttaaaaggtt cgtaattgtt gtttagaaat      1140
   gaaaatggtt ttaaattata attagagatg gaagcgcgtt ttaggggagg gcggggcgtt      1200
   gtgtaatcga gttttttatg cgtaaggcgg atataaacgg tttatggatg tgggcgaagg      1260
   tttttagagt aaagaatgtt tatttttatt agattaaaat atagtttggg aaaataatta      1320
   tttgtttgag attttgggaa agtaaaattt tttggttttt ttgttaggaa gaacgattgt      1380
   aaaataaggg ttgagaattt tttaaataag attttagaat tttatttaaa tagttgtagt      1440
   ttatttttga ggagttttag atttatttgg gtttgatgga tgttgtaata ttaagtttga      1500
   ggttttttta gtttggtgtt aagagtgggt tttaggggat aaatagagaa tagttgtttt      1560
   tttaatcgga gtagaaaata aaattttttt ttttttttat ttttaatttt tttgttttgt      1620
   tgtgaaattg ttattgcgtt tttgaggagt gattaaaatt ttagttatga ttaatttaaa      1680
   gggtaggttt ttaggtaggg aagggaaagg ggtttaaggt aaaaaaaaaa aaaaaaagtt      1740
   agttgaagtg agtttatgga ggatttgggg attataaaag ttatgggatt tgagttaatt      1800
   aaaattaatg gaaaagtatt aatatgtaat ttaaaaaata ataataaaat aaagtagtaa      1860
   ggatattttt ggtttgtttt ttgtttttat ttttgagttt gtttgggaat tttggagtag      1920
   attttttgaa gtatagtatg agtgtttaat ggttgtttta atttattttt ttgtttttta      1980
   tatataaaga ttttgtaatg gagagtggga atgaatggtt tatttatttt tgaatatttt      2040
   ttgtgagttg ggaatgcgtt tagtaaggtt gttggagtgg agggaaaaga aaaggtgttt      2100
   gagtttttta gatatgggta gaaaaggaga gaatttgaga taggttgaga gaaagttgtt      2160
   tcggttggta gtattgttag atttttagat atatcgtata ttttagtttt cgaaaaggtt      2220
   attttgagtt tttttagggt ttttggggtt tttattttat tttaaatatt tggaattgtt      2280
   tgtgtttgtt ataata                                                  2296
<210> 34
   <211> 2296
   <212> DNA
   <213> Artificial Sequence
<220>
   <223> chemically treated genomic DNA (Homo sapiens)
<400> 34
   tattatagta ggtatagata gttttaaata tttgggatga gatgagagtt ttaggggttt      60
   tagagggatt taggatggtt ttttcggggg ttggaatgta cggtgtgttt gagaatttgg     120
   taatgttgtt aatcggagta gttttttttt agtttgtttt aaattttttt tttttttgtt     180
   tatgtttaga gaatttagat attttttttt ttttttttat tttagtagtt ttattgagcg     240
   tatttttaat ttataggaaa tgtttagggg tgggtggatt atttattttt attttttatt     300
   gtagaatttt tgtgtgtgaa gggtaggaaa ataggttaaa atagttattg ggtatttatg     360
   ttgtgtttta ggaagtttgt tttaagattt ttaagtaggt ttaagaatgg aagtagagag     420
   taagttagaa atatttttat tgttttattt tattattatt ttttaaattg tatgttggtg     480
   tttttttatt aattttaatt gatttaagtt ttatgatttt tgtgattttt aaatttttta     540
   taaatttatt ttaattgatt tttttttttt ttttttgttt tgaatttttt tttttttttt     600
   gtttgggaat ttgtttttta aattggttat ggttgggatt ttaattattt tttaaaggcg     660
   tagtgataat tttataataa agtagggagg ttgggggtgg gaaggggagg gagttttgtt     720
   ttttatttcg attaagagag taattgtttt ttgtttgttt tttggggttt atttttggta     780
   ttaggttggg gagattttaa atttggtgtt gtaatattta ttagatttaa gtgagtttgg     840
   ggttttttaa aggtgaattg tagttgtttg gataaaattt taaaattttg tttgggagat     900
   ttttagtttt tgttttataa tcgttttttt tggtaaagaa attaggaagt tttatttttt     960
   taggatttta ggtaggtaat tgttttttta aattgtattt taatttgatg gaaatggata    1020
   ttttttgttt tgagaatttt cgtttatatt tataaatcgt ttgtattcgt tttgcgtatg    1080
   ggaggttcga ttatatagcg tttcgttttt ttttgagacg cgtttttatt tttggttata    1140
   atttagggtt atttttattt ttaaataata gttacgagtt ttttagtttt ttggtatttc    1200
   ggagtgtttt tttgtttagc ggcggattat agttaggtgg tgggtgattt ttcgaggttg    1260
   ggaattggta aaatttttta cgcggtgtta tttttaagta aagtagtata aacggtgacg    1320
   agttggtagt ttttgttata attaatgttt ttatggtttt taggttttaa ttgatttatt    1380
   atgaggtttt ttgtttaggg gatttggttt acgtttgaaa tattagaggt ttcgagtttg    1440
   ggttttgagt ttatattggg attgtgagtt tagggaagtg gttgtattag tttttttgga    1500
   ggtgtgtacg tgtttttttt atgtaggttg atttggcggg gtttttatgg ggaggagggg    1560
   ggttgagtta attttttatt tttgtatttt ttggagatgc ggagagttta ttatagggag    1620
   gtagatttag ttttttttag tgaattaagt gattgtaaat agagttagtt tggaagtttt    1680
   aggtagggtt ttatttttta ttttcgtttt ttttttttgg gggttgtttt ttgatttatt    1740
   attaaaaatg taaaagttaa aacgttttag gggaaaagtt taatttgtta gaataatttt    1800
   gttatttatt aataaatatt gatttattat attttgtgtt aggtgttttt acgttggaga    1860
   agtagttagt ttatttaaaa tgttagtaaa gattttagtt gtttaaacgc gttagggata    1920
   ggtaaggagg ataagtggtg tgaggaggat gttgggattt agttgaagtt ggaaagttta    1980
   tagtcggtat ttagagaatt gtaatagggt tttttgggat attagtacgc ggggaatttg    2040
   ttttagtatt agaggttttg ggttaggttt ggttttttag gtgttttttg tgttattttt    2100
   tattttgtgg agaggtattt ttggtagaag tgaattttgt aaaatatgta aaggtatggt    2160
   tgaatagtga attttttttt tgtttttagg aaaagtatat ttggtatttt ttgttagagg    2220
   ggatttagta atttattaaa gatttttttt ttaatttatt ttttattttt gaaaaatgtt    2280
   ggtagggtgt agtgat                                                    2296
<210> 35
   <211> 4247
   <212> DNA
   <213> Artificial Sequence
<220>
   <223> chemically treated genomic DNA (Homo sapiens)
<400> 35
   ttttaaaaaa attaaaatat aaaataataa taattattaa aaattttatt ttcgtttttt      60
   taaataaaaa atttatttta aaaattttaa tttaaaaatt taaaaacgaa aaacgcgtat     120
   tataattttt aaaaattttc gaatttaaaa acgattttcg tttttcgttt ttttcgcgcg     180
   acgaaatcga atttattaat aaaaattcga aataaaacga ataaaaacga aacgaaacga     240
   aattacgaaa aaataatcga cgaatcgcgt cgcgaattaa tattttaaaa aacgcgaaat     300
   tcgaataatc gcgcgttaaa aatttcgaaa cgaacgtcgc gaaatttttt cgaattataa     360
   ataaacgatt ttacgacgcg attcgaataa aaattcgacg attttataaa aaaatttaaa     420
   atatcgacgt cgaatttttt ttaattttcg taataatcga tacgttatat aaaacgaaaa     480
   aaaattcgaa ataaatttcg aaacgcgaat taaaaaaaac gaattcgatt cgcgatttta     540
   acgtttttcg atcgacgcga aaaaaataaa attaaacgtt cgcgcgaatt tcgaaaaaaa     600
   aaaaaataaa aaattttaac gattaaaaat ttttcgaacg tttatttcga aatttttttc     660
   gtcgtttttt atttaaaata tatcgaattc gatacgattt tatcgtttta ttttttatta     720
   cgatttttat ttcgaattaa atttaattaa tttattataa taattatcga taatcgtttt     780
   ttcgtttcgt aatatttcgt aaattttaaa atcgcgacgt cgtcgaatat aaatttcgaa     840
   tttaaaacgc gcgatttttt tttttacgat cgtcgttttt taaaaaaaat tacgcgaata     900
   taaaaaattt atttaatacg ttttataaaa tttattattt ttttttcgat cgcgaattac     960
   gtcgataatt cgaaaaattc gatttttcgt tttttatttt tttttttttt tttttttttt    1020
   tttttttttt tttatttttt cgtttattcg tcgtaatttt ataaaaaaaa aatcgttttt   1080
   ttaattattt ttttttttta ttttttatat atttttttat tttttttcga tttataaaaa  1140
   aaacgaaata acgattaaaa aacgttaaaa aaaaaaaata aaaacgtaaa ttacgaattt    1200
   ataaacgata ttcgatttcg cgttcgacga aaattacgaa ttaattcgaa ttcgcgtttc    1260
   gttttttcgc gatatttaaa aatcgttcga attaaaatcg cgttattttt cgtttaacgt     1320
   cgacgtaata aaatttacgc gatataaaat tttcgaaaat ttaaaaaatt ttaaaacgtt    1380
   acgaatttaa tattttttta tttataaaat tttaaattaa taaaaaatat ttcgcgaatt    1440
   ttaaaaaaaa ataataaata ttttttattt tattcgaatt tttttaataa aaatttatac    1500
   gtacgataac gttattacgt ttataaataa cgttaaaata taaaaaaaat aaaaaatcga    1560
   aattcgaaaa ttttatttcg taataaaatt taaaatttaa tttaaatttt aaaaattcgt    1620
   ttaaaattta aaatttaata taataaaaat aaaaaaaatt ttcgataatt tatttttata    1680
   attaaaaaac gttaattaat atttttaaat ttttatttcg taaataaaat aaaaatatac    1740
   gaaattattt aaaaaatttt tattattcgt tattattaaa tataatttaa atttcgtaaa 1800
   aaaaaataat tttattttaa aattaaaatt tttaaaatat tttaaaattc gataaaaaaa 1860
   ataaaaaaaa taaaatttaa aaattaataa ttttatattt aatatttttc gtattttttt 1920
   ttttttttat attttttaat aatttttata attttaatta tttttaaatt ttattttttt 1980
   cgtttttttt taaattaaaa tattaaaatt aaattataat ttaaattata tttatatttt 2040
   attaaaaaat aaaaatttta aatttatcgc gttttttttt attattaatt ttaatttaat 2100
   atttaataaa tttttttttt taaattatta aaataaattc gcgtaaattc gatttaatcg 2160
   tcgttttcga attatcgaat aaatcgatcg tttcgtcgtt ttaaaaatta tttttatttt 2220
   attttaattt aaatttaaaa aaatttaaat taataatttt aatcgaaaaa acgaaacgac 2280
   gtttattaat tattattaaa attattatta ttattattat aataattaac gtaatatttt 2340
   taatcgataa ttttttataa aaaaaaatat tatttttttt ttattatatt taaaataaat 2400
   aaaacgaata ataaaaaaat aaaaaaaaaa aaaaaaaaaa aaatatatat ttttaataaa 2460
   tttttaaatt taaaaattat ataattttat attaattatt attttttttt aaataataaa 2520
   aatttataaa tttattacgt ttattttata tttaaaaaat taatattttc gaaatattta 2580
   ttaaataacg aaatatttta aaaaaaaaat aaaataaata aatttttaat ttttttatta 2640
   aatttaatta taatttataa aaaaaatttt tttttttttt ttattttttt ttattaaaaa 2700
   tattaaatat attaaattta atacgtttat tttataaata aaaaaaaaaa aatataaaat 2760
   taataattaa tttaataaaa aatataatta tataatatta aaatttttta ttttatttat 2820
   ttttttaatt tatttttatt tatttttaat ttaatattta ataaaataaa aatttattaa 2880
   ttttttcgta tttatttatt atttaatttt ataattattt tttaatttta attaaatttc 2940
   gtaaaatatt ttaaatttat ttttaataat ttttaattat cgttcgatta tttttatttt 3000
   tttattttta tatatttatt tttatttcgt taaattaatt aaattttaat taaaattaat 3060
   tttttatttt tttaaataat aaataataat tttttaaata aaattttaat aaatattaat 3120
   aaattttttt ttttttttta aaattataaa aataatattt tttatttaaa aataataaat 3180
   tttttaaatt tttttcgatt taaattaaaa aaattaattt ataaaatata aaaattatat 3240
   tttttttcga tttttttttt tttttcgtta ttatttaatt ttttaattaa ttttttttta 3300
   tttttaaatt taaatatttt aataattaat ataaatttat ttttattaat aaaataataa 3360
   aaacgatata ttaaaaaatt atatatttat ttaaaaaaaa aaaataaatt tatatttttt 3420
   aaatataaat aaaaaattta aaattttttt atttttatta aaaaaaaaaa aatttttaat 3480
   ttttaaaaat taacgttttt taaaaattaa attattttaa ttttaaattt taattttatt 3540
   ataaataaat tttataattt taaataaatt attaaatttt tttaaatttt aaatattata 3600
   tttattatat ttaaataatt aaaaaaattt aataatataa tattatataa aatatttaat 3660
   ttatattaat taaaatatta atttaaatcg ataaaatata taatttaaaa ataaataaat 3720
   ttataaaaat attttttatt aaaaataata ataaattaat atttttaata ttaaaatttt 3780
   aaaatttaaa aattaaattt aatttaaata aaaattataa aattaaaaaa aaattaaatt 3840
   tataatataa aaaaaatttt aaataatttt tttatttaat attttatcgt attttttttt 3900
   tatttaataa aaaattaaat tattaaaaat ttaaatttat tttttaacgt aattatttaa 3960
   tttatttcga tttttttttt aataataaaa taaaaaaatc gtaaaaaaat ttttataaaa 4020
   taaattatta ttattaaaat ttttttaata ttatttttta attatataaa atttaaaaaa 4080
   taaattaaaa ataaattttt ttaaaaaaat ataaaaattt tttaaaaaaa aatttttaaa 4140
   aattataaaa aaaaatttaa attataaaaa tatataattt taaaaaaaaa aatataatat 4200
   tataaaaata ttattttttt ttaaaataat ttttaaattt aaatata 4247
<210> 36
   <211> 4247
   <212> DNA
   <213> Artificial Sequence
<220>
   <223> chemically treated genomic DNA (Homo sapiens)
<400> 36
   tgtatttaaa tttagaaatt attttgagga gaaataatat ttttatagta ttgtattttt      60
   ttttttagga ttatgtgttt ttataattta ggtttttttt tgtagttttt agaagttttt     120
   ttttaaaaag tttttgtgtt tttttgggga ggtttatttt tagtttgttt tttgagtttt      180
   gtgtagttag aaaatggtgt tagaaaaatt ttgataatag tagtttattt tataaaagtt      240
   tttttgcgat ttttttattt tattgttggg aagagggtcg aggtaggtta ggtggttgcg      300
   ttagaagatg agtttgggtt tttagtgatt tagtttttta ttaagtagag agggggtgcg      360
   gtagggtgtt gggtaggagg attgtttgag gttttttttg tgttatgagt ttgatttttt     420
   tttaatttta tagtttttgt ttgagttaag tttaattttt agattttaaa attttagtat      480
   tgaagatgtt agtttgttat tgtttttagt agaggatgtt tttataggtt tatttgtttt      540
   taagttatat gttttatcga tttaaattag tgttttggtt ggtgtgggtt aggtatttta      600
   tataatattg tattgttgaa tttttttagt tatttagata taatagatat aatgtttgag    660
   gtttagagag gtttagtaat ttgtttgggg ttatagagtt tatttatggt agagttggga    720
   tttagaatta gggtagtttg atttttgaga gacgttgatt tttgagagtt agagattttt    780
   ttttttttaa tgggagtagg gagattttaa gttttttatt tgtgtttaga ggatgtaaat    840
   ttgttttttt tttttaagta gatgtgtagt tttttagtat atcgtttttg ttattttgtt    900
   aataagagta ggtttgtgtt gattgttaag gtatttaggt ttagggatgg gagggggttg    960
   gttagagagt tgggtagtag cggggagagg gggagggtcg ggaaggggta tggtttttat   1020
   attttatgaa ttaatttttt tggtttgggt cggggagggt ttagaggatt tattattttt   1080
   gaataagggg tgttattttt ataattttga ggaaggagga aagatttgtt gatatttatt   1140
   gagattttat ttaaaaaatt gttatttgtt atttgagaga gtagggggtt agttttggtt    1200
   agggtttaat taatttggcg aagtaaaggt aggtatgtaa aggtaggaag gtgaaggtag   1260
   tcgggcggtg gttgagagtt attgaagata aatttaagat gttttacggg gtttagttag   1320
   gattagaagg tagttatggg gttaggtagt gggtggatgc ggggaagtta gtgggttttt   1380
   gttttgttag atattaagtt ggaagtagat gaaagtgggt taagaggata gatggggtaa   1440
   ggggttttag tattgtatgg ttgtattttt tattaagttg gttattggtt ttgtattttt   1500
   ttttttttat ttataagatg ggcgtgttag atttaatgtg tttaatgttt ttagtagaaa   1560
   aaagtgagaa gagaaaggag gtttttttta taaattatag ttaggtttag tgagaagatt   1620
   agaggtttgt ttgttttgtt ttttttttaa ggtatttcgt tgtttagtag gtgtttcgga    1680
   aatattagtt ttttgggtgt aggatgagcg taatggattt atagattttt gttgtttaga   1740
   gaggggtagt gattagtata aggttatata gtttttaagt ttaagggttt gttggaggta   1800
   tatatttttt tttttttttt tttttttatt tttttgttgt tcgttttatt tattttgggt    1860
   gtgatgggaa ggaagtgata ttttttttta tgaaaagtta tcgattggga atattgcgtt   1920
   aattattata ataatggtaa tagtagtttt aataataatt agtagacgtc gtttcgtttt   1980
   ttcggttgag gttgttggtt taagtttttt taagtttgag ttggagtaaa ataaagatag   2040
   tttttgaagc ggcgaaacgg tcggtttatt cggtagttcg ggagcggcgg ttaggtcgag   2100
   tttacgcgag tttgttttag tagtttgggg aggggggttt gttaagtgtt gagttggagt   2160
   tagtagtgag aagaaacgcg atgggtttgg gatttttatt ttttaatagg gtataggtgt   2220
   agtttaagtt atagtttaat tttagtattt tagtttgggg gagggcgaga gagatagggt   2280
   ttgggggtag ttgaggttat aagagttatt agagggtata gaaagggaag gaggtgcggg   2340
   ggatattgaa tgtgaggtta ttaattttta agttttattt tttttgtttt ttttatcggg   2400
   ttttgggatg ttttaaaagt tttagtttta ggatgggatt gttttttttt acggagtttg    2460
   ggttatattt ggtggtgacg gatagtgaga gttttttaga tggtttcgtg tgtttttatt  2520
   ttatttacgg ggtagaggtt tagaggtgtt agttgacgtt ttttaattgt agaagtagat   2580
   tatcggaggt tttttttatt tttgttatat taagttttag gttttaggcg ggtttttaag  2640
   gtttggattg ggttttgagt tttgttgcgg ggtgaagttt tcgagtttcg attttttatt  2700
   ttttttgtgt tttagcgttg tttgtaggcg tagtgacgtt atcgtgcgtg tgggttttta    2760
   ttagaggagt tcgggtgggg taagaggtat ttgttgtttt tttttggggt tcgcggagta   2820
   ttttttattg gtttaagatt ttgtaaataa agggatattg gattcgtagc gttttgggat    2880
   tttttgggtt ttcgggggtt ttgtatcgcg taagttttat tacgtcggcg ttgggcggag   2940
   ggtgacgcga ttttagttcg ggcgattttt agatatcgcg gagggacgga gcgcgggttc   3000
   gggttggttc gtagttttcg tcgagcgcga agtcgggtat cgtttgtaag ttcgtagttt   3060
   acgtttttgt tttttttttt tgacgttttt tagtcgttat ttcgtttttt ttgtagatcg   3120
   agggaagata aggggatata taagggatag gagaggagag taattaggga agcgattttt   3180
   tttttgtggg gttgcggcgg gtgggcggag agataggaga ggaaaagaga aaagggaaga   3240
   gaaggaaggg gtagggggcg agaagtcggg tttttcggat tatcggcgta gttcgcggtc   3300
   gaaagaggaa tagtggattt tataaagcgt attgagtaag ttttttatat tcgcgtagtt  3360
   ttttttagga aacggcggtc gtagaggaaa gggtcgcgcg ttttggattc ggagtttgtg    3420
   ttcggcggcg tcgcggtttt gaaatttgcg ggatgttacg ggcggagggg gcggttgtcg   3480
   atagttgtta taataaatta gttgagttta gttcgaggtg gaaatcgtag taggaagtgg   3540
   gacgatagag tcgtatcggg ttcggtatat tttaggtaag agacggcgaa aagagtttcg   3600
   agataaacgt tcgggaagtt tttggtcgtt aaaatttttt gttttttttt ttttcgaagt   3660
   tcgcgcggac gtttagtttt atttttttcg cgtcggtcgg ggagcgttga agtcgcgggt   3720
   cgggttcgtt ttttttggtt cgcgtttcga aatttatttc gggttttttt tcgttttata    3780
   tagcgtatcg attgttacga gggttggagg ggattcggcg tcggtatttt gaattttttt   3840
   gtagggtcgt cgagtttttg ttcgggtcgc gtcgtagagt cgtttattta tggttcggag   3900
   aggtttcgcg gcgttcgttt cgagattttt agcgcgcggt tgttcggatt tcgcgttttt   3960
   tgagatattg gttcgcggcg cggttcgtcg gttatttttt cgtagtttcg tttcgtttcg   4020
   tttttgttcg ttttatttcg ggtttttatt ggtaagttcg gtttcgtcgc gcggagaagg   4080
   cggggggcgg ggatcgtttt tgagttcggg gatttttggg aattgtagtg cgcgtttttc    4140
   gtttttaggt ttttagattg gagtttttgg aataggtttt ttatttagag gagcggaggt   4200
   aggattttta gtaattattg ttgttttgtg ttttagtttt tttggag                 4247
<210> 37
   <211> 3905
   <212> DNA
   <213> Artificial Sequence
<220>
   <223> chemically treated genomic DNA (Homo sapiens)
<400> 37
   tttaaaaaag tagatttgaa gatgtttaaa gtaataatgg tgttgttaga atggtgtttt      60
   tttttttttt taggggatga ttttgaagga tattataata atagatgtgt aaattttgaa    120
   atatatggtt aaaatgaggt ttattatttt ttagttatta tttagatatt ttttagttat    180
   ttgtttatga tagtttataa atgagaaaga agtttattta aaatattttt atgaggattt    240
   taaggtaaat aataggaatt gattatttat taatttggtt tggttttggt ttattgaaga    300
   aagttttttt ttgagtgtat ttaaagtttt tatggtttag ttttgtttaa tttgagtagt    360
   attttatttg tttttttttt agatgttttg atgttttgta ttagatttaa gagagagtta    420
   agttttttaa taggttatgt atgtggttat taatatttag tatatgttat ttttggttag    480
   tttttttttt tttttttaag aattaaatat tttttggttt gagttttttt tttttatgtt    540
   attataaagt tgagttattt attattaggg tttatatatg agtagttaaa atatatgggt    600
   ttttatattt aggttaaggg gtttttagta ggagaatatg aataattagg agtgaggtta    660
   tgagatagag gaagaaaagt ttataggaat gggaagatag ttttttagat gagaggtgtg    720
   tggagtgatt gttgtgagga attgtttaat aggaaagtta gttaatttga ggatgttttt    780
   aggggaggaa aagggaaatt tatatggttt tttgttaatt tttaaaaata aggtagattt    840
   ttatttattt agggtgtttg tttgttatga atataggaag attaaggaat gagtatggat    900
   tgtttttatt ttatgttttt ggataagaag aataataaaa aagtattggg tagttggaat    960
   aattgtagag gttttaataa tgatggagat tttttggtgt attgtggtat aggggttttt   1020
   tttaaaggga tttaattaat tttaataggt attatttttt gtgatttaag atgttttttt   1080
   taggagtgtt tgtatttttt aatgtttaga gaagtttgta ttattgattt gggaatattg   1140
   agttttttag tttttgtaaa attattttta ggtttatttt taagtatatt ttttattttg   1200
   gggggttaaa ttttttggtt tttagtgaat attgtggtta tatttgtggt tatttattga   1260
   ttttttattt gtttagttta tttgataata ggtaaatggg gaaagaggtt ttaatatttt   1320
   ttaaaagggg aagattgata attagttatt tattgtatag aattaggtta ggttaatttt   1380
   ttttaagaag aaaagtaatt tggatattgt attttggggt ttttttttat tttggtatta   1440
   taggttttat gaggatgtag tttttttgta tttttttttt tttttttaaa ttgttattta   1500
   tttatttatt ttaattaggt taaagtatta tatattgtag aagagaaagt aattttagtt   1560
   ttttttaaat ggtgttattt ttatgatagt ttgtaaattt tggtttttta tgggtttagg   1620
   atttgtgtgt atttgtttgg ggatttgttg gtggatttat tttttatggt ggagtagtaa   1680
   ggggattttt tagaaaaagt aatgggtgaa gtaattgaaa gagtgatgta gaaagtaata   1740
   gttagaaatg gtggggatgt gagtggttta gataggaagg gaggtggtgg tgtagttttg   1800
   gtgtgtagtg tgttgtagtg atggaatttt tgtaaaagtt gtttgtttgt gtgttattag   1860
   tggtgtgtag gtttgtggtt tttttgtttt tgtaattttg ttttaattgt tggtttattt   1920
   tttgataaat aggatgtttt tatttgaggt tgttagtatt ttaggttttt tgaaagaaaa   1980
   gaagtagagt ggttttattt tagaggtaag gatggggttt gtgttaagag gttttttaga   2040
   gaagtgaaaa ttttgtaggt gtagttgttg ggagagtatt aagaagggta gggtggaggg   2100
   gtagggggtg aagggagggg gtgaagtttg tattttattt ttatatgaaa ttgattttat   2160
   tattttattt ttgtaagtgt ttagaggtag agaggttaat attttgggga tagtttggag   2220
   gtgggagatt taggtagggt tttttaaatt tttatgtgta tgaaaattag gttaattatg   2280
   gggtttttga gtaaatgggg atgatgattt agtaggtttg ggttgaggtt ttagattttg   2340
   tatttttaat aagtttttag gtggtagtga tgttgttagt ttaaagatta tattgtgaat   2400
   agtaaggtgt tgttgggata gtttgtttta gagtaaaatt taatagatgt gtaaatattt   2460
   aaattaagtt gtatttattt ttgaggtttt ggtttaattg gagttatttt gttaatggaa   2520
   aatggaaaat gaaatttttt taggttttaa agggaaggaa agtttgaaat ggttaaggta   2580
   tttaaaaaat tttagttggg agtggttttg gggatttggg agagttgaag ttaggttttg   2640
   agttaagaga tgggggagga agggtgggat gtggtaaata aatataaata tatatatata   2700
   aattttagtt taattatagg ttttttttta ttaatgaaat atttgtattt tgtaggtaga   2760
   atttatatgt gtttatattt tttagaagtt gaatggtttt gtgtaatggt atagaatttg   2820
   gagtttatat ttgaatattt tagatttaat gttattttag attggtttta ggattttgtg   2880
   gtttatattt aaaaatgtat gtttttgagt tatgtttaat tagtagtagt atttgagaat   2940
   tgagagatta gattaagaaa tttatggtat atgaataaaa tttttagata gtagttagaa   3000
   tatagaggtt tttttagagg tagtgttagt tgttagttgt aaaaataggt tttgtttagt   3060
   tttgtttagg tttgaagttt tttgaagatt ttatttagat tttattaaaa ttagttaaag   3120
   attttttgtt atagaaaggt ttttaaagta tggttgggat tatagggtta ttaagatgta   3180
   gatttttggg tttttttgtt ttatttatta tggtttttta gggtgggtgt tgggaatttg   3240
   tattttaata aatgttttga ggatttttat gtattgttaa gtttaggagt ttttggtttg     3300
   taggtttaag tttaattttt ttattgtagt atttaaagat ttggttttaa ttttgttgtt     3360
   tataggtgag ttttagtgat ttttttattg ttttttgagt atttttggag ttatttggtt     3420
   gttttattta tttttttttt tttgtttttt tttagttttg gaataaaatg atttttattt     3480
   ttttttttga atttttttta gattatggtt ttttatttag taagtagtta tttttttggt     3540
   ttgttattgt tgtggtatta atgataggtt atttgatttt tattttgttg attgaatatt     3600
   ttttatgttt aggttttgtt tttttgatta gatagtaagt tttttgaagt tattttatgt     3660
   tatataattt ttatgtttaa gattattttt taatgtagtg ttatgtttat atggattatt     3720
   tagtaaattt attgatggtt tatttattat taatatgttt tatagagtgt tattaagtag     3780
   ggatattttt tatgggttta ggattagaag aagttgggaa gagaaatggg gtagtggtga     3840
   gagtgttatg agaatttggt tatatagtta ttaaagatat agaggaagtg ggaaaaaggg     3900
   agtgt                                                                 3905
<210> 38
   <211> 3905
   <212> DNA
   <213> Artificial Sequence
<220>
   <223> chemically treated genomic DNA (Homo sapiens)
<400> 38
   atattttttt ttttttattt tttttgtgtt tttgatgatt gtataattag atttttatga      60
   tatttttatt attattttat tttttttttt agtttttttt gattttagat ttatagagag     120
   tgtttttgtt taataatatt ttgtaaaata tgttggtgat agataagtta ttggtaaatt     180
   tattgaatga tttatgtgga tatgatattg tgttagaaag tagttttgaa tataaagatt     240
   atatagtatg aaatggtttt agagaattta ttgtttggtt agggaggtaa agtttaagta     300
   tgagaaatat ttagttaata aaatgagaat tgagtggttt attattaata ttatagtagt     360
   ggtagattag gaaagtaatt gtttgttgag tgggaagtta tgatttgaag agagtttgga     420
   gaggaagatg gaagttattt tattttaggg ttggggaaag atagaggaga agaaatgaat     480
   gaggtagtta ggtggtttta ggagtgttta gagggtagtg aggaagttat tgaggtttgt     540
   ttgtgaatag taaaattgaa gttaggtttt tgaatgttgt agtgagaagg ttgaatttaa     600
   atttgtagat tagggatttt taaatttggt agtatataag aatttttaaa gtgtttgtta     660
   aaatgtaggt ttttggtatt tattttgaga aattatgatg agtgaggtag agaggtttaa     720
   gaatttgtat tttaatagtt ttgtagtttt agttatattt taagaatttt tttgtaatag     780
   gaagtttttg attggttttg atgaagttta aatgaaattt ttaggaaatt ttaggtttaa     840
   gtaaaattga gtagaattta tttttatagt tgataattgg tattgttttt ggagggattt     900
   ttatgtttta gttgttgttt ggagatttta tttatatatt atggattttt tagtttagtt     960
   ttttagtttt tagatattat tattaattaa atatgattta gaaatatgta tttttaaatg    1020
   taggttataa gattttaaag ttagtttgag ataatattgg atttagaata tttaaatatg    1080
   ggttttaaat tttgtattat tatataaaat tatttaattt ttgaaaaatg tggatatgtg    1140
   tgagttttat ttgtaagatg taaatgtttt attgatagga aagaatttgt ggttgggttg    1200
   gggtttgtgt gtgtgtgttt atatttattt gttatatttt attttttttt ttttattttt    1260
   tggtttagaa tttaatttta atttttttga atttttaagg ttatttttaa ttaggatttt    1320
   ttaaatattt taattatttt aaattttttt tttttttgga atttaaagga attttatttt    1380
   ttatttttta ttagtaaggt ggttttagtt aaattagggt tttaggaata aatatagttt    1440
   ggtttaaata tttgtatatt tattaggttt tattttaaag tagattgttt tgatgatgtt    1500
   ttgttattta tagtgtggtt tttggattgg tagtattatt attatttggg aatttgttag    1560
   aagtgtagaa tttgaggttt tagtttagat ttgttgaatt attgttttta tttgtttaag    1620
   agttttgtga ttggtttgat ttttatgtat ataaaagttt aaggagtttt gtttaaattt    1680
   tttgttttta agttgttttt gaaatgttgg tttttttgtt tttggatgtt tgtagagatg    1740
   gggtagtgga attagtttta tgtgggggta gggtgtgggt tttatttttt ttttttgttt    1800
   tttgtttttt tattttgttt tttttgatgt ttttttagtg gttgtatttg tagagttttt    1860
   atttttttgg gaagtttttt gatatagatt ttgtttttgt ttttagggtg gggttgtttt    1920
   attttttttt ttttaaagag tttgggatgt tgatagtttt agatggaggt attttgtttg    1980
   ttgaaaaata aattggtagt taaagtaggg ttgtgagagt gagaaaatta taggtttgtg    2040
   tgttgttgat aatgtgtggg taggtagttt ttgtagaagt tttgttgttg tggtgtgttg    2100
   tgtattagag ttgtgttatt gttttttttt ttgtttgggt tgtttgtgtt tttgttgttt    2160
   ttggttgttg ttttttgtgt tgttttttta gttattttgt ttattgtttt ttttaaagga    2220
   tttttttgtt gttttgttat gagggatgga tttattaata agtttttggg tggatgtgtg    2280
   tagattttgg atttatagga ggttaaaatt tatgagttgt tatgaaaatg atattgttta    2340
   agagaagttg aagttgtttt tttttttgta gtatgtaatg ttttgatttg gttaaaataa    2400
   ataaataaat aataatttaa aaagaaagaa agaaatatag aaagattata tttttatgag    2460
   gtttgtaatg ttaaaatgaa aagggatttt aaagtgtaat atttagattg tttttttttt    2520
   tggagagagt tgatttggtt tgattttatg tggtgaataa ttaattatta attttttttt    2580
   tttgaggggt attaaagttt ttttttttat ttgtttgtta ttagataagt taggtagatg    2640
   agaagttagt gggtaattat aggtataatt atgatattta ttggaaatta aaagatttga    2700
   ttttttgagg taaagaatgt atttgaaaat aaatttgaaa atagttttat aggagttgaa    2760
   aaatttagtg tttttgaatt agtaatatga atttttttga gtattaaaaa gtataagtat    2820
   ttttaaaaag gatattttga gttatagaag atgatgtttg ttggggttga ttgagttttt    2880
   ttggaagaga tttttgtgtt atggtgtatt agaaagtttt tattattgtt ggggtttttg    2940
   tagttgtttt agttatttag tgttttttta ttattttttt tgtttgaaaa tatagagtag    3000
   aaatagttta tatttatttt ttaatttttt tgtgtttata atgagtaagt attttagatg    3060
   agtaagaatt tattttattt ttaagaatta gtagagaatt atatgagttt tttttttttt    3120
   tttttagaaa tgtttttagg ttggttaatt tttttattga gtaatttttt atagtaatta    3180
   ttttgtatat tttttatttg agaggttgtt tttttatttt tatgagtttt tttttttttg    3240
   ttttatggtt ttatttttaa ttatttatgt ttttttgttg aaggtttttt gatttaaata    3300
   tagagatttg tatattttgg ttatttatgt atagatttta ataatgaata atttaatttt    3360
   atggtgatat aaaggaggaa aatttaggtt aaagaatgtt taatttttag ggaagaagga    3420
   gagagttggt tagggatagt gtgtgttggg tgttaatagt tatgtgtata gtttgttggg    3480
   gaatttggtt tttttttgaa tttggtgtag ggtattggaa tgtttggggg aggagtagat    3540
   aggatgttgt ttaggttgaa tagggttgga ttatgagagt tttgaatgta tttaagaagg    3600
   aatttttttt agtgggttgg ggttaggtta gattgatgaa tgattgattt ttattattta    3660
   ttttgaggtt tttatggaga tattttaaat ggattttttt tttatttata aattgttata    3720
   aataaatgat taaaagatgt ttaagtggtg attagaaaat aataaatttt attttagtta    3780
   tatattttag aatttatata tttgttattg tagtgttttt taaagttatt ttttgggagg    3840
   gagagggagt attattttaa tagtattatt attattttaa atatttttgg atttgttttt    3900
   ttgaa                                                                3905
<210> 39
   <211> 5338
   <212> DNA
   <213> Artificial Sequence
<220>
   <223> chemically treated genomic DNA (Homo sapiens)
<400> 39
   ggtaattttt tttgttatta ttttttgtat aaaatatgta agatgttttt attttattag      60
   tattgaaatt aaagattaaa agagagaaag gtgttggatt gaaaaggagg tggtgtaatg    120
   gttatttttt ttagtatttt ggttttgttt tttttgggga aggtggttat atttttattt    180
   gttttgtttt tgaatgtagt aaataatttt ataaataatt attgttgttt atgtttttta    240
   tttgtttttt tggttttatt agatttttgt ttttttgtat tttttgggta gggtttttaa    300
   taagtttagg ttgaaagaat gtttgttatt ttttttattt ttgttgaaag aaaaggaaga    360
   aaaatagtag tagtgagaaa tttttggggt gatttttttt ttgtttttaa gtattagtgt    420
   atagtatttt tttttgtttt tgttgtggtt ttttgttgtt ttgggttatg ttgtttagtt    480
   aagtaatttg gatttgagag tgtatagtta ggattagttt agggggtgag gggttggttt    540
   ttgggaaatt aagtgtttag gatagaggtg gaaagtgggt tttgggagtt agaaaagaga    600
   gagaagggta gatggttggg tggtaaatat aaaaatagaa ataatttagg gggatgtttg    660
   ttaggttttt gtgtttgttt tttttttttt aatttggagt aggttttttt tggttttttg   720
   tgttttgggg tgttttttgg tggtagtggt agtagtgggt aatgtgtgga gggtttaggg  780
   ggtgtatagg ggatttttgg gtatatttag agaggtgggt gtggtttttt ggtggtggtg    840
   atgtagttat ttggtgagtg gagttttgtt tttttggttt ggtgggttta tggttgtttt    900
   attaagtatt gtggttgagt agggtaggaa gttgtttttg gaatgtggtg ttgttgtgtt   960
   gtagttgaag tttgagtttt ttttggtttt ggtgtttttg ttgttgttgt tattattatt  1020
   attgttgttg ttattgttat tttgggaatt tagggttgtg gtagttgttg ttgtggttgt   1080
   tgttgttgat tgattgttgt ggtaattgag gtatgagtag ggtgtagagt tgttgttggg   1140
   gggtgtgttg gttgttgttg ttgaggaggt tgtagttgtt gtggttgttg tggttgttgt   1200
   ggtagaggtt gtgttgttga gttttgtggt ggttgtggga gtttggtaga gattagggtg   1260
   gtggaagtta tatagtagtt ttggttgaga gtaggggtgt gagagggtgt ggaaggtgtt   1320
   tagtggttgg atggaagtag tgaagggtga tgaagttgtg gttgttgtgt ttgaggttgt   1380
   agttgtggtt gttgttgttg tgatgtttat gtgtgggtag tagtgtagtg gtatgtgtga   1440
   gtggaagggg tagggtaggt ttttggtggt ggttgtgtgt gttattatgt aggtgtagaa   1500
   gttggggttg gttgggtgtg gttaggatat ggttaggtgt tgttgtttgt tttttatgtg   1560
   ttggttttgg aattatattt gtggggagag atgtgttgta gtttgggtta gggagtgttt 1620
   tgtgttttta gtttttgttt taggattttt gttttttttg gatttttgaa tggtttggtt 1680
   tatttttttt tgattaagtt taattttgag tattttgtgg ttttttagtt gggaaagtgt 1740
   ggatggtagt gtgtggattg ttgtgggtat attgttttta atgaagaggg tttttttttt 1800
   tgtgtttggt tgttgttgtt ttttaggttt ttattttttt attttttgtt gtattttttt 1860
   gttttaattt aatttttttt tttttttttg ttatttatta gtgttggttt tgttgagtat 1920
   ttgtttttta attttaggat ttgtatgggg attttgggta gtttttgaag agaggttgtt 1980
   gtttagtttt tttgagaggt tgttgtgtga agttttgagt tttttgaatt gtaaggattt 2040
   gtttttaggg gtatgggttt gattttgata ttgaagggat gattttgttg gaattgtttg 2100
   ttttaaatga gtgggtagag taatgttttt ataaatgggg aaggggatgt ttttattttt 2160
   tttaatatta tttaataaag atattatttg ttataatttt aaattaaaga aagtttgatt 2220
   aaattagagg gagattttta tatttttttt ttattttgtg gagatttttt tttttttttg 2280
   tagtgttgaa tgttttatga agggtttatt aaattgttta attttttggt tatttttttt 2340
   agatgtataa taaaattaat aatttaaagt tatatataaa taaggataat tttgttgtat 2400
   ttttttttgt aggaggttgt tttttttttg ttgtttaaga ggaaaatgtt taggaaatta 2460
   ttgttttaaa ttaattgatt ttaaagatat agtatttttt tttttgtgta atgatttatg 2520
   tggaaaataa atttttaagt ttaagagtaa atgaaaagtt ttatttttgg tttttgtttg 2580
   aggaataaag attaattggg tttgttgttt aggggaaagt ggggttgtgg gtatgggtga 2640
   gggggtattt ggttaggtgt tgggtataat ggagtagggg tgagtgtttt tagtattgga 2700
   gttattattt gggggttttt ttagatttgt taggttggat aattgtttgg atttggtggt 2760
   tgggaaataa ataagttaat ttttttggtg attatttttt gtggattttt agatttttag 2820
   ttaaatttag ttatttagaa aggggaaagg ggaaaaagaa ataattaatt tagatgtttt 2880
   tggggaggtt agagtaggta tgtattggaa ttgatatttt gatggtggtt ttgggtaggt 2940
   tgagtgttgt ggttagtttg tattggtggg gttgtgatat atagtttttt tggtagaatt 3000
   ttttttttag gtgtgtgatt tgtttgtggg tgaatgttgt atggtagtgt tgtatttgat 3060
   ttgtgttaga gttggagtta tttagtgttg tgtttttgtt gttgtttttg ttgtttttat 3120
   gtaggttttt gaggtttgag tttgatgttg atgttgtggt gttggtagtt gagttgtttt 3180
   ttgtgtattt tggtaaataa atgattaata gtgtatgagt ggttgtagga ttaatagttt 3240
   ggtgttggtg ttgtgtgtgg attggggaag ttttgttagg aaggagagtt gttgttggaa 3300
   ttgatggggt ttgttatgtt tataaattgt tgttgttaat ggaattaata aagtttgggg 3360
   agttttttat aagtaaataa tagaaatgga attaggagat ttttttttta ataattagaa 3420
   atttttaatt aaggaggaaa agtggttgag ggaaaatgtt tatttttggg tgtagtttgg 3480
   gaagttttgg gttttttatg gtttggagat tgtagggtag ttttttatat ggtttttaat 3540
   ttttatttga gtttttgggg ttttaaatat tttaagagtt ttaatatagt agtagtttaa 3600
   atttaagtta atagggggtg aaatatattt tttaattttt ttttttgttg atttaaatag 3660
   agatgttggt gaggtttttt ttgatttatt tagaagaatg ttttttttag ttttgagggt 3720
   ataggggtag gggagtttag agtagttgtt tagggtattt tttttaaaag taatttggtt 3780
   gttgattagg ggtttattgg tttgtttggg tttttagata tgagtgtaga aatatttttt 3840
   tggatttagg agtgagggtg ggtgggtttg gtgttttagg ttttgtaggt ttgagtttgg 3900
   tgggaaagtt taaggagtag aattggaagg tatggtttta gatatgtgtt ttgtttttgt 3960
   ttgtgttagt ttggtgtagt ttgtttgtgg agggtttgag aggggaaaag gtattgggaa 4020
   aggttggtgg gggttgtgga ggagtaaaga ggatgggatt ggagagtgtg gtgtggttgg 4080
   tgtggttatt tttgttattg ttttttttaa gttgagtggt gttgaggttt ttgggggagt 4140
   gaagtgtgga gtagtttatt tttatgttgt tgtttatgtt ggttttagtg gttgtttttg 4200
   aataatggtt tggttttttg tggtttttgg tggtggagga gattttggag gagatggtgt 4260
   ttttgttgtt tgtgtgttgt aggttgaata aagtgtttat tttgaatttg tttttggtgg 4320
   ggagtttttt tagagtgttg tgtagggggg tagatggtag gtgtgggttt aggtgagttg 4380
   ggtgttgtga attttttagg gttttgagta tagtattgtt agttgagttg gataaattgg 4440
   agaatttttt gtttgttgta gggttgtgta gttttttttt tattagaatt attttttttt 4500
   ttattttttt tattatttta gtttttttaa aaatgttata gtggttttgt tgttttgttt 4560
   taatgatagg ttgtgtttag ggttaatttt tatttagttt tagtgaatgt ttttaaatat 4620
   tattattgtt tttggaggga ggtggaaaaa ttgtgggatg ttagagtgag ggaatgattg 4680
   gttaaaatga tttatatatt tttttgattt tgagatgtta tttattaaaa agtagtgttt 4740
   gtttgttatt aaggtatgaa tgatgttgtt tgaataatta tttattgtaa taggtttata 4800
   agtaaataaa tatatttttt tgttagtggg taatgaaggt agttttagag tagataaata 4860
   gatttaggta ggaggtgaga agagataagt gaggaggaag gttttggttt tttgtttgtt 4920
   ttgagttagt tttgtttgtt tgggggtttg gttttggggg tgaaattgat agtttgatta 4980
   atagagtgtg ttgtggtata tttttttttt tatttagggg tattattatg tttttagttt 5040
   gttaggttgt tttttaggtt ttaattatgt agtgtttttt ttattttttt ttggatatag 5100
   atatgtatta aataatagat aatggtttga ttttttagag taggtttttg ggaaggtttg 5160
   gttttttttt tttttttttt tttttttttt attatttata agttgggggt ataagtgggg 5220
   ggagataagg gggaggatgt aggaagagtt gtgtaaggag aagattttgt gtttttttta 5280
   tttggattat ttattttttg gattttggaa ttaaatatgt attttattta ataaatag 5338
<210> 40
   <211> 5338
   <212> DNA
   <213> Artificial Sequence
<220>
   <223> chemically treated genomic DNA (Homo sapiens)
<400> 40
   ttatttgtta aataaaatat atatttggtt ttgaaatttg aaagataagt aatttagata      60
   ggagaggtgt aaggtttttt ttttatgtaa ttttttttgt attttttttt ttattttttt     120
   ttatttatgt ttttagtttg taaatggtaa aaaaaaaaaa aaaaaaaaaa aaaaaaatta     180
   aatttttttg aggatttatt ttggaaagtt aaattattat ttattattta atatgtattt    240
   gtgtttaagg aaaaatgaag aaggtgttgt atgattagga tttaaggggt aatttagtaa     300
   attgagagtg taatgatgtt tttaaatgaa gggggaaatg tgttgtggtg tgttttatta     360
   attagattgt taattttatt tttgaggtta aatttttggg tgagtagaat tggtttggag     420
   tgggtaaagg attggagttt ttttttttat ttgttttttt ttgtttttta tttggattta       480
   tttgtttgtt ttgaagttgt ttttattatt tattgatagg agggtgtatt tatttgttta     540
   taaatttgtt ataataaatg attatttgaa tagtgttatt tgtattttaa tgatgagtgg     600
   gtgttatttt ttgatgaatg atattttggg attggaagga tgtatgggtt attttgatta     660
   gttatttttt tgttttggta ttttataatt tttttgtttt tttttgaaag tgataataat     720
   atttagaggt gtttgttggg gttgaatgag aattagtttt aggtgtagtt tattattagg     780
   atgggatagt agggttattg tgatattttt aagaaagttg agatgatgga aagaataaga     840
   aaagagatga ttttgatgga gagagggttg tatagtttta tggtgggtaa gagatttttt     900
   aatttgttta atttggttgg taatgttgtg tttgaggttt tggaaaattt gtagtatttg     960
   gtttgtttaa gtttgtgttt gttgtttgtt tttttgtata gtgttttggg agaatttttt    1020
   gttaagggta aatttgaaat agatattttg tttaatttgt agtatatggg tagtgaaagt    1080
   attgtttttt ttgaaatttt ttttgttgtt gagagttgta agaagttggg ttattattta    1140
   gaggtggttg ttgaggttga tatgagtagt gatgtggagg tgggttgttt tgtgttttgt    1200
   ttttttgggg gttttggtgt tgtttagttt aaggaaaata atggtaaagg taattgtgtt   1260
   ggttgtattg tgttttttag ttttattttt tttgtttttt tgtggttttt gttagttttt    1320
   tttggtgttt tttttttttt tagatttttt ataggtaagt tatattgagt tagtatgggt    1380
   gggggtggga tgtatgtttg ggattgtgtt ttttagtttt gttttttgag tttttttgtt   1440
   aggtttaggt ttgtggagtt tggaatatta ggtttatttg tttttgtttt tgagtttgag    1500
   aaaatgtttt tgtgtttgtg tttaagagtt tgagtgagtt ggtagatttt tagttagtag    1560
   ttgagttgtt tttgggaaag gtattttggg taattatttt gaattttttt gtttttgtgt    1620
   ttttagggtt ggagggggta tttttttggg taagttggag gaagttttgt tggtgttttt  1680
   gtttaggtta gtgggagaaa gagttgaaaa gtatatttta ttttttattg gtttgggttt    1740
   gagttattgt tgtgttggaa tttttaaaat atttgaaatt ttgaagattt agataaaggt    1800
   tagaggttgt ataaaaagtt gttttgtggt ttttagatta tgggaaattt agagtttttt    1860
   aaattgtgtt tagaggtagg tatttttttt tggttatttt ttttttttgg ttgaaaattt    1920
   ttaattatta aaaaagaaat tttttaattt tgtttttatt atttgtttat gaagggtttt    1980
   ttaaatttta ttgattttat taatggtagt aatttgtaag tatgatagat tttattaatt    2040
   ttggtagtga tttttttttt tgatggggtt tttttgattt gtgtgtagtg ttagtgttgg    2100
   gttgttggtt ttatagttat ttatgtgttg ttggttgttt gtttgttagg gtatgtagag    2160
   agtggtttgg ttgttggtat tatgatgttg gtgttgggtt taggttttgg aagtttgtat    2220
   ggaggtagtg gaggtagtgg tgggagtgtg gtgttgggtg gttttggttt tggtgtggat    2280
   taagtgtggt gttattgtat ggtgtttatt tgtgagtaga ttgtgtgttt ggagaaggag    2340
   ttttattggg agaattatgt gttgtggttt tgttggtgtg agttggttgt ggtatttaat    2400
   ttgtttgaaa ttattattaa ggtattaatt ttagtgtatg tttgttttgg tttttttggg    2460
   ggtatttggg ttgattgttt tttttttttt tttttttttt ttgggtggtt agatttagtt    2520
   aagggtttgg aaatttgtgg ggggtagtta ttaaaggaat tggtttattt attttttggt    2580
   tattgaattt gaatggttgt ttggtttgat ggatttaaga aggtttttga atggtgattt    2640
   taatgttgaa agtatttgtt tttgttttat tgtgtttaat gtttggttag atgttttttt    2700
   gtttatattt atggttttat ttttttttag gtggtaagtt tagttggttt ttgtttttta   2760
   agtaggaatt agaggtgaaa ttttttattt gtttttgagt ttggagattt attttttgta  2820
   taaattgtta tatggagaaa aggatattgt atttttaaaa ttgattggtt tgagatagta    2880
   gttttttgaa tatttttttt ttgggtaatg aaaaaagggt aattttttgt ggggaaaaat  2940
   gtaatgaaat tgtttttatt tatatataat tttaggttat taattttatt atatgtttgg    3000
   gagagatagt tgggaggtta ggtgatttgg tgagtttttt atggagtgtt tgatattgta    3060
   gaaaaaagaa aaaattttta tggggtaggg gaggagtgtg gaagtttttt tttggtttga   3120
   ttgggttttt tttaatttag agttgtgatg aatgatgttt ttattagata gtgttgggag 3180
   gggtggaaat gttttttttt ttgtttatgg agatattgtt ttatttattt atttaaggta 3240
   aatgatttta ataaaattat ttttttaata ttaaaattgg atttgtgttt ttaggggtag 3300
   gtttttgtag tttggagggt ttaagatttt gtgtggtgat tttttagaaa agttgagtgg 3360
   tagttttttt ttaaaggttg tttagaattt ttgtataaat tttgggattg agagatgggt 3420
   gtttagtgag attggtattg gtgggtggtg ggagggagag gaaaggttgg attgggataa 3480
   aaaagtgtag taagaagtaa gggaataaaa gtttgaggag tagtagtagt tggatgtggg 3540
   ggagaggatt ttttttgttg aggatggtgt gtttatggtg gtttatatat tgttgtttat 3600
   atttttttag ttgggagatt atagggtatt tggggttggg tttggttgga gagaagtaga 3660
   ttaagttatt tagaggtttg gaaggggtag aggttttggg ataggagttg ggaatatggg 3720
   gtgtttttta atttaggttg tggtgtgttt ttttttgtag gtgtggtttt agaattggtg 3780
   tatgaaggat aagtggtagt gtttggttat gttttggttg tatttagttg attttagttt 3840
   ttatatttat atgatgatgt atgtggttgt tattggaagt ttgttttatt ttttttattt 3900
   gtatgtgttg ttgtattatt atttgtatgt gggtgttatg gtggtggtgg ttgtggttgt 3960
   agttttaggt gtggtggttg tggttttgtt gttttttgtt atttttattt ggttattgga 4020
   tattttttgt gtttttttgt atttttattt ttggttggag ttgttgtgta gtttttgtta 4080
   ttttggtttt tattaggttt ttgtggttgt tgtggggttt aatagtgtgg tttttgttgt 4140
   ggtagttgtg gtagttgtag tggttgtggt ttttttggtg gtggtggttg gtgtgttttt 4200
   tagtggtggt tttgtatttt gtttgtgttt tagttgttat agtagttagt tggtggtggt      4260
   agttgtggta gtagttgttg tagttttggg tttttggggt ggtggtggtg gtggtggtgg      4320
   tggtggtggt ggtggtggtg ggggtgttgg ggttggggga ggtttggatt ttggttgtag 4380
   tgtggtggtg ttgtgttttg agagtggttt tttgttttat ttggttgtgg tgtttagtaa 4440
   gatggttgtg agtttgttgg attagaggga tgaggttttg tttattagat aattatgttg 4500
   ttattgttag ggggttgtgt ttgttttttt gagtgtgttt gggagttttt tgtgtgtttt 4560
   ttgagttttt tgtgtgttgt ttgttgttgt tgttgttgtt agggggtgtt ttggggtgtg 4620
   ggaggttgaa gggaatttgt tttgaattgg gggagaagga gtaggtgtaa aagtttggtg 4680
   ggtatttttt taaattattt ttatttttgt atttgttatt tagttgtttg tttttttttt 4740
   ttttttttgg tttttgggat ttatttttta tttttgtttt gagtgtttgg ttttttaaag 4800
   attaattttt tgttttttaa gttagttttg gttgtgtatt tttaggtttg ggttgtttgg 4860
   ttgaatggtg tggtttgaag taatggagaa ttataataaa gatagagggg gatgttgtgt 4920
   gttggtgttt gggggtgggg gaggagttgt tttggaggtt ttttgttgtt gttgtttttt 4980
   tttttttttt tttaatgagg gtgggggagg tggtaaatgt ttttttagtt tgagtttatt 5040
   agggattttg tttgaagagt gtgggagaat ggaggtttga taaggttggg aggatggatg 5100
   gagagtgtgg gtagtggtgg ttgtttgtga gattgtttat tatgtttagg agtaaggtgg 5160
   gtagggatgt ggttgttttt tttgggaagg ataaagttga ggtgttggaa ggggtggttg 5220
   ttgtgttgtt ttttttttgg tttaatattt tttttttttt tggtttttgg ttttagtgtt 5280
   gatggaatgg aagtattttg tatattttat gtaaaaagtg atggtaaaag aggttgtt 5338
<210> 41
   <211> 5332
   <212> DNA
   <213> Artificial Sequence
<220>
   <223> chemically treated genomic DNA (Homo sapiens)
<400> 41
   ttagttttaa attttgtatt tgtatttttt agataggaag tttttttttt ttgattaggg 60
   attgagggag aagagattta gaatgtattt ttttgtttta ggttttttgg gggggatatg 120
   gttttagata gttattttga aattgggttt aggtttttat agtaagttga gtgttttttt 180
   atatttttaa ttttggtttt tttattaaag tttaaaagat ttttttgttt tttaggtttt 240
   ttttgtttgg ttgtttttat tattttttaa ttgtggaaat attagttatt atatgtttgt 300
   tggagaataa gtgtgagtgt tgtggtggat agttgttgtt aaaggtgttt agatggatgg 360
   tatttttaaa gtagggattt tagtattttt tttatatatt attatttaag aatggtagag 420
   gaaagagtag gagtggggtt atagatagga gagaaaggag gttgaggtta taaaattggt 480
   atttttttaa ttattaattt ttttttttat tattttgtta ttatttgtta tggtaaagtt 540
   ttttttttgt gttttttttt taataaagaa atttttagtt agtttttatt tatttttttt 600
   gatttgtagg aagttaaatt gttagtagat tgagtttttt aagggatatt tttgggatat 660
   gttggaattt tgatttgttt aagatgatga tggttattag tataaattag aaataatgtt 720
   attttttagg gtgttatttg taagtgtaat aatatggatt tatttgtaaa atttagttaa 780
   attgtgtttg gataagtgat tagatttaaa tgataaattt tttttattat ttagtttttt 840
   ttaagttttg tgggggtagt atagaagatt gttttagggt gaaattaagg tttagtttat 900
   tatttgtgtt agtttgaggg attttggaaa tgttgatttt gagaaaaaga taaaataaaa 960
   taaaataaaa taaaatttaa ttataaaata tattttattg tttataaagt aaatagaaat 1020
   ataaagttgt ttttaaaatt atttttttag taaataagta tgtgatattt tgagtaggag 1080
   taggagggag agaaagattg ttatttaaaa aaatatgaaa gtattaagta ataatttaaa 1140
   atatggttgt ttagaaaaat atttatattt attgtagttg tttaattggt tttgttaaag 1200
   ttggtaagta tttaaattgt gttataattt tatttaaatt ttgttttgtt ttagtaggtt 1260
   gaagagtttg aatagattaa ttattttata atgatgatga atgagaaatt aatttagata 1320
   taagtaagat aatttaggtt tttatttgtt taaatagttt tttaatatta tttgttattg 1380
   tgggttatag attgaattaa ttgtttaatt ttgtgaaagt tatatttttg tgtttttatt 1440
   gagattattt atagtgtagt gagtgttgtt gaaaggtata tgttgttaat agggataatt 1500
   atttaaaggg aagtgtttgt aaaatggaaa ataaatgttg gggttggtaa taaatgggat 1560
   taaaagtagt tattttaatt tggttttttg agggaaagga gtgggtgtgg ttttgtagga 1620
   ttaggggtgt ttttgatttt tttaagattt aggtgaggtt gagggttttt ggtgtgttag 1680
   tttgtgttgt ggttgtggtt gtggtggtga tttgggttgg gttttgtttt tgttggtttt 1740
   aggtttttgt tttttttgtg taggtagtgg gtgtgtgtgg tttgggttgg gtaagttgag 1800
   gaatagtgag tttttggatg ttgattgtag gatgttttag tttgtgtttg ggtttttgtt 1860
   tttttttgta tggggtttgt atttttgggt ttgggtttga tttatagggt gttgaggttt 1920
   ttgtagttga agttggaagg ttttgttgtg agtgtggtat aggttgttgg tagtttttgg 1980
   attttggagg tttggttttt tttttaagtt gatggtgggg aaagaatttt gtttttatag 2040
   tttttttgat ttttgttgtt tgttatttgg ggatgggaag tgtgtttggg ttgttttatg 2100
   tttttttggg ttggagtttt ttttatggtt ggtttttttg ggggtttttg ggtttgtgag 2160
   tagtgtttat tttttttaga gaagaatttt tttttttttt tattgaagtg tttttttttg 2220
   tattttgaaa taattttttt tgggtgaggt tagttttttt ttgttgtttt ttttttgtag 2280
   gtgtttggga gttttgtgag gattttgtgt agttgagttt aggtgatagg tgttttttta 2340
   ggtgtttatt tgtttttttt taatttgttt agggaaagat tagaatgatg tgtgtgggga 2400
   ggtggttttg ttttttttga aattggtgtt tttgggtaaa attgtgtgtt gtgtttatgt 2460
   gggttttgtt agtgtgtgtg ttttagaaga gtgggttttt taggtggtat tttttggaga 2520
   agtaagtgag gtgaatttag aatagagaag tgggaatgat tgtttgatgt gttgagttgg 2580
   aaaaagagag gagagggaga ggtttttagt gagtgtggag atagaggagt ttatgtggta 2640
   tagtatgagt tttatttttg tttgtgtaga ggtgtgtgtg tgtgtgtgtg tgtgtgtgtg 2700
   tgtgtgtgtg tgtgtgtgtt tttttttgta tttttgtgtt tgaaataaaa gtttaattaa 2760
   tggatttttg gtgttttgag gatttatttt tgggagtgat tttgatgtat tgttgtttaa 2820
   ttagtgtttt taattggtgt ttttggagat aggtaggttt ggagttggga tagagttttt 2880
   ttttgttttt tttgtatatt ggaaaggtaa aatttataat atattgtggt tattttttgg 2940
   gagagtgaaa gagagtggga ggtagggaag aaagggagag atgtagttgt ttgtagagga 3000
   tggagttttt agtttggggg gttgtgggga agggtttaga ttattgggtt tgatttagtt 3060
   ttttgtgtaa gagggtttgg gttggttgag ttgttgagga ttatgaattt gtttgtagaa 3120
   aggtttaggg agtttttatt gtttgtataa atttatttta tgtggtttat tattttgtta 3180
   ataatggttg tttgttaata aggatgtgga ggttaaataa tttgggtgga agattaaata 3240
   tgttttatta aggtgtttgg aagttgtgtg gtgtagagat ttgtgttgga gttgtttggt 3300
   ttattggagt ttggagggag atgggagtat agtttgtaag gagttttggg gtagaagagg 3360
   aggtaaggtt tagttttttt ttttagggtg ttttgttatg ttaggatagg tttaattttt 3420
   ttatatgtta aatagaaaat ttatatagaa gttagtttta atgaggtgtg gaggttaaat 3480
   ttttggagta gttggttgga gggtagatat ggagtatttg gggtttgaat taaaggtttt 3540
   tttttgagga ggggtggtta ttttttgttt tttagttttt atatgttatg tatttggtaa 3600
   tttgtgttta tatgggatta gtattggatt agaagataga agatgtggtt tttagtttgg 3660
   gttttgtttt taaattattg tagaattttt attatattat ttttttagtt ttattttttt 3720
   attgtgtaag agaaaggaga tggggtttga tgtagatgat gtatagtatt tttttttttg 3780
   tttagttttt tattagagtt agggaattga atattttata agatgttttg ttttttttaa 3840
   attttgtagg atgtagttaa aaagaatttt atttagtaaa tatttgaggg tttattttgt 3900
   attagttatg gtgtagtatg tattttttaa ttgagagtag aatattagtt tatatttttt 3960
   tgttattttt aaatataatg ttttataata aaagatgaaa gagaaagaat tttaaagaga 4020
   atagaatagg atgggtatgt atgtataggg tagagaaatt atggaggtaa aggtaaaggt 4080
   attgataaaa gttgtgaaga atttgttgaa ttggtaagga agttagtgga taaatattgt 4140
   agaagtagtt ttttaataga gatattatgt aggagagtag agagaaggtt tttttttgaa 4200
   ttttggtgat ttggtagaga ataaaagggt aagggttgta gaatttgatt aggaggttta 4260
   agttgtatgt atttaatatt gttaattttt ggtttatata tttttttagt agttggaatt 4320
   tttttttttt tttttttttt tttttttttt tttgagatgg ggttttgttt tgttgtttag 4380
   gttggagtgt agtggtttta ttaaggttta ttgtagtttt aaatttttag gtttaagtga 4440
   tttttttatt ttagtttttt gagtagtaag ggttataggt atatgttatt atgtttggat 4500
   aatttttttt tttggttgag gggtatagat ggggttttgt tgtgttattt aggttggttt 4560
   taagttttta ggtttaatga atttttttat tttggtgttt taaagtgttg agattatagg 4620
   aatgagttat tgtgtttggt ttaggaagtt tattttttag ttaattttga aatattattt 4680
   tttgaattta ttaattagga aatttggttg ttaggttggg attttttaag tttttgggtg    4740
   atgggtattt agttttaagg aaggtaatga tgtttaaatt attaaattat gttttagttt    4800
   ttgtgagtta ggaatttagt gagtttagtt gtgtgttttt ggttttaggt tattttattg    4860
   gggttgtggt tttatttaaa gatttagttg ggtgaggata tgttttgggt ttatttatgt    4920
   ggttgtttgg taggatttag ttttttgttg gtgggttatt tggttgaagg ttttagtttt    4980
   tttttggtgg ttggttagag gtttttttgt tttttattat atgagttttt ttatagggta    5040
   gtttataaag tggtagttgt ttttttttag attgagtaag taagagtgag agtggagggg    5100
   ggagaaagta tttaagatag aagttatatt tttttggtta tttaattttg gaagtgattt    5160
   tttattatta tgttatatta tatttgttag aagtgagtta ttagatttag tttatattaa    5220
   aggaaagggg attatagaag gtattaaatt taggaggtag ggattattgg ggattgtttt    5280
   agaggttgtt gtttagattt tttttatata atagttttga ggtttatatg gt              5332
<210> 42
   <211> 5332
   <212> DNA
   <213> Artificial Sequence
<220>
   <223> chemically treated genomic DNA (Homo sapiens)
<400> 42
   attatataag ttttagagtt gttatataaa aggggtttgg gtagtagttt ttaagatagt     60
   ttttagtgat ttttgttttt tggatttgat gttttttgta attttttttt ttttagtgtg    120
   ggttggattt agtgatttat ttttaataaa tataatatgg tatagtgatg gaaagttatt    180
   tttaagatta gatgattaaa agagtgtagt ttttgttttg ggtgtttttt ttttttttta    240
   tttttatttt tgtttattta gtttgaggaa agatagttgt tattttataa gttattttat    300
   agagaggttt atatggtaag gaatagagaa atttttggtt aattattaag aaggaattga    360
   ggtttttagt taaatagttt attagtaagg aattgaattt tgttaaataa ttatatgagt    420
   gagtttggaa tatattttta tttagttgag tttttagata agattataat tttagtggaa    480
   tagtttggag ttagggatat atagttaagt ttattagatt tttgatttat agaaattgag    540
   atatggttta gtgatttaag tattattgtt ttttttgagg ttaaatgttt attatttaga    600
   agtttgaaga gttttagttt agtagttgag ttttttgatt ggtggattta gaaagtaatg    660
   ttttaaaatt agttaaagag taaatttttt aggttgggta tagtggttta tttttgtaat    720
   tttaatattt tgggatatta gggtgggagg gtttattgag tttagaagtt tgagattagt    780
   ttgggtaata tagtaagatt ttatttgtat tttttaatta aaaaaaaaaa ttatttaggt    840
   atggtggtat atgtttgtgg tttttgttat ttgggagatt gagatgagag gattatttga    900
   gtttgggagt ttaaggttgt agtgagtttt gatggggtta ttgtatttta gtttgagtaa    960
   tagagtaaga ttttatttta aaaagaaaaa gagagagaga gagagagaga gaaattttaa   1020
   ttattgaaag aatatataaa ttaaaaattg gtaatattag atgtatataa tttgggtttt   1080
   ttagttaaat tttgtaattt ttattttttt gttttttgtt aaattattag gatttaggag   1140
   gggatttttt ttttattttt ttatatagta tttttgttaa ggagttattt ttgtagtatt   1200
   tatttattga tttttttgtt agtttaataa gttttttata gtttttgtta atgtttttat   1260
   ttttattttt atagtttttt tattttgtat atatatattt attttatttt attttttttg   1320
   aagttttttt ttttttattt tttgttataa aatattatat ttgagaataa taggggaata   1380
   tgaattgata ttttgttttt agttaaaaaa tatatattgt attatgattg gtgtagagta   1440
   ggtttttaga tatttgttaa ataaaatttt ttttgattgt attttgtaag gtttagggga   1500
   gatagagtat tttataaggt gtttagtttt ttaattttgg tgggaagttg gataaaagaa   1560
   gaaatgttgt atattattta tattaagttt tatttttttt tttttatata gtagaaaaat   1620
   gaagttggga gaataatgta ataaaaattt tatagtgatt taggggtaga atttagatta   1680
   gaaattatat tttttgtttt ttaatttaat gttaatttta tgtaggtata aattgttaga   1740
   tgtatgatat gtaaggattg ggaggtaggg gatggttatt tttttttaaa agggggtttt   1800
   tggtttagat tttagatgtt ttatgtttgt tttttagtta attattttga gggtttgatt   1860
   tttatatttt attggggttg atttttatgt ggattttttg tttgatatgt ggaagagtta   1920
   gatttgtttt ggtatagtag gatattttga agagggagat tagattttgt tttttttttt   1980
   attttggggt tttttgtaga ttgtgttttt attttttttt aggttttagt gagttaggtg   2040
   gttttagtgt aggtttttgt attgtgtggt ttttaagtgt tttgatgaga tgtgtttaat   2100
   tttttatttg gattatttaa tttttgtatt tttgttaata aatagttatt attaatgaga   2160
   tggtagatta tatggggtgg gtttgtatag ataatgagag ttttttaggt ttttttgtga   2220
   gtaaatttat aatttttagt gatttagttg gtttgaattt ttttatatag aaaattgagt   2280
   tgaatttggt ggtttgggtt tttttttatg atttttgggg ttggaggttt tgttttttgt   2340
   aaatagttgt attttttttt tttttttttg ttttttattt ttttttgttt ttttaagggg   2400
   tggttatagt gtgttataaa ttttattttt ttagtgtgta ggagaggtga gaggaggttt   2460
   tgttttagtt ttgggtttgt ttgtttttga ggatattaat taaaggtatt aattggatag 2520
   taatatatta aagttgtttt tgggagtgag tttttagagt attaggaatt tgttagttgg 2580
   gtttttgttt taaatataga aatatagagg aaaatatata tatatatata tatatatata 2640
   tatatatata tatatatata tttttatgtg ggtaagagta gggtttgtgt tgtgttgtgt 2700
   gggttttttt gtttttgtgt ttgttgggag tttttttttt tttttttttt ttttggtttg 2760
   gtatattaaa tgattgtttt tgtttttttg ttttgagttt attttattta tttttttaaa 2820
   gagtgttgtt tagaaggttt atttttttgg gatatgtata ttaatagagt ttgtgtagat 2880
   gtggtatgtg gttttattta agagtgttag ttttgggaga aataaaatta tttttttgtg 2940
   tatattgttt tggttttttt ttaaataggt tgagggaggg taagtagata tttggggagg 3000
   tatttgttgt ttggatttaa ttgtatgggg tttttatgag gtttttggat gtttgtggga 3060
   ggagggtgat agaggggaat tggttttatt taggaggggt tattttagga tatagaaagg 3120
   gatattttga tgggggaagg aaaggatttt tttttgaggg aagtagatgt tgtttatagg 3180
   tttaagggtt tttagaggag ttagttatgg aaagggtttt ggtttagagg gatgtgaagt 3240
   gatttgggtg tgttttttgt ttttaaatgg taagtggtgg gggttgggga agttgtggaa 3300
   atgaggtttt ttttttgtta ttggtttaga aggaaggtta agtttttaga gtttagaagt 3360
   tattagtaat ttgtgttgtg tttgtaatga ggttttttga ttttagttat aaaggtttta 3420
   gtgttttgtg ggttagattt aggtttgggg gtatgagttt tgtatgggga gggatggaga 3480
   tttgggtata aattgggatg ttttgtagtt agtgtttggg ggtttgttgt tttttggttt 3540
   gtttagtttg ggttatatgt gtttgttgtt tgtgtgaggg aggtgggaat ttgaagttgg 3600
   tgagagtgag gtttggtttg agttgttatt gtagttatag ttatggtgta ggttggtgtg 3660
   ttggggattt ttggttttat ttgggttttg gggaggttag aagtgttttt aattttatag 3720
   ggttatgttt gttttttttt tttggggagt tagattggaa tggttgtttt tgattttatt 3780
   tgttattaat tttggtattt gttttttatt ttataaatgt ttttttttaa gtaattgttt 3840
   ttgttaatag tgtatatttt ttagtagtgt ttattgtgtt ataaataatt ttgatgaaga 3900
   tgtaaggata tgatttttat aagattggat aattgattta atttgtgatt tgtgatggtg 3960
   aataatattg gaaggttatt taaatagatg aaggtttaaa ttgttttgtt tgtatttgaa 4020
   ttaatttttt atttattatt attatgaagt gattggttta tttaagtttt ttagtttgtt 4080
   ggaatggaat gggatttaaa tgagattgta atataattta aatgtttgtt gattttaatg 4140
   aggttaattg agtagttgta ataaatataa atattttttt aaatagttgt gttttaaatt 4200
   attatttaat gtttttatgt ttttttaagt ggtagttttt tttttttttt tgtttttgtt 4260
   tagaatatta tatatttatt tgttgaaaag gtgattttgg aaataatttt gtgtttttgt 4320
   ttgttttgta gatagtagga tatgttttgt gattgggttt tgttttgttt tgttttgttt 4380
   tgtttttttt ttagagttaa tatttttagg gttttttaag ttggtataaa tggtgagttg 4440
   agttttagtt ttattttaaa gtagtttttt gtgttgtttt tgtagagttt gggaggagtt 4500
   gagtgatgga agaaatttgt tgtttggatt tagttattta tttagatgta gtttagttgg 4560
   gttttgtaaa tgaatttgtg ttattgtatt tataaatgat attttgaaga gtggtattgt 4620
   ttttgatttg tattgatgat tgttattatt ttgaataaat tagaatttta atatatttta 4680
   aaggtatttt ttagaaagtt taatttgttg gtagtttggt tttttgtaaa ttaaaaggag 4740
   taggtggagg ttgattgagg atttttttat tagggaaggg gtatagaaag agaattttgt 4800
   tataataaat ggtggtagga tggtggagga gggggttgat aattagagaa atgttaattt 4860
   tgtggtttta attttttttt ttttttattt gtaattttat ttttattttt ttttttatta 4920
   tttttgggtg atgatgtatg gagaggatgt taagattttt attttgagaa tgttatttgt 4980
   ttggatattt ttggtggtaa ttgtttattg tgatatttat gtttattttt tggtggatat 5040
   atggtggttg gtgtttttgt agttggggaa taatggaagt aattaagtgg gagaagtttg 5100
   aggaataaaa gaatttttta aattttagtg aaagggttgg ggttggggat gtggggggat 5160
   atttgattta ttgtgaggat ttgagtttaa ttttggggtg attgtttaag gttatgtttt 5220
   ttttgaaagg tttagggtaa ggagatgtat tttgaatttt ttttttttta atttttggtt 5280
   gagggagagg gattttttgt ttggagagtg tgggtgtagg gtttgaaatt ag 5332
<210> 43
   <211> 4215
   <212> DNA
   <213> Artificial Sequence
<220>
   <223> chemically treated genomic DNA (Homo sapiens)
<400> 43
   ggtgtttgat taagtgtagt ttgtagtttg ggtattgtta attgatttta atttttttta      60
   tggtgagagg atggattttt tgttattttt ttgtttaatt tggtatttat ttatttattt     120
   atttattatg tttgttgggt gtatttaagt ttaattttgg ggtgtatgtg ttagtgtaga     180
   tattgtattt tttttttttt tttggttaat tttaggtaga attttaaaat aattgttttt     240
   ttttttatga tttagatgtt gtggtttgtg gataggaggt ttaagaaata gtatattttg 300
   agtggtaggt agtgaggtgg aaatggttgt tggttttagt ggtggtttta ttggaagttg 360
   agtttaggag tggttaagtg gttgttgggg aaagtattgg ggttttttag ggtttttttt 420
   gagtttttat tttgtatttt tgagggtgtg aaaattatgg gagttgtttt attttgtgtg 480
   tttagttttg ttttagttta gatattgttt tttgttagtt ttttttgtgg tgtttaggga 540
   ggatgtgttt tgtttttttt taatttggtt aatgggtgtt tgggtagtgt gtggtttgtt     600
   tttgtttttg ttagggaaat ttggaggagg agaaaagttt gtatagaggg tggaaaggtg     660
   agagtggagt tttaagtttg gtagtttgag aggaagatga atagttttag gttagagttt 720
   ttggtagagt ggattttgag ttgtttttag gtagttagga gtggttttag tggtagttgt 780
   aaattttagt agttgtttgt gttgtttgtg gttggggtgg agggtagtta gagttgggga 840
   ttaaggtttt gtgttatttg tgtgtatagt tttatatttg aatgttgttt ttttgtagat 900
   gagattggtg ggtattgtaa agttgggatt tgtttttgaa ggaaaaaaaa tagtgagtaa 960
   gaaatttagt attatttttt attgatttat tttgttgtat tttttgtttt ttaagttttt 1020
   gaaagttggt aattttgatt ttggtgttta aaaattgata gttattgaga ttggttttga 1080
   gaagttgaag atttggtagt ttttagattg agtaggataa ggtgaaagta ggttggaggt 1140
   gggtttagga tatttgaggg ttgattttgg gggtttgtga ggttgttatt gttgttgttg 1200
   ttataggtga gatggtgttg ggttgatgtt ggggttaatg ggtagagaat gtagggatgt 1260
   ggtttttgtt gaagagagtt aagaagggaa gagtgtgttt tttaaattgt ttttgtaatt 1320
   tgtttttagt gagtatttta ttgatttaga atttattgag aatagtatta gtgagttatt 1380
   ttttttttga gatgggtttt atttattttg gtaatggagt gagttggatt gtggggagga 1440
   agaggaatgg gaaaattagt ttataaatat taatgttagt aagagtgtgt tgttggtagg 1500
   atgtattgtg agtttggaga ttttggtggt tgtagttggt aagtggttat aatttagaaa 1560
   gtaggattga gttgtttttt ttgttttatt agtgtattgt tttttgggtg tgggtttaat 1620
   attttataag tggtaatttt tgtttatggt agttttgttt tttttttatt atttttagat 1680
   ttagttttgt attttaaggt tgtgtattgt tagttattat tatgtttatt tttggggtta 1740
   atttgtttgt ttttttgagt tttgattggt tgaatagttt agtgattatt ttggtggtga 1800
   tgtttatttt tggggtggtg ggtaatttgg tggttattgt ggtgttgtgt aagttgtgta 1860
   aggagtagaa ggagatgatt ttttatatgt tggtatgtgg gttggttgtt attgatttgt 1920
   tgggtatttt gttggtgagt ttggtgatta ttgttatgta tatgaagggt taatggtttg 1980
   ggggttagtt gttgtgtgag tatagtattt ttattttgtt tttttttagt ttgtttggtt 2040
   ttagtattat ttgtgttatg agtgttgagt gttatttggt tattaattat gtttattttt 2100
   atagttatta tgtggataag tgattggtgg gttttatgtt ttttgtagtt tatgtgttta 2160
   atgtgttttt ttgtgtgttg tttaatatgg gttttggtag tttgtggttg tagtatttag 2220
   atatttggtg ttttattgat tggattatta atgtgatggt gtatgttgtt tatttttata 2280
   tgtatgtggg ttttagtttt ttttttattt ttgttattgt tttttgtaat gtgtttgtgt 2340
   gtggtgtgtt gttttgtatg tattgttagt ttatgtgttg tattttgttg ggtattgagt 2400
   agtattatgt ggttgtggtt gttttggttg ttttttgggg ttattttgtt gtttttttag 2460
   ttttgttgtg ttttagtgat ttttggtgtt gttggagttt ttgttgtatt gtgggtgttg 2520
   agatttagat ggttatttta tttattgtta tttttttggt ggtgtttatt tgttttattt 2580
   tgtttgtggt gagtgattgg ggttggggtt ttatttggtt ttttttttgt atttattttt 2640
   tgtgtttatt ttttgttttt tgtttttttt ttgagttttt ggtagtgaat gtgttgtttt 2700
   taggttgggg ttgggatttt tatattgttt tttagagtag gtttaatttt ttttgaagtt 2760
   ttaattttaa tgagatttag taggtgtttt gtttttatat tttttagttt atgtttttgg 2820
   aagtttgggt tttttttttt attgagatag tttttatttt ttgttgtttg atattggttg 2880
   agttttttaa gaaaattttt tgtttttttt gttagatgtg gaggggagtt tgttgtagtg 2940
   tgatttagtt tatttttttg tattgtgaat tgtgaattgt aaaatttgaa atgttaggga 3000
   tggtgatttg tttgttgtaa attattttta aggaaagtgg ggtttttggt gttttgatgt 3060
   atgtaaggtt ttttgaattt ttttttttgt ggattgtgta gaattttaaa gttagaagag 3120
   atattaagag gtgttttagt ttaatttttt tattttattg agggagtgaa tgatatgttt 3180
   aaaataattt agttgaattt atatagttaa gttaggtttt ttgtttttta ttatggtgtg 3240
   ttttgggagg gtagtttaaa gtatataaat tttaatttat atatagggta gggtttagat 3300
   ttagtttttt ttattttgtt tagattagga aagtaaagtt ttttagggtg attaattatg 3360
   gataggttga tatatatagg gtatgggatt tgaatttttg ataattgtgt gaggtatagt 3420
   attattttag agaaattgag gtttagagat ggttgttttt gtttttttaa gttaaagttt 3480
   tatagtttgt aatggatttt aggttgtttg aggttagatt tagtttatgt gttattatat 3540
   tatgtttttt ttgtagaata ttttttattt tggtgtttgt aaagtgtggg ttggagtttt 3600
   ggtatatttt atttttagtt tattttttaa gaattgtttt tggattaatt tttaaaatat 3660
   gataaataga aaggtttaaa ttttatttag ttttttattt tgtttgtggt taaaatggaa 3720
   tgtattagaa gtagatgggt agaataatgt aatattttat ttagagtttt ttttgaaagt 3780
   aattagttat gagttttttt attttttaaa atattgtatt tttggaaatt ttaaattatt 3840
   aatttaaaaa gagataattg ttttttttta tgtttgttgg gaataagaga atataaaatg 3900
   taatgggtta ttattttttt ttttttttta ttgatattgt ttagggattt ttggtgtaaa 3960
   aatatatttt tgataaaagt gattgtaaat ggaaggaatt tagagggggt ttagagttag 4020
   aatatagtag gtttttatta atttgtataa atagaagtaa atataatagg ttataagagt 4080
   ttttaagtta ttttaggatt taatataatt tatagagtta agttatattt gagttatata 4140
   aatatatttt attgtttgta atggtaattt agaataaagt gagattttgg aagaaataat 4200
   attgtttttg atggg 4215
<210> 44
   <211> 4215
   <212> DNA
   <213> Artificial Sequence
<220>
   <223> chemically treated genomic DNA (Homo sapiens)
<400> 44
   tttattagga gtagtgttgt tttttttaaa attttatttt attttaggtt gttattatag     60
   atagtaaaat atatttatgt ggtttaagtg tgatttaatt ttgtaaatta tattgggttt    120
   tggaatggtt taaaggtttt tatggtttat tatatttatt tttgtttata taaattggta    180
   agaatttatt gtattttggt tttgagtttt ttttaagttt tttttattta tagttatttt    240
   tattaaagat atatttttat attaaagatt tttggatagt attaataagg aaaaggaagg    300
   aataatagtt tattatattt tatatttttt tgtttttaat aagtataaga aaaagtagtt    360
   attttttttt gagttggtag tttagaattt ttaggaatgt agtgttttgg aagatggaag    420
   aatttataat tagttgtttt taagagaaat tttagataaa gtattgtatt attttgttta    480
   tttgtttttg gtgtatttta ttttagttat aggtaaagta gaaaattagg tagggtttga    540
   gtttttttgt ttattatatt ttaaaagtta atttaaaggt aatttttaaa agatgaatta    600
   aaaatgaagt gtattaaaat tttaatttat gttttataaa tgttaaaatg agaaatgttt    660
   tatgagggag atgtgatgta ataatatatg agttgggttt ggttttagat ggtttaaggt    720
   ttattataag ttgtagagtt ttggtttgaa gaaataaaga tagttatttt taaattttgg    780
   tttttttaaa atggtattgt attttgtata gttgttgaga atttaaattt tatattttat    840
   gtatattagt ttgtttataa ttagttattt tgagaaattt tgtttttttg gtttagatag    900
   aatgaaagaa attgagtttg gattttgttt tgtatatgaa ttagaatttg tgtgttttaa    960
   gttgtttttt tagggtatat tatggtaaag agtagggagt ttagtttgat tgtgtaggtt   1020
   tggttggatt attttaagtg tattatttat ttttttagtg aagtgaggga gttagattaa   1080
   aatatttttt gatatttttt ttagttttaa aattttatgt aatttatggg ggaagaaatt   1140
   taagaaattt tgtatatatt aaaatattaa aaattttatt ttttttaaaa atggtttata   1200
   atgaatagat tattattttt gatattttag gttttgtagt ttatagttta tagtatggag   1260
   gaatgggtta agttatatta tagtaggttt ttttttatgt ttaatagagg gggtgggggg   1320
   tttttttgga agatttggtt agtgttaggt agtaaggggt aagggttgtt ttggtggaga   1380
   aagaaattta ggtttttggg aatataaatt gggggatgta ggggtaaagt atttgttaaa   1440
   ttttgttagg gttgggattt taaagagggt tgggtttgtt ttgagaaata gtgtgggaat   1500
   tttagttttg atttaaaggt gatatgttta ttgttaagga tttagaggga aagtagggag   1560
   tggggaatgg atgtgggagg tggatgtgag aaaaaggttg agtagggttt tagttttggt   1620
   tatttattat gagtgggatg gagtagatga gtattattag ggaggtggta atgagtaaga   1680
   tgattatttg gattttggtg tttgtgatgt ggtggaagtt ttggtggtgt tgaaagttgt   1740
   tgaggtgtgg taaggttggg gaggtagtgg ggtggttttg ggaggtaatt gaggtggttg   1800
   tggttgtgtg gtgttgtttg gtgtttagtg aggtgtggtg tatgaattgg tggtgtatgt   1860
   ggagtagtgt gttgtatata agtatgttgt agaggatggt ggtgagaatg aggaaggagt   1920
   tgaagtttgt gtatatgtag gagtaggtgg tgtgtgttgt tatgttggtg gtttagttga   1980
   tgaagtatta ggtgtttggg tattgtagtt gtgagttatt gagatttatg ttgggtagtg   2040
   tgtaaaagag tatgttggat gtatagattg taaagagtgt gaggtttgtt aattgtttgt   2100
   ttatgtagtg gttgtagaaa taggtatggt tgatggttag gtagtgtttg atatttatgg   2160
   tgtagatgat gttgaggttg gataggttga agaagagtag aatgaaggtg ttgtatttgt   2220
   atagtggttg gtttttgggt tattggtttt ttatgtatgt ggtgatggtt attgggttta   2280
   ttaataaagt gtttaatagg ttggtgatag ttagtttata tattagtgtg tagaaggttg   2340
   tttttttttg ttttttgtgt gatttgtata gtattatgat ggttattagg ttgtttatta   2400
   ttttgaagat gaatattatt gttgggatgg ttattgggtt gtttagttgg ttggggttta   2460
   aggaggtgga tgaattgatt ttgggagtgg atatgatagt ggttggtggt gtgtagtttt   2520
   ggagtgtaag gttgggtttg gggatggtag aagagagata aagttgttgt gagtggaaat   2580
   tattatttgt aaggtgttag atttgtgttt gagaaatgat atattgataa gataagggga   2640
   gtaatttgat tttatttttt ggattgtagt tatttattaa ttgtggttat taaaattttt   2700
   aggtttgtga tatgttttgt taatagtata tttttgttga tattaatatt tataaattga   2760
   tttttttatt tttttttttt tttataattt aatttatttt attgttaaga tgaataagat   2820
   ttattttaag ggaaaagtag tttgttagtg ttatttttga tagattttga attaataaaa   2880
   tgtttattga aaataagtta taaaagtaat ttggagagtg tgtttttttt tttttggttt 2940
   tttttggtga gggttgtatt tttgtgtttt ttatttgttg attttaatgt tagtttaatg 3000
   ttattttatt tgtagtggta gtagtggtgg tagttttatg agtttttagg gttagttttt 3060
   agatgttttg gatttgtttt taatttgttt ttattttgtt ttgtttagtt tggaaattgt 3120
   taaatttttg gttttttaaa gttggtttta gtggttgttg atttttggat attgaggtta 3180
   gagttgttag tttttaaaaa tttgggaaat aagaggtgta gtgggatggg ttagtgaaga 3240
   atggtgttgg attttttatt tgttattttt ttttttttaa agatgagttt tagttttgta 3300
   gtgtttgttg gttttgtttg tgggaggata gtgtttaggt gtgaggttgt gtgtgtaggt 3360
   ggtgtggagt tttggttttt agttttggtt gttttttgtt ttagttatgg gtagtatggg 3420
   tggttattgg agtttgtggt tgttgttgag gttgtttttg gttatttggg ggtggtttgg 3480
   ggtttatttt gttagaggtt ttggtttggg gttgtttatt ttttttttgg gttgttgggt 3540
   ttggagtttt gtttttgttt ttttattttt tgtataaatt tttttttttt tttaagtttt 3600
   tttggtggag gtggaggtaa attgtgtgtt gtttgggtgt ttattggttg gattggaagg 3660
   gggtggagtg tgtttttttt gggtgttgta gggaagattg gtggggggtg gtgtttgggt 3720
   tggggtgggg ttgggtgtgt ggggtggggt ggtttttgtg gtttttatgt ttttggaggt 3780
   gtggagtggg aatttggagg ggattttggg aagttttggt gttttttttg gtgattgttt 3840
   agttgttttt gaatttggtt tttagtgggg ttattattga aattggtgat tgtttttgtt 3900
   ttgttgtttg ttgtttggag tgtattattt tttgaatttt ttgtttgtgg gttgtaatat 3960
   ttagattgtg gaagagaggg tagttgtttt aggattttat ttagggttgg ttgagaaagg 4020
   agaagaaata tggtgtttgt gttagtgtgt gtgttttggg gttagatttg ggtgtgttta 4080
   gtggatgtgg taggtgggtg ggtgggtggg tattagattg ggtggggaaa taatgaagaa 4140
   tttatttttt tattataaaa ggagttggag ttagttggta gtgtttagat tgtgggttgt 4200
   gtttggttgg gtatt 4215
<210> 45
   <211> 5473
   <212> DNA
   <213> Artificial Sequence
<220>
   <223> chemically treated genomic DNA (Homo sapiens)
<400> 45
   gtaatgttaa ttagttttat tttaaagaaa aattaaatat tagagtaaat aatgattttt     60
   ttgtggagaa ggagttgtaa gggttgggga gaggtaatta gtgttataaa agttttatta    120
   taaagaagtt tagaattttt ttgttttgtt gtgtagtaga gtgaaatata gtagtagaat    180
   aattgtgtat tattattatg ataatttaat ttgttttttt agaaaaagtt attgtttgta    240
   gttaggatag taggaaaata tttagaaaat aattaaaatg taaataaaag tatgaaataa    300
   taataaagtt tattttttaa gagaaaaaaa gaagagtatt tagtgaaagg agaatgaagt    360
   atatataaag taagtaattt ttaggtattt tgtttttttg gatgttattt gtttaaattt    420
   gataattttt ataggtagta tgatagattt tttaaaattt aaataaagtt tgtttttgta    480
   ttttattttt aataataaag tatattgatt gatttataaa ttaaagtttt taaaatttta    540
   ttaaagtaaa aatattgttt tagagttttt attggtatgt ttttgaaagt aagtgttata    600
   gttaatgtaa atttttaaaa atttttttgt tatttagtat aatagagggt ggattttgtg    660
   ttagggtggt attttttgtg taatttatat ggtttgaggt tttttgtttt tttttttttt    720
   agaagagttg gaattaagat attaataatt ataattgtaa taattattat tatgttttta    780
   aggggttgtt gagtttttgg agattttagt gtatagagtg aagtaggaga ggtttaaaaa    840
   ggaaggtttt gttgttgatg ttgtgatgat taaggtagtt tattattatt tttttttttt    900
   aatgtgattg ttttgttttt tttttttttg attttgttat aagatgtggg gaagtttttt    960
   aagaattgtg tatagtttgt tatagatggt ggtttttgtg tttagagatt gaaagagtat   1020
   ttgttatagt gatattttgt aatagttgta gatgtgtggg tttaaaattg ttttatgtgt   1080
   ttaaattaat aataattatt attttatttt attttattat ttgagtggaa atataatttg   1140
   aaatatttaa atttgttttt tttaaagtta gaaggatatg tgtatgtgaa tgtatataat   1200
   ttgttttttt aaatgttttt aaagtttatt tttgttatta tatttgtttt tataattaat   1260
   aaagaaaatt tgttttagtt ttaattttat tgggtatggt atgtgttgga gggtaggttt   1320
   tggaaatata aatattaata tatgtggttt gttttaaaat ttaatgtatt tagggttagt   1380
   tttgttgttt tggttaaatg aatgaataaa gagtgattaa aatgtgttta tttttttgtt   1440
   attttatata tagtttgaga ttgattgtta atagtagatt gtaaattttt gtatttaata   1500
   gttattaatt ttttttatag tttgtatatt tataaattta attgggatat ttgtttgata   1560
   aatttagatt ttgtagagta gatttttaag gaaatttaat tttagagaat aaaatatttt   1620
   ttggaatttt taaggtattt agagataaaa tgttttttgt gggatttttt tttaagtttt   1680
   ataaaagtag gtttattagg attaaattat attaaagtta gtgtgaggta aggggggtgt   1740
   ggtttataga aattttggga gaaagtttag tatagttttt tttttttggg aatatatgtt 1800
   aggatggaaa aaaaaaaaag gaggggagtg ttataggatt aagatttatt ttggaaaata 1860
   agatttttag taaagatatt tttttttttt ttattttata ttagatatta gaagtattat 1920
   tttttgagtg tgttttttaa gttttttttt aattttaggt tttttttgtt ttttgggttt 1980
   ttgatttttt ggtagttggg tatttgtatt tgggtttttg ataggtttag ttgttggttt 2040
   agtttttttt gtatgaaggt gttgggatag tgggttttgt taaaaagttt ttttagttta 2100
   ttgagttgtt ttagggtgaa attggtttga ttttgttttt gtttgatttt ggtttggttt 2160
   ttgtttttta ttttttttgt attttttttg tgatttttta gttttgggga tattggaggg 2220
   ggtattttag tggggttata tgtgtattta tatgaatata tatatatatg tatatatata 2280
   tggatgaaaa tagtatagta aattttgtta ttagatttgt tgtaaaaaag aatagagaat 2340
   tttttttttt tgtggaattt tggtttggtt ttttaattta tttggaggaa gaatattttg 2400
   gagaagtgta aaggttaagt ttgagtggtt gtttggggta aggtgggtag ttatttatta 2460
   ttttttttat aaatgtattt tgatttattg ttttattatt tattttttag ttattggttt 2520
   tttttagtaa atattaaatg gtttttattt atttttgtat ttatttgttt tgtagtattt 2580
   atagaaatta aaggtgtagt gttaggtttt ttatagagta aagtttggat ataagggtgt 2640
   ggtaaggtta tttattggtt ttttatattg gttattttta gaaggttggt ttgtttttag 2700
   gttagttttt attattgttg gttttttgtt tattgtaaat ttgttggttt aatttaggaa 2760
   taattgggtt gaaaggtatt agtgagagta atagattttg gtgttgtgtt gtatagggag 2820
   ttgtatttgt agatgttttt tgttgttttt gggtttgggt taaattttgt ataaggtttt 2880
   ttggatagtt aggtaatttt tgttttgttt gtttgattgg ggttgtatga gtataggtgt 2940
   ttatgttatg ttggttgttt aaagggtttg ttgtttaagt tgggttagaa ggtaggaggt 3000
   ggaaaattag tttttggtgg tgggtgaaag taattgtttt ttttgttttt tttttgtttt 3060
   tttttgtgga aatgtagatt tgattttaaa tgtttaattt atagagatta ataggtttaa 3120
   gtggaatatt tgtgattttt ggtttttatt ggttgtttaa agttttttta tgtaattagt 3180
   agtttggatg tttaataaaa tatgaatttt tttgttttgg gtttgtatat atttgttgtt 3240
   atttttttgt tttttttttt tttttaaaaa taggagtttt tttagtgtga gtatatatag 3300
   ttattatata tatatttttt tggaattttt attttttgtg tttgaattag agattttggg 3360
   ttttaagaag ttaaagttga gtttagatag ttgaggttgt ttgtagtttt ttttagtgta 3420
   ggatttaggt tagtatgggt gttaaaaatt atttttggaa gagttggttt gtagagaaat 3480
   taatatttgt tttgagtttg gaaattaata attttaggaa ttgtgtttgt tggggtttta 3540
   gatttgaatt ttgttgttag gtgtgttggt gaatttgtag tttgggtttg tagttgtgga 3600
   tttatggttg ggaggttgta ttgggtattt tgatggtaat tttattattt tataaattta 3660
   ggaagatttg gtgttggttt ggaggaagaa gagttgttag attttggttt tgttgggtat 3720
   tttagttatg gtgattttaa atattgttgt attttttagt ttttagagtt agttttgtgg 3780
   atatttgttt tttttttagg aaggaatatt atagtaaata ttatgggtta atttaatagt 3840
   aaagtatttt tattgatatt gtattgaaat ttaaatatga aaatatattt tttaaatttt 3900
   gttaatgagg tgggaaggtt ttgtttatta tatgttattt atatagtgga tgttatttaa 3960
   agttttgggg ttttgaggag atgtttaaat tttaagttga tttatagatt taataaaatt 4020
   tttatttttg tttttaaatt tttaattatt gatttaaata ggaatagatt gtgttataaa 4080
   taatttaatt gtttgtttaa atatgtgatt ttatgtatgt tatatatttt tttaattgga 4140
   aaaatatggt agtatattag ttattgtagg tattaagtgt taaattaatt ttattgttga 4200
   atttttttga attggtatag aatgttttgt tattgagata aagatgtttt ttaatattga 4260
   gttagtatta aaggattttt gttttaagag aattgagtat tgggtgattg aaaatattaa 4320
   aggaattgtg taaatataga tttttatttt ttttgttgtt tttttttttt gtttttaata 4380
   tattaagaaa ttgaaatgtt ttgtttggga gaagtagtgt agtttggatt ttagtttttg 4440
   ttggtttttt tttattttga aagagaatgg gaagttttgg tttttaagat ttaaagattt 4500
   aggggttttg tggagtttga ggggttttgg tggttttaaa tatttaggtg attggagggt 4560
   taagtttttt atttttgttt tttttttttt ttggtaaatt ttgtgttaga ggttagtttt 4620
   agttttgttg tttggtttag tatttttttt tgtagtttgg ttttgtgaat ttttatgttt 4680
   gtttgtgggt tatttgggtt gtgggtttat ttttttgttg gtgaaaaatg tattttggtg 4740
   gaatgggaat atttgggaag ggtgtgtata aattttatat gttttttttt atgttatttt 4800
   tataatatat taggatttta tttatgattg agtatattat tggattttta tttttttatt 4860
   attattttag ttgataaagt tggggtttag ggtttttttt gtgttttttt tttttttttg 4920
   ttgggttttg gagttttttt ttgtttgaga ggagtttgga aggtgggaag gggaattgtt 4980
   gttgggggtt agttaggttg ttattttttg ttagttgtgg gttgtttggt tttttgtttt 5040
   ttttggtggt gtttatgttt agtttttgga tgggagagtg ttttttttta gtttttttgt 5100
   ttgttttttt tttttttata ttttttttgt tttttttgtt gttgtttttg ttttttttgt 5160
   tgttgttttt gttggttgtt tggattgttg ggttgttgtg gttgttgtgg tttgttttgg 5220
   tgtagttggt tggttttttg gttttgtgta gtggtttgtt ttttagtatt ttttggtatg 5280
   tgattgtttt tttttttttt tttatttttt ggttaaaaga tttggagatg aatgttgtaa 5340
   gtttttttat tgttgttgta tgttagtttg gtgtttaatt tttgttagta gagttttgtt 5400
   tttttttttt tttttttttt tttattgttt tagttttttt aataataata tattaatggg 5460
   ataggaggtg ggg 5473
<210> 46
   <211> 5473
   <212> DNA
   <213> Artificial Sequence
<220>
   <223> chemically treated genomic DNA (Homo sapiens)
<400> 46
   ttttattttt tgttttattg atgtgttatt attggggggg ttggagtagt aaaaaaagaa     60
   gaaggaaaaa aagagtgggg ttttgttggt agaggttgag tgttgggttg atgtgtggtg    120
   gtgatggaag aatttatggt gtttgttttt aagttttttg attagaaagt gaaggagaag    180
   aaggaggtga ttatgtattg ggaggtgttg gagagtgggt tgttgtgtgg ggttaaggag    240
   ttgattggtt gtattgaggt gggttgtgat gattgtagta gtttggtagt ttgggtggtt    300
   ggtggaggtg gtggtggagg aggtggaggt ggtggtggag gaggtggagg aggtgtagga    360
   ggaggaggag taggtggagg agttggagga gggtgttttt ttgtttggga gttggatatg    420
   ggtgttgttg agagaagtag ggagttgggt agtttgtgat tgatggaggg taatgatttg    480
   attgattttt ggtagtggtt tttttttttg ttttttggat tttttttggg tgggagagga    540
   ttttgaggtt tagtggggag gggagaggga tatggagagg gttttggatt ttagttttgt    600
   tagttagagt gatagtggaa gggtgggggt ttggtggtat gtttggttat gggtggggtt    660
   ttagtgtgtt gtgggggtgg tgtgggggga agtgtgtggg gtttgtgtgt gtttttttta    720
   gatgttttta ttttattgaa atgtattttt tattagtggg aggatgggtt tgtagtttgg    780
   atggtttgta ggtggatgtg gaagtttgtg gggttgggtt gtaggagggg gtgttgaatt    840
   agatggtggg attaaagtta gtttttagtg tagagtttgt tgggagggag aaggggtaga    900
   ggtggaaagt ttaatttttt ggttgtttag gtgtttgggg ttgttaagat tttttgaatt    960
   ttgtagagtt tttgaatttt tggattttag gggttaaaat tttttatttt tttttaaggt   1020
   agaaggaagt taatgaaagt tgaggtttag gttatgttgt tttttttggg tgaggtgttt   1080
   tggttttttg gtgtgttggg ggtggggggg gggggtggtg ggggaggtgg ggatttatat   1140
   ttatataatt tttttagtgt ttttaattat ttagtatttg gtttttttag agtaaaaatt   1200
   ttttggtgtt agtttagtgt taggaaatgt ttttatttta atggtagagt gttttatatt   1260
   aatttaaagg agtttaataa taaaattgat ttggtatttg gtgtttgtag taattggtgt   1320
   attgttatat ttttttaatt aagaaaatat gtggtatgtg tggaattata tatttaagta   1380
   agtaattaag ttgtttatga tatggtttgt ttttgtttag attagtaatt ggaaatttgg   1440
   ggataaaaat agagatttta ttaagtttat ggattaattt gaaatttgga tatttttttg   1500
   aagttttaaa attttgagtg gtatttattg tgtagatgat atgtaataga taaagttttt   1560
   ttattttatt ggtagaattt gaagggtgtg tttttatatt taaattttaa tataatatta   1620
   gtaaaaatat tttgttatta aattggttta taatgtttat tataatgttt ttttttaaag   1680
   aaaggataaa tgtttataaa attaattttg agaattagaa aatatggtaa tatttaagat   1740
   tattgtagtt agaatgtttg gtagggttga gatttagtga tttttttttt tttaaattag   1800
   tattaggttt ttttaaattt gtaaagtggt ggagttattg ttaaggtgtt tagtgtagtt   1860
   ttttggttat gagtttataa ttataggttt gaattatagg tttgttagtg tgtttgatgg   1920
   tgaggtttag gtttgaattt ttggtgggtg tagtttttgg gattgttgat ttttaagttt   1980
   agggtaagta ttaatttttt tgtagattgg tttttttgga aatgattttt aatatttatg   2040
   ttggtttgga ttttgtgttg ggaagaattg taaatagttt tgattattta aatttagttt   2100
   tgatttttta aaatttggag tttttgattt gggtgtagag gataaaggtt ttagggggat   2160
   atatatatgg tggttgtata tgtttatatt agaagagttt ttgtttttaa agggaggaga   2220
   aggataggaa agtggtaatg gatgtgtgta aatttagagt agaaaaattt atattttatt   2280
   aaatatttga gttgttggtt gtatgaaaag gttttgagta attaatagaa attgaggatt   2340
   gtgaatattt tgtttgaatt tgttgatttt tgtgggttaa gtgtttaggg ttgagtttgt   2400
   gtttttatga gggagggtga agagagaata gaaagagtgg ttgtttttgt ttgttattgg   2460
   aggttggttt tttgtttttt gttttttggt ttggtttggg tggtgagttt tttgggtagt   2520
   taatatggtg tgggtgtttg tgtttgtgtg attttggttg ggtaggtggg atggagatta   2580
   tttggttgtt taggggattt tatgtagggt ttggtttgag tttaggggta gtgaggggtg   2640
   tttgtggatg tggttttttg tgtggtataa tattggggtt tgttgttttt gttgatattt   2700
   tttggtttaa ttgtttttaa attagattag taagtttgtg gtaggtagag agttagtggt   2760
   ggtgagagtt ggtttgagaa tgagttagtt ttttgggaat ggttggtgtg agaggttagt   2820
   gggtggtttt gttgtgtttt tatgtttaga ttttgttttg tgagaggttt ggtgttgtgt   2880
   ttttgatttt tgtgagtgtt gtggagtaag tgagtgtgaa agtgagtgga aattatttgg   2940
   tgtttgttgg ggaaggttgg tgattggggg gtgaatagtg aggtggtgag ttgaggtatg   3000
   tttgtgaagg aggtggtgga tgattgttta ttttgtttta ggtggttgtt tagatttgat   3060
   ttttgtgttt ttttgggata tttttttttt gggtgggttg agaagttgga ttaggatttt   3120
   atgaggggga aggatttttt attttttttt gtgataaatt tggtaatagg atttgttgtg    3180
   ttgtttttgt ttgtgtgtgt gtgtgtgtgt gtgtgtgttt gtgtggatgt atgtgtggtt    3240
   ttgttggggt gtttttttta gtgtttttgg agttgaaaga ttgtaaagag gatgtgaaag    3300
   ggatggagga tgaaggttag attaaaatta agtagaggtg aagttggatt aattttattt    3360
   tggaataatt taatgagttg gagaggtttt ttgatgagat ttattatttt gatgttttta    3420
   tgtgagagga attgagttag tgattgggtt tgttggaggt ttgagtgtag gtatttagtt    3480
   gttaggaggt tgagggttta gggagtagag agagtttggg gttggaaaga ggtttggggg    3540
   atatatttgg gaggtggtgt ttttagtgtt tggtgtaaga tgagaaggaa agagatattt    3600
   ttattagagg ttttattttt taaagtagat tttagtttta tagtattttt tttttttttt    3660
   ttttttttat tttaatatat gtttttagaa gaagaaagtt gtgttgggtt tttttttaga    3720
   gtttttataa attatatttt ttttgttttg tattggtttt aatgtggttt aattttaatg    3780
   ggtttatttt tataaggttt aaaaaaaaat tttatagaaa atgttttatt tttggatgtt    3840
   ttaaaagttt taaagagtat tttgtttttt ggagttggat ttttttgaaa gtttgttttg    3900
   tagggtttag gtttgttaag tggatatttt aattaggttt atagatgtgt agattataaa    3960
   gaaaattagt aattgttaag tgtagaaatt tatagtttgt tgttagtggt tgattttaaa    4020
   ttgtgtgtga aatagtggag aaataaatgt attttggtta ttttttgttt atttatttaa    4080
   ttaaggtaat gaaattgatt ttaaatatat tagattttaa aataaattat atatattagt    4140
   atttatattt ttaaagtttg ttttttagta tgtgttatat ttaatgaagt taggattgaa    4200
   atagattttt tttattggtt gtggaggtaa atgtaatagt aaaaataggt tttgggggta    4260
   tttaaaggag taaattgtgt atatttatat atatatattt ttttagtttt agggggaata    4320
   aatttaaatg ttttagattg tatttttgtt tagataataa aatagaataa aatgatagtt    4380
   attattggtt tgaatatatg gagtagtttt aggtttgtat atttgtagtt gttataaagt    4440
   attgttgtaa tagatatttt tttggttttt gggtatagag attattattt gtagtgaatt    4500
   atgtataatt tttaaggggt ttttttatat tttgtaatag gattaggaaa aaaaaaaata    4560
   gagtagttat gttgaagagg aaaagtaata ataggttgtt ttggttatta tagtattagt    4620
   agtagagttt ttttttttaa gttttttttg ttttgttttg tgtattgaga tttttagagg    4680
   tttagtaatt ttttaaaaat atgatgataa ttattatagt tatgattatt agtattttaa    4740
   ttttaatttt tttaggggaa aaaaaggtaa gaagttttag gttatgtgaa ttatataaag    4800
   gatgttattt tggtataaaa tttatttttt gttgtattaa atagtagaga aatttttgaa    4860
   gatttatatt gattatgata tttgttttta aaaatatatt aatgaaagtt ttgaagtagt    4920
   atttttattt taatggggtt ttaaaaattt taatttgtga gttaattaat atgttttatt    4980
   attgaaagtg gaatataaaa gtaagtttta tttgaatttt aaaaaatttg ttatattgtt    5040
   tgtaaaagtt gttaagttta agtaggtaat atttaggaga ataaagtatt taagagttat    5100
   ttgttttata tatattttat tttttttttg ttgagtgttt tttttttttt tttttaagaa    5160
   gtgaatttta ttgttgtttt atatttttat ttatatttta gttatttttt agatgttttt    5220
   ttattatttt aattgtaaat agtggttttt tttaaagaag taaattagat tattatgata    5280
   atggtgtata attattttgt tattgtgttt tattttgttg tatagtagaa tagagaaatt    5340
   ttaaattttt ttgtagtgag gtttttatga tattaattat ttttttttaa tttttataat    5400
   tttttttttg taaaggagtt attatttgtt ttggtgtttg gttttttttt aagatgaagt    5460
   taattaatat tat                                                        5473
<210> 47
   <211> 5328
   <212> DNA
   <213> Artificial Sequence
<220>
   <223> chemically treated genomic DNA (Homo sapiens)
<400> 47
   ttgataagag aaaaggtgtt gagggtaggt tgtgttgttt tatttatagt ttattgtttg       60
   ttgtagagtt tttgttagga ttggaaagtt aatttagtgt tgattaaaag tattatagag      120
   ttaaaggatt ggtaatgatt gttaatttta ttttagtttt atgtattttt aattgttgta      180
   attaaagata tgttatttaa atataggtga agggtttttt ttttttaagt tgggttgggg      240
   gggggtgggt gggtggatta taattttatt tagatttttt attttatttg ggtgttatgt       300
   agttttaaat tagattttat tattattatt atttagagtt gttttttgtt atttatattt      360
   ggttttgtag aattggaagt gtgaaatgtg ttatttatat aaaagttttt gtttaaagaa      420
   agggtgggat gggggagtgt agaggagtaa ttatttaggg gtgatttaaa tatttaggtt      480
   tttttttttt tttttttaaa ttaatagaaa ggtttttgat atagttaaaa gtatttgaaa      540
   ttgggggtaa ggttaatagg ttaaagggat tagtgttagt tttttaagta tttttaaaaa      600
   gattatgtga atagatggaa aaatatattt ttaattaaat aatttttttt atattatagt    660
   tgtagtgttg gtttagaaat tgtgtaattt tttatttgat aggagttttt ttatgagaat      720
   attaaaggat aaaaagtttt gtatttttgt agttggttaa aattgataat tattttttta 780
   tgtgtgtgtg tgtatatata tatatatata tatatatata tatatattaa tatatatata 840
   tatatatata tatatatata tatatatata tatttttaaa aaagaattta gtttttaatt 900
   tttttttatt tatgaagatt aaagttttgt atttgatttt gtgagtggtt tttgtttttt 960
   ggggttggaa tttgatttag atgtgaagtg gtattgagtt gttgtgtagg tttgtgttta 1020
   gtgttttttg ttggatatag taggagttgt gtagtgttag ttttagaaat tgttggaatt 1080
   ttgttttggg gttagagaaa atttttttat tgttttttaa aaaagaaatg ggatgtgaaa 1140
   ataaattttt atatttttga aaaataatat aatattaatt taataaaaaa ttaaagaggt 1200
   gatattataa ttttaattta ataaaaaata aagattgttt tttttatatt ttagttattt 1260
   tttatttggt ttggttttgt aatttttttg aaaggttttg tgatggtaaa ataggtaaag 1320
   aaaagtgatg aaaaataaag tatttttttg tagttgtgga gaattaagta aagtatattt 1380
   tttttgtttt gtttatattt taaaaaatta aatggaagat gttgttgttt gttgttttga 1440
   tgttgttagt agtgttttga atgtttttag aggttttgta agtattgtaa ttgagttttt 1500
   gagtaaatga aataatttgt ttttatgtgt ttatttttag agtataatta aaagattagt 1560
   tttttttttg agtgtttttt attttttaga ttaattttgg ttaaagtttt ttttttttag 1620
   atattggtta agaaatatat attggttaag aaataggtgt ttggtatttt ttttttttta 1680
   ttttagttgt aggattttgg ggaaggtgtg ttaatggtag ggaaggtttt ttttgaaatt 1740
   tagtattttg ttgattagga atttaaatta ttttagggaa gggggagtat ttttatattt 1800
   ttaaaaaatt atttgtttat ataataaaga gttttgtttt ttttattatt attatttttt 1860
   tttttttttt ttggttagta agtgagggta aaagtagttt ttgaattatt ttagaaagag 1920
   aaattgtaaa aatggtatat tttttgaatt taaaataata gggaaatgtg ggtttgttta 1980
   attgtttaaa attaaataga aataattatt gtgttaaatg aataagatgt tttaagattt 2040
   atatttttag ggttatggtt attgatttta attttatttt taagaagttt agagattgtg 2100
   tttttaaagt ttgtttattg atggaaattg gtttaatttt ttataaaaga atggtttttt 2160
   aaaatattga atattatggt tatgtaaggt ttatgatatt tttatgttta ttttttttat 2220
   tttttaaaat tagtgaattt gagataatat tttaagggga attgttattt agtgtgatga 2280
   gagatgattt ttttatttgg ttttagaatt agattttggt ttaggttttt tagtaagtag 2340
   gtttagatgt aatggaagga gagaagaatt aagttttttg ataaaatttt tattttgaat 2400
   tttatggaaa agataggtaa gtgggatttt agttatagta tttttatttt ttgttttatt 2460
   aaaaggtttg tttttattat agaaataata tagttggtgt tagttttgtg aatttgtgtt 2520
   aattgataag gtaagaaatg gtgtgttgat agttgaggtt tgaaggttgt ttagtggttt 2580
   ttgtagtttg tggggtaggg aggttaaagt tagtaaaatg ttggaggtga tgttagaaag 2640
   agagtatatg tttgttattt ggaagtgatg ttgaatgtgt aatagtagag ttttggagat 2700
   ttttattttg ttttgtattt agggatttta gtttgtgggg tagtaggttg ttgggtgaag 2760
   gtaggaggga gaagttgtat ttggttgagg gtgggtgggt gtgttgggag tgagtgttta 2820
   attttttagg gtttatggag aaagtataat ttttagttga ggaaagagag ttaaataagt 2880
   gggtggttta ggtttgggaa tgttatttag tttttttgat gtagtgttat ttttgtggtt 2940
   ttagggggtt ttagtatttg tttgaatttt attttttaag gttgttagta ggaaggtagt 3000
   ttttttattt ttgagggttg aggagggggt agtgagtgat ttgatttttt agtagtaggg 3060
   aaaattgtag tggttggtaa tgggttgtgt ttttttgtgg tggtaaagtg ttttttaggg 3120
   gtggggtggg tatttttggt tttttgtttt agtgtttagg tttgagtttg tgtgggttgg 3180
   ttgggtgttt tgttttgtaa gtaattaatt ttgttggatt ttgaggatta tttaggtgga 3240
   tgggtttatt tagtttaggt ttattgagat tttgttaatt ttttagattt tttttttttt 3300
   tattttttat attttttatg atgaatttag gaaagaattt gaattttgaa aaatagtata 3360
   tgtggtttga agtttgatag ttatttgggt gtgagggata tgattttatt aataaagatt 3420
   aagatttaag gttgagtttt tatttattaa atttgttttt ttattttgtt tttaaatttt 3480
   ggagattttt tagttttgtt gttttaattt tgatatagat aaaggtgtta gggagaaagt 3540
   ttaggtgggt ttgaagtagt tgaggtatag attgatattt atttttttat tatttagatg 3600
   ggtagataag tattgttaga ttgggggttt taatttaggg tttagtggtt atgtgggttg 3660
   ttgtggggtg ttgaaattat aagtataatt tgatattatt gagtttatgg tgtttgttaa 3720
   taatttgtga attaggtgta gagattgtta ttgtagatta taatagtttt tgaaggattt 3780
   attatagttt attgtgttaa tgatttttaa attaaggagg ggaggtgggg tgagatgaaa 3840
   aaggagtggg gagagttagt aaaaagggaa gaagggtggg agggaagata aaagttagag 3900
   ttttagtttt gaaggagttt ttaaagatta ttaggttagt gatttgagaa ttttattggg 3960
   atgggtttgg aaatttaatt ttatttgagt taggattgga agaggggtgt tgggggtaag 4020
   tagaagtggg tttttgggaa ggttgatttt ttaggtattt tttttgaggt tggaatagtt 4080
   tttgggagag ggttttagat gttaatggga ggttttgagg atgttagttt gtttaggatg 4140
   ggtgtatatt ttgatttaga gtatttttta gggtaagttg tagttgtaga tttttgaaaa 4200
   aagtggtaat agttttgttt agggatgatg gtgattgtag gatgattttt tttttttaat 4260
   ggtttgtttt aatttttttt tatgttatag gtgaaagttt tttttttttt tttttgatag 4320
   tatttttgtt tgttttttat atgttatgta gatttaattt ttttttttaa tttgtttttt 4380
   tgggtgttaa atatattagt tttttgagat tttattattt ttgtggtttg tattgagttt 4440
   aattgtaatt ttggaaaggg aatttgtgga taatatagta gtagtatgtt ttataagatt 4500
   ttagaagaaa gaagattaaa ttttatttat gtggtgtggt aggggttagg ttaagggaaa    4560
   gaggtttttt ttgtttggta aattgttttt gtagtgaaag ggtagtttag tttttattgg    4620
   agtgttattt aatattggtt agagaaaggt taaggaaagt gtaggtttgt tttttttata    4680
   aaagttgtgg ttataattaa atgaagtaat tatagttgga gaatttaaaa taattgtttt    4740
   ttttttaaaa tgagggtaat aattttagtt attggaaaat tgggatttta aggaaaattt    4800
   ttttttttgg tataattaat tgtataagat agtgtggtta attagagata tatgtattta    4860
   gatttaagta atgatttata ggtgtaaaag gtttagtggt tttttgggtt atttggagtt    4920
   tttattgatt attttgtttt ttaaaaaatt tttttttaat tttaaaaata aaagttatag    4980
   atttaatgta attgaaaatt attgatggta gttttttagg tttttagttt atgtttttaa    5040
   agggaattta tatttaaaat aattaaatta tttaatggtt atattatatt tttttttgga    5100
   taatagttgt agtgtaggga tagaatttta atttatatta aaattgtttg ataatttaga    5160
   aatggaaatg aaattgtgaa ttaatttttt tagaaaatat ttgagttaga atataagaag    5220
   gatgtttata tataatattg taagtaatat gatgattatt atatgtagtt tgaaagattt    5280
   atataaatga ttatatatta tttttgttgt tataatttaa agtaaggg                 5328
<210> 48
   <211> 5328
   <212> DNA
   <213> Artificial Sequence
<220>
   <223> chemically treated genomic DNA (Homo sapiens)
<400> 48
   tttttatttt aaattatagt aatagaagta gtatataatt atttgtgtag gttttttaag      60
   ttgtatataa taattattat gttatttata gtattatata tgaatatttt ttttatattt     120
   tagtttaaat attttttgga gaaattaatt tataatttta tttttatttt tgaattatta     180
   agtaatttta atgtaagtta agattttatt tttgtattat agttgttgtt taggagaaaa     240
   tgtagtgtga ttattaaatg atttaattat tttaaatata aatttttttt aaaaatataa     300
   attgaaaatt tgaagagttg ttattagtgg tttttagttg tattaaattt atgattttta     360
   tttttaaaat tagaagaaaa ttttttaaaa ggtaaaatga ttagtaggaa ttttaggtag     420
   tttaaaaagt tattggattt tttatatttg taaattattg tttgagttta aatatatatg     480
   tttttgattg attatattat tttatgtaat taattatatt aaaggaaaag gtttttttta     540
   gaattttaat tttttagtgg ttagggttat tatttttatt ttagagaaaa aatagttgtt     600
   ttaaattttt taattgtgat tattttgttt agttgtgatt ataatttttg tgggaaaaat     660
   aaatttatat tttttttaat ttttttttag ttagtattaa atgatatttt agtgaaggtt     720
   gggttatttt tttattgtaa ggataattta ttaagtaggg aaaatttttt ttttttagtt     780
   tggtttttgt tatattgtat aaatggaatt tagttttttt ttttttggaa ttttatggaa     840
   tatattgtta ttgtgttatt tataagtttt ttttttagaa ttatagttgg atttaatata     900
   aattataaaa gtgataaaat tttagagggt tgatgtgttt ggtatttaaa aaaataagtt     960
   ggggaaagaa attagattta tgtaatatgt gagggataga taggaatatt attagaggaa    1020
   aaggaagaaa gtttttattt gtggtatgaa aagaagttaa agtaaattat tggaaaaaaa    1080
   aagttatttt ataattatta ttatttttaa atgaaattat tattattttt tttaaagatt    1140
   tgtagttgtg gtttgttttg gaaagtgttt taaattaaag tgtatattta ttttgggtgg    1200
   gttggtattt ttaaggtttt ttattgatat ttagaatttt tttttaaaag ttattttggt    1260
   tttaaaagaa atgtttgaga agttggtttt tttaggggtt tatttttgtt tgtttttagt    1320
   gttttttttt tagttttggt ttagatggag ttgggttttt aaatttattt taatggaatt    1380
   tttagattgt tggtttgata atttttaaag gtttttttgg agttggggtt ttgattttta    1440
   tttttttttt tatttttttt ttttttttgt tggttttttt tatttttttt ttgttttgtt    1500
   ttattttttt tttttaattt gaagattatt gatatgatag gttgtaatga gttttttaga    1560
   aattgttatg atttatagtg ataatttttg tatttaattt atagattatt gataagtatt    1620
   gtgggtttaa tggtgttaag ttgtatttgt ggttttaata ttttgtagta atttatgtag    1680
   ttgttgggtt ttggattagg atttttagtt tggtagtgtt tatttgtttg tttgagtgat    1740
   ggagagatga gtattagttt atattttaat tgttttaagt ttgtttgggt tttttttttg    1800
   gtgtttttgt ttgtgttagg gttggagtaa tgaaattgaa agatttttag agtttgaaaa    1860
   tagagtgaaa gagtaaattt aataaatgag agtttagttt tgaattttag tttttattaa    1920
   tagagttatg ttttttatat ttaaataatt attaaatttt aaattatatg tattgttttt    1980
   tagagtttgg gttttttttt gggtttatta tgaaaggtgt gagggatgaa ggggaaaagg    2040
   gtttgaggag ttggtagggt tttagtgggt ttaagttaag tgggtttatt tgtttggata    2100
   gtttttagaa tttagtaaga ttggttattt ataaagtagg gtgtttagtt aatttgtgtg    2160
   ggtttaggtt tggatgttgg gataggagat taaaaatgtt tgttttgttt ttaaagggtg    2220
   ttttgttgtt gtggagaaat gtggtttgtt gttagttgtt gtggtttttt ttgttattaa    2280
   ggaattaagt tatttgttgt ttttttttta gtttttaaag atggaggggt tgtttttttg 2340
   ttggtggttt tggggagtgg gatttgggta ggtgttgggg ttttttggga ttatagaaat 2400
   aatattgtgt tgggagagtt gggtggtatt tttaggtttg ggttatttat ttgtttagtt 2460
   tttttttttt aattaggggt tatgtttttt ttatgggttt tagaaagttg ggtatttgtt 2520
   tttggtatgt ttatttgttt ttagttaggt gtggtttttt ttttttgttt ttatttaata 2580
   atttgttatt ttataggttg ggatttttgg gtgtggaatg gggtggaaat ttttaggatt 2640
   ttgttattgt atgtttgata ttatttttga atagtaggta tatatttttt ttttggtgtt 2700
   gtttttaata ttttgttggt tttggttttt ttattttatg ggttgtggga attattgagt 2760
   agtttttgga ttttggttgt tagtgtattg ttttttattt tattggttgg tgtgggttta 2820
   taaaattagt attgattata ttatttttgt aatggggata agttttttgg tagagtaaaa 2880
   ggtgggggtg ttgtggttgg aattttgttt gtttgttttt tttatagaat ttggggtaga 2940
   gattttatta aaaggtttga tttttttttt tttttattgt atttgaattt atttattaaa 3000
   aaatttaagt tgagatttaa ttttggaatt agatggagaa gttatttttt attgtattaa 3060
   atagtaattt tttttgaaat gttattttga atttgttaat tttgggaggt gggaagaata 3120
   aatataaagg tattataaat tttgtatagt tatagtattt aatattttaa aaagttattt 3180
   ttttatgaaa aattaagttg atttttgtta ataagtaaat tttaaaaatg tgatttttaa 3240
   attttttaag ggtaaagtta aaattaataa ttataatttt gaagatgtaa attttaaaat 3300
   attttgttta tttagtatag taattatttt tatttgattt taaataatta agtaaattta 3360
   tattttttta ttgttttaaa tttagaggat gtgttatttt tatagttttt ttttttgaaa 3420
   taatttaaag gttatttttg tttttatttg ttgattaaaa agaagaagaa gaaataataa 3480
   taataaaaaa ggtaaagttt tttattatgt agataggtag ttttttggaa gtgtaaaaat 3540
   attttttttt ttttaggata atttggattt ttggttaatg aaatgttgaa ttttaaagaa 3600
   aatttttttt attattggta tatttttttt gaaattttat ggttaagatg aaagaaaaaa 3660
   gatattaggt atttgttttt tagttagtat gtgtttttta gttagtattt gggaaggaaa 3720
   aattttgatt aggattggtt taggggatgg gaaatattta aagaagagat tggtttttta 3780
   attatatttt ggaaataaat atataaaagt aagttatttt gtttatttga aggtttagtt 3840
   atgatatttg taggattttt gagggtattt aagatgttgt tgataatgtt aaaatagtaa 3900
   gtagtagtat tttttgttta attttttgaa atgtggataa aatggaggaa gtatattttg 3960
   tttgattttt tatggttata gaaaaatgtt ttgttttttg ttattttttt ttatttgttt 4020
   tattattgta gaatttttta aaagagttat aaaattaaat tgggtggggg atggttggga 4080
   tgtgaggagg atagttttta ttttttattg aattaaaatt gtagtattgt ttttttagtt 4140
   ttttattgag ttaatattat attatttttt aaggatataa aaatttgttt ttgtattttg 4200
   tttttttttt ggagggtaat aaaagagttt tttttaattt tagagtagga ttttagtgat 4260
   ttttgggatt ggtgttgtgt agtttttatt gtgtttaata gggggtgttg ggtatgaatt 4320
   tgtgtagtgg tttagtgttg ttttatattt gagttgggtt ttagttttgg aaggtgagag 4380
   ttgtttgtgg ggttgagtat agagttttag tttttgtgga taaaaagaga ttaaaggtta 4440
   aatttttttt tggggatatg tgtgtgtgtg tgtgtgtgtg tgtgtgtgtg tgtgtgtgtt 4500
   ggtgtgtgtg tgtgtatatg tgtgtatgtg tgtgtgtgtg tgtgtgtatg ggggggtggt 4560
   tattaatttt aattaattat gaaaatgtag agttttttat tttttgatgt ttttataaag 4620
   aaatttttat tggatgagaa attatatggt ttttgagtta atattgtagt tatagtgtgg 4680
   gggaaattat ttaattaaaa atatattttt ttatttgttt atatgatttt tttagaagta 4740
   tttaaaaagt tgatattgat ttttttggtt tgttaatttt gtttttagtt ttaaatattt 4800
   ttaattatat tgaaggtttt tttattaatt tgaaggagaa aaaaaggaga tttaaatatt 4860
   taaattattt ttaaatagtt atttttttgt atttttttgt tttatttttt ttttaaatag 4920
   gggtttttat gtgagtgata tattttatat ttttgatttt gtaaagttag gtataaatga 4980
   taaggagtgg ttttaaataa taataataat aaaatttaat ttgggattat ataatattta 5040
   agtagagtag aagatttaaa taaaattgta atttatttat ttattttttt ttaatttaat 5100
   ttaaaaaaaa aaaatttttt atttgtattt aaatagtgtg tttttaatta tagtagttgg 5160
   ggatgtatga ggttgaaatg aaattagtag ttattattag ttttttaatt ttgtggtgtt 5220
   tttgattagt attaaattag ttttttagtt ttaataaaga ttttatagta agtagtgggt 5280
   tgtaaataag atagtgtgat ttgtttttaa tatttttttt tttgttaa 5328
<210> 49
   <211> 4324
   <212> DNA
   <213> Artificial Sequence
<220>
   <223> chemically treated genomic DNA (Homo sapiens)
<400> 49
   gttttttagt attattattt taaaggtatt tttatttttt ttattattag ggaaggagtg 60
   tgtagttttg atttagtttt ttagtggagt aaagggaatt tagaggaagg gggatttttg 120
   tagaaattgt tagttatata agtaagtttt ttttggtttt ttgttttatt aaaagtgaaa 180
   aattgtttat aggagtgttt atgttttttg tttttttatg tattattaga gttaagaggg 240
   gattttgtta gtaaggtatt ttttgttttg ttttgtagat tatagttttt gatttttatt 300
   ttattgtttg tgtaatgtgt agaattggag tttaaagtag tgggtttagg attatttttt 360
   aggtttggtg ggtttataga tttttttttt ggttgggaag aggggttttt gtgaaagtag 420
   agtttgataa gttttagtat tgttatggtt aagattatta atattattat tgttgtgttt 480
   ataaatatga agattatggt agaggaggag ttgaaagttt gaggagtggt agaggaagtt 540
   gtagaattaa atttggaaat tatgtttttt gatggggtga tagtggtttt tgatttggtt 600
   tgaagggata tttgaagggt agatattgtg ttttgtgatt tttattgagg aattttagga 660
   atagatgtta ttgaattgtt ttgtttaggg ataagtggtg ttttttttag ttttttgttg 720
   attttgattg gttatgtttt ttgtggtatg gggttggttg tagtggttgg tgggtgtttg 780
   gtgggtattt tggttttttt aagggttggt tgtttttttt tattggttat ataagagtgg 840
   ttgttttttt ttagtttgaa gtttgtagta tatttgtagg taaagttttt taagttgttt 900
   aggtagttag ggagttttgt gtatttgtta gtatggaggt attttttggg gtagggatat 960
   aatatattgt ttgagagttt gttttagtga gtgttgattt tgtttgtgat gtaagtaatt 1020
   gagattggga gttgtttttg gtagagtgta tttattttgg ttttaggtgg attgaagttt 1080
   agagtggtgt tgtgtagttg gaagggtgtg tgatagttta agttagaggt ggttttgggg 1140
   tgtggtgtag gatataagat tttaaattgg tatttgtata ggtagttgtt ggtgtgtagg 1200
   tggtattgta ttttttttta gtttgtgggt ttgattttat tggtggtttg gagtattgtg 1260
   tatttttgtg tggtgtagga gtgttggggt ttttttattt attgtagtgt gttgtttttg 1320
   agattgttgg ggttggagga tagttaggag aaattttgta aaggtttgtt ttttagggtg 1380
   tagtgggaat gtttgtgttt tagtgtgatt tagaatagta ggtttttgga gttttttttg 1440
   ggttttgggt ttgtttgtag gagtgtgagt atagtgtgta gtttggtgtt tgtatgtatg 1500
   gtgttgagtg ttttattttg taggatgtag gtttttttgg ttgtttgttg ttttatggta 1560
   gtgtggtgta ggttgtagta ggtttttgag gttgagtagt tagtatggtt ggtagtgggg 1620
   tgtttgttgt tgtttggttt gggttagagt gtttgttaga ggaggtatag ggtgaatgtt 1680
   ggttttattt tttgaggttt tgtatgtaga gttgttttta atttggattt gttttgtttg 1740
   aggatgtaga gttgtgtttg gagtgtggtt attgtttttt atagttggtt tttttaattt 1800
   ggtttttttt tttttttttt ttgtttttgt ttttgatgga ttttttaatt gttaagaatt 1860
   tttttgtggg tttagtggtt aatagttttt tttaggttgt gataggaaat gtgtgtggag 1920
   tgttgttagg tttggtgtag ttttgagtta ttttggggtt aggaatatag ttggtgttgg 1980
   gtatagggga tgtagttgat tagaaggtta aatattttgt ttagagtaag aggagaaatt 2040
   tgttattggg atgaagtgtg tggttttttt ttgtttaggg agttttatta ggattgtaga 2100
   atttataata tgtaagtgaa tataggtagg aaggatttga gaaaggaatt ggtttatgat 2160
   tataagatta tgtttgttta ttgtgttttt gtttggagtt gtggagttag tgttagaata 2220
   atttgtagat ggttttagtg tttggggggt ttgtgtttgt gggggaaaaa tatgttgtga 2280
   aataatttag taattgtttt tgttattaga ggtagtatta tttaattgta tattttttta 2340
   tgggtttttg tttttttatt atgttaagag attttaggag ttttaattat taaagaattt 2400
   ggagttgttt gggtatttta aatgggaaat tttgtttttt ttattagtat ttgttttaga 2460
   aatttatttt ttatttttat gtgttttttt ttgagttaga ttatattgat ttattataga 2520
   aaggaaatgt atttttagga ttaaatgaaa aataatgatt atagatttta atagttaggt 2580
   tgttagtttt ttttgttatt gtattggttt aaaaattttt taagtttata ggttatatat 2640
   aaatgagttt gtggttttat aaatatttga gaataattgt tttatgtgtg attttagttt 2700
   tgaattattt tagtattatt tttttttatg attgagtggt agtatttata atagtaagtt 2760
   gtaaatattt tttttaagga aaattagagt tttttaagaa attaaaatgt ttattttatt 2820
   ataatgaata ttgtatattg ttaaaaaata ttttgtaatt tgaaagatgg gtgagttaat 2880
   ttttttagaa ttttagtggg tgttttgttt taatataaat attttaattt agtgggaagt 2940
   ggtaaggaat taaagatgaa taaagaagta aaggttaaaa attgtattgt tatgaaagga 3000
   ttttttgtta gtaatttaga taatttagta ttagtttaga aaatgtatat tttattttgg 3060
   tttataatgg ttaaatatgt tgttattaga aatatataga gagttttagt taagtttttt 3120
   tagagatgtt tgatgttttt aaatattttt aattagaatg aaagttgtta ttttatggat 3180
   aatgtatata agtttatttt gttttattgt atttaggttg atgaggggtg ggagggaagg 3240
   ttttaggatt atatttgtat gtttttattt tatttttatt ataattttta tttttttttt 3300
   tttttggaaa ttttatgtat tttaataaat aatagtaagt ttatgagtta ttattatatt 3360
   tattttgttt ttgttgagta tggagttttg tattaggatt ttttttttgt tttatttttt 3420
   aatttaaaga atatttttta gttttttttt tagatgtttg agaattattt taaatgtggt 3480
   tttgtgttta aattaggtaa tttagtgttt tataagaaag aaatatgaaa atttagaaat 3540
   taaattgaat ggtttttaaa aaatatttag ttatttgggt tagtttttta tagattattg 3600
   taattttatg attgtgtttg taaatgtgtt tatgtagtgt aatattttta ataaaatttt 3660
   ttttaaattt aagtagttaa tggaaatttt ttagatagtt ttaaaaattg tttgggatga 3720
   gatgaggtgt ttatagtaag aaagaatgaa tagttggaaa gtttaagtaa atatatgatt 3780
   ttattttttg atattttgtt taggggagtg ggattatagt agagaatata ttggttataa 3840
   tgattatatg agagtattga ttatgattat gattatatta tggaatgata taagagttag     3900
   aaaaaattag tttaaagtag gatgtaaata gttttgtaat tagtttgaaa tagatagttt     3960
   atttgattaa attagaaatg gaggaaagtt tttatttttt aataatattt attaagataa     4020
   taatatagaa ttattttatt tttattggat tttaaatgtt taaaaatttt ataatattaa     4080
   atgtgaattt attggttttt ttatatgtta tagattagag taattatttt ggaaaaattt     4140
   gatatttttt tttattaaaa ttgaatattt gtaaatttta gtatttttat gataaggtat     4200
   aaaattaaga gaaatttttg tatatgtata atagaagata tgtaaaaaat gtttatagtg     4260
   atattattta tattaataaa atgttgaatt aattaaaata tttaaaaata gtataaaaat     4320
   aaat                                                                  4324
<210> 50
   <211> 4324
   <212> DNA
   <213> Artificial Sequence
<220>
   <223> chemically treated genomic DNA (Homo sapiens)
<400> 50
   gtttattttt atattgtttt tggatatttt gattgattta atgttttgtt aatatgaata     60
   gtgttattat aaatattttt tatatatttt ttgttgtata tgtgtaaaaa ttttttttag    120
   ttttatattt tattatggaa gtgttagagt ttgtaaatat ttaattttag tgaaaaaaag    180
   tgttaaattt ttttaaagtg gttgttttaa tttgtaatat gtaaaagagt taatagattt    240
   atatttggta ttataagatt tttaggtatt tggaatttag tgggggtaaa atggttttat    300
   attattattt taataaatat tattaaaaaa tagaaatttt tttttatttt taatttggtt    360
   agataaattg tttattttaa attaattata aaattattta tgttttattt tgagttaatt    420
   ttttttaatt tttgtgttat tttatgatat ggttatggtt atggttagta tttttatata    480
   gttgttatgg ttaatgtgtt ttttgttata attttatttt tttagataga atgttaggga    540
   gtgagattat gtatttattt aagtttttta gttatttatt tttttttatt gtagatattt    600
   tattttattt taaatgattt ttaaagttat ttaagaaatt tttattgatt atttaagttt    660
   gaaaaagatt ttattaaaaa tattgtatta tatggatata tttataaata tagttataag    720
   attgtaatgg tttatagaga gttggtttaa gtgattaagt attttttaaa aattatttaa    780
   tttgattttt ggatttttat gttttttttt tataagatat taagttattt aatttaaatg    840
   taaagttatg tttaaggtga tttttagata tttgaaggaa ggattaagga atgtttttta    900
   aattgggaaa tagggtagga gagaaatttt agtgtaaagt tttatattta gtaaaagtaa    960
   ggtaagtatg atgatgattt ataggtttat tgttgtttgt taagatatat ggaattttta   1020
   ggaggaaaga agaatgagag ttatggtaaa aataagatgg aagtatatag atgtgatttt   1080
   gaaatttttt tttttatttt ttattaattt gaatgtagta aaataaagtg agtttatatg   1140
   tattatttat ggaatggtaa tttttatttt gattaaagat atttaaaagt attagatatt   1200
   tttaagaggg tttagttgaa attttttatg tatttttgat ggtaatgtat ttggttatta   1260
   tgaattaaga tagaatgtat gttttttaaa ttgatattga attgtttaag ttgttaataa   1320
   gaaatttttt tataatagta tagtttttaa tttttatttt tttatttatt tttaattttt   1380
   tgttattttt tattgagttg aaatgtttat attaaaataa aatatttatt ggaattttaa   1440
   agaaattggt ttatttattt tttaaattat aagatatttt ttgatagtat ataatattta   1500
   ttataatgga ataaatattt taatttttta aggaatttta atttttttta aaggagatgt   1560
   ttataattta ttattataaa tgttgttatt taattatagg agaaaatgat attaaaatag   1620
   tttaaggtta aaattatata tagggtagtt gtttttgaat atttataagg ttatgaattt   1680
   atttatgtat aatttataaa tttgaagaat ttttgaatta atatagtggt aaggaaagtt   1740
   aataatttaa ttgttaagat ttatggttat tattttttat ttagttttag aaatatattt   1800
   tttttttgta atgaattaat atggtttaat ttaaagagaa atatgtggaa gtgaaaaata   1860
   aatttttaaa gtaagtgttg atggaagaag tagaattttt tatttaaaat atttaaataa   1920
   ttttaaattt tttagtagtt aggatttttg aaattttttg atataataga gaagtaaaga   1980
   tttatagaga gatatgtggt taaatagtgt tgtttttggt ggtaaaagta gttgttaagt   2040
   tgttttataa tatatttttt ttttataggt ataggttttt taaatattgg agttgtttat   2100
   gaattatttt gatgttaatt ttatagtttt ggatagaaat ataatagatg ggtgtggttt   2160
   tataattata agttagtttt ttttttaagt tttttttgtt tatgtttgtt tatgtgttat   2220
   gggttttgtg gttttgatga aattttttga atggagaaaa attatatatt ttattttagt   2280
   ggtgaatttt tttttttgtt ttaagtaggg tgtttgattt tttagttgat tgtgtttttt   2340
   gtatttggtg ttagttgtgt ttttgatttt agaataattt agggttgtat tgggtttggt   2400
   agtgttttgt atatattttt tgttgtggtt taagggaaat tgttggttgt tgggtttgtg   2460
   gggggatttt tggtagttgg ggggtttgtt gggagtgagg gtggagggga agggaggggg   2520
   aattgggttg gggaagttag ttgtagaggg tggtgattgt gttttagata tagttttgtg   2580
   tttttgagtg ggatagattt aagttgggag tagttttgtg tgtggggttt tagagaatga 2640
   ggttggtgtt tgttttgtgt tttttttggt aggtgttttg gtttgggttg ggtggtggtg 2700
   aatattttat tgttgattgt gttggttgtt tggttttggg ggtttgttat agtttgtatt 2760
   atgttattat gaagtggtag gtggttgagg aggtttgtat tttgtgaggt ggggtgttta 2820
   gtattgtgtg tgtgggtgtt gagttgtgtg ttgtgtttgt gtttttgtgg gtaggtttag 2880
   ggtttggagg gggttttaaa gatttgttgt tttgggttgt attggagtgt aggtgttttt 2940
   attgtatttt ggagaatgag tttttgtggg gttttttttg gttgtttttt gattttggtg 3000
   gttttgaaag tgatatgttg tagtgggtgg aggagtttta atgtttttgt attgtgtgga 3060
   gatgtgtggt attttaggtt attggtgggg ttgagtttgt aggttggaag gagatgtgat 3120
   gttatttgtg tgttaatggt tatttgtgta agtattagtt tgaggttttg tgttttgtgt 3180
   tgtgttttgg ggttgttttt aatttgagtt attgtgtgtt tttttagttg tatagtgttg 3240
   ttttggattt tagtttattt gggattgagg tgagtgtgtt ttgttgggga tagtttttga 3300
   ttttagttat ttgtattgtg gatgaaattg gtgtttgttg ggataaattt ttgggtgatg 3360
   tgttgtgttt ttgttttggg aggtattttt gtgttggtaa atgtgtagag ttttttaatt 3420
   gtttagatga tttgggaggt tttgtttgtg aatgtgttat gggttttgag ttggggaagg 3480
   atggttgttt ttgtgtgatt agtggggaag gatagttgat ttttgggggg attggggtgt 3540
   ttattaggtg tttgttggtt attgtaatta gttttgtgtt gtagagaata tggttaatta 3600
   gggttgatga gaagttggga gagatattat ttgtttttga ataagataat ttagtaatat 3660
   ttatttttga gattttttga tggggattat agagtatgat gtttattttt taaatgtttt 3720
   tttaagttga gttaaaggtt attattattt tattagggag tgtgattttt aagtttaatt 3780
   ttatgatttt ttttgttatt ttttaggttt ttgatttttt ttttgttgtg gtttttatat 3840
   ttgtgagtat agtagtagta gtgttggtga ttttgattat gatagtattg ggtttgttta 3900
   agttttgttt ttatgaaagt tttttttttt agttaaggaa ggagtttatg ggtttgttgg 3960
   gtttggagag tgattttgag tttgttgttt tgggttttag ttttgtatat tgtataaata 4020
   atggggtgaa agttggggat tgtgatttgt gggatagagt agagggtgtt ttgttggtgg 4080
   agtttttttt tggttttagt gatgtatagg gaaatagggg atatgggtat ttttgtgaat 4140
   agttttttat ttttgatgaa atggggaatt aagaggaatt tatttgtgta attgataatt 4200
   tttgtagaaa tttttttttt tttaaatttt ttttatttta ttgaggagtt aaattagaat 4260
   tgtatatttt ttttttgatg atagaggaag tggaagtgtt tttaggatgg tgatattggg 4320
   ggat 4324
<210> 51
   <211> 5683
   <212> DNA
   <213> Artificial Sequence
<220>
   <223> chemically treated genomic DNA (Homo sapiens)
<400> 51
   ataatgttaa atgtaaattt tttgttttgt ttaatgtttt tattttatgg ttggtaaata      60
   aaaataataa aatagtattg ataataataa tagaaataat taattgaagt tgtagttttt     120
   aattttttta aagataatag gttaaagtga ggtttttata ataagatgtt agttattagg     180
   aaggaagtta gggaaaattt tttttagggg ggtaattgat ttttattggt ttaatattgt     240
   atgtagtatt gagttaggag gttttatgga gaagtgtttg tttttttttt tttgttgtta     300
   gaataaagtt tataaataat tttgtttttt ttatattttt ggagttagtt agttagggtt     360
   tttatttatg tttaggggat tagatttgga tatttgtaaa agaattgaat atagatggat     420
   agttaagaaa aaaataaatg ttaagattat tattatttat agtttaatga tttagtaatt     480
   taaaaaaatt taagaataat aaaataattt ttaagaagga taagaatttt aaaatatgta     540
   gtttagaaaa ttaaattttt taggtttaga gtttaagtaa aagaaaattt gagataatga     600
   agtttggaat attgggtttg tattttgttt ttgattttgt gtttttgggt aagatattga     660
   atttttttat tttagattta ttttatattt taaggagggt gtaggatttt gtagtttttt     720
   tagtgatggt atattattaa ttagaatttt aattagtttt atttttatta ttttagtaaa     780
   atgatgttaa aaattagatt ttgtgatatg agagaagagg ggaaattaat tatagattat     840
   aaagaaattg tgggatttaa gtttagggtg aggtgtttta ttgaattttt agggttggtt     900
   atagtatttg ttatatagta ggtatttaat aaatgatgtg taatgggtgg ttagattgat     960
   ggaaaggtag gtttttttag tgatggtaaa ggatttgggg taaaagagat ttgtggttga    1020
   gttttgattt ttatatatta gtatattgat tagatgaagt tataagtttt aatttttttt    1080
   tgtgtaaaat ggagataata ttttataggg ttatgaggat taaataagaa aatgtttagt    1140
   ttagtgttta atatatagga agtatattgt aaatattagt tattattatt attaatattg    1200
   tgattatatt ttttatttaa agattttttt gaggagtagg gggtatttag agataaaaag    1260
   tttgtaggtg ggaaatttgt tagtttttta gggtgttagg tttttgtgga attttgtatt    1320
   ttttttttgt tttagttttt aagaatagaa attttttagg gatttttggt gaggtagttg 1380
   tgggaagatg aggtgtagaa gtaagttggg atttgtgagt tttaattttg gttttttttg 1440
   tgttgagatt taagtggatt ttgtttggtt tgtatgtgtt attgggggaa atggatgtgg 1500
   gtttgagtgt ggtaggtgtg agggtgttgt tttggggttg attattttgt ggggatattg 1560
   tagttgttat tttttttttt gtaggtgggt aggggaagtg gtggttaaag tgggagttga 1620
   ttgtttagta tagtttgttt gagtgttgat taggaaagtt taggtttttt ttaaattttg 1680
   ttgttagatt tggtgatgta tttgtatgta tttaaggtgt ttgtatgtag aatgttatta 1740
   tagaatgtag ttattttttt taatggtttg ggaaattaga ggttttttta gttatttagg 1800
   gtagaggaat tttttttatt tttatgtgat attttttgat ttagaagaat taatgttaga 1860
   ttttttttgg gtttttttat ttatagttat agtttttttt ttgtttagtt aaaattgagg 1920
   taggtaggaa aatattattg ggggtataag tttattagtg tgtaaatgta tttttatgaa 1980
   gtgtgttttt agggagttat taaaaattga ttttttaatt atagaaatag atgagatttt 2040
   gagaatattg agaagttgtt ttttgtaaag taaatttgta atggtttttg atgaaggggg 2100
   gttgggggtg gggagaagtt tagttgagag aggagtttat tttatgttat atttttgtag 2160
   ttgaaaagtt gtttaattat tgttaattgt tttttgtata agagttttgg gaagatttta 2220
   gaaataaggt aaatgaagat tttttattgt tttttttggg ttgttgtttt tggaagttga 2280
   agttttagta tgtagagtag taggagaggg ttatttttag gtaattttat tgagtaaaat 2340
   aaattattta atggtataat gttttggttt aaaaaattgg aatttattag aggtaaaaat 2400
   attttttaag aaattatgga tattttgtgt tttatttatt tttatggtag tagagtattt 2460
   gtattttgag ttatttatat agatttatgt tttgtattgt ttttattttt tttttttttg 2520
   aagtaaagtt tttttttaag aaggtggtta gaaagtggat tttattgggg gaaaaagaaa 2580
   aatgaaatgt aaattagttt ggtattgttt gtgtttttta aaatgtggtg ggataaggtt 2640
   attgagttta tagttaattt ttttatgtat ataaaatgta tatatatgta tatattttta 2700
   tatatataaa agaatttata tatatgtata tagttatgtg gagttttgaa gtaatttttt 2760
   atgtttttgt tttttttaag ttttttaggt ggaggtagtt ataagtatta taagttgttt 2820
   tagtgattat tagggatgga aattgttaat tattatgttt ttttttattt ttaggggttt 2880
   tttggtttgt gatataagag gttttggtat tggtgttttt ttagaattgg tttagaggag 2940
   ttagtttaga gtgtgaggtt gggtttgtat tgaatgtata tattgaggtt tattagattt 3000
   ttttgatttt agtgaagggt gttgattgtt gtgttgttag aggttagtaa gttttttgtg 3060
   tgtagttgat gagttttagt aatttgttat ttgggtgttt ttttttaaat tttgggagta 3120
   aattgggaaa ataaaaaaat ttttatgttt attggttttt tttttttttt taattttttt 3180
   ttttgatttg tttgtgggag tttttttttt tttgttggga ttataatgtg atgtgtaatt 3240
   gtttgtgaat gaataaaagt tattggtaag tagggagatg gggatagatt gttgatggta 3300
   gattgagggt ggtagtaaag gtttggtttt taaggataat ggggagttgt ttttttttat 3360
   gtttggtttt tatggttttt tttgtttttt aggtaaaatg aatgtttttt tatttattat 3420
   ttgtagagta tttttaggtg tgtgtagaat ttgttgatga aatttattag tgttgattgg 3480
   gtagttttgt ggagttattt gaggtttttt attgtggttt ttgtttgtag aatttaagta 3540
   tttatataat gtattagtat ggaatttagt atttggtggg gatattgtgt gttaagtttg 3600
   gtgtggttaa tgttttagtg gtttttttat ttggtagttt tttaggatta tttttgagga 3660
   ggtattggag ttgggttgta ggtgtatggg taaagaattt agtattttat ttaagtattt 3720
   tgtttttttt ggttgttttt gggaggttat gtttaaaaat ataaaaataa aaataaaaat 3780
   aaaaataaag ggaggtggat aaagttttgg tgggtgaatt gaagttgggt ttatgtgatt 3840
   ttgaagttgg agaaataaat ttaatatgaa ttttgttttt ttggtgggtg ttgggatttt 3900
   gttgtttttt atgttaattg ttggaagttt tgtatttaat ggttataggt gtttaggagt 3960
   gtagagaggt tttgggttta aattattggt gtgtagggat tggatttgtg ggtagtgatt 4020
   tttttaattt tttttttttt gttttatata tatatttttg tgttggttga aagtatggag 4080
   gatttagggt atttgaaaaa agaggggttg ggtgtggtgg tttatgtttg taattttagt 4140
   ggtttgggag gtttagaagg gtggattatt tgaggttagg agtttgagat tagtttggtt 4200
   aatattagtt tggttaatat ggtgagattt tgtgtttatt aaaaatataa aaattagtta 4260
   ggtgtggtgt ttgtaatatt agttatttgg gaggttgagg tatgagaatt gtttgaattt 4320
   gggaggtgga ggttgtagtg agtttagatt gtgttattgt ttttttgttt tgggtgatag 4380
   agtaaggtat tgttttaaaa taaaataaaa taaaatgaaa gatttggtta ggaaagaatt 4440
   tgtaggtatt tgaggtgttt gtattttgta aagtaaatgt aattttttta ttaagttgaa 4500
   gttttttata tttatttttt tagaggaagg tggtttaatt tagaaatttt ttttaagagg 4560
   attttttatt gaagattatt gtgaagtgtt aggaatttgt tttagttttg ataggtgtag 4620
   gattttttgt gttgttatat tttgggattt tattttttta aataggttat ttaaaagtta 4680
   gtagtgtaat tgggattttg tggtgataaa gaattattgt gtagaatttt ggagttggga 4740
   gttggtttgt ttttttttta aagaaattgg gattttgtgt tttttttgtt gttagtgtag 4800
   tgtgttagtg gtgtttaatt agtgagtttt ttgggtttgt gatggttttt ggttttgttt 4860
   ttttgatggt tataaaaagt tgtagtgaag tttgtatttt tgagtattgt gtgtggtttt 4920
   gtttttggta tggtttttag gtgtgttttt tttttggttg tttgtagtat tggttttatt 4980
   gtttttgttg ttagttattg gttttttatt tgttttattt tttattgtgg tttggttgag 5040
   ttggttgtta ggttttgttg tttttttttg tttttttttt tttttagtgg ttgtattttg 5100
   tgttgtattt tatttggttt gtaggtgagt agtagtgtag tatttttgtt tggtgagttt     5160
   aatttgttta gtttggtttt tgttggagtg gtattggtat ttttttaaga tgtttttttt     5220
   tttgtaggat gaagaatttt atgttggagg tggtgttttt attattggag aagttgtttt     5280
   ttatttttaa ttttatgttt tttagtttgg gtgttgtggg tgaagataaa gggaggaata     5340
   gtttttatga aattagtttt ttttattgag gtgatgtgtt ggaggtgttt tggatttatt     5400
   tgatttatta tggtatttat ttaggagata attgtgttgt ttatatgatg tttgatattt     5460
   tgttggtttt gatagatgat atggggtgta tgtagaaggt ggtttttaat aagtgtttta     5520
   ttttgggtgt tattgttaaa gtggttagta tttgtgtgga tatagtggag gattttgttt     5580
   atggagttaa tattttggtt aattatttgg atgagttttt ttagaaaaag gtattgttta     5640
   atgaggaggt ggtgtggagg gttgaaaagt tgttgggttt tat                       5683
<210> 52
   <211> 5683
   <212> DNA
   <213> Artificial Sequence
<220>
   <223> chemically treated genomic DNA (Homo sapiens)
<400> 52
   gtaaagttta gtagtttttt agttttttgt gttatttttt tgttgagtag tgtttttttt      60
   tggagggatt tgtttaggtg attgattagg atgttagttt tgtaggtgaa gttttttatt     120
   gtgtttatgt ggatgttggt tattttgata ataatgttta ggatgagatg tttgttggag     180
   attatttttt gtgtgtgttt tatgttgttt gttagggtta ataggatgtt gggtattatg     240
   tgggtaatat ggttgttttt taggtagatg ttatagtggg ttaggtgggt ttggggtatt     300
   tttagtatgt tgttttggtg gaaagagttg gttttataaa aattgttttt ttttttgttt     360
   ttgtttgtgg tgtttgaatt aaagagtgtg aagttggaga tgaggagtag tttttttagt     420
   agtaaagata ttatttttag tatggggttt tttattttgt agggaagagg gtgttttgga     480
   gaggtgttgg tgttattttg gtaggggttg ggttgggtaa gttaagtttg ttgggtaggg     540
   gtgttgtgtt gttgtttatt tgtaggttag gtgaggtatg gtgtaaagta tggttgttga     600
   ggagagagga aggtaaagga ggatagtgag gtttggtgat tggtttagtt aagttataat     660
   ggggaatgag atggataagg aattggtggt tgatggtggg gatagtgaga ttagtgttat     720
   aagtagttgg agaaggagtg tatttggggg ttgtgttggg ggtagggttg tgtgtggtgt     780
   ttggaggtgt aggttttgtt gtgatttttt atggttgttg agggggtgga gttgaaggtt     840
   gttatagatt tggaaagttt attgattggg tgttgttggt gtgttgtgtt agtggtgggg     900
   aggatgtggg attttggttt ttttgggagg ggggtgggtt gatttttagt tttagggttt     960
   tgtatagtga ttttttattg ttgtgggatt ttggttgtat tattggtttt taagtggttt    1020
   atttagggag gtagagtttt aaggtgtggt ggtatgaaag gttttgtatt tgttgggatt    1080
   ggggtgagtt tttggtattt tgtggtagtt tttggtggaa agtttttttg ggagagattt    1140
   ttgagttgga ttattttttt ttaaaaggat agatatgagg gattttggtt taataaagag    1200
   attgtattta ttttgtaaag tgtgagtgtt ttgaatgttt gtagattttt ttttgattga    1260
   attttttgtt ttgttttgtt ttgttttgag atagtgtttt gttttattat ttagagtagg    1320
   agggtggtgg tgtgatttgg gtttattgta atttttgttt tttaggttta agtgattttt    1380
   gtgttttagt tttttaagta gttgatatta taggtattat gtttggttaa tttttgtatt    1440
   tttagtagat atggggtttt attatgttgg ttaggttggt gttggttagg ttggttttga    1500
   atttttgatt ttaagtgatt tgtttttttg gattttttaa attgttggga ttataggtgt    1560
   gagttattgt gtttagtttt ttttttttta aatgttttga attttttatg tttttagttg    1620
   gtgtgagggt gtgtgtgtag ggtggggggg ggggggttgg gggggttgtt atttgtgagt    1680
   ttagtttttg tgtgttggtg atttgaattt gaggtttttt tgtgttttta ggtatttgtg    1740
   gttgttgagt atgaagtttt taatggttgg tatgaaaaat ggtggggttt taatgtttgt    1800
   taggaaagta agatttatgt taagtttatt tttttggttt tagggttata tggatttagt    1860
   tttagtttat ttattgaaat tttgtttgtt tttttttatt tttattttta tttttatttt    1920
   tatgttttta agtataattt tttggagatg gttaaggaag gtgaagtatt tggatgagat    1980
   gttaagtttt ttgtttgtgt gtttgtagtt tgattttaat gtttttttga gggtggtttt    2040
   agggagttgt tgggtgaggg agttgttgaa gtgttggttg tgttaggttt ggtatgtgat    2100
   gtttttattg ggtgttgagt tttgtgttaa tgtattatgt aaatgtttaa attttgtaga    2160
   taaaggttat aatggagagt tttgggtggt tttataaagt tgtttagttg gtgttaatag    2220
   gttttattag tagattttat gtatgtttga gggtattttg tggatgatgg atgaaggaat    2280
   atttatttta tttggaagat gaagggggtt atagagatta ggtgtgaggg aaaatggttt    2340
   tttattattt ttggaggttg ggtttttgtt gttattttta atttgttgtt agtgatttgt    2400
   ttttgttttt ttgtttgttg gtgatttttg tttatttata aatgattgtg tattatatta    2460
   tagttttagt aaagaggaga aaatttttat aaatgagttg aaagaggaag ttggagaagg    2520
   ggagagagtt agtggatgtg gagatttttt tattttttta gtttattttt aaaatttaaa    2580
   gaaaagtgtt taggtggtga gttgttgaaa tttattagtt gtgtataggg agtttgttag    2640
   tttttagtag tatagtagtt agtatttttt gttaaaattg ggggagtttg atagattttg    2700
   gtgtgtatgt ttaatgtaga tttgatttta tattttgaat tggttttttt gggttagttt    2760
   tgggaaagtg ttaatattga agttttttgt gttatgggtt aaagggtttt tggagatgaa    2820
   aggaaatgtg ataattaatg gtttttgttt ttggtggtta ttaaggtggt ttataatgtt    2880
   tatgattgtt tttatttggg gaatttgagg gagataaaag tatggagaat tgttttaggg    2940
   ttttatatag ttatatatat atatatgaat ttttttatgt atataaaaat atatatatat    3000
   gtatatattt tatatatatg aaagaattag ttatagattt aatagttttg ttttattgtg    3060
   ttttggaaga tgtaagtagt gttaagttga tttgtgtttt attttttttt tttttttggt    3120
   ggggtttatt ttttggttgt ttttttggga aagggtttta ttttaaaaag aaaggaggtg    3180
   agagtgatat gaggtatgaa tttgtatagg tggtttaaga tgtagatatt ttgttgttat    3240
   ggaaatgaat agggtgtgga gtgtttatag ttttttgaag gatatttttg tttttgataa    3300
   attttaattt tttaagttag aatgttatgt tattaagtga tttattttgt ttagtggaat    3360
   tgtttgaaag taattttttt ttgttgtttt gtgtgttagg attttggttt ttggaagtga    3420
   tagtttgaag gaggtagtga aaagttttta tttgttttgt ttttgaagtt tttttagaat    3480
   ttttatgtgg gaagtggtta gtgatgatta ggtagttttt taattgtaaa aatatagtgt    3540
   ggaatgagtt ttttttttgg ttggattttt ttttgttttt gatttttttt tgttaaggat    3600
   tattgtaaat ttattttgta aaaggtagtt ttttaatgtt tttaaaattt tatttatttt    3660
   tgtggttgag aggttagttt ttaatggttt tttggaggta tattttataa aaatatgttt    3720
   atatattaat aggtttatgt ttttaataat atttttttat ttgttttaat tttagttaaa    3780
   taaagggaag attatagttg taggtggaaa ggtttaagag aaatttaata ttagtttttt    3840
   taaattagaa gatgttatat ggagatagga gaaatttttt tattttgagt agttggagag    3900
   atttttggtt ttttggattg ttaagaaagg tggttatatt ttgtgataat gttttgtgtg    3960
   taaatgtttt aaatatatgt gaatgtgtta ttaggtttgg tggtgagatt tagaaagagt    4020
   ttggattttt ttggttaata tttagataga ttgtgttgag tggttgattt ttattttggt    4080
   tattgttttt tttatttgtt tgtagaagaa ggaatgatag ttatagtgtt tttgtagggt    4140
   ggttggtttt ggggtagtgt ttttgtattt gttgtgttta ggtttatgtt tatttttttt    4200
   agtaatgtat ataggttaag taagatttgt ttgggtttta gtgtagaaga ggttgaaatt    4260
   gaggtttata ggttttagtt tatttttgta ttttattttt ttatggttat tttattagag    4320
   gtttttggag agtttttgtt tttaagaatt aggataggga agaggtgtag agttttatag    4380
   aaatttaatg ttttagaagg ttaatagatt ttttatttgt aggtttttta tttttggatg    4440
   ttttttgttt tttagagaag tttttggatg gaagatatga ttatagtatt agtaataata    4500
   ataattaata tttatggtgt gttttttgtg tattaggtat tgaattaagt atttttttat    4560
   ttaattttta taattttatg aggtattatt tttattttat ataggaagaa attgaagttt    4620
   ataattttgt ttagttagtg tgttaatgtg tggggattag gatttaatta taggtttttt    4680
   ttgttttagg ttttttattg ttattgggaa ggtttgtttt tttattagtt taattattta    4740
   ttatatatta tttattgaat atttgttata tggtaggtgt tatgattaat tttagaggtt    4800
   taataaaata ttttatttta aatttagatt ttataatttt tttgtaattt gtaattaatt    4860
   tttttttttt ttttatgtta tgaggtttaa tttttaatat tattttattg ggatagtgag    4920
   aatagagttg gttaaggttt tgattaatag tatgttatta ttaggaaggt tataaaattt    4980
   tatatttttt ttagggtgta ggatgaattt ggggtgggga agtttaatgt tttatttaag    5040
   ggtatagagt taggagtaga atgtaggttt agtgttttag attttattat tttagatttt    5100
   tttttatttg agttttaggt ttgagaaatt tggtttttta aattgtatgt tttaaaattt    5160
   ttgttttttt tagggattat tttgttgttt ttggattttt ttagattatt aaattattgg    5220
   gttataaata atgataattt taatatttgt ttttttttta gttatttatt tatatttagt    5280
   ttttttataa atgtttaggt ttggtttttt ggatataaat aggggtttta attagttaat    5340
   tttaaaggtg tggggaaaat aaagttgttt gtgaattttg ttttgatagt agaggaaaag    5400
   aagtagatat ttttttatgg agttttttaa tttagtgtta tatgtagtgt taggttaata    5460
   gggattaatt attttttggg aggagatttt ttttggtttt ttttttggtg attagtattt    5520
   tattatgagg attttatttt agtttattgt ttttagaaag attaaaagtt gtagttttag    5580
   ttggttgttt ttgttgttgt tgttaatgtt gttttgttgt ttttgtttgt taattataaa    5640
   ataagaatat tgaataaaat aaagaattta tatttagtat tat                      5683
<210> 53
   <211> 2347
   <212> DNA
   <213> Artificial Sequence
<220>
   <223> chemically treated genomic DNA (Homo sapiens)
<400> 53
<210> 54
   <211> 2347
   <212> DNA
   <213> Artificial Sequence
<220>
   <223> chemically treated genomic DNA (Homo sapiens)
<400> 54
   gtttattagg tttaagtgat ttttttgttt tagttttttg agtagttagg attataggtg 60
   tgtgttatta tgtttggtta attttgtatt tttattttat tttatttttt tgagatagaa 120
   ttttattttt gttgtttagg ttggagtgta atggtatgat tttggtttat tgtaaatttt 180
   gtttattagg tttaagtgat ttttttgttt tagtttttta agtagttggg attatagata 240
   ggagttatta tgtttggtta attttgtatt tttagtagag atggggtttt tttatgttgg 300
   ttaggttagt tttgaatttt tgattttaag tgatttgttt attttggttt ttttaaagtg 360
   ttgggatata ggtatgagat tatattttta ttaaaaattg aaaaaaaaaa aaaaaaagtt 420
   ggatggttga ggggtggtgg tttatgttgg taattttagt tttttgggag gttgaggtta 480
   gtagattatt taaggttagg tatttgagat tagtttggat aatatggtga aattttattt 540
   ttattataaa tataaaaatt agttgggtgt ggtggtgtat gtttgtaatt ttagttattt 600
   aggaggttga ggtaggagaa ttgtttaaat ttaggaggtg gaggttatag tgagttgaaa 660
   ttgtattatt gtattttagt ttgggtgatt gagtaagatt ttgttttaaa agtaaaaaaa 720
   aaaaaaaaaa aaagaaaagt taggtattat ggtggtattg gtttgtgttt tagttatatt 780
   agaggttgag gtagaaggat agtttgaaat tgggaggttg aggttgtagt gagttatgat 840
   tgtgttattg tattttagtt tgggtaatag agtgagattt tgttttaaat aaaaataata 900
   attaaatatt tttaaatgat tttttagagg tattagaaag ttttgattga atgatgaaaa 960
   ttgtttttgt tgatgagtga tgtttatgag tttgttaaat taataaatgg gttgtttttt 1020
   ggttaatttt tatgtatttt ttaatatttt ttttagatga tttttagtga tgttagggtt 1080
   ttttgttata tggagggaag tttggttttt ttttttgggt tgaaggtttt tttttttgag 1140
   aaggtggaag ttgaggaggt ggggaagtgt tgtggttttt tttgttatgt tttttggggt 1200
   ttttgtgatt tgattttttt tgttttattt gtttttgagt ttgtggggta tttgaaagtg 1260
   gggtagtttt gggaatttat tttagatgag tttttggggt agttgggtgg ggttataagg 1320
   ggtttatagg gttttttggt aggtttagag atggagggaa ggaggtgggt ttgttaagat 1380
   gtggtaaggg gaaggtggga tgtggggaat tttagaggga gtgttaggtt gtgattttaa 1440
   taaggggaga aatattattt tatttttgtt tggggtattt ttttagttta tatatttgtt 1500
   atggtttttt attgtttttg gagaaagtta ggttttttag tttggtgttt gaggttttag 1560
   tttattattt tatagaaatt tagatttatt gtgtttattt tggtggtttt taaagtattt 1620
   tttttggatt tttgtttttg ttgtggtttt tgttggagat gttttgtaat tttatatatt 1680
   ttagtttaag tgtttttttt ttaggtagtt ttttttgttt tattttaggg tatttggttt 1740
   ttatttgttt ttgattttaa ggggttaggg gtgtttgtgt ttagtttttt atttttttag 1800
   ttttgtattt tttttttttg atgttttttt agtagttgtt aggtagaggt tattggggaa 1860
   taataataat gattaattat attggttagt gggtggatag tgtttattgt aagttaagtt 1920
   tttttttata tttataaaag tttattttgt aggtgggaaa gatgaggttt gagagggtta 1980
   attttggtgt gggtggggga agttttgagg tatggttttt attttggaag gggtaaaatt 2040
   atattttata aggggtggtt tgaatttatt tttttatttt tagttggggt tttaggtggt 2100
   ttgagtgttt ttggatttag ttttttaatt tgtgaaataa ggtaagtgag ggatttttta 2160
   ggaattggtg gagttgggag ggttttagga tggttaggag ggggttatga tttttttttg 2220
   atttttattt atttttttta ttttttttgt tattttttga gtagggtttt gttttgtggt 2280
   ttaggttgga gtgtagtggt gtaattagag tttattgtag ttttgatttt tttggtttaa 2340
   gttttag 2347
<210> 55
   <211> 6709
   <212> DNA
   <213> Artificial Sequence
<220>
   <223> chemically treated genomic DNA (Homo sapiens)
<400> 55
   atttttaaat tttttaattt atagagggtt ggagagttgg tattatgagt ttttagtttt      60
   tatgggtgag aaaagaggtg tttgtgtgga gggtaggtgt gattttgggg gttagtagag     120
   aggagaggtt gtgggaggat ttgttgtgaa gggtgagggg tggtgttggg tgggatgggt     180
   gtttggggtt gtgtagggtg gtttttgggt gttgtttggg ttgtttgttg tttagtattg     240
   gtgtaggaag atgggtttgt gtagtgtttg gtaatagtgg tttgttttat gtttggaggt     300
   gattaagttt tgagttaaag gtgttgggtt tagttgggaa gtgtatggtg atttagaata     360
   tggagtggag aaggttttag gggatatgag gttattgttt tagtttttgg gtttgtgttt     420
   tgagtggtta gggtgtgtat ggatgggttt tgggatgtta gtggagaggt gataggagtt     480
   tattatttta tggttaagtg tgtgaggttt ttggtgggtg ggggtttttt ttggggtagg     540
   gtagggggag agttaggtat gtttttaatg ggttttttag tagaattgtt aggaaagtgg     600
   tttttaattt taggtatttt tgtggttttt aggtagttgg gtgattaaaa atttattttt     660
   gtttgttgtg aaatgggata ggtggttgtt aggttagaga ttgtgtttat gaagttttgt     720
   ttggtgggtt gggattgatg tggatttggg ttttttttgg aagtttggga atttgtgagt     780
   tttttagttt tttgttttat tttgtttgtt tgaaaaaagg agttgtttga gaggaagagt     840
   taaggtttaa aaggttttaa atgaagagta tgtggtttga tgtggagtgt aaagaagtga     900
   gtagtgaatt gaatttggtg ggattgagat tagaggtgtt tgtatgtggt ggttgttttg     960
   tgttttatta aagtaatttg ttatttatga taattatttt tgtaattatt ttggagtggt    1020
   tgtgatttga tgtttgtggt gttttagggt atgtttttat tggatataga tattttaggg    1080
   tgttttttga tgttagtgtt ggtttagtgg gtgttagtgt tgtgaaggga gggtggtggg    1140
   tgtgaatgtt tttttttttt gggtttgttg taggttttgg tttttttatt ttgtttgatt    1200
   ttggtgggtt tgttatttat tgaggttttt attggtgttt atgttgtttt gagaggtggg    1260
   ataaatgttt ggtgtgtggt gtggagtttg tgaaagtgtt tgggggtttt tattttttgt    1320
   ttgggagttt tttttttggg gtgggaagag gagttgtaga tttgtttgaa ttttgtttta    1380
   tttgcttggt tgtttttttt tgtttggggt gggggtaggg gtggtggatg atttaggttt 1440
   ggggttggga gttttgtttt taaaggtatt attttgtgtt tttttggaag tggtgtgttg 1500
   ttggggtgtt tttgttgttg tagaggtttg gtttgtggtt ttataggggt gtgggtttag 1560
   ggtttttagg gagtaagtga gtttaggttt tttagggggt ggtagagggg aatttttgat 1620
   aattttttat tttttttttt aggtggttgt gaggtttttg gggttttaga ggggtttttt 1680
   tttgttggtg ttggttgttt tgagtgtggt tatgtagttt gtgtttttat gtgggtaagg 1740
   ggtggggatg ttggagggag gttgttttgg atgttggagt ttttggtttt tggttttgtt 1800
   gagtagaaaa gttgaagtta aggaagggtt gaggtttttt tataagggtg tgtgtgtgtt 1860
   tgggtgttgg tttagaagtt gagaatgtgt gtattgggga agggtttttt gtgttgagaa 1920
   gatagaggat ttgttttagt tgttttttgt atagagggtg tggaggttgt gttttagttt 1980
   gaatgtttat ttattggaaa gagggtagtg ttggggttta gggaagtttt ttgggaatga 2040
   atggtttttg ttaagtggtt ttggattttt tgggtttttt gggtttatgg tatggtgttg 2100
   tgtgagtttt tagtgtttat tggttttttt tgttttttgt gtagatgttt ttaggtgggg 2160
   aggtatattg gtttttttgg gtagttttgg ttagtgttgt tgtgggaaag gagagttagg 2220
   gtttgggatg ggggatgagt atttttttgt ttattttggg ttttagtgtg taggaggtaa 2280
   atttgttagt atagataaga tagtttgttt aagttgtatt ttaggttggg ttagagaata 2340
   aaattgaggg ttagaaggtt tagaatgttg gatagtttag tggtatttgt tagggagttt 2400
   taggtgtttg aaagaggtgt tgtatttttg gtgagtttag gatttatttt tttggatttg 2460
   tgttttggag tgtttgtggg tttgggttta atttttgttt ttggtaggtg tttttttttt 2520
   ttgttgttaa tattaggagt gttgtttttg tggtaattta ttttattttt agaagtttgt 2580
   tttgggattg aagtgttagg gttttgtgtg tttttttatg tattgttttt tttagtgtaa 2640
   agttttgtag atatgtttgg ttgaaaagtt ttagtggttt taatttttag attttgtttt 2700
   tgtgtttggt tggaaaatta tttgggattt ggttgttgtt taaggttggg ggaaagtttt 2760
   ttttggagga tgtttattat tattattttt ttttaggttt atttgggttt aaataaatgt 2820
   taaagtttaa atgaatttga gaaggaatag tgaggtagtg gatgttgtta ttttgtggat 2880
   gggagagtta tgtttattgt aggtgtggat attgtattga taggtattga ttattgtaaa 2940
   attgtattta gtattaaata gaaatagttt tgttttttaa tttttataga atgtttaaat 3000
   ttagtttagg gagttagttg gggtgtttta ataaatatgt ttttgagaat tagtgatgtt 3060
   ttttttataa tgaaagttaa ttgttgtata ataattgggt taaagaattt tgttagttta 3120
   aattttaaaa attagtattg tattttgttt ttaagtttgg gtggtaagta ttgaagggga 3180
   gagggaagta gtgtttgtgt ggaaggaagt tttgtttttt gtttaaatat gtgggatttg 3240
   ggtgtttatt tttttagttg agagtttttt gtaggttttt ttttaatttt ttgtattttt 3300
   ttatttttat tttatttgtg gtgtttgggt tttatttttg tttggtgttt tttttttagg 3360
   gtgagtagag gtttttgagt gattggtttg ttagaggtag ttgtagagtt ggtgttttgt 3420
   agggtagggt agggttgggt tgttgggttg gtttagtgtg tgtgggtttt ttttggtttt 3480
   agttagttgt gggagatgtt attttgggtg atagtggtga gatgttgttg tttgtaattt 3540
   ttgggtgggt aggattgttt gttgtttttg tggtttttgg ggtagatttt ttaggagttt 3600
   tttttttttt ttttttgttt gggggttggg attgtgttgt gtttatggag ggaattgggt 3660
   gtttttggtg tattggggtt ttagttgtat gattttagtt tagattttaa gttttagtgg 3720
   ggtgtaggtg ggatgggatg tgtttttagg agatgaggag attttggttg ttgagttgtt 3780
   ttttaaatgt gtagtttttt aattttaggt gatatttgtt ttttggagga atgagtgaag 3840
   gttgtttagg tgaaagaggg gtgggtttta gtatttggat tttttttttt agttttgtta 3900
   attttaggtg tgtttatggt ggaggtgata gaaggaaggt gtgttggggg atttaggttt 3960
   tgtaggtttt tggttgttaa tgaggtattg tttattgatt ttgtgttttg ttttttatag 4020
   tgtggggttt ggtgttgtgt taggtttggg tgtggggtgg agttaggttg ggattggttg 4080
   ggggttgttt tgtttggtgt ttgggttttt gtgttggttt ttggggagga gttatgataa 4140
   tttttttttt attaaggtgt ttgggttttt tggttattgt taggatatat tgtttgggtg 4200
   tggttttttt tgtttgtgga gtggttttga tatttgtggt ggtagtattt atgtttgtag 4260
   agttgttgat gtgtgtttag tgatttggat agtaaggttt gtgtgtggtg ggggtggtgg 4320
   tagatgtttg gttattgtga ttttgatttt ggatttattg tttgggggtt ggggggattt 4380
   tggatttaat tggtgattgt tttgggggat gttggatgtt atgttgtgga aaataattga 4440
   taatgttaag tatgaagagg attgtgaggt gagttggggt tttggggtgt agttttgttt 4500
   tgttgaggat agtttgggag gtaggggtta ttggattgag gttggggatg agggtatagg 4560
   agttttggtt ttttggtggg atgggattta tttttttgga tattttttag tttatttttg 4620
   tggggttttt tttttgtata gggttttttt ttaaatagtt tttttttggg aatgaggttt 4680
   ggaaagagaa tggtaaattt tatttatata gtttattggg tgttttttta agtggtaaat 4740
   agttttgttt tttgtttttt tggaaaagtg ttttgtttgt aatttttaga tgtggttttt 4800
   atgtatgaag tttttgttta aaggggttag atgaggttat ttgttagtga tattttggtg 4860
   gatatttttt taaggttggt ttgttatttg ttttgttatt tttttgggga agtatgaaaa 4920
   taattgaagt ttggttttta aaagaagtgg gggttgggga gtggtggtgg gggtgtttga 4980
   ggttagatgg ggttgtaaga gtttgtgagt gttttagttt gttggatagg ttgagttaga 5040
   gttttggaga gtgggaggga agaaggaggt ggtgagtggg aagaggaaga agatgtaggt 5100
   agtgttaggt gagtttaggg gtgttgggag tgtggagttt gggttatgga tttgtgggag 5160
   tttattgtgt ttttgggttt tggagggttg tttttgtttg taggtttggg tgtttttgtt 5220
   aggaggtgat agtgttatgt ttttttaggt ttttggtgtt ttgagatttt tgggtagatg 5280
   gggatgtatt ttttagaaat tgagttgggt tgtttagggg tgagggaatg tgtgtgggta 5340
   aggttgttta atttttaggg ttttttatgt tttttttgtg ttttgatgtg tgagaatatt 5400
   gtttttggta gtgtagggtg tttttattta ttattttttt tggttgggtt tggttagtag 5460
   ggagggttgt tttgtgtgtg tgtttttttt ttatttttta gggtggtgta gggtggtggg 5520
   ttttgttttt tgagtgttgt ttgtttggag gtttttgttt tgagggtgtg gaagggaggg 5580
   ttttgggggt gggggaggtg tgtatttttt gtgttgtgta ttttatttgt gtttgttgtg 5640
   ttgttatttt tagtagtttt tgtgttatgg gtgggtttta tgagatagtt ttgtattggg 5700
   tgtttggttt ttttgttttg ggttttggtt tttttgtttt gggttttggt tatggatttt 5760
   tttggtttaa gatttagggt ttggatttgg tgtttttaag tgttttgggt ttgggagtag 5820
   tgtttagatt tttgttgttg ggttttgaga atttgttttt ttaggttttt tattagattt 5880
   tttttttttt ttgtattttt tgtttataat gaaattttgg gggtgtattg tttttgggta 5940
   ttttttgatt ttgggtaagt tttgttgggt ggggtgtggt tggttttttg gggttttttg 6000
   tgtagtttgg tgatgttggt tagtttgtag tagtggggtt ttgtattaat ggggtttttt 6060
   gttttttttt ttttttttag gattgttatg atgggagtag taatgggaat ttgtgggttt 6120
   tttatttttt ttttgttggg tagtattttt atagttttgt gttatttttt ttttatattg 6180
   gagttgttga atattagttg ttattttatt tttttttttt ttattagtag ttggtttatt 6240
   tttagttggt tgatttttat ttgtatttgg gggaagtgta tgttgttgtt attaattttt 6300
   tgtattagtt ggtgtttata ggtagttagt agtaggtttg gtttggttgt tagagttagg 6360
   agggagtggg gttgtttttg tattatgggt gtttggttgg tttattgttt tatttttttg 6420
   ggttggaggt gggtgtggtg agtgtttgta gggatgttta ttgttgtttt gatttgttgt 6480
   tgttttatgt atatgttttg gatgttgtgg gtttggttga gaatttgggg ttttatgata 6540
   tgttttatta gatggatgag gtgtaggtga gtggtgttgt ggtttttgat tggatttgtt 6600
   tattttatgg ttttttgttt ttattttgta tttttttagg tttttttgaa tttaagggaa 6660
   ttttgttttt ttttttttag aatgttgatg attagtattt gttgttgta 6709
<210> 56
   <211> 6709
   <212> DNA
   <213> Artificial Sequence
<220>
   <223> chemically treated genomic DNA (Homo sapiens)
<400> 56
   tgtagtaata ggtgttggtt gttgatattt tgggggagag aggataaaat ttttttaagt     60
   ttgggggagt ttagaggagt gtggggtggg ggtagaaggt tgtagggtga ataggtttgg    120
   ttaggagttg tagtgttgtt tatttgtatt ttgtttattt ggtgaggtat gttgtggagt    180
   tttaggtttt tggttaggtt tgtggtattt agggtgtgtg tgtggggtag tagtaggttg    240
   gagtggtggt aggtattttt gtgggtgttt attgtgtttg tttttagttt ggagaggtgg    300
   ggtagtaggt tggttgggtg tttgtggtgt gagggtagtt ttgttttttt ttggttttgg    360
   tggttgggtt aggtttgttg ttggttgttt gtgggtgttg gttggtgtag ggggttgatg     420
   gtggtggtgt atgttttttt tagatgtgag taggggttgg ttgattggga gtaggttagt    480
   tgttggtagg gagggggaaa gtagggtggt ggttgatatt tggtgatttt agtgtgggag    540
   aggggtggtg tggggttgta gaggtgttgt ttggtggagg agaggtgggg gatttgtgga    600
   tttttattgt tgtttttgtt gtggtgattt tggagggaaa aaaaaaaata ggagattttg     660
   ttagtgtgaa attttgttat tgtgaattgg ttggtattgt tgggttatgt agagggtttt    720
   gggggattaa ttgtattttg tttggtgggg tttgtttagg gttggaaggt atttgggggt    780
   aatgtgtttt gaggattttg ttgtaagtaa agagtgtggg gagggaggag agtttggtga    840
   aagatttggg ggaatgagtt tttaaagttt ggtggtgaag gtttaggtgt tgtttttaag    900
   tttgaggtgt ttggaggtgt taagtttgaa ttttgggttt tgggttagaa aagtttgtgg    960
   ttggagtttg gggtgaagga gttagagttt ggggtgaagg agttggatgt ttggtgtaga   1020
   gttattttgt ggagtttgtt tatgatgtag ggattgttgg aggtggtagt gtggtaggtg   1080
   tggatagagt gtgtggtgtg ggaaatgtgt attttttttg tttttgggat tttttttttt   1140
   atgtttttgg gataaagatt tttgagtggg tggtgtttgg aaagtagggt ttgttatttt   1200
   gtgttgtttt gggaagtgaa gagggagtgt gtgtataggg tggttttttt tgttggttgg   1260
   gtttagttga ggggagtaat aagtgggggt gttttgtatt gttaagggta gtgtttttgt   1320
   atgttggggt gtagaggggg tatgaagaat tttggaaatt gggtagtttt gtttgtgtat   1380
   gtttttttgt ttttgggtgg tttgatttag tttttaaagg atgtgttttt atttgtttag   1440
   gggttttggg gtgttgggaa tttggaggaa tatggtgttg ttgttttttg gtggaagtgt   1500
   ttgggtttgt gggtaggggt agttttttag ggtttggagg tgtagtgaat ttttgtgagt   1560
   ttgtagtttg agttttgtgt ttttggtgtt tttgagtttg tttagtgttg tttgtgtttt 1620
   tttttttttt ttgtttgttg tttttttttt tttttttatt ttttgaaatt ttagtttaat 1680
   ttgtttgata ggttggagta tttgtgagtt tttatggttt tatttggttt tgggtatttt 1740
   tattattatt ttttagtttt tatttttttt gaaagttaaa ttttggttgt ttttgtgttt 1800
   ttttgaggag gtagtggggt aggtgataag ttggttttgg aggagtgttt gttaaggtgt 1860
   tgttagtagg tagttttatt tgattttttt ggatagagat tttgtgtgta aaagttatgt 1920
   ttgagggttg tagatggggt atttttttga gaaaatggga agtagggttg tttgttgttt 1980
   aggaaagtgt ttggtaagtt gtgtgggtgg gatttgttat ttttttttta ggttttgttt 2040
   ttgaggggag gttatttagg aaaaggtttt atataggaaa ggggttttgt agaaatggat 2100
   taaggggtgt ttggagaagt ggattttgtt ttattgagag gttagggttt ttatgttttt 2160
   gtttttgatt ttggtttagt ggtttttgtt ttttggattg tttttggtgg ggtggggttg 2220
   tattttggag ttttagttta ttttgtagtt ttttttgtat ttgatattat tggttatttt 2280
   ttataatatg gtgtttggtg ttttttaaaa tagttgttag ttaaatttag gattttttta 2340
   gtttttaagt ggtaaattta aaattggggt tatggtgatt aggtgtttgt tgttgttttt 2400
   gttgtgtgtg ggttttgttg tttgggttat tggatatgta ttggtggttt tgtgagtgta 2460
   gatgttgttg ttgtgagtgt taagattgtt ttgtggatgg ggaaagttgt gtttgaatag 2520
   tgtgttttgg tgatagttgg gggatttgag tgttttaatg agaaggaaat tattataatt 2580
   tttttttggg ggttggtgtg gaggtttagg tgttgggtgg ggtggttttt agttagtttt 2640
   agtttagttt tgttttgtat ttgggtttgg tgtagtgttg agttttatgt tgtgagggat 2700
   gaagtgtgga gttagtgggt ggtgttttgt tgatagttga agatttgtga ggtttgggtt 2760
   ttttgatgtg tttttttttt gttattttta ttgtaaatat atttgagatt ggtagagttg 2820
   ggggaaggga tttaaatatt gaaatttatt ttttttttgt ttaaatggtt tttatttgtt 2880
   tttttaaaag atggatgtta tttggagtta agaaattgtg tatttgggga gtgatttaat 2940
   aattggggtt tttttatttt ttaaaaatat attttatttt atttatattt tattgaggtt 3000
   taaggtttga gttgggatta tgtagttgga gttttagtgt gttagagatg tttagttttt 3060
   tttgtgggta tagtgtggtt ttggttttgg ggtgggagga gagaggaagg gatttttggg 3120
   gagtttgttt taagggttgt agaggtagta gatggtttta tttgtttagg agttgtggat 3180
   agtggtgttt tgttgttgtt gtttgaagtg gtatttttgg tggttggttg aggttagggg 3240
   gaatttgtgt gtattggatt agtttagtgg tttggttttg ttttgttttg tggaatgttg 3300
   gttttgtagt tgtttttggt gggttggtta tttagaagtt tttatttgtt ttgggaaaga 3360
   agtgttgggt aggaataaaa tttgagtgtt atgggtaagg tgagggtgag gaggtgtagg 3420
   aagttgggag agagtttgta gagagttttt gattggagag gtagatattt aggttttata 3480
   tgtttagata gaaaatgaaa ttttttttta tataaatgtt gttttttttt tttttttggt 3540
   atttgttatt taaatttgaa agtaaagtgt aatattaatt tttaaaattt aaattaatga 3600
   aattttttgg tttagttatt atgtagtaat tgatttttat tgtaagggga atattgttaa 3660
   tttttgaggg tgtatttgtt gggatgtttt aattggtttt ttgggttgga tttaaatatt 3720
   ttgtggggat tagaggataa gattattttt atttgatatt gaatatgatt ttgtaataat 3780
   taatgtttat tagtatggtg tttgtatttg tagtagatat gattttttta tttatggaat 3840
   ggtggtattt attgttttgt tgtttttttt tgggtttgtt tgaattttag tatttattta 3900
   gatttaggtg ggtttaaaag aaaataataa taataggtgt tttttaaggg aaattttttt 3960
   ttggttttag atagtagtta aattttaggt gattttttga ttggatgtgg gaataaaatt 4020
   tggaagttga aattattgag attttttggt taagtatatt tgtagggttt tgtattaagg 4080
   agaatagtgt ataaaaagat atataggatt ttgatatttt ggttttaaaa taggtttttg 4140
   ggggtaaaat aagttattgt agaagtagtg tttttggtat tagtggtggg ggagggggat 4200
   atttgttaga agtagaaatt agatttaggt ttgtaggtat tttagagtat aggtttagga 4260
   agatgggttt tagatttgtt agaggtgtgg tgtttttttt gggtgtttgg gattttttga 4320
   tgggtgttgt tgggttgttt gatattttgg gttttttagt ttttggtttt attttttgat 4380
   ttggtttgag gtgtagtttg aataagttgt tttgtttgtg ttggtaagtt tattttttgt 4440
   atgttggggt ttggaatggg taagaaggtg tttatttttt attttaggtt ttggtttttt 4500
   ttttttatag taatattagt taaagttgtt tggaggaatt gatatgtttt tttgtttggg 4560
   agtgtttatg taggaggtga agggagttga tgggtattga gggtttgtgt agtattgtgt 4620
   tgtgggttta aaggatttag aggatttgga attgtttggt aaaggttatt tatttttaag 4680
   gagttttttt ggattttggt gttgtttttt ttttaatggg taagtgtttg aattggagtg 4740
   taatttttgt gttttttgta tggaaggtag ttagaataaa ttttttgttt ttttggtatg 4800
   ggagattttt ttttaatatg tatatttttg gtttttgggt tggtatttag gtatgtgtgt 4860
   gtttttgtga ggaagtttta attttttttt gattttaatt tttttgttta gtagagttgg 4920
   gaattgaagg ttttagtatt taagatagtt tttttttaat gtttttgttt tttgtttatg 4980
   tgaagatgtg gattgtgtgg ttgtgtttaa gatagttggt attggtagaa ggggattttt 5040
   ttggaatttt ggaggttttg tagttgtttg gagaagagga tggaaggttg ttgggggttt 5100
   ttttttattg ttttttaggg agtttaaatt tatttatttt ttggaagttt tggatttatg 5160
   tttttatgaa attgtaaatt aggtttttgt aatagtggga gtgttttaat agtgtgttat 5220
   ttttaaagag gtgtgaggtg gtgtttttgg aaatagggtt tttggtttta ggtttgagtt 5280
   atttgttatt tttgttttta ttttagatgg aagaaggtgg ttaggtgggt agagtaagat 5340
   ttgggtagat ttgtgatttt tttttttgtt ttagggaggg gatttttgag tagaagatgg 5400
   aggtttttga gtgtttttgt agattttgtg ttatgtgttg gatgtttgtt ttgttttttg 5460
   ggataatgtg agtgttggta gggattttag taagtggtag atttgttagg attaagtaag 5520
   gtggaaaggt tggggtttgt ggtaaattta ggaggagggg gtatttatgt ttgttatttt 5580
   ttttttgtgg tattggtatt tattggattg atattggtgt taaagggtat tttgaagtat 5640
   ttatgtttag taagggtatg ttttggggta ttatagatat taagttatag ttattttaaa 5700
   ataattataa aaatgattat tgtaaataat agattgtttt aatagggtgt gaagtggttg 5760
   ttgtatatag atgtttttgg ttttagtttt attagattta gtttattgtt tgtttttttg 5820
   tgttttgtgt taggttgtat gttttttgtt tagaattttt tgagttttaa tttttttttt 5880
   taaataattt ttttttttaa ataggtaaag tgaggtgagg agttgagggg tttataggtt 5940
   tttaagtttt tgaggagggt ttgggtttat gttagttttg gtttgttaaa tgagattttg 6000
   tgggtatagt ttttaatttg ataattattt gttttatttt atagtaggta aaagtgaatt 6060
   tttagttgtt taattatttg aaagttataa aggtgtttag ggttgaaggt tattttttta 6120
   gtggttttat tagggagttt gttagagata tgtttgattt tttttttgtt ttgttttggg 6180
   ggggattttt gtttgttgga ggttttatat gtttggttgt gggatggtag atttttgtta 6240
   tttttttatt aatattttgg agtttatttg tatgtatttt aattgtttgg agtgtaggtt 6300
   tgaggattaa ggtggtagtt ttgtgttttt tgaggttttt tttgttttgt gttttgggtt 6360
   gttatatatt ttttggttaa gtttggtgtt tttaatttag aatttggttg tttttaggtg 6420
   tggagtaggt tattgttgtt agatgttgtg tggatttgtt tttttgtgtt agtattgggt 6480
   gataggtagt ttgggtagta tttaggagtt attttgtatg gttttaggtg tttgttttat 6540
   ttagtgttat tttttgtttt ttgtagtgag tttttttgta gttttttttt tttgttagtt 6600
   tttggggttg tatttgtttt ttatatagat attttttttt ttatttgtag gaattggaag 6660
   tttatggtgt tagtttttta attttttgtg agttgaggag tttaagagt 6709
<210> 57
   <211> 2296
   <212> DNA
   <213> Artificial Sequence
<220>
   <223> chemically treated genomic DNA (Homo sapiens)
<400> 57
   gttattgtat tttgttaata ttttttaaaa gtaaaaaata aattaaaaaa aaagttttta 60
   gtggattgtt gagttttttt tgataggagg tattaaatgt gttttttttg aggatagggg 120
   aagagtttat tatttagtta tatttttgta tattttataa aatttatttt tattaaggat 180
   gtttttttat aaaatgagga gtagtataaa gaatatttaa gaaattaggt ttgatttagg 240
   gtttttgatg ttggggtagg ttttttgtgt gttaatattt taggggattt tgttgtagtt 300
   ttttgaatat tggttgtgag ttttttaatt ttagttgagt tttagtattt tttttatatt 360
   atttattttt tttgtttgtt tttggtgtgt ttagataatt ggaatttttg ttgatatttt 420
   aaatgggttg gttatttttt tagtgtggag gtatttagta taaggtgtga tagattagta 480
   tttgttgatg agtaataaag ttattttggt aaattggatt ttttttttga aatgttttgg 540
   tttttatatt tttaatgatg ggttaagaaa tagtttttaa gaagaagggg tggagatagg 600
   gggtaggatt ttgtttgaag tttttaagtt gattttgttt gtagttattt aatttattag 660
   ggagggttgg atttgttttt ttgtggtgga ttttttgtat ttttagaaag tgtaggggtg 720
   gagaattaat ttagtttttt ttttttttat aagagttttg ttaggttagt ttgtatgagg 780
   agggtatgtg tatattttta aaaggattgg tgtagttatt tttttaagtt tatagtttta 840
   gtgtgagttt agggtttaga tttggggttt ttggtgtttt aggtgtgagt taagtttttt 900
   gagtaagagg ttttatgata aattagttgg agtttggggg ttatgaaagt attggttatg 960
   gtagaaatta ttagtttgtt attgtttatg ttgttttgtt taaaaatgat attgtgtaag 1020
   ggattttgtt aatttttaat tttgggagat tatttattat ttggttgtgg tttgttgttg 1080
   aataggaaag tattttgaga tgttaagaga ttaaaaggtt tgtaattgtt gtttagaaat 1140
   gaaaatggtt ttaaattata attagagatg gaagtgtgtt ttaggggagg gtggggtgtt 1200
   gtgtaattga gttttttatg tgtaaggtgg atataaatgg tttatggatg tgggtgaagg 1260
   tttttagagt aaagaatgtt tatttttatt agattaaaat atagtttggg aaaataatta 1320
   tttgtttgag attttgggaa agtaaaattt tttggttttt ttgttaggaa gaatgattgt 1380
   aaaataaggg ttgagaattt tttaaataag attttagaat tttatttaaa tagttgtagt 1440
   ttatttttga ggagttttag atttatttgg gtttgatgga tgttgtaata ttaagtttga 1500
   ggttttttta gtttggtgtt aagagtgggt tttaggggat aaatagagaa tagttgtttt 1560
   tttaattgga gtagaaaata aaattttttt ttttttttat ttttaatttt tttgttttgt 1620
   tgtgaaattg ttattgtgtt tttgaggagt gattaaaatt ttagttatga ttaatttaaa 1680
   gggtaggttt ttaggtaggg aagggaaagg ggtttaaggt aaaaaaaaaa aaaaaaagtt 1740
   agttgaagtg agtttatgga ggatttgggg attataaaag ttatgggatt tgagttaatt 1800
   aaaattaatg gaaaagtatt aatatgtaat ttaaaaaata ataataaaat aaagtagtaa 1860
   ggatattttt ggtttgtttt ttgtttttat ttttgagttt gtttgggaat tttggagtag 1920
   attttttgaa gtatagtatg agtgtttaat ggttgtttta atttattttt ttgtttttta 1980
   tatataaaga ttttgtaatg gagagtggga atgaatggtt tatttatttt tgaatatttt 2040
   ttgtgagttg ggaatgtgtt tagtaaggtt gttggagtgg agggaaaaga aaaggtgttt 2100
   gagtttttta gatatgggta gaaaaggaga gaatttgaga taggttgaga gaaagttgtt 2160
   ttggttggta gtattgttag atttttagat atattgtata ttttagtttt tgaaaaggtt 2220
   attttgagtt tttttagggt ttttggggtt tttattttat tttaaatatt tggaattgtt 2280
   tgtgtttgtt ataata 2296
<210> 58
   <211> 2296
   <212> DNA
   <213> Artificial Sequence
<220>
   <223> chemically treated genomic DNA (Homo sapiens)
<400> 58
   tattatagta ggtatagata gttttaaata tttgggatga gatgagagtt ttaggggttt      60
   tagagggatt taggatggtt tttttggggg ttggaatgta tggtgtgttt gagaatttgg     120
   taatgttgtt aattggagta gttttttttt agtttgtttt aaattttttt tttttttgtt     180
   tatgtttaga gaatttagat attttttttt ttttttttat tttagtagtt ttattgagtg     240
   tatttttaat ttataggaaa tgtttagggg tgggtggatt atttattttt attttttatt     300
   gtagaatttt tgtgtgtgaa gggtaggaaa ataggttaaa atagttattg ggtatttatg     360
   ttgtgtttta ggaagtttgt tttaagattt ttaagtaggt ttaagaatgg aagtagagag     420
   taagttagaa atatttttat tgttttattt tattattatt ttttaaattg tatgttggtg     480
   tttttttatt aattttaatt gatttaagtt ttatgatttt tgtgattttt aaatttttta     540
   taaatttatt ttaattgatt tttttttttt ttttttgttt tgaatttttt tttttttttt     600
   gtttgggaat ttgtttttta aattggttat ggttgggatt ttaattattt tttaaaggtg     660
   tagtgataat tttataataa agtagggagg ttgggggtgg gaaggggagg gagttttgtt     720
   ttttattttg attaagagag taattgtttt ttgtttgttt tttggggttt atttttggta     780
   ttaggttggg gagattttaa atttggtgtt gtaatattta ttagatttaa gtgagtttgg     840
   ggttttttaa aggtgaattg tagttgtttg gataaaattt taaaattttg tttgggagat     900
   ttttagtttt tgttttataa ttgttttttt tggtaaagaa attaggaagt tttatttttt     960
   taggatttta ggtaggtaat tgttttttta aattgtattt taatttgatg gaaatggata    1020
   ttttttgttt tgagaatttt tgtttatatt tataaattgt ttgtatttgt tttgtgtatg    1080
   ggaggtttga ttatatagtg ttttgttttt ttttgagatg tgtttttatt tttggttata    1140
   atttagggtt atttttattt ttaaataata gttatgagtt ttttagtttt ttggtatttt    1200
   ggagtgtttt tttgtttagt ggtggattat agttaggtgg tgggtgattt tttgaggttg    1260
   ggaattggta aaatttttta tgtggtgtta tttttaagta aagtagtata aatggtgatg    1320
   agttggtagt ttttgttata attaatgttt ttatggtttt taggttttaa ttgatttatt    1380
   atgaggtttt ttgtttaggg gatttggttt atgtttgaaa tattagaggt tttgagtttg    1440
   ggttttgagt ttatattggg attgtgagtt tagggaagtg gttgtattag tttttttgga    1500
   ggtgtgtatg tgtttttttt atgtaggttg atttggtggg gtttttatgg ggaggagggg    1560
   ggttgagtta attttttatt tttgtatttt ttggagatgt ggagagttta ttatagggag    1620
   gtagatttag ttttttttag tgaattaagt gattgtaaat agagttagtt tggaagtttt    1680
   aggtagggtt ttatttttta tttttgtttt ttttttttgg gggttgtttt ttgatttatt    1740
   attaaaaatg taaaagttaa aatgttttag gggaaaagtt taatttgtta gaataatttt    1800
   gttatttatt aataaatatt gatttattat attttgtgtt aggtgttttt atgttggaga    1860
   agtagttagt ttatttaaaa tgttagtaaa gattttagtt gtttaaatgt gttagggata    1920
   ggtaaggagg ataagtggtg tgaggaggat gttgggattt agttgaagtt ggaaagttta    1980
   tagttggtat ttagagaatt gtaatagggt tttttgggat attagtatgt ggggaatttg    2040
   ttttagtatt agaggtttgg ggttaggttt ggttttttag gtgttttttg tgttattttt    2100
   tattttgtgg agaggtattt ttggtagaag tgaattttgt aaaatatgta aaggtatggt    2160
   tgaatagtga attttttttt tgtttttagg aaaagtatat ttggtatttt ttgttagagg    2220
   ggatttagta atttattaaa gatttttttt ttaatttatt ttttattttt gaaaaatgtt    2280
   ggtagggtgt agtgat                                                   2296
<210> 59
   <211> 4247
   <212> DNA
   <213> Artificial Sequence
<220>
   <223> chemically treated genomic DNA (Homo sapiens)
<400> 59
   ttttaaaaaa attaaaatat aaaataataa taattattaa aaattttatt tttgtttttt 60
   taaataaaaa atttatttta aaaattttaa tttaaaaatt taaaaatgaa aaatgtgtat 120
   tataattttt aaaaattttt gaatttaaaa atgatttttg ttttttgttt tttttgtgtg 180
   atgaaattga atttattaat aaaaatttga aataaaatga ataaaaatga aatgaaatga 240
   aattatgaaa aaataattga tgaattgtgt tgtgaattaa tattttaaaa aatgtgaaat 300
   ttgaataatt gtgtgttaaa aattttgaaa tgaatgttgt gaaatttttt tgaattataa 360
   ataaatgatt ttatgatgtg atttgaataa aaatttgatg attttataaa aaaatttaaa 420
   atattgatgt tgaatttttt ttaatttttg taataattga tatgttatat aaaatgaaaa 480
   aaaatttgaa ataaattttg aaatgtgaat taaaaaaaat gaatttgatt tgtgatttta 540
   atgttttttg attgatgtga aaaaaataaa attaaatgtt tgtgtgaatt ttgaaaaaaa 600
   aaaaaataaa aaattttaat gattaaaaat tttttgaatg tttattttga aatttttttt 660
   gttgtttttt atttaaaata tattgaattt gatatgattt tattgtttta ttttttatta 720
   tgatttttat tttgaattaa atttaattaa tttattataa taattattga taattgtttt 780
   tttgttttgt aatattttgt aaattttaaa attgtgatgt tgttgaatat aaattttgaa 840
   tttaaaatgt gtgatttttt tttttatgat tgttgttttt taaaaaaaat tatgtgaata 900
   taaaaaattt atttaatatg ttttataaaa tttattattt tttttttgat tgtgaattat 960
   gttgataatt tgaaaaattt gattttttgt tttttatttt tttttttttt tttttttttt 1020
   tttttttttt tttatttttt tgtttatttg ttgtaatttt ataaaaaaaa aattgttttt 1080
   ttaattattt ttttttttta ttttttatat atttttttat ttttttttga tttataaaaa 1140
   aaatgaaata atgattaaaa aatgttaaaa aaaaaaaata aaaatgtaaa ttatgaattt 1200
   ataaatgata tttgattttg tgtttgatga aaattatgaa ttaatttgaa tttgtgtttt 1260
   gtttttttgt gatatttaaa aattgtttga attaaaattg tgttattttt tgtttaatgt 1320
   tgatgtaata aaatttatgt gatataaaat ttttgaaaat ttaaaaaatt ttaaaatgtt 1380
   atgaatttaa tattttttta tttataaaat tttaaattaa taaaaaatat tttgtgaatt 1440
   ttaaaaaaaa ataataaata ttttttattt tatttgaatt tttttaataa aaatttatat 1500
   gtatgataat gttattatgt ttataaataa tgttaaaata taaaaaaaat aaaaaattga 1560
   aatttgaaaa ttttattttg taataaaatt taaaatttaa tttaaatttt aaaaatttgt 1620
   ttaaaattta aaatttaata taataaaaat aaaaaaaatt tttgataatt tatttttata 1680
   attaaaaaat gttaattaat atttttaaat ttttattttg taaataaaat aaaaatatat 1740
   gaaattattt aaaaaatttt tattatttgt tattattaaa tataatttaa attttgtaaa 1800
   aaaaaataat tttattttaa aattaaaatt tttaaaatat tttaaaattt gataaaaaaa 1860
   ataaaaaaaa taaaatttaa aaattaataa ttttatattt aatatttttt gtattttttt 1920
   ttttttttat attttttaat aatttttata attttaatta tttttaaatt ttattttttt 1980
   tgtttttttt taaattaaaa tattaaaatt aaattataat ttaaattata tttatatttt 2040
   attaaaaaat aaaaatttta aatttattgt gttttttttt attattaatt ttaatttaat 2100
   atttaataaa tttttttttt taaattatta aaataaattt gtgtaaattt gatttaattg 2160
   ttgtttttga attattgaat aaattgattg ttttgttgtt ttaaaaatta tttttatttt 2220
   attttaattt aaatttaaaa aaatttaaat taataatttt aattgaaaaa atgaaatgat 2280
   gtttattaat tattattaaa attattatta ttattattat aataattaat gtaatatttt 2340
   taattgataa ttttttataa aaaaaaatat tatttttttt ttattatatt taaaataaat 2400
   aaaatgaata ataaaaaaat aaaaaaaaaa aaaaaaaaaa aaatatatat ttttaataaa 2460
   tttttaaatt taaaaattat ataattttat attaattatt attttttttt aaataataaa 2520
   aatttataaa tttattatgt ttattttata tttaaaaaat taatattttt gaaatattta 2580
   ttaaataatg aaatatttta aaaaaaaaat aaaataaata aatttttaat ttttttatta 2640
   aatttaatta taatttataa aaaaaatttt tttttttttt ttattttttt ttattaaaaa 2700
   tattaaatat attaaattta atatgtttat tttataaata aaaaaaaaaa aatataaaat 2760
   taataattaa tttaataaaa aatataatta tataatatta aaatttttta ttttatttat 2820
   ttttttaatt tatttttatt tatttttaat ttaatattta ataaaataaa aatttattaa 2880
   tttttttgta tttatttatt atttaatttt ataattattt tttaatttta attaaatttt 2940
   gtaaaatatt ttaaatttat ttttaataat ttttaattat tgtttgatta tttttatttt 3000
   tttattttta tatatttatt tttattttgt taaattaatt aaattttaat taaaattaat 3060
   tttttatttt tttaaataat aaataataat tttttaaata aaattttaat aaatattaat 3120
   aaattttttt ttttttttta aaattataaa aataatattt tttatttaaa aataataaat 3180
   tttttaaatt ttttttgatt taaattaaaa aaattaattt ataaaatata aaaattatat 3240
   ttttttttga tttttttttt ttttttgtta ttatttaatt ttttaattaa ttttttttta 3300
   tttttaaatt taaatatttt aataattaat ataaatttat ttttattaat aaaataataa    3360
   aaatgatata ttaaaaaatt atatatttat ttaaaaaaaa aaaataaatt tatatttttt    3420
   aaatataaat aaaaaattta aaattttttt atttttatta aaaaaaaaaa aatttttaat    3480
   ttttaaaaat taatgttttt taaaaattaa attattttaa ttttaaattt taattttatt    3540
   ataaataaat tttataattt taaataaatt attaaatttt tttaaatttt aaatattata    3600
   tttattatat ttaaataatt aaaaaaattt aataatataa tattatataa aatatttaat    3660
   ttatattaat taaaatatta atttaaattg ataaaatata taatttaaaa ataaataaat    3720
   ttataaaaat atttttattt aaaaataata ataaattaat atttttaata ttaaaatttt    3780
   aaaatttaaa aattaaattt aatttaaata aaaattataa aattaaaaaa aaattaaatt    3840
   tataatataa aaaaaatttt aaataatttt tttatttaat attttattgt attttttttt    3900
   tatttaataa aaaattaaat tattaaaaat ttaaatttat tttttaatgt aattatttaa    3960
   tttattttga tttttttttt aataataaaa taaaaaaatt gtaaaaaaat ttttataaaa    4020
   taaattatta ttattaaaat ttttttaata ttatttttta attatataaa atttaaaaaa    4080
   taaattaaaa ataaattttt ttaaaaaaat ataaaaattt tttaaaaaaa aatttttaaa    4140
   aattataaaa aaaaatttaa attataaaaa tatataattt taaaaaaaaa aatataatat    4200
   tataaaaata ttattttttt ttaaaataat ttttaaattt aaatata                  4247
<210> 60
   <211> 4247
   <212> DNA
   <213> Artificial Sequence
<220>
   <223> chemically treated genomic DNA (Homo sapiens)
<400> 60
   tgtatttaaa tttagaaatt attttgagga gaaataatat ttttatagta ttgtattttt      60
   ttttttagga ttatgtgttt ttataattta ggtttttttt tgtagttttt agaagttttt     120
   ttttaaaaag tttttgtgtt tttttgggga ggtttatttt tagtttgttt tttgagtttt     180
   gtgtagttag aaaatggtgt tagaaaaatt ttgataatag tagtttattt tataaaagtt     240
   tttttgtgat ttttttattt tattgttggg aagagggttg aggtaggtta ggtggttgtg       300
   ttagaagatg agtttgggtt tttagtgatt tagtttttta ttaagtagag agggggtgtg     360
   gtagggtgtt gggtaggagg attgtttgag gttttttttg tgttatgagt ttgatttttt    420
   tttaatttta tagtttttgt ttgagttaag tttaattttt agattttaaa attttagtat     480
   tgaagatgtt agtttgttat tgtttttagt agaggatgtt tttataggtt tatttgtttt     540
   taagttatat gttttattga tttaaattag tgttttggtt ggtgtgggtt aggtatttta     600
   tataatattg tattgttgaa tttttttagt tatttagata taatagatat aatgtttgag    660
   gtttagagag gtttagtaat ttgtttgggg ttatagagtt tatttatggt agagttggga     720
   tttagaatta gggtagtttg atttttgaga gatgttgatt tttgagagtt agagattttt     780
   ttttttttaa tgggagtagg gagattttaa gttttttatt tgtgtttaga ggatgtaaat    840
   ttgttttttt tttttaagta gatgtgtagt tttttagtat attgtttttg ttattttgtt    900
   aataagagta ggtttgtgtt gattgttaag gtatttaggt ttagggatgg gagggggttg     960
   gttagagagt tgggtagtag tggggagagg gggagggttg ggaaggggta tggtttttat    1020
   attttatgaa ttaatttttt tggtttgggt tggggagggt ttagaggatt tattattttt    1080
   gaataagggg tgttattttt ataattttga ggaaggagga aagatttgtt gatatttatt    1140
   gagattttat ttaaaaaatt gttatttgtt atttgagaga gtagggggtt agttttggtt   1200
   agggtttaat taatttggtg aagtaaaggt aggtatgtaa aggtaggaag gtgaaggtag    1260
   ttgggtggtg gttgagagtt attgaagata aatttaagat gttttatggg gtttagttag    1320
   gattagaagg tagttatggg gttaggtagt gggtggatgt ggggaagtta gtgggttttt    1380
   gttttgttag atattaagtt ggaagtagat gaaagtgggt taagaggata gatggggtaa    1440
   ggggttttag tattgtatgg ttgtattttt tattaagttg gttattggtt ttgtattttt    1500
   ttttttttat ttataagatg ggtgtgttag atttaatgtg tttaatgttt ttagtagaaa    1560
   aaagtgagaa gagaaaggag gtttttttta taaattatag ttaggtttag tgagaagatt   1620
   agaggtttgt ttgttttgtt ttttttttaa ggtattttgt tgtttagtag gtgttttgga    1680
   aatattagtt ttttgggtgt aggatgagtg taatggattt atagattttt gttgtttaga    1740
   gaggggtagt gattagtata aggttatata gtttttaagt ttaagggttt gttggaggta    1800
   tatatttttt tttttttttt tttttttatt tttttgttgt ttgttttatt tattttgggt    1860
   gtgatgggaa ggaagtgata ttttttttta tgaaaagtta ttgattggga atattgtgtt    1920
   aattattata ataatggtaa tagtagtttt aataataatt agtagatgtt gttttgtttt    1980
   tttggttgag gttgttggtt taagtttttt taagtttgag ttggagtaaa ataaagatag    2040
   tttttgaagt ggtgaaatgg ttggtttatt tggtagtttg ggagtggtgg ttaggttgag    2100
   tttatgtgag tttgttttag tagtttgggg aggggggttt gttaagtgtt gagttggagt    2160
   tagtagtgag aagaaatgtg atgggtttgg gatttttatt ttttaatagg gtataggtgt    2220
   agtttaagtt atagtttaat tttagtattt tagtttgggg gagggtgaga gagatagggt    2280
   ttgggggtag ttgaggttat aagagttatt agagggtata gaaagggaag gaggtgtggg    2340
   ggatattgaa tgtgaggtta ttaattttta agttttattt tttttgtttt ttttattggg    2400
   ttttgggatg ttttaaaagt tttagtttta ggatgggatt gttttttttt atggagtttg    2460
   ggttatattt ggtggtgatg gatagtgaga gttttttaga tggttttgtg tgtttttatt    2520
   ttatttatgg ggtagaggtt tagaggtgtt agttgatgtt ttttaattgt agaagtagat    2580
   tattggaggt tttttttatt tttgttatat taagttttag gttttaggtg ggtttttaag    2640
   gtttggattg ggttttgagt tttgttgtgg ggtgaagttt ttgagttttg attttttatt    2700
   ttttttgtgt tttagtgttg tttgtaggtg tagtgatgtt attgtgtgtg tgggttttta    2760
   ttagaggagt ttgggtgggg taagaggtat ttgttgtttt tttttggggt ttgtggagta    2820
   ttttttattg gtttaagatt ttgtaaataa agggatattg gatttgtagt gttttgggat    2880
   tttttgggtt tttgggggtt ttgtattgtg taagttttat tatgttggtg ttgggtggag    2940
   ggtgatgtga ttttagtttg ggtgattttt agatattgtg gagggatgga gtgtgggttt    3000
   gggttggttt gtagtttttg ttgagtgtga agttgggtat tgtttgtaag tttgtagttt    3060
   atgtttttgt tttttttttt tgatgttttt tagttgttat tttgtttttt ttgtagattg    3120
   agggaagata aggggatata taagggatag gagaggagag taattaggga agtgattttt    3180
   tttttgtggg gttgtggtgg gtgggtggag agataggaga ggaaaagaga aaagggaaga    3240
   gaaggaaggg gtagggggtg agaagttggg ttttttggat tattggtgta gtttgtggtt    3300
   gaaagaggaa tagtggattt tataaagtgt attgagtaag ttttttatat ttgtgtagtt    3360
   ttttttagga aatggtggtt gtagaggaaa gggttgtgtg ttttggattt ggagtttgtg    3420
   tttggtggtg ttgtggtttt gaaatttgtg ggatgttatg gggtggaggg gtggttgttg    3480
   atagttgtta taataaatta gttgagttta gtttgaggtg gaaattgtag taggaagtgg    3540
   gatgatagag ttgtattggg tttggtatat tttaggtaag agatggtgaa aagagttttg    3600
   agataaatgt ttgggaagtt tttggttgtt aaaatttttt gttttttttt tttttgaagt    3660
   ttgtgtggat gtttagtttt attttttttg tgttggttgg ggagtgttga agttgtgggt    3720
   tgggtttgtt ttttttggtt tgtgttttga aatttatttt gggttttttt ttgttttata    3780
   tagtgtattg attgttatga gggttggagg ggatttggtg ttggtatttt gaattttttt    3840
   gtagggttgt tgagtttttg tttgggttgt gttgtagagt tgtttattta tggtttggag    3900
   aggttttgtg gtgtttgttt tgagattttt agtgtgtggt tgtttggatt ttgtgttttt    3960
   tgagatattg gtttgtggtg tggtttgttg gttatttttt tgtagttttg ttttgttttg    4020
   tttttgtttg ttttattttg ggtttttatt ggtaagtttg gttttgttgt gtggagaagg    4080
   tggggggtgg ggattgtttt tgagtttggg gatttttggg aattgtagtg tgtgtttttt    4140
   gtttttaggt ttttagattg gagtttttgg aataggtttt ttatttagag gagtggaggt    4200
   aggattttta gtaattattg ttgttttgtg ttttagtttt tttggag                  4247
<210> 61
   <211> 20
   <212> DNA
   <213> Artificial Sequence
<220>
   <223> chemically treated genomic DNA (Homo sapiens)
<400> 61
   ggagtggagg aaattgagat 20
<210> 62
   <211> 22
   <212> DNA
   <213> Artificial Sequence
<220>
   <223> chemically treated genomic DNA (Homo sapiens)
<400> 62
   ccacacaaca aatactcaaa ac 22
<210> 63
   <211> 33
   <212> DNA
   <213> Artificial Sequence
<220>
   <223> chemically treated genomic DNA (Homo sapiens)
<400> 63
   tgggtgtttg taatttttgt tttgtgttag gtt 33
<210> 64
   <211> 22
   <212> DNA
   <213> Artificial Sequence
<220>
   <223> chemically treated genomic DNA (Homo sapiens)
<400> 64
   aaccgcaacg actaacaacg aa 22
<210> 65
   <211> 21
   <212> DNA
   <213> Artificial Sequence
<220>
   <223> chemically treated genomic DNA (Homo sapiens)
<400> 65
   agggcgttta gttaattcgc g 21
<210> 66
   <211> 30
   <212> DNA
   <213> Artificial Sequence
<220>
   <223> chemically treated genomic DNA (Homo sapiens)
<400> 66
   cgcgacgaca aaacgccctt taaaaacgaa 30
<210> 67
   <211> 20
   <212> DNA
   <213> Artificial Sequence
<220>
   <223> chemically treated genomic DNA (Homo sapiens)
<400> 67
   cacgcacgat actaaacgcc 20
<210> 68
   <211> 20
   <212> DNA
   <213> Artificial Sequence
<220>
   <223> chemically treated genomic DNA (Homo sapiens)
<400> 68
   tcgatcgtgt tggttgttcg 20
<210> 69
   <211> 27
   <212> DNA
   <213> Artificial Sequence
<220>
   <223> chemically treated genomic DNA (Homo sapiens)
<400> 69
   cgaccgccta ccgcttcata ataacgt 27
<210> 70
   <211> 21
   <212> DNA
   <213> Artificial Sequence
<220>
   <223> chemically treated genomic DNA (Homo sapiens)
<400> 70
   acccgataaa aacatcgcgt a 21
<210> 71
   <211> 22
   <212> DNA
   <213> Artificial Sequence
<220>
   <223> chemically treated genomic DNA (Homo sapiens)
<400> 71
   gttcgtgcgt ttgtagttcg at 22
<210> 72
   <211> 23
   <212> DNA
   <213> Artificial Sequence
<220>
   <223> chemically treated genomic DNA (Homo sapiens)
<400> 72
   aacgcgacca acgcttcaac gac 23
<210> 73
   <211> 24
   <212> DNA
   <213> Artificial Sequence
<220>
   <223> chemically treated genomic DNA (Homo sapiens)
<400> 73
   tttttgttaa gcggtttcgg attt 24
<210> 74
   <211> 22
   <212> DNA
   <213> Artificial Sequence
<220>
   <223> chemically treated genomic DNA (Homo sapiens)
<400> 74
   ccgcctaaaa acgtctacgc aa 22
<210> 75
   <211> 29
   <212> DNA
   <213> Artificial Sequence
<220>
   <223> chemically treated genomic DNA (Homo sapiens)
<400> 75
   taaaaactcg cgcaacaccg taccgtaaa 29
<210> 76
   <211> 30
   <212> DNA
   <213> Artificial Sequence
<220>
   <223> chemically treated genomic DNA (Homo sapiens)
<400> 76
   gttagttttt ttttgtcgtt tttttttcgt 30
<210> 77
   <211> 22
   <212> DNA
   <213> Artificial Sequence
<220>
   <223> chemically treated genomic DNA (Homo sapiens)
<400> 77
   tcgcctaaac tcaactacac ga 22
<210> 78
   <211> 21
   <212> DNA
   <213> Artificial Sequence
<220>
   <223> chemically treated genomic DNA (Homo sapiens)
<400> 78
   gcgttcggga gtttcgtgag g 21
<210> 79
   <211> 4513
   <212> DNA
   <213> Homo Sapiens
<400> 79
   agtgtatggg gaaagaccaa tccaactgtc catctgtggc tgggacagcc cagggggtgt   60
   gcccacggct gacccagggg tgtgcacacg gctgagctgg gagtcccgct ggtctccctg  120
   aggactgagg gtgaacttcg ctctttgcct taaacctctt tatttcattg cagtaatagt  180
   tttacgttgt acataatagt gtaaaccttt ttaaaaagga aagtataaaa acaaaagttg  240
   taatttaaaa gtctgaataa ccatctgctg cttaggaaac tcaatgaaat gacatgcctt  300
   tttagcagga agcaaagttg gtttctgttt tttgttttct ttgttgtttt agtttataaa  360
   acatgtgcat tttacagttc cagtatcaaa tatttataat cttatgagaa atgaatgaat  420
   gtttctattt acaactgtgc ttatcaaaat tgtgaacacc cccacccccg catttttgtg  480
   tgttgaaatt cttgaaggtt acattaaata aaacaaaatc tctttattat aaaatactga  540
   aggtcccagg tggaatgatg agttttctaa gcatgtctga gtccagagga actgcctggg  600
   catcctcccc agtactcagg tgctcatggc cttcgccccg cctgggagca ggtgccaggt  660
   gcctttgtag cgagaggtga ttattatttt tttttaaata agacagtttg gatatgtcac  720
   atgtgagcac agaataacca aaagaagtac acattttgtt accaggagga agtgttcaaa  780
   actacggtgc tattccagtg actacatttt tttaaatttt gcgaagttca acaggtctcc  840
   ctccggctgg gtgctcactg gccctgccac ccgtgctgga ccgaggcgct ttcccctggg  900
   gacaggaact ttttactcca ctgagacaaa gagactctcc ttttcaaagt gtcatttttg  960
   tttacctttt gttgtttccc ccccaaaaca aaagactttg tacaacttcc taagcaggat 1020
   tgggggaggg atcagcttcg cctgtgctga ccgcgcctct ggattccgct catcaggaag 1080
   tgtttggggt ctttgagcga gtgatctacg cgttgaattg ctgtttttcc aaactttcat 1140
   ggcttaaaaa ttttttttct tctccctgta aaagaaactc agagactggt gatctgttac 1200
   cgaagacgac tttttaggcg ttttctgttt ccccaggcag ctgggccgtt ggcgttccca 1260
   ggcggaagcg gggcctctgg gctgggcctc tgccctgcca ggaagcggtc tgggggctgc 1320
   ccctcgggat tttcagaacc ctggaaggag agagctgggg tctcaccgcg gagactcccg 1380
   agttctcgtt gcctgggccc gagctgcgcc ccagggtcat cgctgatggc gcgagaccac 1440
   gcattccccg cgaaccccga cttctgaaca gttcagaaag ttcgtagatt aaaaaccaaa 1500
   caaaaaatac tggcacaact tctattgccg tattggcctc tctcttattt taataaaaat 1560
   acggtaacat gaagcgatca tttttttaaa aatctgtttt caaagtataa cactgaaaag 1620
   gggcaaggca cagctttgta aggtaatgag ttccccgtcc tcagaggtgt tcaagcagac 1680
   gctaggctta cagtggcatg gagagtgttc tttctctttg gaactggatg agtcctcaga 1740
   cccttgaaac gctgagtttt tttttttttt tttactccta tgtttattac attttaatcc 1800
   aacagcatat aatctaagct gctccctcat ggagagccgc ggctgtgcca cctgcctgct 1860
   ttcgccatgg agaccggagt caggcctgca gggaagtcgg tgcgcctttc ccaagtcctc 1920
   tgcccgctgg ttcctctcgg gcctcccggg ctctgcgtgc cgcggatctg gacccggact 1980
   gcagacaccg ccccacgact gggcccccgg ccagactgtt accccacagg aggagggttc 2040
   cctaagtgtg cagaagagga aagctcattt ataaagtcct tgctccttcg agctgaacga 2100
   cccccgcgcc cgaggcctag ctgtgtcccc gccgggccca ggctgagctt actccacact 2160
   gctccatagc cgggcagaga ctccgcgagg gtctgggagt cctggcctcg gaggacccgc 2220
   cgccctgcag gcctgcccaa gcaggcgccc ccgcgagagt gcgggacaga gagggcctgg 2280
   cggggctcct tggcgctgag agccctcctt ctccgctgca cccagctctg ccaacggggc 2340
   tggatgagtg ggggctggag tcagaatccc agggtctaca cccgccgaca gcagcaatgc 2400
   cgacccgccc tgcctttctc ctaaggagcg gagggaagga gccatgtgaa tgaccccagc 2460
   ctgaccacca agagggcgcc agcatttctg ttttcctaga gtgaaaaggt caaatcgatg 2520
   ggggctccta caccgctgga cacaggtgcg cccgggcgtc cagtgcgcgc aacacacgcc 2580
   cagacactct atatgcaaat atgcctgccg ccgcggagcc ggccgggagg cagcgtttgt 2640
   tcgctggggt tctgctagaa gtcattctgt ctcccgctgg tttaatatcc gccctctgcg 2700
   gtctccgctc gcctcggcgg ctcccgaagc gcccccggga gggtcccggc tccgccgaaa 2760
   actgcagcct gccaggctga gccgccgcgc gctgggaggt taatagcgac tgacgacaaa 2820
   gggccaaggt gcaattccta aagcggggat tcgccgggtg aggcagaaat cagcctccgg 2880
   ggagatgggt ccccccttcc cgacgcgccc ctgcacacac tgctgccccg gcgccgggcc 2940
   tccgggcggg gacccctctt ccgggcatcg cggccccatt cggcggagag ctaggccctc 3000
   tccaggcctc caagtaggga gcggacgtgg gtggtgggga agggaagccg ccggcgggac 3060
   ggagagtggg gtcccccgga ccctgggtcg ggcaggcctg gcgctggcag ctctgttgca 3120
   cagatttgtg agcgatgctg gtggccgcat tgccgcgcgg cgcgaacccc aagtagacca 3180
   ttcatgtttt tattaataat ctcaaacact tcattatatt tatgctgcct gcccagagaa 3240
   aataaaaggc cattcttgaa atcgcctgaa atgtaataat attcattaaa gcgcctccta 3300
   attatgtgat ttaatcaagc gtggtgcgcc cgcgctctcg gctctcgctg gaaaggctgg 3360
   cgatgacggt tattattact gctgttgtta gtggcctcgt gatagcagcc ggccagctct   3420
   tcagtccagg cccagtttcc tcagagagac tcggggagcc ccactttgag ggatggggtt   3480
   ccccggaacg actccgaagt cccggagacc aggcgcggcc caggccctct gcccttctcc   3540
   agccccagcc ccagccccag ccccagcccc gggcaaactc ttccaggccg agggcaggac   3600
   tttctccaga cccttggggc cctctgtgtc ccctggggac gcccgacccg gctttctagg   3660
   agggggaact ccgggtcctg agcccgggcc ccgccccgtt ggctgttccg ccccctccag   3720
   ggctgagccc cgcgcaatgc ccgaagcaga aacaaagagt cgccgagatc tgcctcctgg   3780
   gccgcctttt caccccctca gcctggaagg cgtcccagag aggccccacc cccagcctcc   3840
   acggagaccc aggagggaag aagagccggg acccggtagc cctgaggggc tgccgggcct   3900
   gcggcgcttt ctccgccgca gaggggttgg gtgggagacg caggcgggaa ggaccctgtc   3960
   ccagccgaga gtcctctgtg gggtcgagga atggtgagga tcactgtttg caccaaccgt   4020
   ctggaagaaa ccgggcggcc agccgctcga ccccatctgt cgagatgtga aacttggccg   4080
   cgcggtccgg gcgcgcgctg cctgcgcgga aggagccgcc tccctccttt cagcgaacgc   4140
   attcggggtt cccccctccc gccaagtcct ttgtctggaa ggggactggc aggctctgtc   4200
   gtcgccgtgt gcccccctcc tttccccagc ctctaccccc tccaactaac tttcctttct   4260
   tcccttctct tttcttccct ctctggatgg gtaatcacag gctccgctgc caagcgcgct   4320
   gcgaggagcc gctgcccgtt cccgtcggtg cccctccccg cggcagcctg gccgagtccg   4380
   cccagcctgc ctaggacgtc cctgacccgc gcgctcagtt tcattcagtg gccgtgggat   4440
   gttgcatctg agtttttcct ttaggccaaa actttgtccc gaggtttttc tgagagggtt   4500
   ttattctcca aca                                                      4513
<210> 80
   <211> 2280
   <212> DNA
   <213> Homo Sapiens
<400> 80
   tagtggcgag cactcgccgc tggccttcta gggcaggcag tattgccgct ccaggcgtgc 60
   ggagtcgggg agaaaaaaac cgaacacgtg tgctacccag ggcccccaga taagcccctc 120
   cgcgcgcaca cacaccctga accggagtcc agaaggccgg actcgcgtcc cggggtcgag 180
   ccccttactc tccgctccca gttcggaaga tcccagcctg cccgaaggcg gcgtctgacc 240
   cgctggtgga cctggtgggt gtctgttcct gatacctggg gacgaggcgg gagcgccttg 300
   agcgctggga agcttcgcct cgcgagtccc cggcagtgac acgtgggagc gttgggcccg 360
   gcacccccga cgcttccgag gtttcgggtg gtcgttttgc tctctcgggg gcttttattc 420
   cttaagtggc tttaaacgac cgaggatggg cctcccgcgc cgccgcagct gtcacttcct 480
   tcgggaaagg ctagcgacct tggacgcgca ggctctgaaa accaaggtcc agacccatac 540
   cgagcgccca aagaccgcat ttgaattctc ctcgccggcc attgagggag agaaaggaac 600
   gcttagttcc attcacattc acagaaagaa gcgccgaggg tgggggaaac gcagtcttgc 660
   cgggtgagcc gggacaggtt cctcgcctgc cccccggccg ctgcttcctc ttagctgaat 720
   ggggagcgac ccgccccggg cgcggccttc ggggctgaag actgaggtgc agcctcaccc 780
   ccggcctggc agcggcttgg aagagagagg gaaaggagga acatctaccc ggctaagaga 840
   cgccgccaga gtccctaaag ctggcggggg acggaggccg gggtcccgga tcttcagagc 900
   gcccgcagcc gggcttctgg gctcctcggt gcggggaacc tgaccctgta gccttcgcca 960
   gggcgcggcg gggctgcaag aagggaggaa ggagtgggac tagagccttc gagggcgcgg 1020
   gcggcgtgca ctcagccttg tctccccgcg cgccctgccg cctgcttgcc ccctttgctg 1080
   caccgggtgg ccctggtccc cgcgcccgcc cgcccccgac gccttcgcgt ggttccgcgc 1140
   tgcccgcggc cagccccgtt tcgcttcgca aggagcgtgt tgttttctca atcgaaatgg 1200
   gatcaagatc ccagggtttc ctcgtctcct tgttttgagg tgataggatc ttagccaata 1260
   gatcgaaaac gtgaaaagta cccgtcgcct cttgtcccaa gtgcctctgt ggaaggatca 1320
   tttacttgga aaagcagaat aaattctgtt gccgttttcc ttccggaggc gtgctcaccc 1380
   cagagctcct agacccactg gcattcgcta tacttcctcc tgggctttac aggtaggatt 1440
   tcaagaatga tatgggggca cctgggtgat ctgtgggttg caggtcctca agaggccgtt 1500
   ttttcgtgca cggaagtgtc gtagctttat tttagcggaa gcaatatcaa accttatctt 1560
   agaggtttgt tttgcaaaga atccagagtt tactatattt gacactattt taaaataaca 1620
   cttgcttgtt ttaagaagtt gggagaaatc gataaaagaa aagttgaaat ccatgcttcc 1680
   catttctact ctaggagata atcctgatca acagtttggt ttgctcccta cccccattcc 1740
   ttaaaaacaa acaaacaaac aaacaaacca aaacccacta tacttttaga ttgtgtagga 1800
   ttacgtagga ttttgtaaaa ataaaaatgg gattatactt cactcgtttc tgcaacggcc 1860
   tagctcctac tagtaatcat tccctatcag tatagtagaa ttaaccttat tttagtatta 1920
   tcattattat tttgagacag ggtctcactt tgtcacccag gctggagtac agtggcatga 1980
   tcttggctca ttgcagcctc tgcctcctgg gctcaagctg tcctcccgtc tcaccacccg 2040
   gccccttccc ctcaactagc tgggactaca gtcatgcgcc accaagcccg gctaattttt 2100
   gtatattttt gtggagacag gtttcaccat gttgcccagg ctgatctcaa actactgaac 2160
   tcaagggatc cacccacctt ggcctcccaa attgctggga tggcagacct gagccaccac 2220
   accggcaaat ttattcttta taatggttgc gtggtacaga atttccattt tataggtgtg 2280
<210> 81
   <211> 3640
   <212> DNA
   <213> Homo Sapiens
<400> 81
   aatttctaga aattaatttg ttacaatttt atgataccgt aacatctctt ggaagagaca   60
   tatgtggatg cttctatagc aagaccctgt gataagtaat ttttatgttt gtatcaaaat  120
   aatcagattg tcttgtgatt ggtctagtca cctagcccaa tcttattatc tcaggaggaa  180
   attaaacttc gttagtatta acatacttgt attaaaataa gaacctatac aaacctattg  240
   atagaaggta ggaatttatg attgtgcact aaatccattc acataagaaa tatttttctt  300
   tttactggac tctatgagac tgacattttt tatgtagaaa taatgtggct ttcttctccc  360
   aaagatgctt atccatctta ttcaaataga gacacaatga gagtattatt taataaatat  420
   ttgttaaatg ttttctccag cattataaga aatgcaccct ctaacaacca caacttcagt  480
   aacaccttcc ttctgttgct accctaggtt gggggccatt caattttacc aagagtgtgg  540
   aaattctgct gttttaatgg ggactgaaag gaaactagtc atttctcatt caaaccatca  600
   aggctcctag attgatttaa aatattaagt cggtgtttaa atattccaat aaatggactt  660
   tacaaagagc acaagcaatg tcacttgagg aacaaggcct caagctgagg agcaggatat  720
   tggagagcct ccttcttaat tacccacctt tattagactt gaggattttc tgtcttacaa  780
   gaaaatcaga aataaatttt attggtatgc tctgtaagaa cgatttcttc tgataacttg  840
   gggtagcagc atttgtttga ttagctgaat ttgccatgaa tgtggaaata atgacatcaa  900
   aacagctcgt gtaacaacct ccttaaagac ctcgtagatt agagatgact atagattttc  960
   cccagcgcgg atcaaaggga ctgaattgaa cgttttagct taatgatcca acccctgtca  1020
   cacccacagg gacgcgaatt ccgattttat aagcaggtgt gcacgtgcac tcagatactc  1080
   acacaaagcc gcgtggaggg acgaaaagat caaccacccg atcgacgagg ataggtttga  1140
   tcttttcgat tacctcagtg tgccagtgta tattcccggc tgggcctagc gccctaagaa  1200
   acttcggaac tttagctgtt aatttttgtt ttcttactat gacttcccaa agacattcta  1260
   tctgcctacc ggggcgaaga gaaatgggac caggcgtcag ggcggtggga ccctgtccag  1320
   ggtcctgact ccgcgccagg gctccagacc agtcggcctc cgaaggccct ttcacccacc  1380
   ccacacaaga ggaaacaaag acttctcagc tcaaggcctc agggctgcct cctgactccg  1440
   gtcagtttgc aggaagagga aacaacaaaa caaaggaacc gccaatccgc cgggtattat  1500
   atccctcagc tccaacctcc gacttcggac cgccagggtc accttctcta cgctgacccc  1560
   gcttttctta aatgaaaaca cgccaacaaa agcatacttc ggatacaaaa tccaagtacg  1620
   catctttttt ggggaggtta gagctgaggt gtacttcgga agatgagaat tttgttttca  1680
   tgaattgggt aacacccagg cattgttagg taccccgaca gaccctctag atatccttct  1740
   cttcctcctt cacactttct tcctatcaaa atagttattg ttttgaaatt cactagaaca  1800
   acgacgttct aaaaacaaag gcgcagcaag catccctttc ttcgctgccg cgggctgaac  1860
   cacggacgct cgcgggtcgc ccagccccga cggcccgcag ggggcgcgcg ccgcagccgc  1920
   agcacagccc ggctaccccc agaaagggag ccgaatggag ggaagcaggg agcgcggagg  1980
   gctcgaggct tgcagataag gagaggcgca tcctgggatt tgggtcctct gctgctacaa  2040
   cacaaccgtg ctattgttgg cactgtccga cccaagtgtc ggtggtaagc ggcgatgtcg  2100
   gggctgggtc tctagcaacc gctgtgccct gggtcaggct ggtcgcccca gctaccggga  2160
   tccctctccg gatgcttcca gggcgatagg tgctgcaccc attgacggga tagccgcata  2220
   cctccgaaca ggtagtggag ttcgtctctg gcaggcatcc tagctgcgct gacaccaagg  2280
   tcgccacaca atagccatca gcccccctta agccccagaa gtaggtttcc cctgccccag  2340
   ccatagcggt tccagtcgtc gccacgagcc cacttcttat ccccaagcgc acctccctct  2400
   cctcacccgg gtttatgccc gttacacaga gagaactaca cagggggaac tatggtccta  2460
   caccctcgag gggacagaca ccggccgtga gacaggcacc acgcagagcc ttctggtgac  2520
   tgtccgcagg agcgagacct tttttggttt tgcagtcggc taggtgtgtg tgtgtgaggg  2580
   tccccagttg actaccggga tgcactgcca cttttcggcc tggcgggctc tgggactcct  2640
   tggtctccgt aggaggccat cctaggcctt cggaggaggc gctcccagct ggcggcgccc  2700
   ctcgccccgg gctcagaggc ggacacggtc ggtcgcgccc tgctggccct ttgttcgcgc  2760
   cgcagcgggc tgggagcagc tgcgcgacac cagacccaca gcgccaagac gcgaagcgcg  2820
   aggaaaccgc tgcgcttgac tccttctccc ccaactcttg gacccaggaa cgcttccagc  2880
   tcctgcgtcc cacacgccca gtcctggctt ctctcccggc ccagaagtct tcaaggattg  2940
   aagggctctg cctagggccc cgcactcctg cctgactccc atggcccaga aaagcagggg  3000
   gacatttgaa atgtcacccc gggataaata ttaacaaaaa agcaaatgga cttgtgcagg  3060
   ggccagttat caatcaacca ggccgcaagg ccactcaggg caaacacagc ccaggttggg  3120
   ctgggcgaga ctttctcatg gccgcccccc gaccgaaccc ctcccccctt ggctcaggtc  3180
   cttcttaggt cgttctgggg caaacaccgg acgggaaggg ggcgccgcca actcccccgc  3240
   ggggtttgga ggtttcctcg cctccaagtc ccgcagggca gggtcggagt gcccaacacc  3300
   cacccccgcc cgaacctcgg gcctgcgcgc cgccttcccc tgggtccgcg gtgctgcgct  3360
   tgctgttggg tgtgtgtcgc tgtctctttc cgagccggca gctcctgctg tgtggccgaa  3420
   gccctctgga atctttaatt ggaaactaat cttggtcttg atagacgccc acgtcagagg  3480
   cgcgccaccc acccacaccc cccgcctcat cccggaggag acgcggcgag aaccctgtcg  3540
   ctctgctctt taatggggca gcagcaagta gtacatggag ggaacatata ttgattttta  3600
   aaaggtagtt aatttggtgg gaatttcctc ctgtcgccct                        3640
<210> 82
   <211> 3240
   <212> DNA
   <213> Homo Sapiens
<400> 82
   gaaactgagg cacaaggcag caatgattac tgagggtcct gcctccgctc ctctaggtga     60
   ggagcctatt ccaggggctc cagtctgaaa gcctagaggc gaggggcgcg cactacaatt    120
   cccagaggtc cccggactca agggcggtcc ccgccccccg ccttctccgc gcgacgaagc    180
   cgggcttacc aatgggagcc cggagtgggg cgagcagggg cgggacgggg cgggactgcg    240
   gggaggtggc cggcgggccg cgccgcgagc cagtatctca gagagcgcgg ggtccggaca    300
   gccgcgcgct gagggtctcg gggcgggcgc cgcgggacct ctccgggcca tgggtaagcg    360
   gctctgcggc gcggcccgga caggggctcg gcggccctgc agggaggttc aaggtgccgg    420
   cgccgggtcc cctccagccc tcgtggcagt cggtgcgctg tgtggggcgg agaggggccc    480
   ggggtgggtt tcggggcgcg ggccaagggg agcgagcccg gcccgcggct tcagcgctcc    540
   ccggccggcg cggaaggggt gaggctgggc gtccgcgcga gcttcgagaa aaggaaagac    600
   aaaaagtttt ggcggccagg ggcttcccgg gcgtttatct cgaaactctt ttcgccgtct    660
   cttgcctgaa atatgccgag cccggtacgg ctctgtcgtc ccacttcctg ctgcggtttc    720
   cacctcgagc tgggctcagc taatttgttg tgacagctgt cggcagccgc ccctccgccc    780
   cgtgacatcc cgcaggtttc agaaccgcgg cgccgccggg cacagactcc gggtccagag    840
   cgcgcggccc tttcctctgc ggccgccgtt tcctgaaagg ggctgcgcgg gtgtgaagaa    900
   cttgctcaat gcgctttgta ggatccactg ttcctctttc ggccgcgggc tgcgccgatg    960
   gtccggggag cccggcttct cgccccctgc cccttccttc tcttcccttt tctcttttcc   1020
   tctcctgtct ctccgcccac ccgccgcagc cccacaggag aggggtcgct tccctggttg   1080
   ctctcctctc ctgtcccttg tgtgtcccct tgtcttccct cggtctgcag aagggacggg   1140
   gtggcggctg gggagcgtca ggggaagagg gcaagagcgt gggctacgag cttgcaggcg   1200
   atgcccggct tcgcgctcgg cgggagctgc gggccaaccc gggcccgcgc tccgtccctc   1260
   cgcggtgtct aggggtcgcc cgggctgggg tcgcgtcacc ctccgcccag cgccggcgtg   1320
   gtggggcttg cgcggtgcag gacccccgag ggcccagggg gtcccaaggc gctgcgggtc   1380
   cagtgtccct ttgtttgcag agtcttgggc cagtaaggag tgctccgcgg gccccagggg   1440
   aggacaacag gtgcctcttg ccccacccgg gctcctctgg tgggagccca cacgcacggt   1500
   ggcgtcactg cgcctacagg caacgctaga gcacaggagg ggtgggaggt cggggctcga   1560
   gggcttcacc ccgcagcaga gctcagagcc caatccaggc cttagggacc cgcctggggc   1620
   ctagaacttg gtgtggcaga ggtagaaagg gcctccgata atctgcttct acagttgaga   1680
   gacgtcaact ggcacctctg agcctctgcc ccgtaggtga aatgggaaca cacgaaacca   1740
   tctgaggagc tctcactgtc cgtcaccacc aaatgtggcc cagactccgt ggggagggac   1800
   agtcccatcc tggaactggg gcttttagga catcccagag cccggtaaga gggacaggga   1860
   gaatggggct tggaagttgg tagcctcaca ttcagtgtcc cccgcacctc cttccctttc   1920
   tgtaccctct ggtggctctt gtggcctcag ctgcccccag accctgtctc tctcgccctc   1980
   ccccaagctg aggtactgag gttgggctgt ggcttgagct gcacctgtgc cctgttaggg   2040
   agtgggggtc ccaggcccat cgcgtttctt ctcactgctg gctccagctc aacacttggc   2100
   aggcccccct ccccaaactg ctggagcaag ctcgcgtaga ctcggcctgg ccgccgctcc   2160
   cgggctgccg agtggaccgg ccgtttcgcc gcttcaaagg ctgtctttgt tttgctccag   2220
   ctcaagctta gagaaactta agccaacaac ctcagccgag ggggcggggc ggcgtctgct   2280
   ggttgttatt gaggctgctg ttaccattat tgtggtaatt gacgcagtgt tcccagtcgg   2340
   tgacttttca tgaaaggaag tgtcacttcc ttcccatcac acccagggtg agtggggcgg   2400
   gcagcagaaa gatggggagg gagagaagga aaggggtgtg tgcctccagc agacccttaa   2460
   acttgagagc tgtgtggcct tgtactagtc actgcccctc tctgggcaac aggggtctgt   2520
   aagtccattg cgctcatcct gcacccagga ggctgatgtt tccggagcac ctgctgggca   2580
   gcgaggtgcc ttggggaggg agcaggacag gcaggcctct gatcttctca ctgagcctgg   2640
   ctgtggtttg tgggggaagc ctcctttctc ttctcacttt tttctgctga gaacattggg   2700
   cacattgagt ctagcacgcc catcttgtag atggggaaag ggagatacag aaccagtgac   2760
   caacttggta agaggtgcag ccatgcagtg ctggaacccc ttgccccatc tgtcctcttg   2820
   gcccactttc atctgcttcc agcttgatgt ctggcagggc aggggcccac tgacttcccc   2880
   gcatccaccc actgcctggc cccatagcta ccttctagtc ctggctaagc cccgtgagac   2940
   atcttaagtt tgtcttcagt gactctcaac caccgcccga ctgccttcac cttcctgcct   3000
   ttgcatacct gcctttgctt cgccaggttg gttgggccct ggccagagct ggccccctac   3060
   tctctcaggt aacaggtagc agttttttaa atggaatctc agtaggtatc aacaggtctt   3120
   tcctccttcc tcaagattat ggagataaca ccccttattc aaagatggtg ggtcctctgg   3180
   gccctccccg acccaggcca gggaagttga ttcatagagt gtgggggcca tgccccttcc   3240
<210> 83
   <211> 2155
   <212> DNA
   <213> Homo Sapiens
<400> 83
   tctcccttcc ttctcacccc tggagggtcc cagccaccag ccttgaagct gcagcgagag      60
   ccacccactg ccttggcctt tgggacccct tgagcaggag aaaaaaacca gaaaaccaaa     120
   agagagagag agacctggaa ggaagaagta atggaggagg gagacagggg gaacgagagg     180
   ggagcccaca ccacagagag agaactcctt aaaggaagcg caggcgttta cgggacagga     240
   gcgcggaaga aaagagtaga aaaggaaaag cagtttcaag caagagaaag taaggagagg     300
   cggacaggga ggggaagagc caagagaagc caagctgagg gatctgcagc aactttgaga     360
   aataaaagtg aactgtctgt gggcggctgc tgtgatctca gcaaaaagcg gagctcaagg     420
   ctcagggact ccgccgccca cactgtgccc cgccttattt tggatgcctc caacttcagc     480
   ttcaggcaca gtacacgagt aggggcagta aggcctggct agggccggtg ccgcacggga     540
   gctgcgggtc cctgcaacac ccaggtgtgc aggcgctaca agaaggagcc ggggccgctg     600
   cccccgcagc gcggacttcg ccccgtgctg cacacgcttt agaagagcgt gtccggaagt     660
   gtcctccccg gccctcctcg cttcccaaga tttgtttgca acgtctacac ccgtcaccac     720
   cgcataagca ccagggtgag gaggtctctt tgcaggaatt aatatcgatt tcagtctcct     780
   gctccttcaa agtttctctt tgacgatggc gagacttata ttctaagtca gaagaaaata     840
   aaaccttgcc tattctgggc ccatgattgc acaaaatcca aaatgctgcc ggcatgaaga     900
   tgcagctgga agactctaga aacctagcat taggaggcag agactttata gtgcccagag     960
   atgagttcct aaataaacta agaattacgc agtgggaaat taaggaggag ggtctgaggg    1020
   gccgacctca gagggacaga tagagaaatc gagagcgaaa cactcaagga agtggaacaa    1080
   atcgtcgtag ccaggcgtct ctgcctctcc ggaaacccga aatccgccca aacctcatcc    1140
   caggcgctca cggtaccagt cgcagggccc cgcactatag gggctgccac cctctgcccc    1200
   cggagtcccc acgccgagac tcatcatttg ctggctcttt cttccttttc ttcttttgtt    1260
   tttttatcct tcaaatataa gacaaaaata cacctcattt atcatcatag atatatatat    1320
   ttcactctca ccacataaat acaaaacatc caaatatcca aaacattcag aacaaagtta    1380
   gtagcctgga ttcagtgaca cctacccgcg tttgaatcgt tcattatttt cttcactatt    1440
   tgcctagaaa aaaaaaaaag atgaaaaaga gggggagagt ttccgacgta aataatttac    1500
   aataaaatta ctattttaaa aaagtgtttt tcgcttgctt cagatggatg aatacattct    1560
   agacactgcg gcacctcgca gtctgggtct gtgattttgg ttaaaatgtg ggtggccgtg    1620
   gctgtaggga cgatcgccgc tgcgcagggt aaggaaagag ggtgtcgcgg ttcgtgttct    1680
   gatttgggac cactgaaacc ctaagactgg ggaggcgagg ggagtgtctt tcctatcaat    1740
   cacacaagac gctatctgga ctccgagact actgctagag gaggcccgac cacccagcgg    1800
   cgtccctgcc tccccttccg caaagaactg ctcagaaatc cagacgtttc ctgcgtgcag    1860
   gaagggtttt gtgaatccgg gtgtttggga gaggaggcaa tgagtgctga ctcgttttcc    1920
   aaaccgagca attgtgcccg aagctacgcg cctgggaggc cgtagggtga agcgccggct    1980
   gcgcaggcta ccgccggcag ccgctttgct ctttcctgga ggagtgggaa gctgtcggcc    2040
   accgccccga acaggctcgg agaaaagatt ctgaatttcc tttgattagc ggcgttacct    2100
   tttcctttgc ttttccccca ttaaattcct gtttaaagtt acaacaccca gaacc         2155
<210> 84
   <211> 4513
   <212> DNA
   <213> Artificial Sequence
<220>
   <223> chemically treated genomic DNA (Homo sapiens)
<400> 84
   agtgtatggg gaaagattaa tttaattgtt tatttgtggt tgggatagtt tagggggtgt 60
   gtttacggtt gatttagggg tgtgtatacg gttgagttgg gagtttcgtt ggtttttttg 120
   aggattgagg gtgaatttcg ttttttgttt taaatttttt tattttattg tagtaatagt 180
   tttacgttgt atataatagt gtaaattttt ttaaaaagga aagtataaaa ataaaagttg 240
   taatttaaaa gtttgaataa ttatttgttg tttaggaaat ttaatgaaat gatatgtttt 300
   tttagtagga agtaaagttg gtttttgttt tttgtttttt ttgttgtttt agtttataaa 360
   atatgtgtat tttatagttt tagtattaaa tatttataat tttatgagaa atgaatgaat 420
   gtttttattt ataattgtgt ttattaaaat tgtgaatatt tttattttcg tatttttgtg 480
   tgttgaaatt tttgaaggtt atattaaata aaataaaatt tttttattat aaaatattga 540
   aggttttagg tggaatgatg agttttttaa gtatgtttga gtttagagga attgtttggg 600
   tatttttttt agtatttagg tgtttatggt tttcgtttcg tttgggagta ggtgttaggt 660
   gtttttgtag cgagaggtga ttattatttt tttttaaata agatagtttg gatatgttat 720
   atgtgagtat agaataatta aaagaagtat atattttgtt attaggagga agtgtttaaa 780
   attacggtgt tattttagtg attatatttt tttaaatttt gcgaagttta ataggttttt 840
   tttcggttgg gtgtttattg gttttgttat tcgtgttgga tcgaggcgtt tttttttggg 900
   gataggaatt ttttatttta ttgagataaa gagatttttt tttttaaagt gttatttttg 960
   tttatttttt gttgtttttt ttttaaaata aaagattttg tataattttt taagtaggat 1020
   tgggggaggg attagtttcg tttgtgttga tcgcgttttt ggatttcgtt tattaggaag 1080
   tgtttggggt ttttgagcga gtgatttacg cgttgaattg ttgttttttt aaatttttat 1140
   ggtttaaaaa tttttttttt tttttttgta aaagaaattt agagattggt gatttgttat 1200
   cgaagacgat tttttaggcg ttttttgttt ttttaggtag ttgggtcgtt ggcgttttta 1260
   ggcggaagcg gggtttttgg gttgggtttt tgttttgtta ggaagcggtt tgggggttgt 1320
   ttttcgggat ttttagaatt ttggaaggag agagttgggg ttttatcgcg gagattttcg 1380
   agttttcgtt gtttgggttc gagttgcgtt ttagggttat cgttgatggc gcgagattac 1440
   gtatttttcg cgaatttcga tttttgaata gtttagaaag ttcgtagatt aaaaattaaa 1500
   taaaaaatat tggtataatt tttattgtcg tattggtttt ttttttattt taataaaaat 1560
   acggtaatat gaagcgatta tttttttaaa aatttgtttt taaagtataa tattgaaaag 1620
   gggtaaggta tagttttgta aggtaatgag tttttcgttt ttagaggtgt ttaagtagac 1680
   gttaggttta tagtggtatg gagagtgttt tttttttttg gaattggatg agtttttaga 1740
   tttttgaaac gttgagtttt tttttttttt tttattttta tgtttattat attttaattt 1800
   aatagtatat aatttaagtt gtttttttat ggagagtcgc ggttgtgtta tttgtttgtt 1860
   ttcgttatgg agatcggagt taggtttgta gggaagtcgg tgcgtttttt ttaagttttt 1920
   tgttcgttgg tttttttcgg gtttttcggg ttttgcgtgt cgcggatttg gattcggatt 1980
   gtagatatcg ttttacgatt gggttttcgg ttagattgtt attttatagg aggagggttt 2040
   tttaagtgtg tagaagagga aagtttattt ataaagtttt tgttttttcg agttgaacga 2100
   ttttcgcgtt cgaggtttag ttgtgttttc gtcgggttta ggttgagttt attttatatt 2160
   gttttatagt cgggtagaga tttcgcgagg gtttgggagt tttggtttcg gaggattcgt 2220
   cgttttgtag gtttgtttaa gtaggcgttt tcgcgagagt gcgggataga gagggtttgg 2280
   cggggttttt tggcgttgag agtttttttt tttcgttgta tttagttttg ttaacggggt 2340
   tggatgagtg ggggttggag ttagaatttt agggtttata ttcgtcgata gtagtaatgt 2400
   cgattcgttt tgtttttttt ttaaggagcg gagggaagga gttatgtgaa tgattttagt 2460
   ttgattatta agagggcgtt agtatttttg tttttttaga gtgaaaaggt taaatcgatg 2520
   ggggttttta tatcgttgga tataggtgcg ttcgggcgtt tagtgcgcgt aatatacgtt 2580
   tagatatttt atatgtaaat atgtttgtcg tcgcggagtc ggtcgggagg tagcgtttgt 2640
   tcgttggggt tttgttagaa gttattttgt ttttcgttgg tttaatattc gttttttgcg 2700
   gttttcgttc gtttcggcgg ttttcgaagc gttttcggga gggtttcggt ttcgtcgaaa 2760
   attgtagttt gttaggttga gtcgtcgcgc gttgggaggt taatagcgat tgacgataaa 2820
   gggttaaggt gtaattttta aagcggggat tcgtcgggtg aggtagaaat tagttttcgg 2880
   ggagatgggt tttttttttt cgacgcgttt ttgtatatat tgttgtttcg gcgtcgggtt 2940
   ttcgggcggg gatttttttt tcgggtatcg cggttttatt cggcggagag ttaggttttt 3000
   tttaggtttt taagtaggga gcggacgtgg gtggtgggga agggaagtcg tcggcgggac 3060
   ggagagtggg gtttttcgga ttttgggtcg ggtaggtttg gcgttggtag ttttgttgta 3120
   tagatttgtg agcgatgttg gtggtcgtat tgtcgcgcgg cgcgaatttt aagtagatta 3180
   tttatgtttt tattaataat tttaaatatt ttattatatt tatgttgttt gtttagagaa 3240
   aataaaaggt tatttttgaa atcgtttgaa atgtaataat atttattaaa gcgtttttta 3300
   attatgtgat ttaattaagc gtggtgcgtt cgcgttttcg gttttcgttg gaaaggttgg 3360
   cgatgacggt tattattatt gttgttgtta gtggtttcgt gatagtagtc ggttagtttt 3420
   ttagtttagg tttagttttt ttagagagat tcggggagtt ttattttgag ggatggggtt 3480
   tttcggaacg atttcgaagt ttcggagatt aggcgcggtt taggtttttt gttttttttt 3540
   agttttagtt ttagttttag ttttagtttc gggtaaattt ttttaggtcg agggtaggat 3600
   tttttttaga tttttggggt tttttgtgtt ttttggggac gttcgattcg gttttttagg 3660
   agggggaatt tcgggttttg agttcgggtt tcgtttcgtt ggttgtttcg ttttttttag 3720
   ggttgagttt cgcgtaatgt tcgaagtaga aataaagagt cgtcgagatt tgttttttgg 3780
   gtcgtttttt tattttttta gtttggaagg cgttttagag aggttttatt tttagttttt 3840
   acggagattt aggagggaag aagagtcggg attcggtagt tttgaggggt tgtcgggttt 3900
   gcggcgtttt tttcgtcgta gaggggttgg gtgggagacg taggcgggaa ggattttgtt 3960
   ttagtcgaga gttttttgtg gggtcgagga atggtgagga ttattgtttg tattaatcgt 4020
   ttggaagaaa tcgggcggtt agtcgttcga ttttatttgt cgagatgtga aatttggtcg 4080
   cgcggttcgg gcgcgcgttg tttgcgcgga aggagtcgtt tttttttttt tagcgaacgt 4140
   attcggggtt tttttttttc gttaagtttt ttgtttggaa ggggattggt aggttttgtc 4200
   gtcgtcgtgt gttttttttt tttttttagt ttttattttt tttaattaat tttttttttt 4260
   tttttttttt tttttttttt ttttggatgg gtaattatag gtttcgttgt taagcgcgtt 4320
   gcgaggagtc gttgttcgtt ttcgtcggtg ttttttttcg cggtagtttg gtcgagttcg 4380
   tttagtttgt ttaggacgtt tttgattcgc gcgtttagtt ttatttagtg gtcgtgggat 4440
   gttgtatttg agtttttttt ttaggttaaa attttgtttc gaggtttttt tgagagggtt 4500
   ttatttttta ata 4513
<210> 85
   <211> 4513
   <212> DNA
   <213> Artificial Sequence
<220>
   <223> chemically treated genomic DNA (Homo sapiens)
<400> 85
   tgttggagaa taaaattttt ttagaaaaat ttcgggataa agttttggtt taaaggaaaa      60
   atttagatgt aatattttac ggttattgaa tgaaattgag cgcgcgggtt agggacgttt     120
   taggtaggtt gggcggattc ggttaggttg tcgcggggag gggtatcgac gggaacgggt     180
   agcggttttt cgtagcgcgt ttggtagcgg agtttgtgat tatttattta gagagggaag     240
   aaaagagaag ggaagaaagg aaagttagtt ggagggggta gaggttgggg aaaggagggg     300
   ggtatacggc gacgatagag tttgttagtt tttttttaga taaaggattt ggcgggaggg     360
   gggaatttcg aatgcgttcg ttgaaaggag ggaggcggtt tttttcgcgt aggtagcgcg     420
   cgttcggatc gcgcggttaa gttttatatt tcgatagatg gggtcgagcg gttggtcgtt     480
   cggttttttt tagacggttg gtgtaaatag tgatttttat tatttttcga ttttatagag     540
   gattttcggt tgggataggg tttttttcgt ttgcgttttt tatttaattt ttttgcggcg     600
   gagaaagcgt cgtaggttcg gtagtttttt agggttatcg ggtttcggtt tttttttttt     660
   tttgggtttt cgtggaggtt gggggtgggg tttttttggg acgtttttta ggttgagggg     720
   gtgaaaaggc ggtttaggag gtagatttcg gcgatttttt gtttttgttt cgggtattgc     780
   gcggggttta gttttggagg gggcggaata gttaacgggg cggggttcgg gtttaggatt     840
   cggagttttt ttttttagaa agtcgggtcg ggcgttttta ggggatatag agggttttaa     900
   gggtttggag aaagttttgt tttcggtttg gaagagtttg ttcggggttg gggttggggt     960
   tggggttggg gttggagaag ggtagagggt ttgggtcgcg tttggttttc gggatttcgg    1020
   agtcgtttcg gggaatttta ttttttaaag tggggttttt cgagtttttt tgaggaaatt    1080
   gggtttggat tgaagagttg gtcggttgtt attacgaggt tattaataat agtagtaata    1140
   ataatcgtta tcgttagttt ttttagcgag agtcgagagc gcgggcgtat tacgtttgat    1200
   taaattatat aattaggagg cgttttaatg aatattatta tattttaggc gattttaaga    1260
   atggtttttt attttttttg ggtaggtagt ataaatataa tgaagtgttt gagattatta    1320
   ataaaaatat gaatggttta tttggggttc gcgtcgcgcg gtaatgcggt tattagtatc    1380
   gtttataaat ttgtgtaata gagttgttag cgttaggttt gttcgattta gggttcgggg    1440
   gattttattt ttcgtttcgt cggcggtttt ttttttttat tatttacgtt cgttttttat    1500
   ttggaggttt ggagagggtt tagtttttcg tcgaatgggg tcgcgatgtt cggaagaggg    1560
   gttttcgttc ggaggttcgg cgtcggggta gtagtgtgtg taggggcgcg tcgggaaggg    1620
   gggatttatt ttttcggagg ttgatttttg ttttattcgg cgaattttcg ttttaggaat    1680
   tgtattttgg ttttttgtcg ttagtcgtta ttaatttttt agcgcgcggc ggtttagttt    1740
   ggtaggttgt agttttcggc ggagtcggga ttttttcggg ggcgtttcgg gagtcgtcga    1800
   ggcgagcgga gatcgtagag ggcggatatt aaattagcgg gagatagaat gatttttagt    1860
   agaattttag cgaataaacg ttgtttttcg gtcggtttcg cggcggtagg tatatttgta    1920
   tatagagtgt ttgggcgtgt gttgcgcgta ttggacgttc gggcgtattt gtgtttagcg    1980
   gtgtaggagt ttttatcgat ttgatttttt tattttagga aaatagaaat gttggcgttt    2040
   ttttggtggt taggttgggg ttatttatat ggtttttttt tttcgttttt taggagaaag    2100
   gtagggcggg tcggtattgt tgttgtcggc gggtgtagat tttgggattt tgattttagt    2160
   ttttatttat ttagtttcgt tggtagagtt gggtgtagcg gagaaggagg gtttttagcg    2220
   ttaaggagtt tcgttaggtt ttttttgttt cgtatttcgc cgggggcgtt tgtttgggta    2280
   ggtttgtagg gcggcgggtt tttcgaggtt aggattttta gattttcgcg gagtttttgt    2340
   tcggttatgg agtagtgtgg agtaagttta gtttgggttc ggcggggata tagttaggtt    2400
   tcgggcgcgg gggtcgttta gttcgaagga gtaaggattt tataaatgag tttttttttt    2460
   ttgtatattt agggaatttt ttttttgtgg ggtaatagtt tggtcggggg tttagtcgtg    2520
   gggcggtgtt tgtagttcgg gtttagattc gcggtacgta gagttcggga ggttcgagag    2580
   gaattagcgg gtagaggatt tgggaaaggc gtatcgattt ttttgtaggt ttgatttcgg    2640
   tttttatggc gaaagtaggt aggtggtata gtcgcggttt tttatgaggg agtagtttag    2700
   attatatgtt gttggattaa aatgtaataa atataggagt aaaaaaaaaa aaaaaaattt    2760
   agcgttttaa gggtttgagg atttatttag ttttaaagag aaagaatatt ttttatgtta    2820
   ttgtaagttt agcgtttgtt tgaatatttt tgaggacggg gaatttatta ttttataaag    2880
   ttgtgttttg ttttttttta gtgttatatt ttgaaaatag atttttaaaa aaatgatcgt    2940
   tttatgttat cgtattttta ttaaaataag agagaggtta atacggtaat agaagttgtg    3000
   ttagtatttt ttgtttggtt tttaatttac gaattttttg aattgtttag aagtcggggt     3060
   tcgcggggaa tgcgtggttt cgcgttatta gcgatgattt tggggcgtag ttcgggttta    3120
   ggtaacgaga attcgggagt tttcgcggtg agattttagt tttttttttt tagggttttg    3180
   aaaatttcga ggggtagttt ttagatcgtt ttttggtagg gtagaggttt agtttagagg    3240
   tttcgttttc gtttgggaac gttaacggtt tagttgtttg gggaaataga aaacgtttaa    3300
   aaagtcgttt tcggtaatag attattagtt tttgagtttt ttttataggg agaagaaaaa    3360
   aaatttttaa gttatgaaag tttggaaaaa tagtaattta acgcgtagat tattcgttta    3420
   aagattttaa atattttttg atgagcggaa tttagaggcg cggttagtat aggcgaagtt     3480
   gatttttttt ttaattttgt ttaggaagtt gtataaagtt ttttgttttg ggggggaaat     3540
   aataaaaggt aaataaaaat gatattttga aaaggagagt ttttttgttt tagtggagta   3600
   aaaagttttt gtttttaggg gaaagcgttt cggtttagta cgggtggtag ggttagtgag    3660
   tatttagtcg gagggagatt tgttgaattt cgtaaaattt aaaaaaatgt agttattgga    3720
   atagtatcgt agttttgaat attttttttt ggtaataaaa tgtgtatttt ttttggttat    3780
   tttgtgttta tatgtgatat atttaaattg ttttatttaa aaaaaaataa taattatttt    3840
   tcgttataaa ggtatttggt atttgttttt aggcggggcg aaggttatga gtatttgagt    3900
   attggggagg atgtttaggt agtttttttg gatttagata tgtttagaaa atttattatt    3960
   ttatttggga tttttagtat tttataataa agagattttg ttttatttaa tgtaattttt    4020
   aagaatttta atatataaaa atgcgggggt gggggtgttt ataattttga taagtatagt     4080
   tgtaaataga aatatttatt tattttttat aagattataa atatttgata ttggaattgt    4140
   aaaatgtata tgttttataa attaaaataa taaagaaaat aaaaaataga aattaatttt    4200
   gttttttgtt aaaaaggtat gttattttat tgagtttttt aagtagtaga tggttattta   4260
   gatttttaaa ttataatttt tgtttttata tttttttttt taaaaaggtt tatattatta    4320
   tgtataacgt aaaattatta ttgtaatgaa ataaagaggt ttaaggtaaa gagcgaagtt    4380
   tatttttagt ttttagggag attagcggga tttttagttt agtcgtgtgt atatttttgg    4440
   gttagtcgtg ggtatatttt ttgggttgtt ttagttatag atggatagtt ggattggttt    4500
   ttttttatat att                                                      4513
<210> 86
   <211> 2280
   <212> DNA
   <213> Artificial Sequence
<220>
   <223> chemically treated genomic DNA (Homo sapiens)
<400> 86
   tagtggcgag tattcgtcgt tggtttttta gggtaggtag tattgtcgtt ttaggcgtgc 60
   ggagtcgggg agaaaaaaat cgaatacgtg tgttatttag ggtttttaga taagtttttt 120
   cgcgcgtata tatattttga atcggagttt agaaggtcgg attcgcgttt cggggtcgag 180
   ttttttattt ttcgttttta gttcggaaga ttttagtttg ttcgaaggcg gcgtttgatt 240
   cgttggtgga tttggtgggt gtttgttttt gatatttggg gacgaggcgg gagcgttttg 300
   agcgttggga agtttcgttt cgcgagtttt cggtagtgat acgtgggagc gttgggttcg 360
   gtattttcga cgttttcgag gtttcgggtg gtcgttttgt tttttcgggg gtttttattt 420
   tttaagtggt tttaaacgat cgaggatggg tttttcgcgt cgtcgtagtt gttatttttt 480
   tcgggaaagg ttagcgattt tggacgcgta ggttttgaaa attaaggttt agatttatat 540
   cgagcgttta aagatcgtat ttgaattttt ttcgtcggtt attgagggag agaaaggaac 600
   gtttagtttt atttatattt atagaaagaa gcgtcgaggg tgggggaaac gtagttttgt 660
   cgggtgagtc gggataggtt tttcgtttgt ttttcggtcg ttgttttttt ttagttgaat 720
   ggggagcgat tcgtttcggg cgcggttttc ggggttgaag attgaggtgt agttttattt 780
   tcggtttggt agcggtttgg aagagagagg gaaaggagga atatttattc ggttaagaga 840
   cgtcgttaga gtttttaaag ttggcggggg acggaggtcg gggtttcgga tttttagagc 900
   gttcgtagtc gggtttttgg gtttttcggt gcggggaatt tgattttgta gttttcgtta 960
   gggcgcggcg gggttgtaag aagggaggaa ggagtgggat tagagttttc gagggcgcgg 1020
   gcggcgtgta tttagttttg ttttttcgcg cgttttgtcg tttgtttgtt ttttttgttg 1080
   tatcgggtgg ttttggtttt cgcgttcgtt cgttttcgac gttttcgcgt ggtttcgcgt 1140
   tgttcgcggt tagtttcgtt tcgtttcgta aggagcgtgt tgttttttta atcgaaatgg 1200
   gattaagatt ttagggtttt ttcgtttttt tgttttgagg tgataggatt ttagttaata 1260
   gatcgaaaac gtgaaaagta ttcgtcgttt tttgttttaa gtgtttttgt ggaaggatta 1320
   tttatttgga aaagtagaat aaattttgtt gtcgtttttt tttcggaggc gtgtttattt 1380
   tagagttttt agatttattg gtattcgtta tatttttttt tgggttttat aggtaggatt 1440
   ttaagaatga tatgggggta tttgggtgat ttgtgggttg taggttttta agaggtcgtt 1500
   ttttcgtgta cggaagtgtc gtagttttat tttagcggaa gtaatattaa attttatttt 1560
   agaggtttgt tttgtaaaga atttagagtt tattatattt gatattattt taaaataata 1620
   tttgtttgtt ttaagaagtt gggagaaatc gataaaagaa aagttgaaat ttatgttttt 1680
   tatttttatt ttaggagata attttgatta atagtttggt ttgtttttta tttttatttt 1740
   ttaaaaataa ataaataaat aaataaatta aaatttatta tatttttaga ttgtgtagga 1800
   ttacgtagga ttttgtaaaa ataaaaatgg gattatattt tattcgtttt tgtaacggtt 1860
   tagtttttat tagtaattat tttttattag tatagtagaa ttaattttat tttagtatta 1920
   ttattattat tttgagatag ggttttattt tgttatttag gttggagtat agtggtatga 1980
   ttttggttta ttgtagtttt tgttttttgg gtttaagttg ttttttcgtt ttattattcg 2040
   gttttttttt tttaattagt tgggattata gttatgcgtt attaagttcg gttaattttt 2100
   gtatattttt gtggagatag gttttattat gttgtttagg ttgattttaa attattgaat 2160
   ttaagggatt tatttatttt ggttttttaa attgttggga tggtagattt gagttattat 2220
   atcggtaaat ttatttttta taatggttgc gtggtataga atttttattt tataggtgtg 2280
<210> 87
   <211> 2280
   <212> DNA
   <213> Artificial Sequence
<220>
   <223> chemically treated genomic DNA (Homo sapiens)
<400> 87
<210> 88
   <211> 3640
   <212> DNA
   <213> Artificial Sequence
<220>
   <223> chemically treated genomic DNA (Homo sapiens)
<400> 88
   aatttttaga aattaatttg ttataatttt atgatatcgt aatatttttt ggaagagata 60
   tatgtggatg tttttatagt aagattttgt gataagtaat ttttatgttt gtattaaaat 120
   aattagattg ttttgtgatt ggtttagtta tttagtttaa ttttattatt ttaggaggaa 180
   attaaatttc gttagtatta atatatttgt attaaaataa gaatttatat aaatttattg 240
   atagaaggta ggaatttatg attgtgtatt aaatttattt atataagaaa tatttttttt 300
   tttattggat tttatgagat tgatattttt tatgtagaaa taatgtggtt tttttttttt 360
   aaagatgttt atttatttta tttaaataga gatataatga gagtattatt taataaatat 420
   ttgttaaatg ttttttttag tattataaga aatgtatttt ttaataatta taattttagt 480
   aatatttttt ttttgttgtt attttaggtt gggggttatt taattttatt aagagtgtgg 540
   aaattttgtt gttttaatgg ggattgaaag gaaattagtt atttttattt taaattatta 600
   aggtttttag attgatttaa aatattaagt cggtgtttaa atattttaat aaatggattt 660
   tataaagagt ataagtaatg ttatttgagg aataaggttt taagttgagg agtaggatat 720
   tggagagttt tttttttaat tatttatttt tattagattt gaggattttt tgttttataa 780
   gaaaattaga aataaatttt attggtatgt tttgtaagaa cgattttttt tgataatttg 840
   gggtagtagt atttgtttga ttagttgaat ttgttatgaa tgtggaaata atgatattaa 900
   aatagttcgt gtaataattt ttttaaagat ttcgtagatt agagatgatt atagattttt 960
   tttagcgcgg attaaaggga ttgaattgaa cgttttagtt taatgattta atttttgtta 1020
   tatttatagg gacgcgaatt tcgattttat aagtaggtgt gtacgtgtat ttagatattt 1080
   atataaagtc gcgtggaggg acgaaaagat taattattcg atcgacgagg ataggtttga 1140
   ttttttcgat tattttagtg tgttagtgta tattttcggt tgggtttagc gttttaagaa 1200
   atttcggaat tttagttgtt aatttttgtt tttttattat gattttttaa agatatttta 1260
   tttgtttatc ggggcgaaga gaaatgggat taggcgttag ggcggtggga ttttgtttag 1320
   ggttttgatt tcgcgttagg gttttagatt agtcggtttt cgaaggtttt tttatttatt 1380
   ttatataaga ggaaataaag attttttagt ttaaggtttt agggttgttt tttgatttcg 1440
   gttagtttgt aggaagagga aataataaaa taaaggaatc gttaattcgt cgggtattat 1500
   attttttagt tttaattttc gatttcggat cgttagggtt atttttttta cgttgatttc 1560
   gtttttttta aatgaaaata cgttaataaa agtatatttc ggatataaaa tttaagtacg 1620
   tatttttttt ggggaggtta gagttgaggt gtatttcgga agatgagaat tttgttttta 1680
   tgaattgggt aatatttagg tattgttagg tatttcgata gattttttag atattttttt 1740
   tttttttttt tatatttttt ttttattaaa atagttattg ttttgaaatt tattagaata 1800
   acgacgtttt aaaaataaag gcgtagtaag tatttttttt ttcgttgtcg cgggttgaat 1860
   tacggacgtt cgcgggtcgt ttagtttcga cggttcgtag ggggcgcgcg tcgtagtcgt 1920
   agtatagttc ggttattttt agaaagggag tcgaatggag ggaagtaggg agcgcggagg 1980
   gttcgaggtt tgtagataag gagaggcgta ttttgggatt tgggtttttt gttgttataa 2040
   tataatcgtg ttattgttgg tattgttcga tttaagtgtc ggtggtaagc ggcgatgtcg 2100
   gggttgggtt tttagtaatc gttgtgtttt gggttaggtt ggtcgtttta gttatcggga 2160
   ttttttttcg gatgttttta gggcgatagg tgttgtattt attgacggga tagtcgtata 2220
   ttttcgaata ggtagtggag ttcgtttttg gtaggtattt tagttgcgtt gatattaagg 2280
   tcgttatata atagttatta gtttttttta agttttagaa gtaggttttt tttgttttag 2340
   ttatagcggt tttagtcgtc gttacgagtt tattttttat ttttaagcgt attttttttt 2400
   ttttattcgg gtttatgttc gttatataga gagaattata tagggggaat tatggtttta 2460
   tattttcgag gggatagata tcggtcgtga gataggtatt acgtagagtt ttttggtgat 2520
   tgttcgtagg agcgagattt tttttggttt tgtagtcggt taggtgtgtg tgtgtgaggg 2580
   tttttagttg attatcggga tgtattgtta tttttcggtt tggcgggttt tgggattttt 2640
   tggttttcgt aggaggttat tttaggtttt cggaggaggc gtttttagtt ggcggcgttt 2700
   ttcgtttcgg gtttagaggc ggatacggtc ggtcgcgttt tgttggtttt ttgttcgcgt 2760
   cgtagcgggt tgggagtagt tgcgcgatat tagatttata gcgttaagac gcgaagcgcg 2820
   aggaaatcgt tgcgtttgat tttttttttt ttaatttttg gatttaggaa cgtttttagt 2880
   ttttgcgttt tatacgttta gttttggttt ttttttcggt ttagaagttt ttaaggattg 2940
   aagggttttg tttagggttt cgtatttttg tttgattttt atggtttaga aaagtagggg 3000
   gatatttgaa atgttatttc gggataaata ttaataaaaa agtaaatgga tttgtgtagg 3060
   ggttagttat taattaatta ggtcgtaagg ttatttaggg taaatatagt ttaggttggg 3120
   ttgggcgaga tttttttatg gtcgtttttc gatcgaattt tttttttttt ggtttaggtt 3180
   ttttttaggt cgttttgggg taaatatcgg acgggaaggg ggcgtcgtta attttttcgc 3240
   ggggtttgga ggttttttcg tttttaagtt tcgtagggta gggtcggagt gtttaatatt 3300
   tattttcgtt cgaatttcgg gtttgcgcgt cgtttttttt tgggttcgcg gtgttgcgtt 3360
   tgttgttggg tgtgtgtcgt tgtttttttt cgagtcggta gtttttgttg tgtggtcgaa 3420
   gttttttgga atttttaatt ggaaattaat tttggttttg atagacgttt acgttagagg 3480
   cgcgttattt atttatattt ttcgttttat ttcggaggag acgcggcgag aattttgtcg 3540
   ttttgttttt taatggggta gtagtaagta gtatatggag ggaatatata ttgattttta 3600
   aaaggtagtt aatttggtgg gaattttttt ttgtcgtttt 3640
<210> 89
   <211> 3640
   <212> DNA
   <213> Artificial Sequence
<220>
   <223> chemically treated genomic DNA (Homo sapiens)
<400> 89
   agggcgatag gaggaaattt ttattaaatt aattattttt taaaaattaa tatatgtttt      60
   ttttatgtat tatttgttgt tgttttatta aagagtagag cgatagggtt ttcgtcgcgt     120
   ttttttcggg atgaggcggg gggtgtgggt gggtggcgcg tttttgacgt gggcgtttat     180
   taagattaag attagttttt aattaaagat tttagagggt ttcggttata tagtaggagt     240
   tgtcggttcg gaaagagata gcgatatata tttaatagta agcgtagtat cgcggattta     300
   ggggaaggcg gcgcgtaggt tcgaggttcg ggcgggggtg ggtgttgggt atttcgattt     360
   tgttttgcgg gatttggagg cgaggaaatt tttaaatttc gcgggggagt tggcggcgtt     420
   tttttttcgt tcggtgtttg ttttagaacg atttaagaag gatttgagtt aaggggggag     480
   gggttcggtc ggggggcggt tatgagaaag tttcgtttag tttaatttgg gttgtgtttg     540
   ttttgagtgg ttttgcggtt tggttgattg ataattggtt tttgtataag tttatttgtt     600
   tttttgttaa tatttatttc ggggtgatat tttaaatgtt tttttgtttt tttgggttat     660
   gggagttagg taggagtgcg gggttttagg tagagttttt taatttttga agatttttgg     720
   gtcgggagag aagttaggat tgggcgtgtg ggacgtagga gttggaagcg tttttgggtt     780
   taagagttgg gggagaagga gttaagcgta gcggtttttt cgcgtttcgc gttttggcgt     840
   tgtgggtttg gtgtcgcgta gttgttttta gttcgttgcg gcgcgaataa agggttagta     900
   gggcgcgatc gatcgtgttc gtttttgagt tcggggcgag gggcgtcgtt agttgggagc     960
   gtttttttcg aaggtttagg atggtttttt acggagatta aggagtttta gagttcgtta    1020
   ggtcgaaaag tggtagtgta tttcggtagt taattgggga tttttatata tatatattta    1080
   gtcgattgta aaattaaaaa aggtttcgtt tttgcggata gttattagaa ggttttgcgt    1140
   ggtgtttgtt ttacggtcgg tgtttgtttt ttcgagggtg taggattata gttttttttg    1200
   tgtagttttt tttgtgtaac gggtataaat tcgggtgagg agagggaggt gcgtttgggg    1260
   ataagaagtg ggttcgtggc gacgattgga atcgttatgg ttggggtagg ggaaatttat    1320
   ttttggggtt taaggggggt tgatggttat tgtgtggcga ttttggtgtt agcgtagtta    1380
   ggatgtttgt tagagacgaa ttttattatt tgttcggagg tatgcggtta tttcgttaat    1440
   gggtgtagta tttatcgttt tggaagtatt cggagaggga tttcggtagt tggggcgatt    1500
   agtttgattt agggtatagc ggttgttaga gatttagttt cgatatcgtc gtttattatc    1560
   gatatttggg tcggatagtg ttaataatag tacggttgtg ttgtagtagt agaggattta    1620
   aattttagga tgcgtttttt tttatttgta agtttcgagt ttttcgcgtt ttttgttttt    1680
   ttttattcgg tttttttttt gggggtagtc gggttgtgtt gcggttgcgg cgcgcgtttt    1740
   ttgcgggtcg tcggggttgg gcgattcgcg agcgttcgtg gtttagttcg cggtagcgaa    1800
   gaaagggatg tttgttgcgt ttttgttttt agaacgtcgt tgttttagtg aattttaaaa    1860
   taataattat tttgatagga agaaagtgtg aaggaggaag agaaggatat ttagagggtt    1920
   tgtcggggta tttaataatg tttgggtgtt atttaattta tgaaaataaa atttttattt    1980
   ttcgaagtat atttagtttt taattttttt aaaaaagatg cgtatttgga ttttgtattc    2040
   gaagtatgtt tttgttggcg tgtttttatt taagaaaagc ggggttagcg tagagaaggt 2100
   gattttggcg gttcgaagtc ggaggttgga gttgagggat ataatattcg gcggattggc 2160
   ggtttttttg ttttgttgtt tttttttttt gtaaattgat cggagttagg aggtagtttt 2220
   gaggttttga gttgagaagt ttttgttttt ttttgtgtgg ggtgggtgaa agggttttcg 2280
   gaggtcgatt ggtttggagt tttggcgcgg agttaggatt ttggataggg ttttatcgtt 2340
   ttgacgtttg gttttatttt ttttcgtttc ggtaggtaga tagaatgttt ttgggaagtt 2400
   atagtaagaa aataaaaatt aatagttaaa gtttcgaagt tttttagggc gttaggttta 2460
   gtcgggaata tatattggta tattgaggta atcgaaaaga ttaaatttat tttcgtcgat 2520
   cgggtggttg attttttcgt ttttttacgc ggttttgtgt gagtatttga gtgtacgtgt 2580
   atatttgttt ataaaatcgg aattcgcgtt tttgtgggtg tgataggggt tggattatta 2640
   agttaaaacg tttaatttag tttttttgat tcgcgttggg gaaaatttat agttattttt 2700
   aatttacgag gtttttaagg aggttgttat acgagttgtt ttgatgttat tatttttata 2760
   tttatggtaa atttagttaa ttaaataaat gttgttattt taagttatta gaagaaatcg 2820
   tttttataga gtatattaat aaaatttatt tttgattttt ttgtaagata gaaaattttt 2880
   aagtttaata aaggtgggta attaagaagg aggtttttta atattttgtt ttttagtttg 2940
   aggttttgtt ttttaagtga tattgtttgt gttttttgta aagtttattt attggaatat 3000
   ttaaatatcg atttaatatt ttaaattaat ttaggagttt tgatggtttg aatgagaaat 3060
   gattagtttt tttttagttt ttattaaaat agtagaattt ttatattttt ggtaaaattg 3120
   aatggttttt aatttagggt agtaatagaa ggaaggtgtt attgaagttg tggttgttag 3180
   agggtgtatt ttttataatg ttggagaaaa tatttaataa atatttatta aataatattt 3240
   ttattgtgtt tttatttgaa taagatggat aagtattttt gggagaagaa agttatatta 3300
   tttttatata aaaaatgtta gttttataga gtttagtaaa aagaaaaata ttttttatgt 3360
   gaatggattt agtgtataat tataaatttt tattttttat taataggttt gtataggttt 3420
   ttattttaat ataagtatgt taatattaac gaagtttaat ttttttttga gataataaga 3480
   ttgggttagg tgattagatt aattataaga taatttgatt attttgatat aaatataaaa 3540
   attatttatt atagggtttt gttatagaag tatttatata tgtttttttt aagagatgtt 3600
   acggtattat aaaattgtaa taaattaatt tttagaaatt 3640
<210> 90
   <211> 3240
   <212> DNA
   <213> Artificial Sequence
<220>
   <223> chemically treated genomic DNA (Homo sapiens)
<400> 90
   gaaattgagg tataaggtag taatgattat tgagggtttt gttttcgttt ttttaggtga 60
   ggagtttatt ttaggggttt tagtttgaaa gtttagaggc gaggggcgcg tattataatt 120
   tttagaggtt ttcggattta agggcggttt tcgtttttcg tttttttcgc gcgacgaagt 180
   cgggtttatt aatgggagtt cggagtgggg cgagtagggg cgggacgggg cgggattgcg 240
   gggaggtggt cggcgggtcg cgtcgcgagt tagtatttta gagagcgcgg ggttcggata 300
   gtcgcgcgtt gagggtttcg gggcgggcgt cgcgggattt tttcgggtta tgggtaagcg 360
   gttttgcggc gcggttcgga taggggttcg gcggttttgt agggaggttt aaggtgtcgg 420
   cgtcgggttt tttttagttt tcgtggtagt cggtgcgttg tgtggggcgg agaggggttc 480
   ggggtgggtt tcggggcgcg ggttaagggg agcgagttcg gttcgcggtt ttagcgtttt 540
   tcggtcggcg cggaaggggt gaggttgggc gttcgcgcga gtttcgagaa aaggaaagat 600
   aaaaagtttt ggcggttagg ggtttttcgg gcgtttattt cgaaattttt ttcgtcgttt 660
   tttgtttgaa atatgtcgag ttcggtacgg ttttgtcgtt ttattttttg ttgcggtttt 720
   tatttcgagt tgggtttagt taatttgttg tgatagttgt cggtagtcgt tttttcgttt 780
   cgtgatattt cgtaggtttt agaatcgcgg cgtcgtcggg tatagatttc gggtttagag 840
   cgcgcggttt ttttttttgc ggtcgtcgtt ttttgaaagg ggttgcgcgg gtgtgaagaa 900
   tttgtttaat gcgttttgta ggatttattg tttttttttc ggtcgcgggt tgcgtcgatg 960
   gttcggggag ttcggttttt cgttttttgt tttttttttt tttttttttt tttttttttt 1020
   ttttttgttt tttcgtttat tcgtcgtagt tttataggag aggggtcgtt tttttggttg 1080
   tttttttttt ttgttttttg tgtgtttttt tgtttttttt cggtttgtag aagggacggg 1140
   gtggcggttg gggagcgtta ggggaagagg gtaagagcgt gggttacgag tttgtaggcg 1200
   atgttcggtt tcgcgttcgg cgggagttgc gggttaattc gggttcgcgt ttcgtttttt 1260
   cgcggtgttt aggggtcgtt cgggttgggg tcgcgttatt tttcgtttag cgtcggcgtg 1320
   gtggggtttg cgcggtgtag gattttcgag ggtttagggg gttttaaggc gttgcgggtt 1380
   tagtgttttt ttgtttgtag agttttgggt tagtaaggag tgtttcgcgg gttttagggg 1440
   aggataatag gtgttttttg ttttattcgg gtttttttgg tgggagttta tacgtacggt 1500
   ggcgttattg cgtttatagg taacgttaga gtataggagg ggtgggaggt cggggttcga 1560
   gggttttatt tcgtagtaga gtttagagtt taatttaggt tttagggatt cgtttggggt 1620
   ttagaatttg gtgtggtaga ggtagaaagg gttttcgata atttgttttt atagttgaga 1680
   gacgttaatt ggtatttttg agtttttgtt tcgtaggtga aatgggaata tacgaaatta 1740
   tttgaggagt ttttattgtt cgttattatt aaatgtggtt tagatttcgt ggggagggat 1800
   agttttattt tggaattggg gtttttagga tattttagag ttcggtaaga gggataggga 1860
   gaatggggtt tggaagttgg tagttttata tttagtgttt ttcgtatttt tttttttttt 1920
   tgtatttttt ggtggttttt gtggttttag ttgtttttag attttgtttt tttcgttttt 1980
   ttttaagttg aggtattgag gttgggttgt ggtttgagtt gtatttgtgt tttgttaggg 2040
   agtgggggtt ttaggtttat cgcgtttttt tttattgttg gttttagttt aatatttggt 2100
   aggttttttt ttttaaattg ttggagtaag ttcgcgtaga ttcggtttgg tcgtcgtttt 2160
   cgggttgtcg agtggatcgg tcgtttcgtc gttttaaagg ttgtttttgt tttgttttag 2220
   tttaagttta gagaaattta agttaataat tttagtcgag ggggcggggc ggcgtttgtt 2280
   ggttgttatt gaggttgttg ttattattat tgtggtaatt gacgtagtgt ttttagtcgg 2340
   tgatttttta tgaaaggaag tgttattttt tttttattat atttagggtg agtggggcgg 2400
   gtagtagaaa gatggggagg gagagaagga aaggggtgtg tgtttttagt agatttttaa 2460
   atttgagagt tgtgtggttt tgtattagtt attgtttttt tttgggtaat aggggtttgt 2520
   aagtttattg cgtttatttt gtatttagga ggttgatgtt ttcggagtat ttgttgggta 2580
   gcgaggtgtt ttggggaggg agtaggatag gtaggttttt gattttttta ttgagtttgg 2640
   ttgtggtttg tgggggaagt tttttttttt tttttatttt tttttgttga gaatattggg 2700
   tatattgagt ttagtacgtt tattttgtag atggggaaag ggagatatag aattagtgat 2760
   taatttggta agaggtgtag ttatgtagtg ttggaatttt ttgttttatt tgtttttttg 2820
   gtttattttt atttgttttt agtttgatgt ttggtagggt aggggtttat tgattttttc 2880
   gtatttattt attgtttggt tttatagtta ttttttagtt ttggttaagt ttcgtgagat 2940
   attttaagtt tgtttttagt gatttttaat tatcgttcga ttgtttttat ttttttgttt 3000
   ttgtatattt gtttttgttt cgttaggttg gttgggtttt ggttagagtt ggttttttat 3060
   ttttttaggt aataggtagt agttttttaa atggaatttt agtaggtatt aataggtttt 3120
   tttttttttt ttaagattat ggagataata ttttttattt aaagatggtg ggttttttgg 3180
   gtttttttcg atttaggtta gggaagttga tttatagagt gtgggggtta tgtttttttt 3240
<210> 91
<211> 3240
   <212> DNA
   <213> Artificial Sequence
<220>
   <223> chemically treated genomic DNA (Homo sapiens)
<400> 91
   ggaaggggta tggtttttat attttatgaa ttaatttttt tggtttgggt cggggagggt 60
   ttagaggatt tattattttt gaataagggg tgttattttt ataattttga ggaaggagga 120
   aagatttgtt gatatttatt gagattttat ttaaaaaatt gttatttgtt atttgagaga 180
   gtagggggtt agttttggtt agggtttaat taatttggcg aagtaaaggt aggtatgtaa 240
   aggtaggaag gtgaaggtag tcgggcggtg gttgagagtt attgaagata aatttaagat 300
   gttttacggg gtttagttag gattagaagg tagttatggg gttaggtagt gggtggatgc 360
   ggggaagtta gtgggttttt gttttgttag atattaagtt ggaagtagat gaaagtgggt 420
   taagaggata gatggggtaa ggggttttag tattgtatgg ttgtattttt tattaagttg 480
   gttattggtt ttgtattttt ttttttttat ttataagatg ggcgtgttag atttaatgtg 540
   tttaatgttt ttagtagaaa aaagtgagaa gagaaaggag gtttttttta taaattatag 600
   ttaggtttag tgagaagatt agaggtttgt ttgttttgtt ttttttttaa ggtatttcgt 660
   tgtttagtag gtgtttcgga aatattagtt ttttgggtgt aggatgagcg taatggattt 720
   atagattttt gttgtttaga gaggggtagt gattagtata aggttatata gtttttaagt 780
   ttaagggttt gttggaggta tatatttttt tttttttttt tttttttatt tttttgttgt 840
   tcgttttatt tattttgggt gtgatgggaa ggaagtgata ttttttttta tgaaaagtta 900
   tcgattggga atattgcgtt aattattata ataatggtaa tagtagtttt aataataatt 960
   agtagacgtc gtttcgtttt ttcggttgag gttgttggtt taagtttttt taagtttgag 1020
   ttggagtaaa ataaagatag tttttgaagc ggcgaaacgg tcggtttatt cggtagttcg 1080
   ggagcggcgg ttaggtcgag tttacgcgag tttgttttag tagtttgggg aggggggttt 1140
   gttaagtgtt gagttggagt tagtagtgag aagaaacgcg atgggtttgg gatttttatt 1200
   ttttaatagg gtataggtgt agtttaagtt atagtttaat tttagtattt tagtttgggg 1260
   gagggcgaga gagatagggt ttgggggtag ttgaggttat aagagttatt agagggtata 1320
   gaaagggaag gaggtgcggg ggatattgaa tgtgaggtta ttaattttta agttttattt 1380
   tttttgtttt ttttatcggg ttttgggatg ttttaaaagt tttagtttta ggatgggatt 1440
   gttttttttt acggagtttg ggttatattt ggtggtgacg gatagtgaga gttttttaga 1500
   tggtttcgtg tgtttttatt ttatttacgg ggtagaggtt tagaggtgtt agttgacgtt 1560
   ttttaattgt agaagtagat tatcggaggt tttttttatt tttgttatat taagttttag 1620
   gttttaggcg ggtttttaag gtttggattg ggttttgagt tttgttgcgg ggtgaagttt 1680
   tcgagtttcg attttttatt ttttttgtgt tttagcgttg tttgtaggcg tagtgacgtt 1740
   atcgtgcgtg tgggttttta ttagaggagt tcgggtgggg taagaggtat ttgttgtttt 1800
   tttttggggt tcgcggagta ttttttattg gtttaagatt ttgtaaataa agggatattg 1860
   gattcgtagc gttttgggat tttttgggtt ttcgggggtt ttgtatcgcg taagttttat 1920
   tacgtcggcg ttgggcggag ggtgacgcga ttttagttcg ggcgattttt agatatcgcg 1980
   gagggacgga gcgcgggttc gggttggttc gtagttttcg tcgagcgcga agtcgggtat 2040
   cgtttgtaag ttcgtagttt acgtttttgt tttttttttt tgacgttttt tagtcgttat 2100
   ttcgtttttt ttgtagatcg agggaagata aggggatata taagggatag gagaggagag 2160
   taattaggga agcgattttt tttttgtggg gttgcggcgg gtgggcggag agataggaga 2220
   ggaaaagaga aaagggaaga gaaggaaggg gtagggggcg agaagtcggg tttttcggat 2280
   tatcggcgta gttcgcggtc gaaagaggaa tagtggattt tataaagcgt attgagtaag 2340
   ttttttatat tcgcgtagtt ttttttagga aacggcggtc gtagaggaaa gggtcgcgcg 2400
   ttttggattc ggagtttgtg ttcggcggcg tcgcggtttt gaaatttgcg ggatgttacg 2460
   gggcggaggg gcggttgtcg atagttgtta taataaatta gttgagttta gttcgaggtg 2520
   gaaatcgtag taggaagtgg gacgatagag tcgtatcggg ttcggtatat tttaggtaag 2580
   agacggcgaa aagagtttcg agataaacgt tcgggaagtt tttggtcgtt aaaatttttt 2640
   gttttttttt ttttcgaagt tcgcgcggac gtttagtttt atttttttcg cgtcggtcgg 2700
   ggagcgttga agtcgcgggt cgggttcgtt ttttttggtt cgcgtttcga aatttatttc 2760
   gggttttttt tcgttttata tagcgtatcg attgttacga gggttggagg ggattcggcg 2820
   tcggtatttt gaattttttt gtagggtcgt cgagtttttg ttcgggtcgc gtcgtagagt 2880
   cgtttattta tggttcggag aggtttcgcg gcgttcgttt cgagattttt agcgcgcggt 2940
   tgttcggatt tcgcgttttt tgagatattg gttcgcggcg cggttcgtcg gttatttttt 3000
   cgtagtttcg tttcgtttcg tttttgttcg ttttatttcg ggtttttatt ggtaagttcg 3060
   gtttcgtcgc gcggagaagg cggggggcgg ggatcgtttt tgagttcggg gatttttggg 3120
   aattgtagtg cgcgtttttc gtttttaggt ttttagattg gagtttttgg aataggtttt 3180
   ttatttagag gagcggaggt aggattttta gtaattattg ttgttttgtg ttttagtttt 3240
<210> 92
   <211> 2155
   <212> DNA
   <213> Artificial Sequence
<220>
   <223> chemically treated genomic DNA (Homo sapiens)
<400> 92
   tttttttttt tttttatttt tggagggttt tagttattag ttttgaagtt gtagcgagag 60
   ttatttattg ttttggtttt tgggattttt tgagtaggag aaaaaaatta gaaaattaaa 120
   agagagagag agatttggaa ggaagaagta atggaggagg gagatagggg gaacgagagg 180
   ggagtttata ttatagagag agaatttttt aaaggaagcg taggcgttta cgggatagga 240
   gcgcggaaga aaagagtaga aaaggaaaag tagttttaag taagagaaag taaggagagg 300
   cggataggga ggggaagagt taagagaagt taagttgagg gatttgtagt aattttgaga 360
   aataaaagtg aattgtttgt gggcggttgt tgtgatttta gtaaaaagcg gagtttaagg 420
   tttagggatt tcgtcgttta tattgtgttt cgttttattt tggatgtttt taattttagt 480
   tttaggtata gtatacgagt aggggtagta aggtttggtt agggtcggtg tcgtacggga 540
   gttgcgggtt tttgtaatat ttaggtgtgt aggcgttata agaaggagtc ggggtcgttg 600
   ttttcgtagc gcggatttcg tttcgtgttg tatacgtttt agaagagcgt gttcggaagt 660
   gtttttttcg gtttttttcg ttttttaaga tttgtttgta acgtttatat tcgttattat 720
   cgtataagta ttagggtgag gaggtttttt tgtaggaatt aatatcgatt ttagtttttt 780
   gtttttttaa agtttttttt tgacgatggc gagatttata ttttaagtta gaagaaaata 840
   aaattttgtt tattttgggt ttatgattgt ataaaattta aaatgttgtc ggtatgaaga 900
   tgtagttgga agattttaga aatttagtat taggaggtag agattttata gtgtttagag 960
   atgagttttt aaataaatta agaattacgt agtgggaaat taaggaggag ggtttgaggg 1020
   gtcgatttta gagggataga tagagaaatc gagagcgaaa tatttaagga agtggaataa 1080
   atcgtcgtag ttaggcgttt ttgttttttc ggaaattcga aattcgttta aattttattt 1140
   taggcgttta cggtattagt cgtagggttt cgtattatag gggttgttat tttttgtttt 1200
   cggagttttt acgtcgagat ttattatttg ttggtttttt tttttttttt tttttttgtt 1260
   tttttatttt ttaaatataa gataaaaata tattttattt attattatag atatatatat 1320
   tttattttta ttatataaat ataaaatatt taaatattta aaatatttag aataaagtta 1380
   gtagtttgga tttagtgata tttattcgcg tttgaatcgt ttattatttt ttttattatt 1440
   tgtttagaaa aaaaaaaaag atgaaaaaga gggggagagt tttcgacgta aataatttat 1500
   aataaaatta ttattttaaa aaagtgtttt tcgtttgttt tagatggatg aatatatttt 1560
   agatattgcg gtatttcgta gtttgggttt gtgattttgg ttaaaatgtg ggtggtcgtg 1620
   gttgtaggga cgatcgtcgt tgcgtagggt aaggaaagag ggtgtcgcgg ttcgtgtttt 1680
   gatttgggat tattgaaatt ttaagattgg ggaggcgagg ggagtgtttt ttttattaat 1740
   tatataagac gttatttgga tttcgagatt attgttagag gaggttcgat tatttagcgg 1800
   cgtttttgtt ttttttttcg taaagaattg tttagaaatt tagacgtttt ttgcgtgtag 1860
   gaagggtttt gtgaattcgg gtgtttggga gaggaggtaa tgagtgttga ttcgtttttt 1920
   aaatcgagta attgtgttcg aagttacgcg tttgggaggt cgtagggtga agcgtcggtt 1980
   gcgtaggtta tcgtcggtag tcgttttgtt tttttttgga ggagtgggaa gttgtcggtt 2040
   atcgtttcga ataggttcgg agaaaagatt ttgaattttt tttgattagc ggcgttattt 2100
   ttttttttgt ttttttttta ttaaattttt gtttaaagtt ataatattta gaatt 2155
<210> 93
   <211> 2155
   <212> DNA
   <213> Artificial Sequence
<220>
   <223> chemically treated genomic DNA (Homo sapiens)
<400> 93
   ggttttgggt gttgtaattt taaataggaa tttaatgggg gaaaagtaaa ggaaaaggta 60
   acgtcgttaa ttaaaggaaa tttagaattt ttttttcgag tttgttcggg gcggtggtcg 120
   atagtttttt atttttttag gaaagagtaa agcggttgtc ggcggtagtt tgcgtagtcg 180
   gcgttttatt ttacggtttt ttaggcgcgt agtttcgggt ataattgttc ggtttggaaa 240
   acgagttagt atttattgtt ttttttttta aatattcgga tttataaaat tttttttgta 300
   cgtaggaaac gtttggattt ttgagtagtt ttttgcggaa ggggaggtag ggacgtcgtt 360
   gggtggtcgg gtttttttta gtagtagttt cggagtttag atagcgtttt gtgtgattga 420
   taggaaagat atttttttcg tttttttagt tttagggttt tagtggtttt aaattagaat 480
   acgaatcgcg atattttttt tttttatttt gcgtagcggc gatcgttttt atagttacgg 540
   ttatttatat tttaattaaa attatagatt tagattgcga ggtgtcgtag tgtttagaat 600
   gtatttattt atttgaagta agcgaaaaat atttttttaa aatagtaatt ttattgtaaa 660
   ttatttacgt cggaaatttt tttttttttt tttatttttt tttttttttt aggtaaatag 720
   tgaagaaaat aatgaacgat ttaaacgcgg gtaggtgtta ttgaatttag gttattaatt 780
   ttgttttgaa tgttttggat atttggatgt tttgtattta tgtggtgaga gtgaaatata 840
   tatatttatg atgataaatg aggtgtattt ttgttttata tttgaaggat aaaaaaataa 900
   aagaagaaaa ggaagaaaga gttagtaaat gatgagtttc ggcgtgggga tttcgggggt 960
   agagggtggt agtttttata gtgcggggtt ttgcgattgg tatcgtgagc gtttgggatg 1020
   aggtttgggc ggatttcggg ttttcggaga ggtagagacg tttggttacg acgatttgtt 1080
   ttattttttt gagtgtttcg ttttcgattt ttttatttgt ttttttgagg tcggtttttt 1140
   agattttttt ttttaatttt ttattgcgta atttttagtt tatttaggaa tttatttttg 1200
   ggtattataa agtttttgtt ttttaatgtt aggtttttag agttttttag ttgtattttt 1260
   atgtcggtag tattttggat tttgtgtaat tatgggttta gaataggtaa ggttttattt 1320
   ttttttgatt tagaatataa gtttcgttat cgttaaagag aaattttgaa ggagtaggag 1380
   attgaaatcg atattaattt ttgtaaagag atttttttat tttggtgttt atgcggtggt 1440
   gacgggtgta gacgttgtaa ataaattttg ggaagcgagg agggtcgggg aggatatttt 1500
   cggatacgtt tttttaaagc gtgtgtagta cggggcgaag ttcgcgttgc gggggtagcg 1560
   gtttcggttt ttttttgtag cgtttgtata tttgggtgtt gtagggattc gtagttttcg 1620
   tgcggtatcg gttttagtta ggttttattg tttttattcg tgtattgtgt ttgaagttga 1680
   agttggaggt atttaaaata aggcggggta tagtgtgggc ggcggagttt ttgagttttg 1740
   agtttcgttt tttgttgaga ttatagtagt cgtttataga tagtttattt ttatttttta 1800
   aagttgttgt agatttttta gtttggtttt ttttggtttt tttttttttt gttcgttttt 1860
   ttttattttt ttttgtttga aattgttttt ttttttttat tttttttttt cgcgtttttg 1920
   tttcgtaaac gtttgcgttt tttttaagga gttttttttt tgtggtgtgg gttttttttt 1980
   cgtttttttt gttttttttt tttattattt ttttttttta ggtttttttt ttttttttgg 2040
   ttttttggtt tttttttttt gtttaagggg ttttaaaggt taaggtagtg ggtggttttc 2100
   gttgtagttt taaggttggt ggttgggatt ttttaggggt gagaaggaag ggaga 2155
<210> 94
   <211> 4513
   <212> DNA
   <213> Artificial Sequence
<220>
   <223> chemically treated genomic DNA (Homo sapiens)
<400> 94
   agtgtatggg gaaagattaa tttaattgtt tatttgtggt tgggatagtt tagggggtgt     60
   gtttatggtt gatttagggg tgtgtatatg gttgagttgg gagttttgtt ggtttttttg    120
   aggattgagg gtgaattttg ttttttgttt taaatttttt tattttattg tagtaatagt    180
   tttatgttgt atataatagt gtaaattttt ttaaaaagga aagtataaaa ataaaagttg    240
   taatttaaaa gtttgaataa ttatttgttg tttaggaaat ttaatgaaat gatatgtttt    300
   tttagtagga agtaaagttg gtttttgttt tttgtttttt ttgttgtttt agtttataaa    360
   atatgtgtat tttatagttt tagtattaaa tatttataat tttatgagaa atgaatgaat    420
   gtttttattt ataattgtgt ttattaaaat tgtgaatatt tttatttttg tatttttgtg    480
   tgttgaaatt tttgaaggtt atattaaata aaataaaatt tttttattat aaaatattga    540
   aggttttagg tggaatgatg agttttttaa gtatgtttga gtttagagga attgtttggg    600
   tatttttttt agtatttagg tgtttatggt ttttgttttg tttgggagta ggtgttaggt    660
   gtttttgtag tgagaggtga ttattatttt tttttaaata agatagtttg gatatgttat    720
   atgtgagtat agaataatta aaagaagtat atattttgtt attaggagga agtgtttaaa    780
   attatggtgt tattttagtg attatatttt tttaaatttt gtgaagttta ataggttttt    840
   ttttggttgg gtgtttattg gttttgttat ttgtgttgga ttgaggtgtt tttttttggg    900
   gataggaatt ttttatttta ttgagataaa gagatttttt tttttaaagt gttatttttg    960
   tttatttttt gttgtttttt ttttaaaata aaagattttg tataattttt taagtaggat    1020
   tgggggaggg attagttttg tttgtgttga ttgtgttttt ggattttgtt tattaggaag    1080
   tgtttggggt ttttgagtga gtgatttatg tgttgaattg ttgttttttt aaatttttat   1140
   ggtttaaaaa tttttttttt tttttttgta aaagaaattt agagattggt gatttgttat    1200
   tgaagatgat tttttaggtg ttttttgttt ttttaggtag ttgggttgtt ggtgttttta    1260
   ggtggaagtg gggtttttgg gttgggtttt tgttttgtta ggaagtggtt tgggggttgt   1320
   tttttgggat ttttagaatt ttggaaggag agagttgggg ttttattgtg gagatttttg    1380
   agtttttgtt gtttgggttt gagttgtgtt ttagggttat tgttgatggt gtgagattat   1440
   gtattttttg tgaattttga tttttgaata gtttagaaag tttgtagatt aaaaattaaa      1500
   taaaaaatat tggtataatt tttattgttg tattggtttt ttttttattt taataaaaat     1560
   atggtaatat gaagtgatta tttttttaaa aatttgtttt taaagtataa tattgaaaag    1620
   gggtaaggta tagttttgta aggtaatgag ttttttgttt ttagaggtgt ttaagtagat    1680
   gttaggttta tagtggtatg gagagtgttt tttttttttg gaattggatg agtttttaga    1740
   tttttgaaat gttgagtttt tttttttttt tttattttta tgtttattat attttaattt   1800
   aatagtatat aatttaagtt gtttttttat ggagagttgt ggttgtgtta tttgtttgtt    1860
   tttgttatgg agattggagt taggtttgta gggaagttgg tgtgtttttt ttaagttttt    1920
   tgtttgttgg ttttttttgg gttttttggg ttttgtgtgt tgtggatttg gatttggatt   1980
   gtagatattg ttttatgatt gggtttttgg ttagattgtt attttatagg aggagggttt    2040
   tttaagtgtg tagaagagga aagtttattt ataaagtttt tgtttttttg agttgaatga   2100
   tttttgtgtt tgaggtttag ttgtgttttt gttgggttta ggttgagttt attttatatt    2160
   gttttatagt tgggtagaga ttttgtgagg gtttgggagt tttggttttg gaggatttgt    2220
   tgttttgtag gtttgtttaa gtaggtgttt ttgtgagagt gtgggataga gagggtttgg    2280
   tggggttttt tggtgttgag agtttttttt ttttgttgta tttagttttg ttaatggggt    2340
   tggatgagtg ggggttggag ttagaatttt agggtttata tttgttgata gtagtaatgt    2400
   tgatttgttt tgtttttttt ttaaggagtg gagggaagga gttatgtgaa tgattttagt    2460
   ttgattatta agagggtgtt agtatttttg tttttttaga gtgaaaaggt taaattgatg   2520
   ggggttttta tattgttgga tataggtgtg tttgggtgtt tagtgtgtgt aatatatgtt     2580
   tagatatttt atatgtaaat atgtttgttg ttgtggagtt ggttgggagg tagtgtttgt    2640
   ttgttggggt tttgttagaa gttattttgt tttttgttgg tttaatattt gttttttgtg   2700
   gtttttgttt gttttggtgg tttttgaagt gtttttggga gggttttggt tttgttgaaa  2760
   attgtagttt gttaggttga gttgttgtgt gttgggaggt taatagtgat tgatgataaa    2820
   gggttaaggt gtaattttta aagtggggat ttgttgggtg aggtagaaat tagtttttgg    2880
   ggagatgggt tttttttttt tgatgtgttt ttgtatatat tgttgttttg gtgttgggtt    2940
   tttgggtggg gatttttttt ttgggtattg tggttttatt tggtggagag ttaggttttt    3000
   tttaggtttt taagtaggga gtggatgtgg gtggtgggga agggaagttg ttggtgggat    3060
   ggagagtggg gttttttgga ttttgggttg ggtaggtttg gtgttggtag ttttgttgta     3120
   tagatttgtg agtgatgttg gtggttgtat tgttgtgtgg tgtgaatttt aagtagatta    3180
   tttatgtttt tattaataat tttaaatatt ttattatatt tatgttgttt gtttagagaa 3240
   aataaaaggt tatttttgaa attgtttgaa atgtaataat atttattaaa gtgtttttta 3300
   attatgtgat ttaattaagt gtggtgtgtt tgtgtttttg gtttttgttg gaaaggttgg 3360
   tgatgatggt tattattatt gttgttgtta gtggttttgt gatagtagtt ggttagtttt 3420
   ttagtttagg tttagttttt ttagagagat ttggggagtt ttattttgag ggatggggtt 3480
   ttttggaatg attttgaagt tttggagatt aggtgtggtt taggtttttt gttttttttt 3540
   agttttagtt ttagttttag ttttagtttt gggtaaattt ttttaggttg agggtaggat 3600
   tttttttaga tttttggggt tttttgtgtt ttttggggat gtttgatttg gttttttagg 3660
   agggggaatt ttgggttttg agtttgggtt ttgttttgtt ggttgttttg ttttttttag 3720
   ggttgagttt tgtgtaatgt ttgaagtaga aataaagagt tgttgagatt tgttttttgg 3780
   gttgtttttt tattttttta gtttggaagg tgttttagag aggttttatt tttagttttt 3840
   atggagattt aggagggaag aagagttggg atttggtagt tttgaggggt tgttgggttt 3900
   gtggtgtttt ttttgttgta gaggggttgg gtgggagatg taggtgggaa ggattttgtt 3960
   ttagttgaga gtttttgtgg gggttgagga atggtgagga ttattgtttg tattaattgt 4020
   ttggaagaaa ttgggtggtt agttgtttga ttttatttgt tgagatgtga aatttggttg 4080
   tgtggtttgg gtgtgtgttg tttgtgtgga aggagttgtt tttttttttt tagtgaatgt 4140
   atttggggtt tttttttttt gttaagtttt ttgtttggaa ggggattggt aggttttgtt 4200
   gttgttgtgt gttttttttt tttttttagt ttttattttt tttaattaat tttttttttt 4260
   tttttttttt tttttttttt ttttggatgg gtaattatag gttttgttgt taagtgtgtt 4320
   gtgaggagtt gttgtttgtt tttgttggtg tttttttttg tggtagtttg gttgagtttg 4380
   tttagtttgt ttaggatgtt tttgatttgt gtgtttagtt ttatttagtg gttgtgggat 4440
   gttgtatttg agtttttttt ttaggttaaa attttgtttt gaggtttttt tgagagggtt 4500
   ttatttttta ata 4513
<210> 95
   <211> 4513
   <212> DNA
   <213> Artificial Sequence
<220>
   <223> chemically treated genomic DNA (Homo sapiens)
<400> 95
   tgttggagaa taaaattttt ttagaaaaat tttgggataa agttttggtt taaaggaaaa     60
   atttagatgt aatattttat ggttattgaa tgaaattgag tgtgtgggtt agggatgttt    120
   taggtaggtt gggtggattt ggttaggttg ttgtggggag gggtattgat gggaatgggt    180
   agtggttttt tgtagtgtgt ttggtagtgg agtttgtgat tatttattta gagagggaag    240
   aaaagagaag ggaagaaagg aaagttagtt ggagggggta gaggttgggg aaaggagggg    300
   ggtatatggt gatgatagag tttgttagtt tttttttaga taaaggattt ggtgggaggg    360
   gggaattttg aatgtgtttg ttgaaaggag ggaggtggtt ttttttgtgt aggtagtgtg    420
   tgtttggatt gtgtggttaa gttttatatt ttgatagatg gggttgagtg gttggttgtt    480
   tggttttttt tagatggttg gtgtaaatag tgatttttat tattttttga ttttatagag    540
   gatttttggt tgggataggg ttttttttgt ttgtgttttt tatttaattt ttttgtggtg    600
   gagaaagtgt tgtaggtttg gtagtttttt agggttattg ggttttggtt tttttttttt    660
   tttgggtttt tgtggaggtt gggggtgggg tttttttggg atgtttttta ggttgagggg    720
   gtgaaaaggt ggtttaggag gtagattttg gtgatttttt gtttttgttt tgggtattgt    780
   gtggggttta gttttggagg gggtggaata gttaatgggg tggggtttgg gtttaggatt    840
   tggagttttt ttttttagaa agttgggttg ggtgttttta ggggatatag agggttttaa    900
   gggtttggag aaagttttgt ttttggtttg gaagagtttg tttggggttg gggttggggt    960
   tggggttggg gttggagaag ggtagagggt ttgggttgtg tttggttttt gggattttgg   1020
   agttgttttg gggaatttta ttttttaaag tggggttttt tgagtttttt tgaggaaatt   1080
   gggtttggat tgaagagttg gttggttgtt attatgaggt tattaataat agtagtaata   1140
   ataattgtta ttgttagttt ttttagtgag agttgagagt gtgggtgtat tatgtttgat   1200
   taaattatat aattaggagg tgttttaatg aatattatta tattttaggt gattttaaga   1260
   atggtttttt attttttttg ggtaggtagt ataaatataa tgaagtgttt gagattatta   1320
   ataaaaatat gaatggttta tttggggttt gtgttgtgtg gtaatgtggt tattagtatt   1380
   gtttataaat ttgtgtaata gagttgttag tgttaggttt gtttgattta gggtttgggg   1440
   gattttattt tttgttttgt tggtggtttt ttttttttat tatttatgtt tgttttttat   1500
   ttggaggttt ggagagggtt tagttttttg ttgaatgggg ttgtgatgtt tggaagaggg   1560
   gtttttgttt ggaggtttgg tgttggggta gtagtgtgtg taggggtgtg ttgggaaggg   1620
   gggatttatt tttttggagg ttgatttttg ttttatttgg tgaatttttg ttttaggaat   1680
   tgtattttgg ttttttgttg ttagttgtta ttaatttttt agtgtgtggt ggtttagttt   1740
   ggtaggttgt agtttttggt ggagttggga tttttttggg ggtgttttgg gagttgttga 1800
   ggtgagtgga gattgtagag ggtggatatt aaattagtgg gagatagaat gatttttagt 1860
   agaattttag tgaataaatg ttgttttttg gttggttttg tggtggtagg tatatttgta 1920
   tatagagtgt ttgggtgtgt gttgtgtgta ttggatgttt gggtgtattt gtgtttagtg 1980
   gtgtaggagt ttttattgat ttgatttttt tattttagga aaatagaaat gttggtgttt 2040
   ttttggtggt taggttgggg ttatttatat ggtttttttt ttttgttttt taggagaaag 2100
   gtagggtggg ttggtattgt tgttgttggt gggtgtagat tttgggattt tgattttagt 2160
   ttttatttat ttagttttgt tggtagagtt gggtgtagtg gagaaggagg gtttttagtg 2220
   ttaaggagtt ttgttaggtt ttttttgttt tgtatttttg tgggggtgtt tgtttgggta 2280
   ggtttgtagg gtggtgggtt ttttgaggtt aggattttta gatttttgtg gagtttttgt 2340
   ttggttatgg agtagtgtgg agtaagttta gtttgggttt ggtggggata tagttaggtt 2400
   ttgggtgtgg gggttgttta gtttgaagga gtaaggattt tataaatgag tttttttttt 2460
   ttgtatattt agggaatttt ttttttgtgg ggtaatagtt tggttggggg tttagttgtg 2520
   gggtggtgtt tgtagtttgg gtttagattt gtggtatgta gagtttggga ggtttgagag 2580
   gaattagtgg gtagaggatt tgggaaaggt gtattgattt ttttgtaggt ttgattttgg 2640
   tttttatggt gaaagtaggt aggtggtata gttgtggttt tttatgaggg agtagtttag 2700
   attatatgtt gttggattaa aatgtaataa atataggagt aaaaaaaaaa aaaaaaattt 2760
   agtgttttaa gggtttgagg atttatttag ttttaaagag aaagaatatt ttttatgtta 2820
   ttgtaagttt agtgtttgtt tgaatatttt tgaggatggg gaatttatta ttttataaag 2880
   ttgtgttttg ttttttttta gtgttatatt ttgaaaatag atttttaaaa aaatgattgt 2940
   tttatgttat tgtattttta ttaaaataag agagaggtta atatggtaat agaagttgtg 3000
   ttagtatttt ttgtttggtt tttaatttat gaattttttg aattgtttag aagttggggt 3060
   ttgtggggaa tgtgtggttt tgtgttatta gtgatgattt tggggtgtag tttgggttta 3120
   ggtaatgaga atttgggagt ttttgtggtg agattttagt tttttttttt tagggttttg 3180
   aaaattttga ggggtagttt ttagattgtt ttttggtagg gtagaggttt agtttagagg 3240
   ttttgttttt gtttgggaat gttaatggtt tagttgtttg gggaaataga aaatgtttaa 3300
   aaagttgttt ttggtaatag attattagtt tttgagtttt ttttataggg agaagaaaaa 3360
   aaatttttaa gttatgaaag tttggaaaaa tagtaattta atgtgtagat tatttgttta 3420
   aagattttaa atattttttg atgagtggaa tttagaggtg tggttagtat aggtgaagtt 3480
   gatttttttt ttaattttgt ttaggaagtt gtataaagtt ttttgttttg ggggggaaat 3540
   aataaaaggt aaataaaaat gatattttga aaaggagagt ttttttgttt tagtggagta 3600
   aaaagttttt gtttttaggg gaaagtgttt tggtttagta tgggtggtag ggttagtgag 3660
   tatttagttg gagggagatt tgttgaattt tgtaaaattt aaaaaaatgt agttattgga 3720
   atagtattgt agttttgaat attttttttt ggtaataaaa tgtgtatttt ttttggttat 3780
   tttgtgttta tatgtgatat atttaaattg ttttatttaa aaaaaaataa taattatttt 3840
   ttgttataaa ggtatttggt atttgttttt aggtggggtg aaggttatga gtatttgagt 3900
   attggggagg atgtttaggt agtttttttg gatttagata tgtttagaaa atttattatt 3960
   ttatttggga tttttagtat tttataataa agagattttg ttttatttaa tgtaattttt 4020
   aagaatttta atatataaaa atgtgggggt gggggtgttt ataattttga taagtatagt 4080
   tgtaaataga aatatttatt tattttttat aagattataa atatttgata ttggaattgt 4140
   aaaatgtata tgttttataa attaaaataa taaagaaaat aaaaaataga aattaatttt 4200
   gttttttgtt aaaaaggtat gttattttat tgagtttttt aagtagtaga tggttattta 4260
   gatttttaaa ttataatttt tgtttttata tttttttttt taaaaaggtt tatattatta 4320
   tgtataatgt aaaattatta ttgtaatgaa ataaagaggt ttaaggtaaa gagtgaagtt 4380
   tatttttagt ttttagggag attagtggga tttttagttt agttgtgtgt atatttttgg 4440
   gttagttgtg ggtatatttt ttgggttgtt ttagttatag atggatagtt ggattggttt 4500
   ttttttatat att 4513
<210> 96
   <211> 2280
   <212> DNA
   <213> Artificial Sequence
<220>
   <223> chemically treated genomic DNA (Homo sapiens)
<400> 96
   tagtggtgag tatttgttgt tggtttttta gggtaggtag tattgttgtt ttaggtgtgt      60
   ggagttgggg agaaaaaaat tgaatatgtg tgttatttag ggtttttaga taagtttttt     120
   tgtgtgtata tatattttga attggagttt agaaggttgg atttgtgttt tggggttgag     180
   ttttttattt tttgttttta gtttggaaga ttttagtttg tttgaaggtg gtgtttgatt     240
   tgttggtgga tttggtgggt gtttgttttt gatatttggg gatgaggtgg gagtgttttg     300
   agtgttggga agtttgtttt tgtgagtttt tggtagtgat atgtgggagt gttgggtttg     360
   gtatttttga tgtttttgag gttttgggtg gttgttttgt ttttttgggg gtttttattt     420
   tttaagtggt tttaaatgat tgaggatggg ttttttgtgt tgttgtagtt gttatttttt     480
   ttgggaaagg ttagtgattt tggatgtgta ggttttgaaa attaaggttt agatttatat     540
   tgagtgttta aagattgtat ttgaattttt tttgttggtt attgagggag agaaaggaat     600
   gtttagtttt atttatattt atagaaagaa gtgttgaggg tgggggaaat gtagttttgt     660
   tgggtgagtt gggataggtt ttttgtttgt tttttggttg ttgttttttt ttagttgaat     720
   ggggagtgat ttgttttggg tgtggttttt ggggttgaag attgaggtgt agttttattt     780
   ttggtttggt agtggtttgg aagagagagg gaaaggagga atatttattt ggttaagaga     840
   tgttgttaga gtttttaaag ttggtggggg atggaggttg gggttttgga tttttagagt     900
   gtttgtagtt gggtttttgg gttttttggt gtggggaatt tgattttgta gtttttgtta     960
   gggtgtggtg gggttgtaag aagggaggaa ggagtgggat tagagttttt gagggtgtgg    1020
   gtggtgtgta tttagttttg tttttttgtg tgttttgttg tttgtttgtt ttttttgttg    1080
   tattgggtgg ttttggtttt tgtgtttgtt tgtttttgat gtttttgtgt ggttttgtgt    1140
   tgtttgtggt tagttttgtt ttgttttgta aggagtgtgt tgttttttta attgaaatgg    1200
   gattaagatt ttagggtttt tttgtttttt tgttttgagg tgataggatt ttagttaata    1260
   gattgaaaat gtgaaaagta tttgttgttt tttgttttaa gtgtttttgt ggaaggatta    1320
   tttatttgga aaagtagaat aaattttgtt gttgtttttt ttttggaggt gtgtttattt    1380
   tagagttttt agatttattg gtatttgtta tatttttttt tgggttttat aggtaggatt    1440
   ttaagaatga tatgggggta tttgggtgat ttgtgggttg taggttttta agaggttgtt    1500
   tttttgtgta tggaagtgtt gtagttttat tttagtggaa gtaatattaa attttatttt    1560
   agaggtttgt tttgtaaaga atttagagtt tattatattt gatattattt taaaataata    1620
   tttgtttgtt ttaagaagtt gggagaaatt gataaaagaa aagttgaaat ttatgttttt    1680
   tatttttatt ttaggagata attttgatta atagtttggt ttgtttttta tttttatttt    1740
   ttaaaaataa ataaataaat aaataaatta aaatttatta tatttttaga ttgtgtagga    1800
   ttatgtagga ttttgtaaaa ataaaaatgg gattatattt tatttgtttt tgtaatggtt    1860
   tagtttttat tagtaattat tttttattag tatagtagaa ttaattttat tttagtatta    1920
   ttattattat tttgagatag ggttttattt tgttatttag gttggagtat agtggtatga    1980
   ttttggttta ttgtagtttt tgttttttgg gtttaagttg tttttttgtt ttattatttg    2040
   gttttttttt tttaattagt tgggattata gttatgtgtt attaagtttg gttaattttt    2100
   gtatattttt gtggagatag gttttattat gttgtttagg ttgattttaa attattgaat    2160
   ttaagggatt tatttatttt ggttttttaa attgttggga tggtagattt gagttattat    2220
   attggtaaat ttatttttta taatggttgt gtggtataga atttttattt tataggtgtg    2280
<210> 97
   <211> 2280
   <212> DNA
   <213> Artificial Sequence
<220>
   <223> chemically treated genomic DNA (Homo sapiens)
<400> 97
   tatatttata aaatggaaat tttgtattat gtaattatta taaagaataa atttgttggt 60
   gtggtggttt aggtttgtta ttttagtaat ttgggaggtt aaggtgggtg gattttttga 120
   gtttagtagt ttgagattag tttgggtaat atggtgaaat ttgtttttat aaaaatatat 180
   aaaaattagt tgggtttggt ggtgtatgat tgtagtttta gttagttgag gggaaggggt 240
   tgggtggtga gatgggagga tagtttgagt ttaggaggta gaggttgtaa tgagttaaga 300
   ttatgttatt gtattttagt ttgggtgata aagtgagatt ttgttttaaa ataataatga 360
   taatattaaa ataaggttaa ttttattata ttgataggga atgattatta gtaggagtta 420
   ggttgttgta gaaatgagtg aagtataatt ttatttttat ttttataaaa ttttatgtaa 480
   ttttatataa tttaaaagta tagtgggttt tggtttgttt gtttgtttgt ttgtttttaa 540
   ggaatggggg tagggagtaa attaaattgt tgattaggat tattttttag agtagaaatg 600
   ggaagtatgg attttaattt tttttttatt gatttttttt aattttttaa aataagtaag 660
   tgttatttta aaatagtgtt aaatatagta aattttggat tttttgtaaa ataaattttt 720
   aagataaggt ttgatattgt ttttgttaaa ataaagttat gatatttttg tgtatgaaaa 780
   aatggttttt tgaggatttg taatttatag attatttagg tgtttttata ttatttttga 840
   aattttattt gtaaagttta ggaggaagta tagtgaatgt tagtgggttt aggagttttg 900
   gggtgagtat gtttttggaa ggaaaatggt aatagaattt attttgtttt tttaagtaaa 960
   tgattttttt atagaggtat ttgggataag aggtgatggg tattttttat gtttttgatt 1020
   tattggttaa gattttatta ttttaaaata aggagatgag gaaattttgg gattttgatt 1080
   ttattttgat tgagaaaata atatgttttt tgtgaagtga aatggggttg gttgtgggta 1140
   gtgtggaatt atgtgaaggt gttgggggtg ggtgggtgtg gggattaggg ttatttggtg 1200
   tagtaaaggg ggtaagtagg tggtagggtg tgtggggaga taaggttgag tgtatgttgt 1260
   ttgtgttttt gaaggtttta gtttattttt tttttttttt tttgtagttt tgttgtgttt 1320
   tggtgaaggt tatagggtta ggttttttgt attgaggagt ttagaagttt ggttgtgggt 1380
   gttttgaaga tttgggattt tggtttttgt tttttgttag ttttagggat tttggtggtg 1440
   ttttttagtt gggtagatgt tttttttttt tttttttttt ttaagttgtt gttaggttgg 1500
   gggtgaggtt gtattttagt ttttagtttt gaaggttgtg tttggggtgg gttgtttttt 1560
   atttagttaa gaggaagtag tggttggggg gtaggtgagg aatttgtttt ggtttatttg 1620
   gtaagattgt gtttttttta tttttggtgt ttttttttgt gaatgtgaat ggaattaagt 1680
   gttttttttt tttttttaat ggttggtgag gagaatttaa atgtggtttt tgggtgtttg 1740
   gtatgggttt ggattttggt ttttagagtt tgtgtgttta aggttgttag ttttttttga 1800
   aggaagtgat agttgtggtg gtgtgggagg tttatttttg gttgtttaaa gttatttaag 1860
   gaataaaagt ttttgagaga gtaaaatgat tatttgaaat tttggaagtg ttgggggtgt 1920
   tgggtttaat gtttttatgt gttattgttg gggatttgtg aggtgaagtt ttttagtgtt 1980
   taaggtgttt ttgttttgtt tttaggtatt aggaatagat atttattagg tttattagtg 2040
   ggttagatgt tgtttttggg taggttggga ttttttgaat tgggagtgga gagtaagggg 2100
   tttgattttg ggatgtgagt ttggtttttt ggattttggt ttagggtgtg tgtgtgtgtg 2160
   gaggggttta tttgggggtt ttgggtagta tatgtgtttg gttttttttt ttttgatttt 2220
   gtatgtttgg agtggtaata ttgtttgttt tagaaggtta gtggtgagtg tttgttatta 2280
<210> 98
   <211> 3640
   <212> DNA
   <213> Artificial Sequence
<220>
   <223> chemically treated genomic DNA (Homo sapiens)
<400> 98
   aatttttaga aattaatttg ttataatttt atgatattgt aatatttttt ggaagagata 60
   tatgtggatg tttttatagt aagattttgt gataagtaat ttttatgttt gtattaaaat 120
   aattagattg ttttgtgatt ggtttagtta tttagtttaa ttttattatt ttaggaggaa 180
   attaaatttt gttagtatta atatatttgt attaaaataa gaatttatat aaatttattg 240
   atagaaggta ggaatttatg attgtgtatt aaatttattt atataagaaa tatttttttt 300
   tttattggat tttatgagat tgatattttt tatgtagaaa taatgtggtt tttttttttt 360
   aaagatgttt atttatttta tttaaataga gatataatga gagtattatt taataaatat 420
   ttgttaaatg ttttttttag tattataaga aatgtatttt ttaataatta taattttagt 480
   aatatttttt ttttgttgtt attttaggtt gggggttatt taattttatt aagagtgtgg 540
   aaattttgtt gttttaatgg ggattgaaag gaaattagtt attttttatt taaattatta 600
   aggtttttag attgatttaa aatattaagt tggtgtttaa atattttaat aaatggattt 660
   tataaagagt ataagtaatg ttatttgagg aataaggttt taagttgagg agtaggatat 720
   tggagagttt tttttttaat tatttatttt tattagattt gaggattttt tgttttataa 780
   gaaaattaga aataaatttt attggtatgt tttgtaagaa tgattttttt tgataatttg 840
   gggtagtagt atttgtttga ttagttgaat ttgttatgaa tgtggaaata atgatattaa 900
   aatagtttgt gtaataattt ttttaaagat tttgtagatt agagatgatt atagattttt 960
   tttagtgtgg attaaaggga ttgaattgaa tgttttagtt taatgattta atttttgtta 1020
   tatttatagg gatgtgaatt ttgattttat aagtaggtgt gtatgtgtat ttagatattt 1080
   atataaagtt gtgtggaggg atgaaaagat taattatttg attgatgagg ataggtttga 1140
   tttttttgat tattttagtg tgttagtgta tatttttggt tgggtttagt gttttaagaa 1200
   attttggaat tttagttgtt aatttttgtt tttttattat gattttttaa agatatttta 1260
   tttgtttatt ggggtgaaga gaaatgggat taggtgttag ggtggtggga ttttgtttag 1320
   ggttttgatt ttgtgttagg gttttagatt agttggtttt tgaaggtttt tttatttatt 1380
   ttatataaga ggaaataaag attttttagt ttaaggtttt agggttgttt tttgattttg 1440
   gttagtttgt aggaagagga aataataaaa taaaggaatt gttaatttgt tgggtattat 1500
   attttttagt tttaattttt gattttggat tgttagggtt atttttttta tgttgatttt 1560
   gtttttttta aatgaaaata tgttaataaa agtatatttt ggatataaaa tttaagtatg 1620
   tatttttttt ggggaggtta gagttgaggt gtattttgga agatgagaat tttgttttta 1680
   tgaattgggt aatatttagg tattgttagg tattttgata gattttttag atattttttt 1740
   tttttttttt tatatttttt ttttattaaa atagttattg ttttgaaatt tattagaata 1800
   atgatgtttt aaaaataaag gtgtagtaag tatttttttt tttgttgttg tgggttgaat 1860
   tatggatgtt tgtgggttgt ttagttttga tggtttgtag ggggtgtgtg ttgtagttgt 1920
   agtatagttt ggttattttt agaaagggag ttgaatggag ggaagtaggg agtgtggagg 1980
   gtttgaggtt tgtagataag gagaggtgta ttttgggatt tgggtttttt gttgttataa 2040
   tataattgtg ttattgttgg tattgtttga tttaagtgtt ggtggtaagt ggtgatgttg 2100
   gggttgggtt tttagtaatt gttgtgtttt gggttaggtt ggttgtttta gttattggga 2160
   tttttttttg gatgttttta gggtgatagg tgttgtattt attgatggga tagttgtata 2220
   tttttgaata ggtagtggag tttgtttttg gtaggtattt tagttgtgtt gatattaagg 2280
   ttgttatata atagttatta gtttttttta agttttagaa gtaggttttt tttgttttag 2340
   ttatagtggt tttagttgtt gttatgagtt tattttttat ttttaagtgt attttttttt 2400
   ttttatttgg gtttatgttt gttatataga gagaattata tagggggaat tatggtttta 2460
   tatttttgag gggatagata ttggttgtga gataggtatt atgtagagtt ttttggtgat 2520
   tgtttgtagg agtgagattt tttttggttt tgtagttggt taggtgtgtg tgtgtgaggg 2580
   tttttagttg attattggga tgtattgtta ttttttggtt tggtgggttt tgggattttt 2640
   tggtttttgt aggaggttat tttaggtttt tggaggaggt gtttttagtt ggtggtgttt 2700
   tttgttttgg gtttagaggt ggatatggtt ggttgtgttt tgttggtttt ttgtttgtgt 2760
   tgtagtgggt tgggagtagt tgtgtgatat tagatttata gtgttaagat gtgaagtgtg 2820
   aggaaattgt tgtgtttgat tttttttttt ttaatttttg gatttaggaa tgtttttagt 2880
   ttttgtgttt tatatgttta gttttggttt tttttttggt ttagaagttt ttaaggattg 2940
   aagggttttg tttagggttt tgtatttttg tttgattttt atggtttaga aaagtagggg 3000
   gatatttgaa atgttatttt gggataaata ttaataaaaa agtaaatgga tttgtgtagg 3060
   ggttagttat taattaatta ggttgtaagg ttatttaggg taaatatagt ttaggttggg 3120
   ttgggtgaga tttttttatg gttgtttttt gattgaattt tttttttttt ggtttaggtt 3180
   ttttttaggt tgttttgggg taaatattgg atgggaaggg ggtgttgtta atttttttgt 3240
   ggggtttgga ggtttttttg tttttaagtt ttgtagggta gggttggagt gtttaatatt 3300
   tatttttgtt tgaattttgg gtttgtgtgt tgtttttttt tgggtttgtg gtgttgtgtt 3360
   tgttgttggg tgtgtgttgt tgtttttttt tgagttggta gtttttgttg tgtggttgaa 3420
   gttttttgga atttttaatt ggaaattaat tttggttttg atagatgttt atgttagagg 3480
   tgtgttattt atttatattt tttgttttat tttggaggag atgtggtgag aattttgttg 3540
   ttttgttttt taatggggta gtagtaagta gtatatggag ggaatatata ttgattttta 3600
   aaaggtagtt aatttggtgg gaattttttt ttgttgtttt 3640
<210> 99
   <211> 3640
   <212> DNA
   <213> Artificial Sequence
<220>
   <223> chemically treated genomic DNA (Homo sapiens)
<400> 99
   agggtgatag gaggaaattt ttattaaatt aattattttt taaaaattaa tatatgtttt      60
   ttttatgtat tatttgttgt tgttttatta aagagtagag tgatagggtt tttgttgtgt     120
   tttttttggg atgaggtggg gggtgtgggt gggtggtgtg tttttgatgt gggtgtttat     180
   taagattaag attagttttt aattaaagat tttagagggt tttggttata tagtaggagt     240
   tgttggtttg gaaagagata gtgatatata tttaatagta agtgtagtat tgtggattta     300
   ggggaaggtg gtgtgtaggt ttgaggtttg ggtgggggtg ggtgttgggt attttgattt     360
   tgttttgtgg gatttggagg tgaggaaatt tttaaatttt gtgggggagt tggtggtgtt     420
   ttttttttgt ttggtgtttg ttttagaatg atttaagaag gatttgagtt aaggggggag     480
   gggtttggtt ggggggtggt tatgagaaag ttttgtttag tttaatttgg gttgtgtttg     540
   ttttgagtgg ttttgtggtt tggttgattg ataattggtt tttgtataag tttatttgtt     600
   tttttgttaa tatttatttt ggggtgatat tttaaatgtt tttttgtttt tttgggttat     660
   gggagttagg taggagtgtg gggttttagg tagagttttt taatttttga agatttttgg     720
   gttgggagag aagttaggat tgggtgtgtg ggatgtagga gttggaagtg tttttgggtt     780
   taagagttgg gggagaagga gttaagtgta gtggtttttt tgtgttttgt gttttggtgt     840
   tgtgggtttg gtgttgtgta gttgttttta gtttgttgtg gtgtgaataa agggttagta     900
   gggtgtgatt gattgtgttt gtttttgagt ttggggtgag gggtgttgtt agttgggagt     960
   gttttttttg aaggtttagg atggtttttt atggagatta aggagtttta gagtttgtta    1020
   ggttgaaaag tggtagtgta ttttggtagt taattgggga tttttatata tatatattta    1080
   gttgattgta aaattaaaaa aggttttgtt tttgtggata gttattagaa ggttttgtgt    1140
   ggtgtttgtt ttatggttgg tgtttgtttt tttgagggtg taggattata gttttttttg    1200
   tgtagttttt tttgtgtaat gggtataaat ttgggtgagg agagggaggt gtgtttgggg    1260
   ataagaagtg ggtttgtggt gatgattgga attgttatgg ttggggtagg ggaaatttat    1320
   ttttggggtt taaggggggt tgatggttat tgtgtggtga ttttggtgtt agtgtagtta    1380
   ggatgtttgt tagagatgaa ttttattatt tgtttggagg tatgtggtta ttttgttaat    1440
   gggtgtagta tttattgttt tggaagtatt tggagaggga ttttggtagt tggggtgatt 1500
   agtttgattt agggtatagt ggttgttaga gatttagttt tgatattgtt gtttattatt 1560
   gatatttggg ttggatagtg ttaataatag tatggttgtg ttgtagtagt agaggattta 1620
   aattttagga tgtgtttttt tttatttgta agttttgagt tttttgtgtt ttttgttttt 1680
   ttttatttgg tttttttttt gggggtagtt gggttgtgtt gtggttgtgg tgtgtgtttt 1740
   ttgtgggttg ttggggttgg gtgatttgtg agtgtttgtg gtttagtttg tggtagtgaa 1800
   gaaagggatg tttgttgtgt ttttgttttt agaatgttgt tgttttagtg aattttaaaa 1860
   taataattat tttgatagga agaaagtgtg aaggaggaag agaaggatat ttagagggtt 1920
   tgttggggta tttaataatg tttgggtgtt atttaattta tgaaaataaa atttttattt 1980
   tttgaagtat attttagttt taattttttt aaaaaagatg tgtatttgga ttttgtattt 2040
   gaagtatgtt tttgttggtg tgtttttatt taagaaaagt ggggttagtg tagagaaggt 2100
   gattttggtg gtttgaagtt ggaggttgga gttgagggat ataatatttg gtggattggt 2160
   ggtttttttg ttttgttgtt tttttttttt gtaaattgat tggagttagg aggtagtttt 2220
   gaggttttga gttgagaagt ttttgttttt ttttgtgtgg ggtgggtgaa agggtttttg 2280
   gaggttgatt ggtttggagt tttggtgtgg agttaggatt ttggataggg ttttattgtt 2340
   ttgatgtttg gttttatttt tttttgtttt ggtaggtaga tagaatgttt ttgggaagtt 2400
   atagtaagaa aataaaaatt aatagttaaa gttttgaagt tttttagggt gttaggttta 2460
   gttgggaata tatattggta tattgaggta attgaaaaga ttaaatttat ttttgttgat 2520
   tgggtggttg atttttttgt ttttttatgt ggttttgtgt gagtatttga gtgtatgtgt 2580
   atatttgttt ataaaattgg aatttgtgtt tttgtgggtg tgataggggt tggattatta 2640
   agttaaaatg tttaatttag tttttttgat ttgtgttggg gaaaatttat agttattttt 2700
   aatttatgag gtttttaagg aggttgttat atgagttgtt ttgatgttat tatttttata 2760
   tttatggtaa atttagttaa ttaaataaat gttgttattt taagttatta gaagaaattg 2820
   tttttataga gtatattaat aaaatttatt tttgattttt ttgtaagata gaaaattttt 2880
   aagtttaata aaggtgggta attaagaagg aggtttttta atattttgtt ttttagtttg 2940
   aggttttgtt ttttaagtga tattgtttgt gttttttgta aagtttattt attggaatat 3000
   ttaaatattg atttaatatt ttaaattaat ttaggagttt tgatggtttg aatgagaaat 3060
   gattagtttt tttttagttt ttattaaaat agtagaattt ttatattttt ggtaaaattg 3120
   aatggttttt aatttagggt agtaatagaa ggaaggtgtt attgaagttg tggttgttag 3180
   agggtgtatt ttttataatg ttggagaaaa tatttaataa atatttatta aataatattt 3240
   ttattgtgtt tttatttgaa taagatggat aagtattttt gggagaagaa agttatatta 3300
   tttttatata aaaaatgtta gttttataga gtttagtaaa aagaaaaata ttttttatgt 3360
   gaatggattt agtgtataat tataaatttt tattttttat taataggttt gtataggttt 3420
   ttattttaat ataagtatgt taatattaat gaagtttaat ttttttttga gataataaga 3480
   ttgggttagg tgattagatt aattataaga taatttgatt attttgatat aaatataaaa 3540
   attatttatt atagggtttt gttatagaag tatttatata tgtttttttt aagagatgtt 3600
   atggtattat aaaattgtaa taaattaatt tttagaaatt 3640
<210> 100
   <211> 3240
   <212> DNA
   <213> Artificial Sequence
<220>
   <223> chemically treated genomic DNA (Homo sapiens)
<400> 100
   gaaattgagg tataaggtag taatgattat tgagggtttt gtttttgttt ttttaggtga 60
   ggagtttatt ttaggggttt tagtttgaaa gtttagaggt gaggggtgtg tattataatt 120
   tttagaggtt tttggattta agggtggttt ttgttttttg ttttttttgt gtgatgaagt 180
   tgggtttatt aatgggagtt tggagtgggg tgagtagggg tgggatgggg tgggattgtg 240
   gggaggtggt tggtgggttg tgttgtgagt tagtatttta gagagtgtgg ggtttggata 300
   gttgtgtgtt gagggttttg gggtgggtgt tgtgggattt ttttgggtta tgggtaagtg 360
   gttttgtggt gtggtttgga taggggtttg gtggttttgt agggaggttt aaggtgttgg 420
   tgttgggttt tttttagttt ttgtggtagt tggtgtgttg tgtggggtgg agaggggttt 480
   ggggtgggtt ttggggtgtg ggttaagggg agtgagtttg gtttgtggtt ttagtgtttt 540
   ttggttggtg tggaaggggt gaggttgggt gtttgtgtga gttttgagaa aaggaaagat 600
   aaaaagtttt ggtggttagg ggttttttgg gtgtttattt tgaaattttt tttgttgttt 660
   tttgtttgaa atatgttgag tttggtatgg ttttgttgtt ttattttttg ttgtggtttt 720
   tattttgagt tgggtttagt taatttgttg tgatagttgt tggtagttgt ttttttgttt 780
   tgtgatattt tgtaggtttt agaattgtgg tgttgttggg tatagatttt gggtttagag 840
   tgtgtggttt ttttttttgt ggttgttgtt ttttgaaagg ggttgtgtgg gtgtgaagaa 900
   tttgtttaat gtgttttgta ggatttattg tttttttttt ggttgtgggt tgtgttgatg 960
   gtttggggag tttggttttt tgttttttgt tttttttttt tttttttttt tttttttttt 1020
   ttttttgttt ttttgtttat ttgttgtagt tttataggag aggggttgtt tttttggttg 1080
   tttttttttt ttgttttttg tgtgtttttt tgtttttttt tggtttgtag aagggatggg 1140
   gtggtggttg gggagtgtta ggggaagagg gtaagagtgt gggttatgag tttgtaggtg 1200
   atgtttggtt ttgtgtttgg tgggagttgt gggttaattt gggtttgtgt tttgtttttt 1260
   tgtggtgttt aggggttgtt tgggttgggg ttgtgttatt ttttgtttag tgttggtgtg 1320
   gtggggtttg tgtggtgtag gatttttgag ggtttagggg gttttaaggt gttgtgggtt 1380
   tagtgttttt ttgtttgtag agttttgggt tagtaaggag tgttttgtgg gttttagggg 1440
   aggataatag gtgttttttg ttttatttgg gtttttttgg tgggagttta tatgtatggt 1500
   ggtgttattg tgtttatagg taatgttaga gtataggagg ggtgggaggt tggggtttga 1560
   gggttttatt ttgtagtaga gtttagagtt taatttaggt tttagggatt tgtttggggt 1620
   ttagaatttg gtgtggtaga ggtagaaagg gtttttgata atttgttttt atagttgaga 1680
   gatgttaatt ggtatttttg agtttttgtt ttgtaggtga aatgggaata tatgaaatta 1740
   tttgaggagt ttttattgtt tgttattatt aaatgtggtt tagattttgt ggggagggat 1800
   agttttattt tggaattggg gtttttagga tattttagag tttggtaaga gggataggga 1860
   gaatggggtt tggaagttgg tagttttata tttagtgttt tttgtatttt tttttttttt 1920
   tgtatttttt ggtggttttt gtggttttag ttgtttttag attttgtttt ttttgttttt 1980
   ttttaagttg aggtattgag gttgggttgt ggtttgagtt gtatttgtgt tttgttaggg 2040
   agtgggggtt ttaggtttat tgtgtttttt tttattgttg gttttagttt aatatttggt 2100
   aggttttttt ttttaaattg ttggagtaag tttgtgtaga tttggtttgg ttgttgtttt 2160
   tgggttgttg agtggattgg ttgttttgtt gttttaaagg ttgtttttgt tttgttttag 2220
   tttaagttta gagaaattta agttaataat tttagttgag ggggtggggt ggtgtttgtt 2280
   ggttgttatt gaggttgttg ttattattat tgtggtaatt gatgtagtgt ttttagttgg 2340
   tgatttttta tgaaaggaag tgttattttt tttttattat atttagggtg agtggggtgg 2400
   gtagtagaaa gatggggagg gagagaagga aaggggtgtg tgtttttagt agatttttaa 2460
   atttgagagt tgtgtggttt tgtattagtt attgtttttt tttgggtaat aggggtttgt 2520
   aagtttattg tgtttatttt gtatttagga ggttgatgtt tttggagtat ttgttgggta 2580
   gtgaggtgtt ttggggaggg agtaggatag gtaggttttt gattttttta ttgagtttgg 2640
   ttgtggtttg tgggggaagt tttttttttt tttttatttt tttttgttga gaatattggg 2700
   tatattgagt ttagtatgtt tattttgtag atggggaaag ggagatatag aattagtgat 2760
   taatttggta agaggtgtag ttatgtagtg ttggaatttt ttgttttatt tgtttttttg 2820
   gtttattttt atttgttttt agtttgatgt ttggtagggt aggggtttat tgattttttt 2880
   gtatttattt attgtttggt tttatagtta ttttttagtt ttggttaagt tttgtgagat 2940
   attttaagtt tgtttttagt gatttttaat tattgtttga ttgtttttat ttttttgttt 3000
   ttgtatattt gtttttgttt tgttaggttg gttgggtttt ggttagagtt ggttttttat 3060
   ttttttaggt aataggtagt agttttttaa atggaatttt agtaggtatt aataggtttt 3120
   tttttttttt ttaagattat ggagataata ttttttattt aaagatggtg ggttttttgg 3180
   gttttttttg atttaggtta gggaagttga tttatagagt gtgggggtta tgtttttttt 3240
<210> 101
   <211> 3240
   <212> DNA
   <213> Artificial Sequence
<220>
   <223> chemically treated genomic DNA (Homo sapiens)
<400> 101
   ggaaggggta tggtttttat attttatgaa ttaatttttt tggtttgggt tggggagggt      60
   ttagaggatt tattattttt gaataagggg tgttattttt ataattttga ggaaggagga     120
   aagatttgtt gatatttatt gagattttat ttaaaaaatt gttatttgtt atttgagaga     180
   gtagggggtt agttttggtt agggtttaat taatttggtg aagtaaaggt aggtatgtaa     240
   aggtaggaag gtgaaggtag ttgggtggtg gttgagagtt attgaagata aatttaagat     300
   gttttatggg gtttagttag gattagaagg tagttatggg gttaggtagt gggtggatgt     360
   ggggaagtta gtgggttttt gttttgttag atattaagtt ggaagtagat gaaagtgggt     420
   taagaggata gatggggtaa ggggttttag tattgtatgg ttgtattttt tattaagttg     480
   gttattggtt ttgtattttt ttttttttat ttataagatg ggtgtgttag atttaatgtg     540
   tttaatgttt ttagtagaaa aaagtgagaa gagaaaggag gtttttttta taaattatag     600
   ttaggtttag tgagaagatt agaggtttgt ttgttttgtt ttttttttaa ggtattttgt     660
   tgtttagtag gtgttttgga aatattagtt ttttgggtgt aggatgagtg taatggattt     720
   atagattttt gttgtttaga gaggggtagt gattagtata aggttatata gtttttaagt     780
   ttaagggttt gttggaggta tatatttttt tttttttttt tttttttatt tttttgttgt     840
   ttgttttatt tattttgggt gtgatgggaa ggaagtgata ttttttttta tgaaaagtta     900
   ttgattggga atattgtgtt aattattata ataatggtaa tagtagtttt aataataatt     960
   agtagatgtt gttttgtttt tttggttgag gttgttggtt taagtttttt taagtttgag    1020
   ttggagtaaa ataaagatag tttttgaagt ggtgaaatgg ttggtttatt tggtagtttg    1080
   ggagtggtgg ttaggttgag tttatgtgag tttgttttag tagtttgggg aggggggttt    1140
   gttaagtgtt gagttggagt tagtagtgag aagaaatgtg atgggtttgg gatttttatt    1200
   ttttaatagg gtataggtgt agtttaagtt atagtttaat tttagtattt tagtttgggg    1260
   gagggtgaga gagatagggt ttgggggtag ttgaggttat aagagttatt agagggtata    1320
   gaaagggaag gaggtgtggg ggatattgaa tgtgaggtta ttaattttta agttttattt    1380
   tttttgtttt ttttattggg ttttgggatg ttttaaaagt tttagtttta ggatgggatt    1440
   gttttttttt atggagtttg ggttatattt ggtggtgatg gatagtgaga gttttttaga    1500
   tggttttgtg tgtttttatt ttatttatgg ggtagaggtt tagaggtgtt agttgatgtt    1560
   ttttaattgt agaagtagat tattggaggt tttttttatt tttgttatat taagttttag    1620
   gttttaggtg ggtttttaag gtttggattg ggttttgagt tttgttgtgg ggtgaagttt    1680
   ttgagttttg attttttatt ttttttgtgt tttagtgttg tttgtaggtg tagtgatgtt    1740
   attgtgtgtg tgggttttta ttagaggagt ttgggtgggg taagaggtat ttgttgtttt    1800
   tttttggggt ttgtggagta ttttttattg gtttaagatt ttgtaaataa agggatattg    1860
   gatttgtagt gttttgggat tttttgggtt tttgggggtt ttgtattgtg taagttttat    1920
   tatgttggtg ttgggtggag ggtgatgtga ttttagtttg ggtgattttt agatattgtg    1980
   gagggatgga gtgtgggttt gggttggttt gtagtttttg ttgagtgtga agttgggtat    2040
   tgtttgtaag tttgtagttt atgtttttgt tttttttttt tgatgttttt tagttgttat    2100
   tttgtttttt ttgtagattg agggaagata aggggatata taagggatag gagaggagag    2160
   taattaggga agtgattttt tttttgtggg gttgtggtgg gtgggtggag agataggaga    2220
   ggaaaagaga aaagggaaga gaaggaaggg gtagggggtg agaagttggg ttttttggat    2280
   tattggtgta gtttgtggtt gaaagaggaa tagtggattt tataaagtgt attgagtaag    2340
   ttttttatat ttgtgtagtt ttttttagga aatggtggtt gtagaggaaa gggttgtgtg    2400
   ttttggattt ggagtttgtg tttggtggtg ttgtggtttt gaaatttgtg ggatgttatg    2460
   gggtggaggg gtggttgttg atagttgtta taataaatta gttgagttta gtttgaggtg    2520
   gaaattgtag taggaagtgg gatgatagag ttgtattggg tttggtatat tttaggtaag    2580
   agatggtgaa aagagttttg agataaatgt ttgggaagtt tttggttgtt aaaatttttt    2640
   gttttttttt tttttgaagt ttgtgtggat gtttagtttt attttttttg tgttggttgg    2700
   ggagtgttga agttgtgggt tgggtttgtt ttttttggtt tgtgttttga aatttatttt    2760
   gggttttttt ttgttttata tagtgtattg attgttatga gggttggagg ggatttggtg    2820
   ttggtatttt gaattttttt gtagggttgt tgagtttttg tttgggttgt gttgtagagt    2880
   tgtttattta tggtttggag aggttttgtg gtgtttgttt tgagattttt agtgtgtggt    2940
   tgtttggatt ttgtgttttt tgagatattg gtttgtggtg tggtttgttg gttatttttt    3000
   tgtagttttg ttttgttttg tttttgtttg ttttattttg ggtttttatt ggtaagtttg    3060
   gttttgttgt gtggagaagg tggggggtgg ggattgtttt tgagtttggg gatttttggg    3120
   aattgtagtg tgtgtttttt gtttttaggt ttttagattg gagtttttgg aataggtttt    3180
   ttatttagag gagtggaggt aggattttta gtaattattg ttgttttgtg ttttagtttt    3240
<210> 102
   <211> 2155
   <212> DNA
   <213> Artificial Sequence
<220>
   <223> chemically treated genomic DNA (Homo sapiens)
<400> 102
   tttttttttt tttttatttt tggagggttt tagttattag ttttgaagtt gtagtgagag      60
   ttatttattg ttttggtttt tgggattttt tgagtaggag aaaaaaatta gaaaattaaa     120
   agagagagag agatttggaa ggaagaagta atggaggagg gagatagggg gaatgagagg     180
   ggagtttata ttatagagag agaatttttt aaaggaagtg taggtgttta tgggatagga     240
   gtgtggaaga aaagagtaga aaaggaaaag tagttttaag taagagaaag taaggagagg     300
   tggataggga ggggaagagt taagagaagt taagttgagg gatttgtagt aattttgaga     360
   aataaaagtg aattgtttgt gggtggttgt tgtgatttta gtaaaaagtg gagtttaagg     420
   tttagggatt ttgttgttta tattgtgttt tgttttattt tggatgtttt taattttagt     480
   tttaggtata gtatatgagt aggggtagta aggtttggtt agggttggtg ttgtatggga     540
   gttgtgggtt tttgtaatat ttaggtgtgt aggtgttata agaaggagtt ggggttgttg     600
   tttttgtagt gtggattttg ttttgtgttg tatatgtttt agaagagtgt gtttggaagt     660
   gttttttttg gttttttttg ttttttaaga tttgtttgta atgtttatat ttgttattat        720
   tgtataagta ttagggtgag gaggtttttt tgtaggaatt aatattgatt ttagtttttt     780
   gtttttttaa agtttttttt tgatgatggt gagatttata ttttaagtta gaagaaaata     840
   aaattttgtt tattttgggt ttatgattgt ataaaattta aaatgttgtt ggtatgaaga     900
   tgtagttgga agattttaga aatttagtat taggaggtag agattttata gtgtttagag      960
   atgagttttt aaataaatta agaattatgt agtgggaaat taaggaggag ggtttgaggg    1020
   gttgatttta gagggataga tagagaaatt gagagtgaaa tatttaagga agtggaataa    1080
   attgttgtag ttaggtgttt ttgttttttt ggaaatttga aatttgttta aattttattt    1140
   taggtgttta tggtattagt tgtagggttt tgtattatag gggttgttat tttttgtttt    1200
   tggagttttt atgttgagat ttattatttg ttggtttttt tttttttttt tttttttgtt    1260
   tttttatttt ttaaatataa gataaaaata tattttattt attattatag atatatatat    1320
   tttattttta ttatataaat ataaaatatt taaatattta aaatatttag aataaagtta    1380
   gtagtttgga tttagtgata tttatttgtg tttgaattgt ttattatttt ttttattatt    1440
   tgtttagaaa aaaaaaaaag atgaaaaaga gggggagagt ttttgatgta aataatttat    1500
   aataaaatta ttattttaaa aaagtgtttt ttgtttgttt tagatggatg aatatatttt    1560
   agatattgtg gtattttgta gtttgggttt gtgattttgg ttaaaatgtg ggtggttgtg    1620
   gttgtaggga tgattgttgt tgtgtagggt aaggaaagag ggtgttgtgg tttgtgtttt    1680
   gatttgggat tattgaaatt ttaagattgg ggaggtgagg ggagtgtttt ttttattaat    1740
   tatataagat gttatttgga ttttgagatt attgttagag gaggtttgat tatttagtgg    1800
   tgtttttgtt tttttttttg taaagaattg tttagaaatt tagatgtttt ttgtgtgtag    1860
   gaagggtttt gtgaatttgg gtgtttggga gaggaggtaa tgagtgttga tttgtttttt    1920
   aaattgagta attgtgtttg aagttatgtg tttgggaggt tgtagggtga agtgttggtt    1980
   gtgtaggtta ttgttggtag ttgttttgtt tttttttgga ggagtgggaa gttgttggtt   2040
   attgttttga ataggtttgg agaaaagatt ttgaattttt tttgattagt ggtgttattt    2100
   ttttttttgt ttttttttta ttaaattttt gtttaaagtt ataatattta gaatt          2155
<210> 103
   <211> 2155
   <212> DNA
   <213> Artificial Sequence
<220>
   <223> chemically treated genomic DNA (Homo sapiens)
<400> 103
   ggttttgggt gttgtaattt taaataggaa tttaatgggg gaaaagtaaa ggaaaaggta 60
   atgttgttaa ttaaaggaaa tttagaattt tttttttgag tttgtttggg gtggtggttg 120
   atagtttttt atttttttag gaaagagtaa agtggttgtt ggtggtagtt tgtgtagttg 180
   gtgttttatt ttatggtttt ttaggtgtgt agttttgggt ataattgttt ggtttggaaa 240
   atgagttagt atttattgtt ttttttttta aatatttgga tttataaaat tttttttgta 300
   tgtaggaaat gtttggattt ttgagtagtt ttttgtggaa ggggaggtag ggatgttgtt 360
   gggtggttgg gtttttttta gtagtagttt tggagtttag atagtgtttt gtgtgattga 420
   taggaaagat attttttttg tttttttagt tttagggttt tagtggtttt aaattagaat 480
   atgaattgtg atattttttt tttttatttt gtgtagtggt gattgttttt atagttatgg 540
   ttatttatat tttaattaaa attatagatt tagattgtga ggtgttgtag tgtttagaat 600
   gtatttattt atttgaagta agtgaaaaat atttttttaa aatagtaatt ttattgtaaa 660
   ttatttatgt tggaaatttt tttttttttt tttatttttt tttttttttt aggtaaatag 720
   tgaagaaaat aatgaatgat ttaaatgtgg gtaggtgtta ttgaatttag gttattaatt 780
   ttgttttgaa tgttttggat atttggatgt tttgtattta tgtggtgaga gtgaaatata 840
   tatatttatg atgataaatg aggtgtattt ttgttttata tttgaaggat aaaaaaataa 900
   aagaagaaaa ggaagaaaga gttagtaaat gatgagtttt ggtgtgggga ttttgggggt 960
   agagggtggt agtttttata gtgtggggtt ttgtgattgg tattgtgagt gtttgggatg 1020
   aggtttgggt ggattttggg tttttggaga ggtagagatg tttggttatg atgatttgtt 1080
   ttattttttt gagtgttttg tttttgattt ttttatttgt ttttttgagg ttggtttttt 1140
   agattttttt ttttaatttt ttattgtgta atttttagtt tatttaggaa tttatttttg 1200
   ggtattataa agtttttgtt ttttaatgtt aggtttttag agttttttag ttgtattttt 1260
   atgttggtag tattttggat tttgtgtaat tatgggttta gaataggtaa ggttttattt 1320
   ttttttgatt tagaatataa gttttgttat tgttaaagag aaattttgaa ggagtaggag 1380
   attgaaattg atattaattt ttgtaaagag atttttttat tttggtgttt atgtggtggt 1440
   gatgggtgta gatgttgtaa ataaattttg ggaagtgagg agggttgggg aggatatttt 1500
   tggatatgtt tttttaaagt gtgtgtagta tggggtgaag tttgtgttgt gggggtagtg 1560
   gttttggttt ttttttgtag tgtttgtata tttgggtgtt gtagggattt gtagtttttg 1620
   tgtggtattg gttttagtta ggttttattg tttttatttg tgtattgtgt ttgaagttga 1680
   agttggaggt atttaaaata aggtggggta tagtgtgggt ggtggagttt ttgagttttg 1740
   agttttgttt tttgttgaga ttatagtagt tgtttataga tagtttattt ttatttttta 1800
   aagttgttgt agatttttta gtttggtttt ttttggtttt tttttttttt gtttgttttt 1860
   ttttattttt ttttgtttga aattgttttt ttttttttat tttttttttt tgtgtttttg 1920
   ttttgtaaat gtttgtgttt tttttaagga gttttttttt tgtggtgtgg gttttttttt 1980
   tgtttttttt gttttttttt tttattattt ttttttttta ggtttttttt ttttttttgg 2040
   ttttttggtt tttttttttt gtttaagggg ttttaaaggt taaggtagtg ggtggttttt 2100
   gttgtagttt taaggttggt ggttgggatt ttttaggggt gagaaggaag ggaga 2155
<210> 104
   <211> 28
   <212> DNA
   <213> Artificial Sequence
<220>
   <223> chemically treated genomic DNA (Homo sapiens)
<400> 104
   tatcgcggag attttcgagt tttcgttg 28
<210> 105
   <211> 19
   <212> DNA
   <213> Artificial Sequence
<220>
   <223> chemically treated genomic DNA (Homo sapiens)
<400> 105
   ggggttgttt ttcgggatt 19
<210> 106
   <211> 24
   <212> DNA
   <213> Artificial Sequence
<220>
   <223> chemically treated genomic DNA (Homo sapiens)
<400> 106
   gataacccta aaacgcaact cgaa 24
<210> 107
   <211> 20
   <212> DNA
   <213> Artificial Sequence
<220>
   <223> chemically treated genomic DNA (Homo sapiens)
<400> 107
   cgtttcgtaa ggagcgtgtt 20
<210> 108
   <211> 24
   <212> DNA
   <213> Artificial Sequence
<220>
   <223> chemically treated genomic DNA (Homo sapiens)
<400> 108
   cgcgttgttc gcggttagtt tcgt 24
<210> 109
   <211> 16
   <212> DNA
   <213> Artificial Sequence
<220>
   <223> chemically treated genomic DNA (Homo sapiens)
<400> 109
   cgacgttttc gcgtgg 16
<210> 110
   <211> 29
   <212> DNA
   <213> Artificial Sequence
<220>
   <223> chemically treated genomic DNA (Homo sapiens)
<400> 110
   gttttgaaat ttattagaat aacgacgtt 29
<210> 111
   <211> 31
   <212> DNA
   <213> Artificial Sequence
<220>
   <223> chemically treated genomic DNA (Homo sapiens)
<400> 111
   ctttctaaaa ataaccgaac tatactacga c 31
<210> 112
   <211> 21
   <212> DNA
   <213> Artificial Sequence
<220>
   <223> chemically treated genomic DNA (Homo sapiens)
<400> 112
   tacggacgtt cgcgggtcgt t 21
<210> 113
   <211> 24
   <212> DNA
   <213> Artificial Sequence
<220>
   <223> chemically treated genomic DNA (Homo sapiens)
<400> 113
   agtaagttcg cgtagattcg gttt 24
<210> 114
   <211> 21
   <212> DNA
   <213> Artificial Sequence
<220>
   <223> chemically treated genomic DNA (Homo sapiens)
<400> 114
   taaaacgacg aaacgaccga t 21
<210> 115
   <211> 24
   <212> DNA
   <213> Artificial Sequence
<220>
   <223> chemically treated genomic DNA (Homo sapiens)
<400> 115
   tcgtcgtttt cgggttgtcg agtg 24
<210> 116
   <211> 24
   <212> DNA
   <213> Artificial Sequence
<220>
   <223> chemically treated genomic DNA (Homo sapiens)
<400> 116
   aaggaagtgg aataaatcgt cgta 24
<210> 117
   <211> 33
   <212> DNA
   <213> Artificial Sequence
<220>
   <223> chemically treated genomic DNA (Homo sapiens)
<400> 117
   aggcgttttt gttttttcgg aaattcgaaa ttc 33
<210> 118
   <211> 25
   <212> DNA
   <213> Artificial Sequence
<220>
   <223> chemically treated genomic DNA (Homo sapiens)
<400> 118
   ctacgactaa taccgtaaac gccta 25